(19) Europäisches Patentamt
European Patent Office
Office européen des brevets

(11) EP 2 281 043 B1

(12) **EUROPEAN PATENT SPECIFICATION**

(45) Date of publication and mention of the grant of the patent:
**13.03.2013 Bulletin 2013/11**

(21) Application number: **09735585.3**

(22) Date of filing: **22.04.2009**

(51) Int Cl.:
***C12N 15/117*** *(2010.01)* ***A61K 31/713*** *(2006.01)*

(86) International application number:
**PCT/IB2009/006036**

(87) International publication number:
**WO 2009/130616 (29.10.2009 Gazette 2009/44)**

(54) **IMPROVED TLR3 AGONIST COMPOSITIONS**

VERBESSERTE TLR3-AGONIST-ZUSAMMENSETZUNGEN

COMPOSITIONS AGONISTES DE TLR3 AMÉLIORÉES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **25.04.2008 US 47969 P**
**20.02.2009 US 154229 P**

(43) Date of publication of application:
**09.02.2011 Bulletin 2011/06**

(73) Proprietor: **Innate Pharma**
**13009 Marseille (FR)**

(72) Inventors:
- **AUBIN, Eric**
  **F-13009 Marseille (FR)**
- **BELMANT, Christian**
  **F-83140 Six-Fours-les-Plages (FR)**
- **GAUTHIER, Laurent**
  **F-13008 Marseille (FR)**
- **MOREL, Yannis**
  **F-13006 Marseille (FR)**
- **PATUREL, Carine**
  **F-69280 Marcy l'Etoile (FR)**
- **BREGEON, Delphine**
  **F-13001 Marseille (FR)**

(74) Representative: **Völlmy, Lukas**
**Innate Pharma**
**IP Department**
**117, avenue de Luminy**
**13009 Marseille (FR)**

(56) References cited:
**WO-A-2004/087877 WO-A-2006/054177**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 281 043 B1

SEQUENCE LISTING

<110> INNATE PHARMA

<120> IMPROVED TLR3 AGONIST COMPOSITIONS

<130> dsRNA

<160> 16

<170> PatentIn version 3.3

<210> 1
<211> 3057
<212> DNA
<213> homo sapiens

<400> 1

cactttcgag agtgccgtct atttgccaca cacttccctg atgaaatgtc tggatttgga 60
ctaaagaaaa aaggaaaggc tagcagtcat ccaacagaat catgagacag actttgcctt 120
gtatctactt ttggggggc cttttgccct ttgggatgct gtgtgcatcc tccaccacca 180
agtgcactgt tagccatgaa gttgctgact gcagccacct gaagttgact caggtacccg 240
atgatctacc cacaaacata acagtgttga accttaccca taatcaactc agaagattac 300
cagccgccaa cttcacaagg tatagccagc taactagctt ggatgtagga tttaacacca 360
tctcaaaact ggagccagaa ttgtgccaga aacttcccat gttaaaagtt ttgaacctcc 420
agcacaatga gctatctcaa ctttctgata aaacctttgc cttctgcacg aatttgactg 480
aactccatct catgtccaac tcaatccaga aaattaaaaa taatcccttt gtcaagcaga 540
agaatttaat cacattagat ctgtctcata atggcttgtc atctacaaaa ttaggaactc 600
aggttcagct ggaaaatctc caagagcttc tattatcaaa caataaaatt caagcgctaa 660
aaagtgaaga actggatatc tttgccaatt catctttaaa aaaattagag ttgtcatcga 720
atcaaattaa agagttttct ccagggtgtt ttcacgcaat tggaagatta tttggcctct 780
ttctgaacaa tgtccagctg ggtcccagcc ttacagagaa gctatgtttg gaattagcaa 840
acacaagcat tcggaatctg tctctgagta acagccagct gtccaccacc agcaatacaa 900
ctttcttggg actaaagtgg acaaatctca ctatgctcga tctttcctac aacaacttaa 960
atgtggttgg taacgattcc tttgcttggc ttccacaact agaatatttc ttcctagagt 1020
ataataatat acagcatttg ttttctcact ctttgcacgg gcttttcaat gtgaggtacc 1080
tgaatttgaa acggtctttt actaaacaaa gtatttccct tgcctcactc cccaagattg 1140
atgatttttc ttttcagtgg ctaaaatgtt tggagcacct taacatggaa gataatgata 1200
ttccaggcat aaaaagcaat atgttcacag gattgataaa cctgaaatac ttaagtctat 1260
ccaactcctt tacaagtttg cgaactttga caaatgaaac atttgtatca cttgctcatt 1320

ctcccttaca catactcaac ctaaccaaga ataaaatctc aaaaatagag agtgatgctt 1380
tctcttggtt gggccaccta gaagtacttg acctgggcct taatgaaatt gggcaagaac 1440
tcacaggcca ggaatggaga ggtctagaaa atattttcga aatctatctt tcctacaaca 1500
agtacctgca gctgactagg aactcctttg ccttggtccc aagccttcaa cgactgatgc 1560
tccgaagggt ggcccttaaa aatgtggata gctctccttc accattccag cctcttcgta 1620
acttgaccat tctggatcta agcaacaaca acatagccaa cataaatgat gacatgttgg 1680
agggtcttga gaaactagaa attctcgatt tgcagcataa caacttagca cggctctgga 1740
aacacgcaaa ccctggtggt cccatttatt tcctaaaggg tctgtctcac ctccacatcc 1800
ttaacttgga gtccaacggc tttgacgaga tcccagttga ggtcttcaag gatttatttg 1860
aactaaagat catcgattta ggattgaata atttaaacac acttccagca tctgtcttta 1920
ataatcaggt gtctctaaag tcattgaacc ttcagaagaa tctcataaca tccgttgaga 1980
agaaggtttt cgggccagct ttcaggaacc tgactgagtt agatatgcgc tttaatccct 2040
ttgattgcac gtgtgaaagt attgcctggt ttgttaattg gattaacgag acccatacca 2100
acatccctga gctgtcaagc cactaccttt gcaacactcc acctcactat catgggttcc 2160
cagtgagact tttgatacaa tcatcttgca aagacagtgc cccctttgaa ctctttttca 2220
tgatcaatac cagtatcctg ttgattttta tctttattgt acttctcatc cactttgagg 2280
gctggaggat atctttttat tggaatgttt cagtacatcg agttcttggt ttcaaagaaa 2340
tagacagaca gacagaacag tttgaatatg cagcatatat aattcatgcc tataaagata 2400
aggattgggt ctgggaacat ttctcttcaa tggaaaagga agaccaatct ctcaaatttt 2460
gtctggaaga aagggacttt gaggcgggtg tttttgaact agaagcaatt gttaacagca 2520
tcaaaagaag cagaaaaatt atttttgtta taacacacca tctattaaaa gacccattat 2580
gcaaaagatt caaggtacat catgcagttc aacaagctat tgaacaaaat ctggattcca 2640
ttatattggt tttccttgag gagattccag attataaact gaaccatgca ctctgtttgc 2700
gaagaggaat gtttaaatct cactgcatct tgaactggcc agttcagaaa gaacggatag 2760
gtgcctttcg tcataaattg caagtagcac ttggatccaa aaactctgta cattaaattt 2820
atttaaatat tcaattagca aaggagaaac tttctcaatt taaaaagttc tatggcaaat 2880
ttaagttttc cataaaggtg ttataatttg tttattcata tttgtaaatg attatattct 2940
atcacaatta catctcttct aggaaaatgt gtctccttat ttcaggccta tttttgacaa 3000
ttgacttaat tttacccaaa ataaaacata taagcacgta aaaaaaaaaa aaaaaaa 3057

<210> 2
<211> 904
<212> PRT
<213> homo sapiens

<400> 2

```
Met Arg Gln Thr Leu Pro Cys Ile Tyr Phe Trp Gly Gly Leu Leu Pro
1               5                   10                  15

Phe Gly Met Leu Cys Ala Ser Ser Thr Thr Lys Cys Thr Val Ser His
            20                  25                  30

Glu Val Ala Asp Cys Ser His Leu Lys Leu Thr Gln Val Pro Asp Asp
        35                  40                  45

Leu Pro Thr Asn Ile Thr Val Leu Asn Leu Thr His Asn Gln Leu Arg
    50                  55                  60

Arg Leu Pro Ala Ala Asn Phe Thr Arg Tyr Ser Gln Leu Thr Ser Leu
65                  70                  75                  80

Asp Val Gly Phe Asn Thr Ile Ser Lys Leu Glu Pro Glu Leu Cys Gln
                85                  90                  95

Lys Leu Pro Met Leu Lys Val Leu Asn Leu Gln His Asn Glu Leu Ser
            100                 105                 110

Gln Leu Ser Asp Lys Thr Phe Ala Phe Cys Thr Asn Leu Thr Glu Leu
        115                 120                 125

His Leu Met Ser Asn Ser Ile Gln Lys Ile Lys Asn Asn Pro Phe Val
    130                 135                 140

Lys Gln Lys Asn Leu Ile Thr Leu Asp Leu Ser His Asn Gly Leu Ser
145                 150                 155                 160

Ser Thr Lys Leu Gly Thr Gln Val Gln Leu Glu Asn Leu Gln Glu Leu
                165                 170                 175

Leu Leu Ser Asn Asn Lys Ile Gln Ala Leu Lys Ser Glu Glu Leu Asp
            180                 185                 190

Ile Phe Ala Asn Ser Ser Leu Lys Lys Leu Glu Leu Ser Ser Asn Gln
        195                 200                 205

Ile Lys Glu Phe Ser Pro Gly Cys Phe His Ala Ile Gly Arg Leu Phe
    210                 215                 220

Gly Leu Phe Leu Asn Asn Val Gln Leu Gly Pro Ser Leu Thr Glu Lys
225                 230                 235                 240

Leu Cys Leu Glu Leu Ala Asn Thr Ser Ile Arg Asn Leu Ser Leu Ser
                245                 250                 255

Asn Ser Gln Leu Ser Thr Thr Ser Asn Thr Thr Phe Leu Gly Leu Lys
            260                 265                 270

Trp Thr Asn Leu Thr Met Leu Asp Leu Ser Tyr Asn Asn Leu Asn Val
        275                 280                 285

Val Gly Asn Asp Ser Phe Ala Trp Leu Pro Gln Leu Glu Tyr Phe Phe
```

```
    290                 295                 300

Leu Glu Tyr Asn Asn Ile Gln His Leu Phe Ser His Ser Leu His Gly
305                 310                 315                 320

Leu Phe Asn Val Arg Tyr Leu Asn Leu Lys Arg Ser Phe Thr Lys Gln
                325                 330                 335

Ser Ile Ser Leu Ala Ser Leu Pro Lys Ile Asp Asp Phe Ser Phe Gln
            340                 345                 350

Trp Leu Lys Cys Leu Glu His Leu Asn Met Glu Asp Asn Asp Ile Pro
        355                 360                 365

Gly Ile Lys Ser Asn Met Phe Thr Gly Leu Ile Asn Leu Lys Tyr Leu
    370                 375                 380

Ser Leu Ser Asn Ser Phe Thr Ser Leu Arg Thr Leu Thr Asn Glu Thr
385                 390                 395                 400

Phe Val Ser Leu Ala His Ser Pro Leu His Ile Leu Asn Leu Thr Lys
                405                 410                 415

Asn Lys Ile Ser Lys Ile Glu Ser Asp Ala Phe Ser Trp Leu Gly His
            420                 425                 430

Leu Glu Val Leu Asp Leu Gly Leu Asn Glu Ile Gly Gln Glu Leu Thr
        435                 440                 445

Gly Gln Glu Trp Arg Gly Leu Glu Asn Ile Phe Glu Ile Tyr Leu Ser
    450                 455                 460

Tyr Asn Lys Tyr Leu Gln Leu Thr Arg Asn Ser Phe Ala Leu Val Pro
465                 470                 475                 480

Ser Leu Gln Arg Leu Met Leu Arg Arg Val Ala Leu Lys Asn Val Asp
                485                 490                 495

Ser Ser Pro Ser Pro Phe Gln Pro Leu Arg Asn Leu Thr Ile Leu Asp
            500                 505                 510

Leu Ser Asn Asn Asn Ile Ala Asn Ile Asn Asp Asp Met Leu Glu Gly
        515                 520                 525

Leu Glu Lys Leu Glu Ile Leu Asp Leu Gln His Asn Asn Leu Ala Arg
    530                 535                 540

Leu Trp Lys His Ala Asn Pro Gly Gly Pro Ile Tyr Phe Leu Lys Gly
545                 550                 555                 560

Leu Ser His Leu His Ile Leu Asn Leu Glu Ser Asn Gly Phe Asp Glu
                565                 570                 575

Ile Pro Val Glu Val Phe Lys Asp Leu Phe Glu Leu Lys Ile Ile Asp
            580                 585                 590

Leu Gly Leu Asn Asn Leu Asn Thr Leu Pro Ala Ser Val Phe Asn Asn
        595                 600                 605

Gln Val Ser Leu Lys Ser Leu Asn Leu Gln Lys Asn Leu Ile Thr Ser
    610                 615                 620
```

Val Glu Lys Lys Val Phe Gly Pro Ala Phe Arg Asn Leu Thr Glu Leu
625 630 635 640

Asp Met Arg Phe Asn Pro Phe Asp Cys Thr Cys Glu Ser Ile Ala Trp
645 650 655

Phe Val Asn Trp Ile Asn Glu Thr His Thr Asn Ile Pro Glu Leu Ser
660 665 670

Ser His Tyr Leu Cys Asn Thr Pro Pro His Tyr His Gly Phe Pro Val
675 680 685

Arg Leu Phe Asp Thr Ser Ser Cys Lys Asp Ser Ala Pro Phe Glu Leu
690 695 700

Phe Phe Met Ile Asn Thr Ser Ile Leu Leu Ile Phe Ile Phe Ile Val
705 710 715 720

Leu Leu Ile His Phe Glu Gly Trp Arg Ile Ser Phe Tyr Trp Asn Val
725 730 735

Ser Val His Arg Val Leu Gly Phe Lys Glu Ile Asp Arg Gln Thr Glu
740 745 750

Gln Phe Glu Tyr Ala Ala Tyr Ile Ile His Ala Tyr Lys Asp Lys Asp
755 760 765

Trp Val Trp Glu His Phe Ser Ser Met Glu Lys Glu Asp Gln Ser Leu
770 775 780

Lys Phe Cys Leu Glu Glu Arg Asp Phe Glu Ala Gly Val Phe Glu Leu
785 790 795 800

Glu Ala Ile Val Asn Ser Ile Lys Arg Ser Arg Lys Ile Ile Phe Val
805 810 815

Ile Thr His His Leu Leu Lys Asp Pro Leu Cys Lys Arg Phe Lys Val
820 825 830

His His Ala Val Gln Gln Ala Ile Glu Gln Asn Leu Asp Ser Ile Ile
835 840 845

Leu Val Phe Leu Glu Glu Ile Pro Asp Tyr Lys Leu Asn His Ala Leu
850 855 860

Cys Leu Arg Arg Gly Met Phe Lys Ser His Cys Ile Leu Asn Trp Pro
865 870 875 880

Val Gln Lys Glu Arg Ile Gly Ala Phe Arg His Lys Leu Gln Val Ala
885 890 895

Leu Gly Ser Lys Asn Ser Val His
900

<210> 3
<211> 21
<212> DNA
<213> artificial sequence

<220>
<223> primer

<400> 3
ctcagaagat taccagccgc c 21

<210> 4
<211> 22
<212> DNA
<213> artificial sequence

<220>
<223> primer

<400> 4
ccattatgag acagatctaa tg 22

<210> 5
<211> 20
<212> RNA
<213> artificial sequence

<220>
<223> strand 1 of AU20 dsRNA

<400> 5
aaaaaaaaaa aaaaaaaaaa 20

<210> 6
<211> 20
<212> RNA
<213> artificial sequence

<220>
<223> strand 2 of AU20 dsRNA

<400> 6
uuuuuuuuuu uuuuuuuuuu 20

<210> 7
<211> 19
<212> RNA
<213> artificial sequence

<220>
<223> strand 1 of 19-mer 5'-phosphate dsRNA

<220>
<221> misc_feature
<223> 5' phosphate modification

<400> 7
aaaaaaaaaa aaaaaaaaa 19

<210> 8
<211> 19
<212> RNA
<213> artificial sequence

<220>
<223> strand 2 of 19-mer 5'-phosphate dsRNA

<220>
<221> misc_feature
<223> 5' phosphate modification

<400> 8
uuuuuuuuuu uuuuuuuuu 19

<210> 9
<211> 45
<212> RNA
<213> artificial sequence

<220>
<223> strand 1 of AU45 dsRNA

<400> 9
aaaaaaaaaa aaaaaaaaaa aaaaaaaaaa aaaaaaaaaa aaaaa 45

<210> 10
<211> 45
<212> RNA
<213> artificial sequence

<220>
<223> strand 2 of AU45 dsRNA

<400> 10
uuuuuuuuuu uuuuuuuuuu uuuuuuuuuu uuuuuuuuuu uuuuu 45

<210> 11
<211> 50
<212> RNA
<213> artificial sequence

<220>
<223> strand 1 of AxU50 dsRNA

<400> 11
aaaauuuuuu aaaaaaaaau uuuuuuuuuu uaaauuuuaa aauuaauaau 50

<210> 12
<211> 50
<212> RNA
<213> artificial sequence

<220>
<223> strand 2 of AxU50 dsRNA

<400> 12
uuuuaaaaaa uuuuuuuuua aaaaaaaaaa auuuaaaauu uuaauuauua 50

<210> 13
<211> 40
<212> RNA
<213> artificial sequence

<220>
<223> strand 1 of AU20-18s-20 dsRNA

<220>
<221> misc_feature
<222> (20)..(20)
<223> hexaethylene glycol spacer between residue 20 and 21

<400> 13
aaaaaaaaaa aaaaaaaaaa aaaaaaaaaa aaaaaaaaaa 40

<210> 14
<211> 40
<212> RNA
<213> artificial sequence

<220>
<223> strand 2 of AU20-18s-20 dsRNA

<220>
<221> misc_feature
<222> (20)..(20)
<223> hexaethylene glycol spacer between residue 20 and 21

<400> 14
uuuuuuuuuu uuuuuuuuuu uuuuuuuuuu uuuuuuuuuu 40

<210> 15
<211> 52
<212> DNA
<213> artificial sequence

<220>
<223> Artificial sequence

<400> 15
gaaggagggt gacctgatat tcaagagata tcaggtcacc ctccttcttt tt 52

<210> 16
<211> 51
<212> RNA
<213> artificial sequence

<220>
<223> Artificial sequence

<400> 16
aagaccagac gccacuccaa cucaagaggu uggaguggcg ucuggucuuu u 51

## Description

The present invention relates generally to the fields of medicine. More specifically, the present invention relates to improved TLR3 agonists.

## INTRODUCTION

Alexopoulou et al. (2001) Nature 413: 732-738-showed that toll-Like Receptor 3 (TLR3) is another dsRNA receptor, and that it is able to recognize in particular polyinosine-polycytidylic acid. Salaun et al (2006) J. Immunol. 176: 4894-4901. Using mice deficient in MDA5, Kato et al. (2006) Nature 441: 101-105 showed that MDA5 (OMIM reference 606951) and RIGI (OMIM reference 609631) recognize different types of double-stranded RNAs: MDA5 recognizes polyinosine-polycytidylic acid and RIGI detects in vitro transcribed double-stranded RNAs. RNA viruses are also differentially recognized by RIGI and MDA5. However, it has not been established whether dsRNA acts through one or several of these receptors, nor whether there are properties of a dsRNA that are important for activity one or another of the particular receptors.

Kang et al (2002) P.N.A.S. U.S.A. 99(2): 637-642 described MDA-5 as an interferon-inducible RNA helicase with a double stranded RNA dependent ATPase activity and a caspase recruitment domain. The expression of MDA-5 polypeptides is induced during apoptosis and they were reported to have melanoma growth suppressing properties. Kato et al. (2006) found that RIGI is essential for the production of interferons in response to RNA viruses including paramyxoviruses, influenza virus, and Japanese encephalitis virus, whereas MDA5 is critical for picornavirus detection. A wide range of agents have for long been referred to as "interferon inducers", in many cases without an understanding of the mechanism(s) by which these compounds exert their immunostimulatory effect. Included among interferon inducing agents are nucleic acids, including single and double stranded DNA and RNA, and binding to RIGI and/or MDA-5 may explain the action of the double-stranded RNA molecules (dsRNA). DsRNA of various sorts, such as dsRNA of viral origin and homopolymers such as poly A-poly U (also referred to as polyAU) and poly I-poly C (also referred to as polyIC) are among the compounds reported to induce interferons. However, attempts to use double-stranded RNA for treating cancer provided at best mixed results. PolyIC which induces high levels of interferon has also demonstrated high levels of toxicity in humans and has not been further developed for therapy. Greater induction of interferon was generally associated with greater toxicity, and these properties have therefore been seen as inseparable (Stewart et al (1972) P.N.A.S. U.S.A. 69(7): 1851-1854). As a result, less potent derivatives of polyIC have been tested, such as a substituted poly IC known as Ampligen™ and polyICLC (derived from polyinosinic:polycytidylic acid by complexing the double-stranded RNA with polylysine and carboxymethylcellulose). Other dsRNA tested included polyAU composition known as Polyadenur™ (Ipsen Beaufour, France), pursued as a candidate medicament in the 1980s. Polyadenur™ was reported to be highly polydisperse with an average molecular weight of about 500 kDa (Michelson et al. (1985) Proc. Soc. Exp. Biol. And Med. 179: 1-8). Another product, Poludan™(Lens Pharma) is described as containing polyAU and useful as an interferon inducer for ocular administration for the treatment of virus diseases of eyes. International patent application nos. WO 03/078595 (Astral Inc.) and WO 04/087877 (Astral Inc.) describe test of a polyAU compositions and state that lower molecular weight fractions (less than 10 or 50 kDa) contained the observed immumodulatory and/or apoptosis-inducing activity. PolyAU and polyIC have also been used extensively in research, and examples include U.S. patent no. 6,780,429, describing preparation of "chain shortened" dsRNA compositions intended to have less toxicity. PolyAU is also sold commercially for research use, Sigma-Aldrich Inc. (St. Louis, MO), product ref. p1537, as a sodium salt. Russian patent publication no. RU2165937 describes polyAU compositions complexed with divalent platinum compounds, intended to stabilize the polyAU and increase its interferon inducing activity. However, platinum salts themselves are known to have strong cytokine and interferon inducing activity (Di Gioacchino et al. Ann Clin Lab Sci. 2004 Spring; 34(2):195-202). More recently, numerous dsRNA have been developed as siRNA reagents. While these reagents are designed to target a particular nucleotide sequence in a host cells, it has been observed that these too have interferon inducing activity, and it has been suggested that that TLRs are involved in this. SiRNA are short ssRNA, typically about 21 base pairs in length.

TLR3 contributes to induction of apoptosis of TLR3-expressing tumor cells (Salaun et al (2006) J. Immunol. 176:4894-4901 and WO2006/014653), augmenting cross-priming of CTL in response to antigens, DsRNA treatment correlates with increased survival following therapy with polyAU therapy (Andre et al Journal of Clinical Oncology, 2004 ASCO Annual Meeting Proceedings (Post-Meeting Edition). Vol 22, No 14S, 2004: 9619) and WO 2006/054177 (Andre et al.)), although these reports do not describe compositions having preferentially high molecular weight polyAU polymers. TLR3 also inhibits angiogenesis (Kleinman et al (2008) Nature). TLR3 agonists are therefore of great potential value in therapy and prevention of disease. There is a therefore a need in the art to identify properties of dsRNA compositions that would favor activity at TLR3 while minimizing toxicity observed with TLR3 agonists to date.

## SUMMARY

The invention provides a pharmaceutical composition comprising (a) an isolated high molecular weight polyAU composition capable of inducing TLR3 mediated signalling and characterized by an $M_n$ of at least 250 kDa, optionally 300 kDa, optionally an $M_w$ greater than 500 kDa, wherein less than 5% of fragments have a molecular weight less than 100 kDa, as determined by SEC-MALLS, and an Ip (index of polydispersity) of less than 2, and (b) a pharmaceutically acceptable carrier or excipient, wherein the composition has an endotoxin level of no more than about 1.0 EU/mg.

The present invention is based in part on the discovery that the nature of the ribonucleic acid residues in dsRNA has an important role in determining the dsRNA receptor-binding profile of dsRNA molecules. PolyAU demonstrates selectivity towards TLR3 over non-TLR3 dsRNA and ssRNA receptors (e.g. TLR7, TLR8, RIGI, MDA-5 and PKR), permitting the polyAU agents to be used at higher potency at TLR3 than other dsRNA agonists (such as e.g. polyIC which act on RIGI and MDA-5), in TLR3-responsive disorders. The invention also provides dsRNA of this type which have increased activity at TLR3 over others of the same type, without the associated toxicity observed with other dsRNA type compositions such as polyIC.

The presently described dsRNA are based on studies presented herein in which several structural aspects were investigated in order to determine those relevant to TLR3 stimulation. Surprisingly, it was discovered that the dsRNA of high molecular weight, and in particular dsRNA made of uridine and adenine homopolymers, have selectivity in activation of TLR3 receptors over other receptors. Furthermore, activating TLR3 selectively over other dsRNA or ssRNA receptors such as TLR7, TLR8, RIG-I or MDA-5 yielded decreased toxicity, enabling more potent TLR3 activation to be achieved. Accordingly, in one embodiment, the present disclosure provides an at least partially double stranded molecule comprising or consisting of complexes of polyadenylic acid (polyA) and polyuridylic acid (polyU), wherein the polyA is substantially free of non-adenine containing nucleotides (e.g. containing less than 10% or 5% non-adenine-containing nucleotides), and the polyU is substantially free of non-uracil containing nucleotides (e.g. containing less than 10% or 5% non-uracil containing nucleotides). In one embodiment, the disclosed polyA and polyU each comprise at least 18, 20, 40, 45, 50, 100, 200, 500, 1000 or 1500 contiguous adenine-containing or uracil-containing nucleotides, respectively. In one embodiment, the polyU and polyA contain no non-uracil (in the case of polyU) or non-adenine (in the case of polyA)-containing nucleotides, respectively. Optionally, the molecule is further characterized by any one or combination of the following:

a) comprising a polyuridylic acid strand which comprises at least one non-uracil-containing nucleotide and/or a polyadenylic acid strand comprising at least one non-adenine-containing nucleotide;
b) comprising at least one nucleotide having a base modification;
c) comprising at least one non-natural linkage; and
d) comprising a polyadenylic acid which comprises two or more polyadcnylic acids joined by a non-nucleotide spacer and/or a polyuridylic acid which comprises two or more polyuridylic acids joined by a non-nucleotide spacer.

The polyAU compositions of the invention may be used to treat TLR3-responsive disease states. It has been found that TLR3 can itself (e.g. without the need for substantial signalling by other dsRNA receptors like RIG-1 or MDA-5) mediate a therapeutically useful effect. In the examples herein, it is demonstrated that different tumors are responsive to TLR, and that specifically inhibiting TLR3 activity results in a corresponding decrease in the activity (e.g. anti-tumor activity) mediated by the polyAU compositions. Moreover, these examples included in vivo immunodeficient models demonstrating this anti-tumor activity is direct (e.g. not dependent upon an immune-cell mediated effect). It is therefore envisaged that the polyAU compositions of the invention can be used as a single therapeutic agent in the treatment of a TLR3-responsive disorder, e.g. without being present in a composition comprising other active ingredients (e.g. vaccines, antibodies, anti-cancer agents, etc.) or used in combination with such other ingredients. Alternatively, polyAU compositions can be used in combination with other agents, but in an amount where they are effective as a single therapeutic agent. The polyAU compositions can however also be used in combination with other therapeutic or prophylactic agents. For example, in view of the pro-apoptotic effect of the polyAU compositions on tumor cells without reliance on immune cells, the polyAU compositions can advantageously be used in combination with chemotherapeutic agents, including but not limited to agents which impair immune system function, for the treatment of cancer.

In another example, it has been reported that long dsRNA (polyIC) and various short sequence-specific and non-specific dsRNA can inhibit ocular angiogenesis (Kleinman 2008), and that this effect is TLR3-mediated. Certain conditions are therefore TLR3-responsive, and they can be treated using TLR3 selective compositions, including the polyAU compositions of the invention, without inducing significant activity across non-TLR3 dsRNA receptors, particularly those that individually or collectively lead to toxicity (e.g. toxic levels of interferon induction). In addition to the conditions exemplified herein, TLR3-responsive conditions can be assessed by testing whether a selective TLR3 agonist ameliorates the condition or has an effect associated with amelioration (e.g. upregulation of TLR3, in turn associated with sensitization to therapy), or by determining whether decreasing TLR3 function (e.g. in a TLR3 knock-out animal, or using TLR3 or TRIF shRNA) substantially eliminates a beneficial effect observed upon treatment with a TLR3 agonist dsRNA. In the case of tumors for example, tumor cells can be contacted with a selective TLR3 agonist and it can be assessed whether the tumor cells upregulate TLR3, produce cytokines, undergo apoptosis, etc.

Accordingly, such disease states can potentially be much more effectively treated by using a ligand which has better selectivity for TLR3, in a disease state where induction of TLR3 activity is able to ameliorate or prevent the disease state. Indeed, in view of the potential for the use of dsRNA therapy in the treatment of many disease states, it would be desirable to have the ability to selectively treat subjects, with compounds which selectively interact as ligands with the specific dsRNA receptor involved in the disease state without having the e.g. high levels of cytokine production associated with toxicity as appears to be mediated by other dsRNA receptors activation pathways, e.g. RIG-I and MDA-5 activation pathways, or by the ssRNA receptor TLR7 or TLR8. Such selective compositions could be used at higher doses, or in repeated administrations (e.g. daily doses) or combined with another therapeutic agent to obtain a precise therapeutic effect, or as vaccine adjuvants where toxicity is sought to be minimized.

The inventors also demonstrate that for the production of stable pharmaceutical compositions which can be used in therapy for the regulation of TLR3, particularly where TLR3 is active in endosomes, human-suitable dsRNA compositions having increased potency at TLR3 can be obtained by controlling the length of the dsRNA polymers. Potency can further be increased by controlling hybridization quality, as evidenced by melting temperature, hyperchromicity and size distribution profile measurements particular, polyAU's activity appears to be mediated substantially exclusively by TLR3. PolyIC, in contrast, has high levels of activity at other dsRNA receptors RIGI and MDA-5.

The invention is further based on the results of studies demonstrating that polyAU compositions having increased TLR3 agonist activity without increased toxicity can be used in treatment regimens involving higher dosing (e.g. a greater total amount, in one or more administrations, over a given period of time) than for other dsRNA compositions. In particular, polyAU demonstrates increased activity at greater than once-weekly (e.g. 2 or more days per week, consecutive or not) administration of the compositions. PolyAU can also be used at doses exceeding 60 mg per patient per week, or exceeding 1 or 2 mg/kg per patient per week. These regimens avoid toxicity and are believed to maximize benefit to a patient (e.g. having a tumor). The invention is further based on comparative tests of different polyAU compositions. Increased induction of TLR3 mediated signalling was observed for high molecular weight polyAU compositions, and any of the polyAU compositions of the invention may be characterized by an $M_n$ of at least 250 kDa, optionally 300 kDa, wherein less than 5% of fragments have a molecular weight less than 100 kDa, as determined by SEC-MALLS. Optionally the polyAU comprises less than 5% of fragments have a molecular weight less than 200 kDa. The invention further demonstrates that Tm and hyperchromicity have a role in TLR3 signaling ability, and any of the polyAU compositions of the invention may be characterized by a Tm of at least 59 °C and/or a hyperchromicity of at least 40 %, preferably 50%, 51% or 55%. In one aspect, the high molecular polyAU compositions may have a $M_n$ of between about 250 kDa, optionally 300 kDa and 2000 kDa; optionally in another aspect the high molecular polyAU compositions have an $M_n$ of between about 1500 and 3000 kDa, 4000 kDa or 5000 kDa.

In one embodiment the isolated polyAU composition is capable of inducing TLR3 mediated signalling, preferably selective TLR3 signalling, and characterized by an $M_n$ of at least 250 kDa, optionally 300 kDa, optionally an $M_w$ greater than 500 kDa, wherein less than 5% of fragments have a molecular weight less than 100 kDa, as determined by SEC-MALLS, a Tm of at least 59 °C, and a hyperchromicity of at least 40 %, preferably 50 %. Optionally the composition is further characterized by a FWHM of less than 5 °C. In one embodiment the isolated polyAU composition is capable of inducing a specific TLR3 mediated signalling and characterized by a Tm of at least 59 °C, and a hyperchromicity of at least 40 %, preferably 50 %. In one embodiment the isolated polyAU composition is capable of inducing a specific TLR3 mediated signalling and characterized by an Ip of less than 2. In one embodiment the isolated polyAU composition is capable of inducing TLR3 mediated signalling and characterized by FWHM of less than 5°C and an Ip of less than 2. In a preferred embodiment, the above mentioned polyAU composition have an endotoxin content of less than 1 EU/mg.

In another aspect, any of the high molecular weight polyAU compositions may be characterized by a $M_n$ of at least 800 kDa, by a $M_n$ of about 800 kDa, or by a $M_n$ between 500 and 1000 kDa. In another aspect, any of the dsRNA compositions may be characterized by a $M_n$ of at least 1400 kDa. In another aspect, any of the dsRNA compositions may be characterized by a $M_n$ of no more than about 1300 kDa. In another aspect, any of the dsRNA compositions may be characterized by a $M_n$ of about 1490 kDa, optionally between 1400 and 1600 kDa.

In another aspect, any of the high molecular weight polyAU compositions may be characterized by less than 50% of dsRNA species having a molecular weight less than 300 kDa, optionally less than 600 kDa, as determined by SEC-MALLS. In another aspect, less than 5% of dsRNA species have a molecular weight less than 300 kDa, optionally less than 600 kDa, as determined by SEC-MALLS.

In another aspect, the inventors demonstrate that yet longer dsRNA, e.g.dsRNA having a $M_n$ of more than 1500 kDa, are very potent TLR3 ligands. These dsRNA typically have a $M_n$ between 1500 kDa and 2000 kDa, optionally 3000 kDa, 4000 kDa or 5000 kDa. Exemplary dsRNA compositions (e.g. polyAU compositions) can be further characterized by a hyperchromicity of at least 45, preferably at least 50% and/or a melting temperature of at least 59°C, preferably at least 60°C. An exemplary dsRNA composition has a Mn of at least 1500 kDa, a hyperchromicity of at least 50% and a melting temperature of at least 60°C. Furthermore, the polydispersity index of such compositions is not more than 2.0.

In another aspect, the dsRNA according to the present invention may be characterized by less than 50% of dsRNA species having a molecular weight less than 1000 kDa as determined by SEC-MALLS. In another aspect, less than 20% of dsRNA species have a molecular weight less than 1000 kDa as determined by SEC-MALLS. In another aspect, less than 5% of dsRNA species have a molecular weight less than 500 kDa, as determined by SEC-MALLS.

In another aspect, any of the high molecular weight polyAU compositions may be characterized by:

a) less than about 20 % of dsRNA species having a molecular weight less than about 300 kDa;
b) less than about 50 % of dsRNA species having a molecular weight less than about 600 kDa;
c) less than about 60 % of dsRNA species having a molecular weight less than about 800 kDa; and
d) less than about 70 % of dsRNA species having a molecular weight less than about 1000 kDa.

In yet another aspect, any of the high molecular weight polyAU compositions may be characterized by:

a) less than about 5 % of dsRNA species having a molecular weight less than about 600 kDa;
b) less than about 15 % of dsRNA species having a molecular weight less than about 800 kDa; and
c) less than about 25 % of dsRNA species having a molecular weight less than about 1000 kDa.

In another aspect, any of the high molecular weight polyAU compositions, may be characterized by:

a) less than about 5% of dsRNA species having a molecular weight less than about 500 kDa;
b) less than about 20% of dsRNA species having a molecular weight less than about 1000 kDa; and
c) less than about 50% of dsRNA species having a molecular weight less than about 1500 kDa.

In another aspect, any of the high molecular weight polyAU compositions, may be characterized by:

a) less than about 5% of dsRNA species having a molecular weight less than about 500 kDa;
b) less than about 15% of dsRNA species having a molecular weight less than about 1000 kDa; and
c) less than about 25% of dsRNA species having a molecular weight less than about 1500 kDa.

In another aspect, any of the high molecular weight polyAU compositions, may be characterized by:

a) less than about 1% of dsRNA species having a molecular weight less than about 500 kDa;
b) less than about 5% of dsRNA species having a molecular weight less than about 1000 kDa;
c) less than about 10% of dsRNA species having a molecular weight less than about 1500 kDa; and
d) less than about 15% of dsRNA species having a molecular weight less than about 2000 kDa.

Any of the polyAU compositions provided herein can be characterized, without limitation, by any one or combination of the following properties:

a) specified molecular weight or chain length characteristics;
b) at least partly, and most preferably in substantially, double stranded form, and/or having specified melting temperature, hyperchromicity and/or value for the FWHM of the $1^{st}$ derivative of the melting temperature curve;
c) characteristics of the starting materials (e.g. ssRNA compositions) and/or methods used to make the dsRNA composition;
d) ability to bind TLR3, e.g. binding affinity, dissociation kinetics, selective binding to TLR3 over other double stranded and/or single stranded RNA receptors e.g. R1G-I, MDA5, TLR7, TLR8;
e) reduced or substantially lacking TLR7, TLR8, RIGI and/or MDA-5, agonist activity;
f) having TLR3 agonist activity, for example inducing TLR3-mediated signalling;
g) having TLR3 agonist activity, when delivered (to a cell, to a subject) in a formulation with other compounds designed to enhance their ability to enter cells, and/or when delivered without such cell entry enhancing compounds;
h) having biological activity which is neutralized by blocking TLR3 (e.g. using an antibody specific for TLR3, inhibiting TLR3 or the TLR3 signaling pathway using an shRNA, etc.)
i) suitability for parenteral administration, including intravenous, subcutaneous, intramuscular, intravitreal injection, and/or in the presence or absence of a cell entry enhancing agent (e.g. a liposome or lipid formulation);
j) presence or absence of non-dsRNA molecules, including for example endotoxin level (e.g. no more than about 1.0 EU/mg);

k) decreased toxicity and/or type I interferon induction, optionally without a commensurate decrease in TLR3 signalling or binding activity compared to polyIC (e.g. a polyIC composition having physical characteristics similar to the polyAU to which it is compared, or a commercially available polyIC);
l) inducing the apoptosis of TLR3-expressing cells, particularly tumor cells;
m) inducing the expression of TLR3 in cells, particularly tumor cells;
n) sensitizing a cell, particularly a tumor cell, to treatment with a TLR3 agonist, e.g. by increasing apoptosis of or cytokine production by said cell following treatment with the TLR3 agonist;
o) inducing cytokine production by TLR3-expressing cells, particularly dendritic cells, NK cells, T cells, epithelial cells, endothelial cells, and tumor cells;
p) inducing an immune reaction in a mammal toward an antigen, tumor, infected cell or pathogen;
q) inducing elimination in a mammal of a tumor, infected cell or pathogen; and
r) suitability for administration to a mammal on more than one day per week.

Also provided are a series of exemplary compounds having increased activity at TLR3. Most notably, the invention includes dsRNA compositions having the characteristics described for Group 0, Group 1, Group 2, and Group 3 compositions, and any of the polyAU compositions of the invention can further comprises the characteristics of Group 0, 1, 2 or 3 compositions.

Group 3 compositions, preferably polyAU compositions, can be characterized by a $M_n$ of between about 250 kDa and 500 kDa, and any one or a combination of:

a) less than about 5%, optionally 10% or 25 % of fragments having a molecular weight less than about 100 kDa;
b) less than about 50 %, optionally less than about 40%, of fragments having a molecular weight less than about 200 kDa;
c) less than about 70 %, optionally less than about 60%, of fragments having a molecular weight less than about 300 kDa;
d) an $M_w$ of greater than 450 kDa, optionally 500 kDa;
e) an Ip of less than 2;
f) a Tm of at least 59 °C;
g) a hyperchromicity of at least 40%, preferably 50%; and
h) optionally, an FWHM of less than 5 °C, optionally less than 3° C.

A preferred characterization of a Group 3 composition is a polyAU composition having a $M_n$ of between 250 kDa and 500 kDa, as determined by SEC-MALLS, and characterized by the following combination:

a) less than about 10% of dsRNA species having a molecular weight less than about 100 kDa;
b) less than about 40 % of dsRNA species having a molecular weight less than about 200 kDa;
c) less than about 60 % of dsRNA species having a molecular weight less than about 300 kDa;
d) an Ip of less than 2;
e) a Tm of at least 59 °C;
f) a hyperchromicity of at least 40%, preferably 50%; and
g) optionally, an FWHM of less than 3° C.

Group 3 compositions have been found to have TLR3 activity resulting in a maximum fold increase in same luciferase assay of 50 to 250, with an EC50 of 10 to 50 μg/ml. The Group 3 compositions can be further or alternatively characterized as comprising complexes of a first composition comprising single-stranded polymers (e.g. polyA) characterized by an $M_n$ of between 50 kDa and 150 kDa, preferably about 81 kDa or within a range of 50, 20 or 10 kDa thereof, and a second composition comprising single-stranded polymers (e.g. polyU) characterized by an $M_n$ of between 100 kDa and 300 kDa, preferably about 149 kDa or within a range of 50, 20 or 10 kDa thereof. In another example, Group 3 compositions can be characterized as comprising complexes of a first composition comprising single-stranded polymers (e.g. polyA) characterized by an $M_n$ of between 300 kDa and 600 kDa, between 400 kDa and 600 kDa, optionally about 500 kDa or within 100, 50, 20 or 10 kDa thereof, and a second composition comprising single-stranded polymers (e.g. polyU) characterized by an $M_n$ of between 150 kDa and 400 kDa, optionally about 149 kDa or within 50, 20 or 10 kDa thereof.

Group 2 compositions, preferably polyAU compositions, can be characterized by a $M_n$ of between about 500 kDa and 800 kDa, optionally between 600 kDa and 800 kDa, and any one or a combination of:

a) less than about 5 % of fragments having a molecular weight less than about 100 kDa;
b) less than about 20 % of fragments having a molecular weight less than about 200 kDa;

c) less than about 40 % of fragments having a molecular weight less than about 300 kDa;
d) less than about 50 % of fragments having a molecular weight less than about 500 kDa;
e) less than about 70 % of fragments having a molecular weight less than about 600 kDa;
f) less than about 75 % of fragments having a molecular weight less than about 700 kDa;
g) less than about 80 % of fragments having a molecular weight less than about 800 kDa;
h) an $M_w$ of greater than 700 kDa or 900 kDa;
i) an Ip of less than 2;
j) a Tm of at least 59 °C;
k) a hyperchromicity of at least 40%, preferably 50%; and
l) optionally, an FWHM of less than 5 °C, optionally less than 3° C.

A preferred characterization of a Group 2 composition is a polyAU composition having a $M_n$ of between 500 kDa and 800 kDa, and characterized by the following combination:

a) less than about 40 % of dsRNA species having a molecular weight less than about 300 kDa;
b) less than about 50 % of dsRNA species having a molecular weight less than about 500 kDa;
c) a Tm of at least 59 °C;
d) a hyperchromicity of at least 40%, preferably 50%; and
e) optionally, an FWHM of less than 5 °C, optionally less than 3° C.

Group 2 compositions have been found to have TLR3 activity resulting in a maximum fold increase in same luciferase assay of 50 to 250, with an EC50 of 1 to 10 μg/ml. The Group 2 compositions can be further or alternatively characterized as comprising complexes of a first composition comprising single-stranded polymers (e.g. polyA) characterized by an $M_n$ of between 300 kDa and 600 kDa, between 400 kDa and 600 kDa, optionally about 500 kDa or within 100, 50, 20 or 10 kDa thereof, and a second composition comprising single-stranded polymers (e.g. polyU) characterized by an $M_n$ of between 50 kDa and 300 kDa, between 150 kDa and 400 kDa, optionally about 149 kDa or within 50, 20 or 10 kDa thereof.

Group 1 compositions, preferably polyAU compositions, can be characterized by a $M_n$ of between about 800 kDa and 2000 kDa, optionally between about 800 kDa and 1500 kDa, optionally further at least 1400 kDa or at least 1500 kDa, and any one or a combination of:

a) less than about 5 % of fragments having a molecular weight less than about 200 kDa;
b) less than about 10 % of fragments having a molecular weight less than about 300 kDa;
c) less than about 20 % of fragments having a molecular weight less than about 400 kDa;
d) less than about 25 % of fragments having a molecular weight less than about 500 kDa;
e) less than about 30 % of fragments having a molecular weight less than about 600 kDa;
f) less than about 40 % of fragments having a molecular weight less than about 800 kDa;
g) less than about 50 % of fragments having a molecular weight less than about 1000 kDa;
h) less than about 60 % or 70 % of fragments having a molecular weight less than about 1400 or 1500 kDa, respectively;
i) an $M_w$ of greater than 1000 kDa;
j) a Tm of at least 59 °C;
k) a hyperchromicity of at least 40%, preferably 50%; and
l) optionally, an FWHM of less than 5 °C, optionally less than 3° C.

A preferred characterization of a Group 1 composition is a polyAU composition having a $M_n$ of between 800 kDa and 1500 kDa, and characterized by the following combination:

a) less than about 30 % of dsRNA species having a molecular weight less than about 600 kDa;
b) less than about 40 % of dsRNA species having a molecular weight less than about 800 kDa;
c) less than about 50 % of dsRNA species having a molecular weight less than about 1000 kDa;
d) an Ip of less than 2;
e) a Tm of at least 59 °C; and
f) a hyperchromicity of at least 40%, preferably 50%; and
g) optionally, an FWHM of less than 5 °C, optionally less than 3° C.

Another preferred set of compositions are Group 0 compositions. Group 0 compositions generally have an $M_n$ between 1500 kDa and 2000 kDa, optionally 3000 kDa, 4000 kDa or 5000 kDa. A preferred characterization of Group 0 compositions is a dsRNA preferably a polyAU composition, having a $M_n$ of more than 1500 kDa, and characterized by the following combination:

a) less than about 5% of dsRNA species having a molecular weight less than about 500 kDa;
b) less than about 20% of dsRNA species having a molecular weight less than about 1000 kDa; and
c) less than about 50% of dsRNA species having a molecular weight less than about 1500 kDa.
d) an Ip of less than 2;
e) a Tm of at least 60 °C; and
f) a hyperchromicity of at least 40%, preferably 50%; and
g) optionally, an FWHM of less than 5 °C, optionally less than 3° C.

Another preferred characterization of a Group 0 composition, preferably a polyAU composition, has a $M_n$ of more than 1500 kDa, and is characterized by the following combination:

a) less than about 5% of dsRNA species having a molecular weight less than about 500 kDa;
b) less than about 15% of dsRNA species having a molecular weight less than about 1000 kDa; and
c) less than about 25% of dsRNA species having a molecular weight less than about 1500 kDa.
d) an Ip of less than 2;
e) a Tm of at least 60 °C;
f) a hyperchromicity of at least 40%, preferably 50%; and
g) optionally, an FWHM of less than 5 °C, optionally less than 3° C.

In another aspect, any of the high molecular weight Group 0 composition, preferably a polyAU composition, may be characterized by:

a) less than about 1% of dsRNA species having a molecular weight less than about 500 kDa;
b) less than about 5% of dsRNA species having a molecular weight less than about 1000 kDa;
c) less than about 10% of dsRNA species having a molecular weight less than about 1500 kDa;
d) less than about 15% of dsRNA species having a molecular weight less than about 2000 kDa;
e) an Ip of less than 2;
f) a Tm of at least 60 °C;
g) a hyperchromicity of at least 40%, preferably 50%; and
h) optionally, an FWHM of less than 5 °C, optionally less than 3° C.

Group 1 compositions have been found to have TLR3 activity resulting in a maximum fold increase in same luciferase assay of 50 to 250, with an EC50 of 0.1 to 2 μg/ml. In one aspect, the Group 1 compositions can be further or alternatively characterized as comprising complexes of a first composition comprising single-stranded polymers (e.g. polyA) characterized by an $M_n$ of between 300 kDa and 600 kDa, between 400 kDa and 600 kDa, optionally about 500 kDa or within 100, 50, 20 or 10 kDa thereof, and a second composition comprising single-stranded polymers (e.g. polyU) characterized by an $M_n$ of between 150 kDa and 400 kDa, optionally about 149 kDa or within 50, 20 or 10 kDa thereof. In another aspect the Group 1 compositions can be characterized as comprising complexes of a first and a second composition, each of said compositions comprising single-stranded polymers characterized by an $M_n$ of between 300 kDa and 600 kDa, between 400 kDa and 600 kDa optionally about 500 kDa or within 100, 50, 20 or 10 kDa thereof.

Group 0 compositions have been found to have TLR3 activity resulting in a maximum fold increase in same luciferase assay of 50 to 250, with an EC50 of 0.1 to 2 μg/ml. In one aspect, the Group 0 compositions can be further or alternatively characterized as comprising complexes of a first composition comprising single-stranded polymers (e.g. polyA) characterized by an $M_n$ of between 300 kDa and 600 kDa, between 400 kDa and 600 kDa, optionally about 500 kDa or within 100, 50, 20 or 10 kDa thereof, and a second composition comprising single-stranded polymers (e.g. polyU) characterized by an $M_n$ of between 100 kDa and 600 kDa, between 400 kDa and 600 kDa optionally about 500 kDa or within 100, 50, 20 or 10 kDa thereof.

Any of the high molecular weight compositions, including the Group 0, 1, 2 and 3 compositions, can be further characterized by any one or combination of the following:

a) comprising a polyuridylic acid strand which comprises at least one non-uracil-containing nucleotide and/or a polyadenylic acid strand comprising at least one non-adenine-containing nucleotide;
b) comprising at least one nucleotide having a base modification;
c) comprising at least one non-natural linkage; and
d) comprising a ssRNA strand made up of two or more ssRNA strands joined by a non-nucleotide spacer, for example a polyadenylic acid which comprises two or more polyadenylic acids joined by a non-nucleotide spacer and/or a polyuridylic acid which comprises two or more polyuridylic acids joined by a non-nucleotide spacer.

The compositions of the disclosure will preferably be a heterogeneous mixture of a plurality of dsRNA polymers, as individual dsRNA polymer species (fragments) within the dsRNA composition will differ for example in terms of the molecular weight or chain length, quality of hybridization. The compositions will preferably be made by a process comprising a step of performing a polymerization reaction, in the presence of an enzyme (e.g. a polynucleotide phosphorylase) and a plurality of ribonucleotide monomers as a substrate, to generate an ssRNA composition, and mixing said composition with a second ssRNA composition to form dsRNA polymers. In one embodiment, for preferred selective TLR3 agonists the type of base on each strand of substantially all dsRNA in a mixture will preferably be homogenous, e.g. a polyAU composition will consist of complexes of polyA and polyU strands. Preferably, as shown herein, when the dsRNA composition is a mixture, the dsRNA fragments in the composition will typically be characterized by a distribution of molecular weights that adopt a bell-shaped pattern. That is, the majority of the dsRNA fragments will be found within a selected molecular weight range, and a smaller number of dsRNA fragments will be found within molecular weight ranges below or above the selected range. Such distribution can be combined with the additional characteristics described herein that were correlated to increased TLR3 agonist activity. Optionally, the compositions can be characterized as comprising a plurality of dsRNA polymer species of heterogeneous molecular weight or chain length, wherein at least 40%, 50%, 75%, or 90% of the dsRNA species in the composition have a molecular weight within about 800, 600, 500, 400, 300, 200 kDa, 100 kDa or 50 kDa of the $M_n$ (so long as such is consistent with the $M_n$ of the dsRNA composition).

Although a heterogeneous mixture of a plurality of dsRNA polymers is usually obtained upon manufacturing of dsRNA compositions, a narrow size distribution profile is preferred. Preferred compositions according to the invention show a narrow beel-shaped size distribution, where the majority of the dsRNA fragments have a similar mass. The size distribution is controlled upon the hybridization step, by controlling reaction conditions. A well hybridized polymer, i.e. having a hyperchromicity of more than 50% and a Tm of more than 60°C, will have a narrower size distribution. Hybridization conditions are discussed herein.

In one aspect the invention provides a process for producing a polyAU composition comprising mixing a poly A composition characterized by an $M_n$ of at least 300 kDa and poly U composition characterized by an $M_n$ of at least 100 kDa as determined by SEC-MALLS, in a buffer for a period of time of between 5 minutes and 4 hours and at a temperature of between room temperature and 100 °C. In one embodiment, the polyA composition is characterized by a $M_n$ of between 400 kDa and 600 kDa and polyU composition is characterized by an $M_n$ of between 150 kDa and 400 kDa. Optionally, said temperature is between 60 °C and 75 °C and said period of time is between 5 minutes and 4 hours, optionally in an aqueous buffer of NaCl 0.15M and optionally in an organic buffer. Optionally, the polyU has a $M_n$ that is between 20% and 100%, preferably between 25% and 80% of the $M_n$ for the polyA. Optionally, the polyU has a $M_n$ that is between 20% and 110%, preferably between 90% and 110% of the $M_n$ for the polyA. In another embodiment, the polyU has a $M_n$ that is between 20% and 110%, preferably between 40% and 60% of the $M_n$ for the polyA. The resulting polyAU composition can be any polyAU composition of the invention, and indicated ssRNA compositions (e.g. having specified Mn ranges for the particular polyAU composition, such as Group 0, 1, 2 and 3 compositions) can be produced by this method. Optionally the method further comprises admixing the resulting composition comprising high-molecular weight double-stranded RNA polymers with a pharmaceutically acceptable carrier or excipient (e.g. a carrier adapted or suitable for intravenous, intravitreal or subcutaneous administration).

In another aspect, the disclosure features, a dsRNA composition made by a method described herein. In another aspect, the method includes an intermediate or reaction mixture from any of the methods for making or analyzing a dsRNA described herein. In another aspect, the disclosure features, a pharmaceutical composition that includes a dsRNA composition described herein. In one embodiment, the pharmaceutical composition further includes a pharmaceutically acceptable carrier. In one embodiment, the pharmaceutical composition is in a form suitable for systemic administration. In a preferred embodiment, the pharmaceutical composition is suitable for subcutaneous, intravenous, intra-arterial, intrasynoval, intramuscular, intraperitoneal, intravitreous, epidural, subdural or intrathecal administration. In one embodiment, a pharmaceutical composition for systemic administration can be an isotonic solution, e.g., an isotonic solution with or without preservatives. Examples of preservative include, but are not limited to, benzyl alcohol, mannitol and leucine. A unit dosage amount of a pharmaceutical composition of the invention can be disposed within a package or a device suitable for administration. E.g., a composition suitable for subcutaneous delivery can be disposed within a syringe configured for subcutaneous delivery, a composition suitable for intravenous delivery can be disposed within a syringe configured for intravenous delivery or within another device for intravenous delivery, e.g., an intravenous drip bag or bottle. In one embodiment, the dsRNA composition is not in an oil-in-water emulsion.

In another aspect, the annealing dsRNA composition is the final product. In other embodiments, the method can include one or more additional processing steps to obtain a final product. The method can include processing the annealed dsRNA composition to obtain a dsRNA composition where e.g. less than 5% of fragments have a molecular weight less than a particular molecular weight (e.g. 100, 200, 300, 400 or 500 kDa), as determined by SEC-MALLS, where the dsRNA composition has a particular hybridization characteristic (e.g. hyperchromicity, Tm or FWHM). The method can include processing the annealed dsRNA composition to obtain a dsRNA composition where e.g. less than 20% of fragments have a molecular weight less than 1000 or 1500 kDa, as determined by SEC-MALLS, where the dsRNA composition has a particular hybridization characteristic (e.g. hyperchromicity, Tm or FWHM). For example, protocols for annealing, or selection of fractions by size exclusion chromatography, ion exchange chromatography and/or filtration can be used to obtain a dsRNA composition having such characteristics.

In another aspect, the disclosure features, methods of evaluating or processing an ssRNA composition to determine suitability of the ssRNA composition for processing into a dsRNA composition, e.g., a dsRNA composition described herein. The method includes determining the molecular weight characteristics (e.g. Mn, Mw) and/or polydispersity and/or endotoxin content, comparing said characteristics to a preselected criterion and making a decision about the ssRNA composition based upon the whether the preselected criterion is met. In a preferred embodiment, a decision or step is taken, e.g., the ssRNA composition is classified, accepted or discarded, processed into a dsRNA, or a record made or altered to reflect the determination, depending upon whether the preselected criterion is met. In some embodiments, when the preselected criterion is not met, a decision can be made about altering one or more steps in manufacturing of a ssRNA composition. In a preferred embodiment, said method is carried out for two complementary ssRNA compositions (e.g. a polyA composition and a polyU composition).

In one embodiment of the disclosure, the preselected criterion is the molecular weight characteristics (e.g. $M_w$, $M_n$) for the single-stranded RNA polymers, optionally with criteria of polydispersity or distribution of ssRNA polymers having a particular molecular weight or weight range. For example, a determination that an ssRNA preparation has an $M_n$ for the single-stranded polymers of at least about 50, 100, 150 or 300 kDa, optionally with a polydispersity (Ip value) of no more than about 1.4, 1.6, or 2.0, optionally with an endotoxin content of less than 2.0 EU/mg, is indicative that a ssRNA preparation is suitable for processing into a dsRNA composition having an $M_n$ for the double-stranded polymers of at least about 250 kDa (or further having any of the characteristics described herein, e.g. those of Group 0, 1, 2 or 3 compositions). In another example, a determination that a first ssRNA preparation has an $M_n$ for the single-stranded polymers of between about 150 and 400 kDa, and that a second ssRNA preparation has an $M_n$ for the single-stranded polymers of between about 400 and 600 kDa, optionally each of first and second compositions having a polydispersity (Ip value) of no more than about 1.4, 1.6, or 2.0, is indicative that an ssRNA preparation(s) is suitable for processing into a dsRNA composition having an $M_n$ for the double-stranded polymers of at least about 800 kDa, e.g., a dsRNA composition described herein. Similarly, each of first and second ssRNA compositions can be determined to have an $M_n$ for the single-stranded polymers of between about 400 and 600 kDa and be suitable for processing into a dsRNA composition having an $M_n$ for the double-stranded polymers of at least about 1500 kDa In such embodiments, when this preselected criterion is met, the ssRNA preparation is accepted and processed into intermediates, drug substance or drug product. In one embodiment, the molecular weight of ssRNA polymers in a dsRNA preparation can be determined using, e.g., SEC-MALLS.

Methods disclosed herein are useful from a process standpoint, e.g., to monitor or ensure batch-to-batch consistency or quality, or to evaluate a sample with regard to a preselected criterion. In one aspect, the invention features, a method of evaluating or processing an intermediate dsRNA preparation, e.g., produced by a method described herein, to determine suitability of the intermediate preparation for processing into a final dsRNA composition. The intermediate dsRNA preparation can be a composition obtained, e.g., by mixing two compositions of complementary ssRNA as described herein. The method includes comparing the molecular weight characteristics (e.g., Mn, Mw, Ip, molecular weight distributions such as percentage of fragments having a molecular weight less than a threshold value), and/or a characteristic indicative of the quality of hybridization of the dsRNA (e.g. Tm, hyperchromicity, FWHM), in the intermediate dsRNA preparation to a property (e.g. Mn, Mw, Ip, molecular weight distributions such as percentage of fragments having a molecular weight less than a threshold value) of the starting material(s) (e.g. ssRNA compositions), and making a decision about the intermediate dsRNA preparation based upon whether a preselected criterion between the starting material(s) and intermediate dsRNA preparation is met. For example, as described herein, when starting materials comprise a first ssRNA composition having an $M_n$ of between about 400 kDa and about 600 kDa (and optionally an Ip value of no more than 2.0, 1.6, or 1.4) and a second ssRNA composition comprising ssRNA complementary to the ssRNA in the first composition, the second ssRNA composition having an $M_n$ of between about 150 kDa and about 400 kDa (and optionally an Ip value of no more than 2.0, 1.6, or 1.4) or a second ssRNA composition having an $M_n$ of between about 400 kDa and about 600 kDa (and optionally an Ip value of no more than 2.0, 1.6, or 1.4), the criteria can be that the intermediate dsRNA compositions shall have an $M_n$ of at least about 500 kDa, optionally at least about 600 kDa, 800 kDa, 1000 kDa, 1500 kDa, optionally wherein less than 10%, less than 5% of fragments have a molecular weight less than about 100, 200, 300, 400, 500 or 600 kDa, as determined by SEC-MALLS, optionally further where the dsRNA is characterized by a Tm of at least 59 °C or preferably 60°C, a hyperchromicity of at least 40% or preferably 50%, a FWHM value of no more than 5.0 °C, 3.0°C, 2.0°C, 1.5 °C and/or an Ip value of no more than about 2.0, 1.5, 1.4 or 1.2. In a preferred embodiment, a decision or step is taken, e.g., the intermediate dsRNA preparation is classified, accepted or discarded, processed into a drug substance or drug product, or a record made or altered to reflect the determination, depending upon whether the a preselected relationship is met. In some embodiments, when the preselected criterion is not met, a decision can be made about altering one or more steps in manufacturing of a dsRNA composition.

The selected ssRNA can be hybridized in a reproducible manner to obtain compositions belonging to Group 0. Such compositions are obtained using the process conditions as disclosed herein. Such process conditions are able to provide a high molecular weight dsRNA having desirable hyperchromicity, Tm and Ip values. Desired dsRNA composition have a Mn higher than 1500 kDa, optionally at least 1600, 1700, 1800, 1900 or 2000 kDa, an Ip of less than 2.0, a hyperchromicity of more than 45%, more than 50%, 51% or 55%, a Tm of not less than 58°C, 59°C or 60°C.

The preselected criterion can be a predetermined $M_n$ or $M_w$ or range of $M_n$ or $M_w$ for the intermediate dsRNA preparation, based on the properties of the particular starting materials used. In one embodiment, the preselected criterion is a decrease in the proportion of fragments having a molecular weight less than 100, 150, 200, 300, 400, 500, 600, 800, 1000, 1500 or 2000 kDa in the intermediate preparation, based on the properties of the particular starting materials used. In another disclosure, the preselected criterion is a distribution of fragments within a predetermined range of molecular weight in the intermediate preparation (e.g. 50%, 75% or 90% of the dsRNA fragments having a molecular weight between about 100 kDa and 1200 kDa, between about 300 and 1200 kDa, between about 1500 and 3000, 4000 or 5000 kDa or having a molecular weight greater than a particular molecular weight, based on the properties of the particular starting materials used. In other disclosure, the preselected criterion is the polydispersity of the intermediate preparation (e.g. Ip value of no more than 2.0, 1.5, 1.4 or 1.2), the Tm (e.g. at least 59 °C or 60°C), hyperchromicity, (e.g. at least 40%, 50%, 51% or 55%), or FWHM (e.g. no more than 5, or preferably no more than 3) based on the properties of the particular starting materials used. In such embodiments, when this preselected criterion is met, the intermediate preparation is accepted and processed into further intermediates, drug substance or drug product.

The preselected criterion can be an indicator of hybridization quality of the intennediate dsRNA composition, for example a Tm of at least 59 °C or 60°C, hyperchromicity of at least 40%, 50%, 51% or 55%,, or an FWHM of no more than 5, or preferably no more than 3. In such embodiments, when this preselected criterion is met, the intermediate preparation is accepted and processed into further intermediates, drug substance or drug product.

The preselected criterion can be bioactivity of the intermediate dsRNA preparation based on the properties of the particular starting materials used, as compared to a reference value for bioactivity based on a reference dsRNA, for example a reference dsRNA generated a comparable starting material as the intermediate dsRNA. Examples of such criterion of bioactivity include but are not limited any one or combination of the TLR3 activities disclosed herein (e.g. induction of TLR3 signaling based on a reporter gene). When this preselected criterion is met, the intermediate preparation is accepted and processed into further intermediates, drug substance or drug product.

In one embodiment, the presence, amounts and molecular weights of a moiety in the starting material and/or intermediate preparation is determined using one or more of SEC-MALLS, nuclear magnetic resonance (NMR), capillary electrophoresis (CE) and high performance liquid chromatography (HPLC). The bioactivity can be determined using any of the assay methods described herein.

Methods disclosed herein are useful from a process standpoint, e.g., to monitor or ensure batch-to-batch consistency or quality, or to evaluate a sample with regard to a preselected criterion.

Certain characteristics can make an ssRNA sample a more preferred starting material for making a dsRNA. Accordingly, disclosed herein is a method of evaluating a ssRNA preparation as a starting material to make a dsRNA composition described herein. The method includes providing an evaluation of the ssRNA preparation for a parameter related to suitability of the ssRNA sample for use in the making of a dsRNA described herein; and optionally, providing a determination of whether a value (e.g., a value correlated to presence, amount, distribution, or absence) determined for the parameter meets a preselected criterion, e.g., is present, or is present within a preselected range, thereby evaluating the ssRNA sample. In a preferred embodiment, the criterion is satisfied and the ssRNA sample is selected and processed into the dsRNA. In a preferred embodiment, a value for the parameter in a dsRNA intermediate used in making the final dsRNA (e.g. drug product) is also determined and optionally, that value must also meet a predetermined criterion to select the ssRNA for use in making the dsRNA. In one aspect, disclosed herein is a method of evaluating an ssRNA preparation, as a starting material to make a dsRNA composition described herein. The method includes optionally, performing an operation, e.g., analysis of molecular weight characteristics (e.g. Mn, Mw, distribution of molecular weight of fragments, polydispersity value, etc.) of the ssRNA composition, analysis of molecular weight characteristics or hybridization quality, or bioactivity of the intermediate dsRNA compositions; and optionally, providing a determination of whether a value determined for the parameter meets a preselected criterion, e.g., is present, or is present within a preselected range, thereby evaluating the ssRNA preparation. In a preferred embodiment, the criterion is satisfied and the ssRNA sample is selected and processed into the dsRNA.

Either of these methods, a decision or step is taken, e.g., the sample is classified, selected, accepted or discarded, released or withheld, processed into a drug product, shipped, moved to a different location, made human-suitable, formulated, labeled, packaged, released into commerce, or sold or offered for sale, or a record made or altered to reflect the determination, depending on whether the preselected criterion is met. E.g., based on the result of the determination or whether one or more subject entities is present, or upon comparison to a reference standard, the batch from which the sample is taken can be processed, e.g., as just described.

Either method can include providing a comparison of the value determined for a parameter with a reference value or values, to thereby evaluate the sample. In preferred embodiments, the comparison includes determining if the test value has a preselected relationship with the reference value, e.g., determining if it meets the reference value. The value need not be a numerical value but, e.g., can be merely an indication of whether the subject entity is present.

Either method can include determining if a test value is equal to or greater than a reference value, if it is less than or equal to a reference value, or if it falls within a range (either inclusive or exclusive of one or both endpoints). In preferred embodiments of either method, the test value, or an indication of whether the preselected criterion is met, can be memorialized, e.g., in a computer readable record.

The method can include determining if a parameter or criterion falls within a preselected range, e.g., a value which corresponds to a value from Tables 3, 5, 8, 13 or 14. In a preferred embodiment: the amount of each structure is within 100 kDa, 50 kDa or 20 kDa of a value that found in Tables 3, 5, 8, 13 or 14.

Some methods described herein include making a determination of whether a subject entity is present at a preselected level or within a preselected range and that level or range is expressed in specific units of measurement, e.g., mole %, e.g., present in a range of X-Y mole %. One can perform the method by determining the amount of subject entity in terms of mole % and then compare that with a reference expressed in mole %, in this example, X-Y mole %. One need not, however, make the measurement in terms of mole % and compare it with reference values expressed in mole %. The sample has an actual level of subject entity, which can be expressed as X-Y when described in units of mole %. That actual level can also be expressed in other units, e.g., weight %.

That actual level is the same regardless of the units in which it is expressed. The specification of mole % in the method is merely to indicate the actual prevalence of the subject entity. The level of subject entity can be measured in terms of other units and the reference value can be expressed in terms of other units, as long as the reference value as expressed in terms of alternative units corresponds to the same amount of subject entity as the reference value expressed in mole %, e.g., X-Ymole % in this example. Thus, a method which requires showing the subject entity is present at X-Y mole % can be performed by showing that the subject entity is present in a range expressed in an alternative unit of measure, e.g., weight %, chain number, or %AUC, wherein the range, as described in the alternative unit of measure, corresponds to the same amount of subject entity which would give the mole % referred to, in this example X-Y mole %. One can establish a functionally equivalent range for an alternative unit of measure by applying art known methods in conjunction with this specification. E.g., one can provide samples in the range of X-Y mole %, and then establish the corresponding range for those samples for in terms of an alternative unit of measure.

Unless specified otherwise, molecular weight figures expressed in kDa shall be as determined by SEC-MALLS. Unless specified otherwise, values for Tm, hyperchromicity and FWHM shall be as determined using methods as described in the Examples here.

In another embodiment, the invention provides a composition of the invention for use in treating or preventing a tumor or infection Optionally, the is a melanoma or a breast cancer. a composition of the invention for use in treating of preventing an ocular angiogenesis disorder.

In another embodiment, the invention provides a composition of the invention for use treating a tumor, the method comprising: a) selecting a patient having a tumor which expresses TLR3 and b) administering to the patient an effective amount of a polyAU composition of the invention.

In another embodiment the composition is for administration in combination with a second therapeutic agent. Optionally, the second therapeutic agent is an antigen, optionally wherein the antigen comprises one or a plurality of purified viral or tumor antigen(s). Optionally, the second therapeutic agent is a chemotherapeutic agent effective in the treatment of a tumor.

In any of the embodiments where a polyAU composition of the invention is administered to a human, the dose may optionally be between 1 mg/kg and 50 mg/kg. In another embodiment, wherein the polyAU composition is administered in a dose to humans that is calculated according to the formula (I):

$$(\text{single dose (mg/kg)}=(0.1 \text{ to } 50) * d) * w \qquad (I)$$

where d is the number of days of treatment per week, w is the number of weeks of treatment, wherein d is at least 2 and wherein the successive treatments are separated by no more than 72 hours. Optionally, w is at least 2, 3, 4, 6, 8 or 12.

In another embodiment the disclosure provides a method of assessing the biological activity of a candidate dsRNA composition, comprising: (a) providing a polyAU composition of the invention; and (b) assessing the ability of the composition to selectively bind and/or to selectively induce TLR3 activity. Optionally, assessing the ability of the composition to induce TLR3 activity comprises assessing pro-apoptotic activity, or assessing the ability of the composition to activate a cell, to induce cytokine production by a cell, or to induce a TLR3-mediated signal in a cell. Optionally, assessing the ability of the composition to selectively bind and/or induce TLR3 activity comprises determining whether the composition binds and/or induces TLR7, RIGI and/or MDA-5 activity. Optionally, a determination that said composition selectively binds and/or induces TLR3 activity, induces apoptosis, cytokine production and/or induce a TLR3-mediated signal indicates that said composition is a TLR3 agonist. Optionally the methods may further comprise making a composition identified as a selective TLR3 agonist suitable for human administration, optionally further comprising admixing the resulting composition with a pharmaceutically acceptable carrier or excipient.

## DESCRIPTION OF THE FIGURES

Figure 1 shows the results of binding of oligomers to immobilized human TLR3 polypeptides, as assessed by Biacore. The upper (dotted) line shows binding to TLR3 and the lower line shows binding to dextran (control). Time is indicated on the x-axis and binding (response, RU) on the y-axis. Figure 2 shows binding of high molecular weight dsRNA. Time is indicated on x-axis, binding response (RU) is indicated on the y-axis.

Figure 3 shows experiments for dose response of gene reporter assay on 293T-T3-ISRE luciferase (range: 0.001 to 10000 μg/ml, 1/10$^{th}$ dilution) for the dsRNA products Am1:Us-2 (black lozenges, full line), commercial polyAU (white lozenge, dotted line), polyIC (black squares, dashed bolded line) and polyAU "pre-run" (white squares, dashed line).

Figure 4A shows induction of apoptosis in vitro in 48h assay, in HCC38 tumor cells for dsRNA products Am1:Us-2 (black lozenges, full line), commercial polyAU (white lozenge, dotted line), polyIC (black squares, dashed bolded line) and polyAU "pre-run" (white squares, dashed line). Ligand concentration is indicated (in μg/ml) on the x-axis and fold increase in either luciferase activity (Fig 3) or % AnnexinV + apoptotic cells (minus % in control untreated cells) is indicated on the y-axis. Figure 4B shows IP-10 chemokine secretion (in 48h assyas) on HCC38 tumor (range: 0.001 to 10000 μg/ml, 1/10$^{th}$ dilution) and Figure 4C show and IL-6 cytokine secretion (in 48h assays) on HCC38 tumor (range: 0.001 to 10000 μg/ml, 1/10$^{th}$ dilution) upon treatment with the same dsRNA products Am1:Us-2 (black lozenges, full line), commercial polyAU (white lozenge, dotted line), polyIC (black squares, dashed bolded line) and polyAU "pre-run" (white squares, dashed line). DsRNA concentration (in μg/ml) is indicated on the x-axis and cytokine concentration in supernatant (in pg/ml) is indicated on the y-axis.

Figure 5 shows the results of hybridization of different ssRNA compositions, illustrating that molecular weight of the dsRNA composition (Mn are indicated as white columns, Mw are indicated as black columns) increases as a function of the longer chain length ssRNA whether the latter is a polyA or polyU.

Figure 6 shows a dose response curve for a gene reporter assay on 293T-T3-ISRE luciferase (range: 0.001 to 1000 μg/ml, 1/10$^{th}$ dilution) for the dsRNA products Axs:Us (white lozenges, dotted line), Am1:Us (black lozenges, full line) and Am2:Um (black squares, dashed line). Ligand concentration in μg/ml (Figure 6A) or in μM (Figure 6B) is indicated on the x-axis and fold increase in luciferase activity is indicated on the y-axis. Induction of TLR3 signaling activity was greatest for Am2:Um, followed by Am1:Us and then Axs:Us having lowest activity.

Figures 7A, 7B and 7C, respectively shows apoptosis induction (Caspase Glow assay in 4h30, figure 7A) and IP-10 (Figure 7B) and IL-6 (Figure 7C) cytokine secretion (in 48h assays) on HCC38 tumor (range: 0.001 to 1000 μg/ml, 1/10$^{th}$ dilution) upon treatment with Axs:Us (white lozenges, dotted line), Am1:Us (black lozenges, full line) and Am2:Um (black squares, dashed line). DsRNA concentration is indicated on the x-axis (in μg/ml) and either fold increase in caspase activity or cytokine concentration in supernatant (in pg/ml) is indicated on the y-axis.

Figures 8A and 8B, respectively shows in vitro cellular activation (CD69 upregulation on CD8+ T cells among total human PBMC) and IP-10 production by PBMC treated for 24h with Axs:Us (white lozenges, dotted line), Am1:Us (black lozenges, full line) and Am2:Um (black squares, dashed line). DsRNA concentration is indicated on the x-axis (in μM) and % of CD69+ cells (8A) or cytokine concentration in supernatant (pg/ml) (8B) is indicated on the y-axis.

Figures 9A and 9B, respectively shows in vivo cellular activation of spleen CD8+ T cells (at 24h post injection) and IP-10 production in sera (at 2h post injection) from mice intravenously treated with Axs:Us (white lozenges, dotted line), Am1:Us (black lozenges, full line) and Am2:Um (black squares, dashed line). DsRNA concentration is indicated on the x-axis (in μg injected per mice) and % of CD69+ cells (9A) or cytokine sera concentration (pg/ml) (9B) is indicated on the y-axis.

Figure 10 shows anti-tumor efficacy of Am2:Um (black squares), Am1:Us (black lozenges) and Axs:Us (white lozenges) compared to physiological serum (white dots) in an HCC1806 xenogenic tumor model, in SCID-NOD mice (5 daily intravenous injections/week of 500 μg/mice dose, starting at day 5 post tumor cells injection). Individual tumor volume in mice (in $mm^3$) on the y-axis is shown at day 33 post tumor cells injection.

Figure 11 shows molecular weight in Mn (g/mol) on the x-axis as a function of heating time and temperature on the y-axis for polyA ssRNA (grey triangles, full line) and polyAU dsRNA (black squares, full line), first point corresponds to reaction at room temperature for two hours, second point corresponds to a reaction time of 10 minutes, at 70°C, third point corresponds to a reaction time of 2 hours, at 70°C, fourth point point corresponds to a reaction time of 10 minutes, at 95°C, fifth point point corresponds to a reaction time of 2 hours, at 95°C. These data demonstrate that the molecular weight of ssRNA and dsRNA decrease as a function of heating (both temperature and time). PolyAU had an Mn of 3360 kDa at room temperature (not shown).

Figure 12 shows dose response of gene reporter assay on 293T-T3-ISRE luciferase (range: 0.001 to 1000 μg/ml, $1/10^{th}$ dilution) Am1:Us (black lozenges, full line), Am1:Us-2 (white lozenges, dotted line) and Am1:Us-3 (black squares, dashed line). Ligand concentration is indicated on the x-axis (in μg/ml) and fold increase in luciferase activity is indicated on the y-axis. There was a clear increase in TLR3 activity correlating to the Mn of the compositions, where Am1:Us-3 was more potent than Am1:Us, itself more potent than Am1:Us-2, and polyIC (white dots, dashed line) was no more potent than the best polyAU.

Figures 13A, 13B and 13C show respectively apoptosis induction (Caspase Glow assay in 4h30, figure 13A) and IP-10 (figure 13B) and IL-6 (figure 13C) cytokine secretion (in 24h) assays on HCC38 tumor cells (range: 0.001 to 1000 μg/ml, $1/10^{th}$ dilution) for compositions Am1:Us-2 (white lozenges, dashed line) and Am1:Us-3 (black squares, full line) in Figures 13B and 13C, and additionally Am1:Us (black lozenges, dashed line) and polyIC (white dots, dashed line) in Figure 13A. DsRNA concentration is indicated on the x-axis (in μg/ml) and either fold increase in caspase activity or cytokine concentration in supernatant (in pg/ml) is indicated on the y-axis. Results demonstrate that Am 1:Us-3 has higher potency than Am 1:Us in pro-apoptotic activity and IP-10 assays.

Figures 14A and 14B shows cellular activation (CD69 upregulation on CD8+ T cells, figure 14A) and IP-10 production (figure 14B), in PBMC treated with polyIC (white dots, dotted line), Am1:Us (black lozenges, full line), Am1:Us-2 (white lozenges, dotted line) and Am1:Us-3 (black squares, dashed line) and following 24h dose-response activation on human PBMC from a single donor (range: 0.001 to 1000 μg/ml, $1/10^{th}$ dilution). DsRNA concentration is indicated on the x-axis (in μg/ml) and percent of CD69-positive cells or cytokine concentration in supernatant (in pg/ml) is indicated on the y-axis. Results demonstrated that Am1:Us-3 and Am1:Us are more potent than Am1:Us-2 in activation of PBMC.

Figure 15A shows spleen cell activation (CD69 up-regulation on T CD8 spleen cells) following 24h dose-response intravenous injection of dsRNA in B6 mice (0, 20, 50 and 200 μg/mice dose) with polyIC (white dots, dotted line), Am1:Us (black lozenges, full line), Am1:Us-2 (white lozenges, dotted line) and Am1:Us-3 (black squares, dashed line). Results show a dose-dependant increase in cellular activation for Am1:Us-3 and Am1:Us. Figures 15B and 15C show cytokine secretion in sera (IP-10, IFN-alpha) following 2h dose-response intravenous injection of dsRNA in B6 mice (0, 20, 50 and 200 μg/mice dose) with polyIC, Am1:Us, Am1:Us-2 and Am1:Us-3. Results show a general dose-dependant increase in cytokine production for Am1:Us-3, Am1:Us-2 and Am1:Us, although significantly lower than that induced by polyIC. DsRNA concentration is indicated on the x-axis (in μg/ml) and percent of CD69-positive cells or cytokine concentration in supernatant (in pg/ml) is indicated on the y-axis

Figure 16 shows anti-tumor efficacy of Am1:Us (black lozenges), Am1:Us-2 (white lozenges) and Am1:Us-3 (black squares) in an HCC 1806 xenogenic tumor model, in SCID-NOD mice (5 daily injections/week of 500 μg/mice dose) compared to physiological serum (white dots). Individual tumor volume in mice on the y-axis is shown in $mm^3$ at day 43 post tumor cells injection.

Figure 17 shows EC50 from dose response assays of gene reporter assay on 293T-T3-ISRE luciferase (range: 0.001 to 1000 μg/ml, $1/10^{th}$ dilution) as a function of Mn for the dsRNA Axs:Us, Am1:Us and Am2:Um, AU690, Am1: Us-2 and Axs:Us. EC50 (μg/ml) is indicated on the x-axis and Mn (kDa) is indicated on the y-axis.

Figure 18 shows experiments for dose response of gene reporter assay on 293T-T3-ISRE luciferase (range: 0.001 to 10000 μg/ml, $1/10^{th}$ dilution) for the dsRNA products, polyIC (full lozenges, full line) and Am1:Us-2 (full dots, dashed line) and Am3:Um3 (open dots, dotted line). Am3:Um3 is 0.5 log times more efficient than Am1:Us-2 in triggering a TLR3 response. DsRNA concentration is indicated on the x-axis (in μg/ml), and fold increase on the y-axis.

Figure 19 shows dose response in vivo cellular activation of spleen NK cells (at 24h post injection) (Figure 19A), IP-10 (Figure 19B), IFNalpha (Figure 19C) production in sera (at 2h post injection) from mice intravenously treated with polyIC (full lozenges, full line) and Am1:Us-2 (full dots, dashed line) and Am3:Um3 (open dots, dotted line). DsRNA dose per injection per mouse (in μg) is indicated on the x-axis and percent of CD69-positive cells among NK cells or cytokine concentration in supernatant (in pg/ml) is indicated on the y-axis. Results demonstrated that Am3:Um3 is significantly more potent than Am1:Us-2 in activation of NK mice spleen cells, IP-10 and IFNα secretion. Moreover, Am3:Um3 is able to trigger IFNα secretion unlike Am1:Us-2 which cannot.

Figure 20 shows EC50 from dose response assays of TLR3 (Figure 20A), MDA-5 (Figure 20B) and RIGI (Figure 20C) gene reporter assay on 293T-T3-ISRE luciferase (range: 0.001 to 1000 μg/ml, 1/10$^{th}$ dilution), in comparison with control (Figure 20D) for the dsRNA: polyIC (full lozenges) and Am1:Us-2 (full dots, dashed line) and Am3:Um3 (open dots, dotted line) delivered with lipofectamine, 3pRNA is evaluated as a control for RIG-like receptor signaling. DsRNA concentration is indicated on the x-axis (in μg/ml) and fold increase in luciferase activity is indicated on the y-axis. This data supports the selectivity of polyAU over polyIC in inducing a selective TLR3 signalling.

Figure 21 shows EC50 dose response assays of huTLR7 (Figure 21B) and huTLR8 (Figure 21C) gene reporter assay of 293T-T3-ISRE luciferase (range: 0.001 to 1000 μg/ml, 1/10$^{th}$ dilution) for the dsRNA polyIC (full lozenges) and Am1:Us-2 (full dots, dashed line) and Am3:Um3 (open dots, dotted line), R848 (resiquimod™) is evaluated as a control for TLR7/8 signaling (control is Figure 21A). DsRNA concentration is indicated on the x-axis (in μg/ml) and fold increase in luciferase activity is indicated on the y-axis. This data supports the selectivity of polyAU over polyIC in inducing a selective TLR3 signalling.

Figures 22A, B and C show anti-tumor efficacy of Am1:Us and Am3:Um3 in an HCC 1806 xenogenic tumor model, in SCID-NOD mice (5 daily intravenous injections/week of 500 μg/mice dose, starting at day 5 post tumor cells injection). Figure 22A reports the comparison between the mean of tumor size in the mice in $mm^3$ of the control group (NaCl, 0.15M, full squares, full line), the Am1:Us group (full dots, full line) and the Am3:Um3 group (open dots, dashed line). Figure 22B reports on the x-axis the individual tumor volume in mice in $mm^3$ in the Am1:Us-3 group, according to time in days on the y-axis. Figure 22C reports on the x-axis the individual tumor volume in mice in $mm^3$ in the Am3:Um3 group, according to time in days on the y-axis. These results supports
the improved antitumoral effect of Am3:Um3 compared to Am1:Us-3 composition in a xenogenic mice model.

Figure 23 shows EC50 from dose response assays of TLR3 (figure 23B), RIGI (Figure 23C) and MDA-5 (Figure 23D) gene reporter assay on 293T-ISRE luciferase (range: 0.001 to 1000 μg/ml, 1/10$^{th}$ dilution) for the dsRNA Am1: Us-2 (white lozenges, dotted line), Am1:Us-3 (black lozenges full line) and polyIC (black squares, dashed line) compared to control (Figure 23A). DsRNA concentration is indicated on the x-axis in μg/ml and fold increase in luciferase activity is indicated on the y-axis.

Figure 24 shows EC50 from dose response assays of TLR3 (Figure 24B) , RIGI (Figure 24C) and MDA-5 (Figure 24D) gene reporter assay on 293T-T3-ISRE luciferase (range: 0.001 to 1000 μg/ml, 1/10$^{th}$ dilution) for the dsRNA Am1:Us-2 (white lozenges, dotted line), Am1:Us-3 (black lozenges full line) and polyIC (black squares, dashed line) delivered with lipofectamine compared to control (Figure 24A). DsRNA concentration is indicated on the x-axis in μg/ml and fold increase in luciferase activity is indicated on the y-axis.

Figures 25A and 25B show apoptosis induction by Am1:Us-3 in HCC38 (figure 25A) and HCC 1806 (Figure 25B) tumor cells in the presence (white lozenges, dotted line) and absence (control, shLamin, black lozenges, full line) of pre-treatment of the cells with lentivirus shRNA targeting TRIF. DsRNA concentration is indicated on the x-axis in μg/ml and % apoptotic cells (minus % apoptotic cells in medium) is indicated on the y-axis.

Figure 26 shows apoptosis induction by Am1:Us-3 in A375 melanoma cells in the presence (black dots, full line) and absence (white dots, dashed line) of pre-treatment of the cells with IFN-alpha. DsRNA concentration is indicated on the x-axis in μg/ml and apoptotic cells (minus % apoptotic cells in medium) is indicated on the y-axis.

Figure 27 shows the schedules of administration of used in a comparison of in vivo anti-tumor efficacy of Am1:Us-3 in different administration schemes in a Nod-SCID mouse model, with or without IFN-alpha. X groups have been set up, each group containing 6 mice. The experiment has been conducted for 6 consecutive weeks. On day 1, mice were injected $5.10^6$ cells of A375 tumor cell line.

Figure 28 shows the result of different schedules of administration of used in a comparison of in vivo anti-tumor efficacy of Am1:Us-3 in different administration schemes in a Nod-SCID mouse A375 melanoma tumor model (schedule of administration of Figure 27), with or without IFN-alpha pre-treatment. The x-axis shows the treatment schedule (in grey dots: physiological serum, in grey squares: IFNapha and Am1:Us-3, two times a week, in light grey triangles, IFNalpha alone, in dark grey triangles, Am1:Us-3 alone, two times a week, in grey lozenges: IFNapha and Am1:Us-3, once a week, in white dots: IFNapha and Am1:Us-3, five times a week) and the y-axis shows the tumor volume in $mm^3$, the results have been assessed on day 35. The schedule involving 5 consecutive days of administration of Am1:Us-3 achieved the best results, and 2 days of administration per week separated by two days was better than once-weekly.

Figures 29A and 29B show the result of treatment in Nod-SCID mice bearing melanoma tumors, where the twice a week Am1:Us-3 treatment regimens were repeated in mice who received A375 cells stably infected in vitro with lentivirus construction for either control LaminA/C shRNA (Figure 29A, treatment with physiological serum (control) in black, treatment with Am1:Us-3 in grey) or TRIF shRNA in order to inhibit TLR3 signaling (Figure 29B, treatment with physiological serum (control) in black, treatment with Am1:Us-3 in grey). The x-axis shows the time in days and the y-axis shows the tumor volume in mm3. Only TRIF shRNA inhibited Am1:Us-3's antitumor effect.

## DETAILED DESCRIPTION

### Definitions

As used in the specification, "a" or "an" may mean one or more. As used in the claim(s), when used in conjunction with the word "comprising", the words "a" or "an" may mean one or more than one. As used herein "another" may mean at least a second or more.

Where "comprising" is used, this can preferably be replaced by "consisting essentially of', more preferably by "consisting of'.

Where "about" is used in connection with a number, this preferably means the number +/-15%, more preferably the number plus 5%, most preferably the number itself without "about". For example, "about 100" would stand for "from and including 85 to and including 115". Where "about" is used in connection with numeric ranges, for example "about 1 to about 3", or "between about one and about three", preferably the definition of "about" given for a number in the last sentence is applied to each number defining the start and the end of a range separately. Preferably, where "about" is used in connection with any numerical values, the "about" can be deleted.

As used herein, the term "in combination", or "in conjunction with" refers to the use of more than one therapies (e. g., more than one prophylactic agent and/or therapeutic agent). The use of the term "in combination" does not restrict the order in which therapies (e. g. prophylactic or therapeutic agents) are administered to a subject.

As used herein, the terms "patient", "subject" and "subjects" refer to an animal, preferably a mammal including, but not limited to, a non-primate (e.g., a cow, pig, horse, cat, dog, rat, and mouse) and a non-human primates (e.g., a monkey such as a cynomolgous monkey), and more preferably a human. In a specific embodiment, the subject is a human with cancer or susceptible to developing cancer.

As used herein, the term "effective amount" refers to the amount of an agent (e. g. a prophylactic or therapeutic agent) which is sufficient to cause the desired effect in the particular context, such as induce apoptosis of a cell, induce cytokine production in a cell, induce cellular activation, induce TLR3 expression in a cell, induce TLR3-mediated signaling in a cell, prevent, reduce or ameliorate the severity, duration and/or progression of a disease (e.g. cancer, infection) or one or more symptoms thereof, ameliorate one or more symptoms of a disease, prevent the advancement of a disease, cause regression of a disease, prevent the recurrence, development, or onset of a disease or one or more symptoms thereof, or enhance or improve the prophylactic or therapeutic effect (s) of another therapy (e. g., prophylactic or therapeutic agent).

As used herein, the term "host cell" includes a particular subject cell transfected with a nucleic acid molecule and the progeny or potential progeny of such a cell.

The term "specifically binds to" or "selectively binds to" means that a compound (e.g. dsRNA) can bind preferably in a competitive binding assay to the binding partner, e.g. TLR3, TLR7, MDA5, RIGI. Competitive binding assays and other methods for determining specific or selective binding are further described below and are well known in the art.

As used interchangeably herein, a "TLR3 activity", "biological activity of TLR3" or "TLR3 bioactivity", refers to an activity exerted by a TLR3 polypeptide or nucleic acid molecule, or a biologically active fragment or homologue thereof comprising a TLR3 as determined in vivo, or in vitro, according to standard techniques. In one embodiment, a TLR3 activity is a direct activity, such as an association with a TLR3-target molecule or most preferably apoptosis induction activity, or induction of cytokine production (e.g. IFN-gamma, IFN-alpha, IL-6, IP10, MCP-1), induction of cellular activation, or induction of TLR3 expression. The activity can be, for example, in an epithelial cell, an endothelial cell, a tumor cell, a T cell, a B cell, an NK cell or dendritic cell. As used herein, a "TLR3 target molecule" is a molecule with which a TLR3 protein binds or interacts in nature, such that a TLR3- mediated function is achieved. For example, a TLR3 target molecule can be a molecule involved in a signalling pathway, e.g. TRIF. Binding or interaction with a TLR3 target molecule can be detected for example using a two hybrid-based assay in yeast to find drugs that disrupt interaction of the TLR3 family bait with the target prey, or an in vitro interaction assay with recombinant TLR3 and target proteins. Alternatively, a TLR3 activity may be an indirect activity, such as an activity mediated by interaction of the TLR3 protein with a TLR3 target molecule such that the target molecule modulates a downstream cellular activity (e.g., interaction of a TLR3 molecule with a TLR3 target molecule can modulate the activity of that target molecule on an intracellular signaling pathway, e.g. involving TRIF, IRF3, IRF7, IKKε, TBK1). TLR3 activity is not limited to the induction of a specified activity, but may also involve enhancing the activity.

The term "TLR3 agonist" refers to an affinity agent (i.e., a molecule that binds a target molecule) capable of activating a TLR3 polypeptide to induce a full or partial receptor-mediated response. An agonist of TLR3 may induce any TLR3 activity, for example TLR3-mediated signalling, either directly or indirectly. A TLR3 agonist, as used herein, may but is not required to bind a TLR3 polypeptide, and may or may not interact directly with the TLR3 polypeptide. As employed herein, the phrases "selective TLR3 agonist" and "TLR3 agonist which selectively induces TLR3 activity" refer to compositions which induce TLR3-mediated signalling to a significantly greater extent than signalling by one or more other dsRNA receptors. When the TLR3 agonist is a dsRNA composition, a "TLR3 agonist which selectively induces TLR3 activity" refers to compositions which induce TLR3-mediated signalling to a significantly greater extent than signalling by one or more other dsRNA receptors (e.g. TLR7, RIGI, MDA-5, PKR and/or other dsRNA or ssRNA receptors). In one embodiment, "significantly greater extent", as applied to interaction between TLR3 agonist and a receptor, refers to agonists which have a significantly higher therapeutic index (i.e., the ratio of efficacy to toxicity) for treatment of the target disease state than for activation of pathways mediated by other receptors. The toxicity of therapeutic compounds frequently arises from the non-selective interaction of the therapeutic compound with other receptors. Thus, the present invention provides a means to reduce the incidence of side-reactions commonly associated dsRNA therapy. Preferably, a composition which induces TLR3-mediated signalling to a significantly greater extent than signalling by other another receptor(s) will have an EC50 for induction of TLR3 signalling that is less than the EC50 for signalling by the other receptor(s).

"EC50" refers to the molar concentration of an agonist which produces 50% of the maximum response for that agonist.

As employed herein, the phrase "TLR3-responsive disease state" refers to any disease state where induction of TLR3 signalling by a TLR3 agonist which selectively induces TLR3 activity can give rise to prevention or amelioration of disease, including but not limited to:

a) diseases where induction of TLR3 signalling by a TLR3 agonist which selectively induces TLR3 activity induces TLR3 polypeptide expression in a cell (e.g. a tumor cell);
b) diseases where induction of TLR3 signalling by a TLR3 agonist which selectively induces TLR3 activity induces apoptosis of a cell involved in a disease (e.g. tumor cell);
c) diseases where induction of TLR3 signalling by a TLR3 agonist which selectively induces TLR3 activity induces cytokine production by a cell involved in disease (e.g. tumor cell, epithelial cell, endothelial or epithelial cells) or immune cell (e.g. PBMC, NK, CD4+ T, CD8+ T, DC);
d) diseases where induction of TLR3 signalling by a TLR3 agonist which selectively induces TLR3 activity induces activation of immune cells and/or induction of immunity toward a diseased cell (e.g. tumor cell, infected cell, vaccination or treatment of a tumor or infections); and/or
e) diseases involving ocular angiogenesis (e.g. macular degeneration), optionally where induction of TLR3 signalling by a TLR agonist which selectively induces TLR3 activity inhibits angiogenesis.

"PolyI", "polyC", "polyA", "polyU", mean polyinosinic acid, polycytidylic acid, polyadenylic acid, and polyuridylic acid, respectively, each optionally substituted with other monomers.

"PolyAU", used interchangeably with "pApU", "polyA:U", poly(A):poly(U), means an at least partially double stranded molecule made of polyadenylic acid(s) and polyuridylic acid(s), each optionally substituted with other monomers so long as the biological function (e.g. immunomodulatory activity, TLR3 agonism or binding) is preserved.

A "homopolymer" is a polymer made of substantially only a single monomer; for example a polyA homopolymer is substantially all A (adenosine) monomers. A homopolymer can be a single longer polymer or can consist of a plurality of shorter polymers concatenated (e.g. using a linker) to form a longer polymer, etc.

"copolymer" is a polymer made of two or more monomers; for example a poly A copolymer comprises A (adenosine) monomers and one or more monomers other than adenosine.

"poly AxU" mean copolymer of adenylic acid and uridylic acid where one uridylic acid is substituted for about every x adenylic acids, respectively. For example "poly C12U" is a copolymer of cytidylic acid and uridylic acid where one uridylic acid is substituted for about every 12 cytidylic acids, respectively.

"dsRNA" and "double-stranded RNA" refer to complexes of polyribonucleotides which are at least partly double stranded. DsRNA need not be double stranded over the length of the molecule, nor over the length of one or more of the single-strand nucleic acid polymers that form the dsRNA.

The term "base pair" (abbreviated as "bp") frequently used to indicate the molecular size of nucleic acid is used to indicate the molecular size by the numbers of bases in the nucleic acid (i.e. 10 bp means the double strand polymer having ten bases) in each complementary strand. The term "biological sample" as used herein includes but is not limited to a biological fluid (for example scrum, lymph, blood), cell sample or tissue sample (for example bone marrow).

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (nonrecombinant) form of the cell or express native genes that are otherwise abnormally expressed, under expressed or not expressed at all.

The term "human-suitable" when referring to an agent or composition refers to any agent or composition that can be safely used in humans for, e.g. the therapeutic methods described herein. For example human suitable agents do not cause effects such as severe cytokine induction at a level that would preclude their use in humans, or contain levels of substances (e.g. endotoxins) that are incompatible with use in humans, in the particular context (e.g. mode of administration) in which the agent is used.

"Average chain length" as used herein refers to the average chain length of ribonucleotide monomers that occur within a chain. The presence of non-nucleotide building blocks (e.g., attached stabilizing moieties) are not included in determining the average chain length. Average chain length is determined by dividing the number average molecular weight (Mn) by the average molecular weight for the nucleotide monomer pairs (e.g. 660 Da is used herein for a pair of unmodified ribonucleotide monomers which assumes the chain is entirely double stranded). Methods of determining number average molecular weight are described below using SEC MALLS.

"Weight average molecular weight", "mass average" or "$M_w$" (herein expressed in g/mol), as used herein refers to the weight average in daltons of chains (or complexed chains) of nucleotide monomers (e.g. ssRNA or dsRNA polymers). The presence of non-nucleotide building blocks are not included in determining the weight average molecular weight. Thus, the molecular weight of non-nucleotide building blocks within a chain or chains in the preparation should not be included in determining the weight average molecular weight. The weight average molecular weight ($M_w$) is calculated from the following equation: $M_w = \sum(C_iM_i)/ EC_i$. The variable Ci is the mass concentration of the polymer in slice i and Mi is the average molecular weight of the polymer in slice i. The summations are taken over a chromatographic peak, which contains many slices of data. A slice of data can be pictured as a vertical line on a plot of chromatographic peak versus time. The elution peak can therefore be divided into many slices. The weight average molecular weight calculation is average dependent on the summation of all slices of the concentration and molecular weight. The weight average molar weight can be measured, e.g., using the Wyatt Astra software or any appropriate software.

"Number average molecular weight", "mean molecular weight" or "$M_n$", expressed in g/mol, as used herein refers to the number average in daltons of chains of nucleotide monomers. The number average molecular weight (Mn) is calculated from the following equation: $Mn = \sum ci/(\sum ci/mi)$. The variable ci is the concentration of the polysaccharide in slice i and Mi is the molecular weight of the polysaccharide in slice i. The summations are taken over a chromatographic peak, which contains many slices of data. A slice of data can be pictured as a vertical line on a plot of chromatographic peak versus time. The elution peak can therefore be divided into many slices. The number average molecular weight is a calculation dependent on the molecular weight and concentration at each slice of data.

The term "polydisperse" or "polydispersity" refers to the weight average molecular weight of a composition (Mw) divided by the number average molecular weight (Mn). The polydispersity of ssRNA and dsRNA preparations provided herein is about 3.0 or less, preferably 2.0 or less, e.g., about 2.0 to 1.1, and numbers in between.

The term "narrow size distribution" refers to a dsRNA composition having a homogeneous size distribution, e.g. a bell shape distribution pattern, a low Ip (e.g. less than 2.0), and where most of the dsRNA strands are of a similar weight. In particular, a dsRNA composition will have a narrow size distribution when more than 60, 70, 80, 90% of the dsRNA strands have a Mn being within 100, 50 kDa of the mean Mn of the whole composition.

For any of the ranges described herein, e.g., for a given structure or activity, the ranges can be those ranges disclosed as well as other ranges. For example, a range constructed from a lower endpoint of one range, e.g., for a given building block or activity, can be combined with the upper endpoint of another range, e.g., for the given building block or activity, to give a range.

An "isolated" or "purified" preparation (e.g. dsRNA or ssRNA preparation) is substantially free of material or other contaminating compounds from the source from which the preparation (e.g. dsRNA or ssRNA) is derived, or substantially free from chemical precursors or other chemicals when chemically synthesized. "Substantially free" means that a preparation of dsRNA or ssRNA is at least 50% pure (wt/wt). In a preferred embodiment, the preparation of dsRNA has less than about 20%, 10%, 5% and more preferably 2% (by dry weight), of free ribonucleotide monomers, proteins or chemical precursors and/or other chemicals, endotoxins, and/or free ssRNA (in the case of a dsRNA preparation), e.g., from manufacture. These also referred to herein as "contaminants". Examples of contaminants that can be present in a dsRNA or ssRNA preparation provided herein include, but are not limited to, calcium, sodium, ribonucleotide monomers, free ssRNA (in the case of a dsRNA preparation), endotoxin, polynucleotide phosphoylase enzyme (or other enzyme having similar substrate specificity), methanol, ethanol, chloride, sulfate, dermatan sulfate, and chondrotin sulfate. Purity and homogeneity are typically determined using analytical chemistry techniques such as polyacrylamide gel electrophoresis or high performance liquid chromatography.

*Preparation of dsRNA*

PolyAU can be natural or synthetic, and may be chemically modified derivatives or analogs of natural nucleotides. Various examples of modifications and derivatives are provided herein. It will be appreciated that a variety of methods are available that can be used to generate a dsRNA. The methods used may depend on the nature of the dsRNA to be prepared. One well known method, particularly for use in generating polymers not having a specific sequence (e.g. homopolymers, or substituted homopolymers), is enzymatic synthesis (e.g. using a polynucleotide phosphorylase) in solution to generate a composition of first single-stranded RNA polymers and a composition of second single-stranded RNA polymers which can at least partly form double strands, and then mixing the compositions such that the first single-stranded RNA polymers and second single-stranded RNA polymers form at least partly double-stranded RNA polymers. However, several other means for preparing double-stranded RNA polymers are known, and furthermore several means for preparing single-stranded RNA (ssRNA) polymers are known. Examples include methods starting from template dsRNA, such as PCR based methods, methods using an expression vector engineered to produce double-stranded RNA (dsRNA). Other examples include automated synthesis of shorter (e.g. 10-50 mer) oligonucleotides, either joined together by linkers (e.g. a non-nucleic acid spacer moiety) or hybridized to a longer complementary single-stranded RNA, which longer ssRNA may optionally itself be comprised of a plurality of oligonucleotides joined by linkers. As shown in the Examples herein, dsRNA made of a plurality of polyA oligonucleotides joined by linkers complexed with a plurality of polyU oligonucleotides joined by linkers demonstrated TLR3 binding. Thus, the invention encompasses an isolated dsRNA polymer composition capable of binding to TLR3, wherein the polymer comprises a complex of: (a) a first single-stranded RNA polymer comprising a plurality of single-stranded RNA polymer joined by a linker; and (b) a second single-stranded RNA polymer capable of complexing with the first single-stranded RNA polymer to form a double stranded; optionally wherein the second single-stranded RNA polymer comprising a plurality of single-stranded RNA polymer joined by a linker.

Other examples include purifying a dsRNA or ssRNA polymer from an RNA virus. Generally, depending on the nature of the dsRNA to be prepared, including for example the introduction of modifications, specific sequences or sequence motifs to be included, or length specifications, different methods adapted to the particular situation can be used. Generally, the dsRNA will arise by forming complexes (e.g. by mixing) two single-strand nucleic acid polymers which can at least partly form double strands. In order to be able to at least partly form double strands, polynucleotides will typically exist as two single stranded complementary nucleic acid polymers that can be so aligned so that they can form double strands over at least a portion of their length at physiological conditions. The extent to which the polymers at least partly form double strands varies depending on base sequences of the two single strand nucleic acid polymers and the length of each polymer. In general, the number of complementary bases is 20 or more.

Preferred modifications are stabilizing modifications, and thus can include at least one modification in the phosphodiester linkage and/or on the sugar, and/or on the base. For example, one or both strands of the dsRNA can independently include one or more phosphorothioate linkages, phosphorodithioate linkages, and/or methylphosphonate linkages; modifications at the 2'-position of the sugar, such as 2'-O-methyl modifications, 2'-O-methoxyethyl modifications, 2'-amino modifications, 2'-deoxy modifications, 2'-halo modifications such as 2'-fluoro; combinations of the above, such as 2'-deoxy-2'-fluoro modifications; acyclic nucleotide analogs, and can also include at least one phosphodiester linkage.

Nucleotides used in the dsRNA TLR3 agonist of this invention may also include base modifications or substitutions. Modified bases include other synthetic and naturally-occurring bases such as 5-methylcytosine (5-Me-C), 5-hydroxymethyl cytosine, xanthine, hypoxanthine, 2-aminoadenine, 6-methyl and other alkyl derivatives of adenine and inosine, 2-propyl and other alkyl derivatives of adenine and inosine, 2-thiouracil and 2-thiocytosine, 5-halouracil and cytosine, 5-propynl(-C=C-CH3) uracil and cytosine and other alkynyl derivatives of pyrimidine bases, 6-azo uracil and cytosine, 5-uracil (pseudouracil), 4-thiouracil, 8-halo, 8-amino, 8-thiol, S-thioalkyl, 8-hydroxyl and other 8-substituted adenines and inosines, 5-halo particularly 5-bromo, 5-trifluoromethyl and other 5-substituted uracils and cytosines, 7-methylinosine and 7-ethyladenine, 2-F-adenine, 2-amino-adenine, 8-azainosine and 8-azaadenine, 7-deazainosine and 7-deazaadenine and 3-deazainosine and 3-deazaadenine.

Other modifications include a 3'- and/or 5'-terminal cap, a terminal 3'-5' linkage, and a 5'-terminal phosphate group or modified phosphate group. Examples of terminal cap moieties include, but are not limited to, an inverted deoxy abasic moiety, an inverted deoxynucleotides, or a glyceryl moiety. All of these modifications are well known in the art (see, for example, Kandimalla et al. ((2003) Nucl. Acid. Res. 31(9): 2393-2400).

The single-stranded nucleic acid polymer may be a homopolymer or a copolymer. A copolymer can comprise two, three, four or more different bases, in any ratio, and the sequence of bases making up the copolymer may be regular or irregular. The term "regular" means that the constituent bases are arranged alternately or a block formed by a given number of units of one base and that of the other base are alternate. In one embodiment, the regular copolymer comprises two bases in each strand. In other embodiments, the copolymer comprises a block of units having a specific functional sequence, such as a siRNA unit or a sequence designed to bind or sequester a protein. In other embodiments, the copolymer comprises or consists of a sequence derived from the genome of an RNA virus. In preferred embodiments, the dsRNA will be a polyAU made complexes of polyA and polyU copolymers (e.g. a poly AxU where one uridylic acid is substituted for every x adenylic acids) or preferably polyA and polyU homopolymers. As discussed herein, polyAU compositions have TLR3 selective binding and/or agonist activity. In one embodiment, the dsRNA TLR3 agonist will consist mostly of a single type of nucleotide base on each of the two strands. Preferably at least 90%, 95% or 99% of the bases on each strand are of the same type (e.g. U and A for the first and second strand, respectively). Thus, such dsRNA TLR3 agonists will be made of single stranded polymers that are essentially homopolymers. Optionally, single stranded polymers include substitutions to include a different base. The base in such homopolymer strands may be any naturally occurring base (e.g., polyA, polyU) or non-naturally occurring (e.g., chemically synthesized or modified) base. Polynucleotides typified by polyadenylic-polyuridylic acid, i.e., poly(A):poly(U) or poly AU, are well-known compounds in the art and have been known to act as TLR3 agonists.

It will be thus appreciated that dsRNA TLR3 agonists can comprise any combination of bases and be designed using any suitable method. Preferably, the basic requirement of a region of double-strandedness and the preferences for chain length are taken into account, as well as optionally stability and resistance to nuclease attack. These properties, as well as relative TLR3 agonistic activity of any dsRNA TLR3 agonist can be tested and assessed with reference to the dsRNA complex for example. Measures can be taken to increase stability and resistance to nucleases, or to increase or optionally modify the biological activity.

Typically, a method for preparing double-stranded RNA will comprise providing a first single-stranded RNA polymer, and then providing a second single-stranded RNA polymer, at least partially complementary to the first single-stranded RNA polymer. In addition one or both strands (in a two-stranded dsRNA TLR3 agonist) may be or include a concatemer consisting of two or more oligonucleotide sequences joined by a linker(s). Generally, any method to provide nucleotide bases that will lead to the formation, together with the first single-stranded RNA polymer, of a double-stranded RNA polymer can be used. The nucleotide bases which form the complementary strand can be provided any suitable way; for example this may be by bases that bind the bases of the first strand, or more commonly by single-stranded RNA polymers capable of hybridizing with said first single-stranded RNA polymer. The single-stranded RNA polymers capable of hybridizing with said first single-stranded RNA polymer can be shorter polymers (e.g. oligoribonucleotides), or they may be long polymers (e.g. having a length similar or greater than the first single-stranded RNA polymer). Any suitable method or source can be used to provide single stranded RNA polymers that can be complexed to form dsRNA polymers. For example it is possible to provide two single strand nucleic acid polymers obtained by separating a double strand nucleic acid polymer by conventional manipulations may be used. Specifically, the manipulations can include non-enzymatic treatments such as heating at 60°C or more, or enzymatic treatments.

Typically, dsRNA which are made of homopolymers or irregular copolymers (e.g. having a certain ratio of two bases in a single strand, but no specific predetermined sequence) will be prepared by complexing single stranded RNA polymers prepared by enzymatic synthesis. Generally, polynucleotides are obtained by the action of a polynucleotide phosphorylase (PnPase) on the appropriate nucleotide monomer. The various possible monomers, such as ADP, CDP, GDP, IDP and UDP, are obtained by well-known techniques. If a homopolymer is desired, the PnPase obtained is used for polymerisation of the selected monomer in the presence of usual agents. If a copolymer is desired, the selected monomer is replaced by a mixture, in appropriate proportions, of the selected monomers. Said copolymer will be designated as Poly X/Poly Y, wherein X and Y are each a monomer, preferably selected from A, C, G, I and U.

The homopolymers can be easily synthesized by means of enzymatic catalysis using bacterial PNPase. The nucleotide diphosphates (ADP or UDP) are dissolved in buffered water in the presence of an enzyme (e.g. 1 to 30 Units of enzyme per gram of nucleotide) and enzyme cofactors, such as magnesium chloride. The reaction medium is heated at 37 °C and kept while stirring at least for several hours or even days. The temperature and pH are controlled over the entire reaction. The reaction advancement is monitored by RP-HPLC (C 18). The homopolymers can be recovered by precipitation using ethanol for instance or by removal of the enzyme with any suitable reagent. PnPase is available from commercial sources, for example Polynucleotide Phosphorylase from *Bacillus stearothermophilus* from Sigma-Aldrich

(St. Louis, MO), product number P2869, E.C. enzyme reference 2.7.7.8. The homopolymers can be further purified, by liquid extraction or by filtration.

PnPase can also be obtained by well-known techniques, starting from a culture of bacteria, then proceeding with a lysis of the bacteria obtained in said culture, followed by extraction of PnPase obtained in the lysis from the medium. Following lysis of bacteria, PnPase must be isolated from a complex medium comprising various enzymes, such as kinases, phosphatases, nucleases, diesterases, etc, all products which are competing agents in the further step of polymerisation of the selected nucleotide monomer by the PnPase. The presence of the various non-desired enzymes leads to parallel undesired reactions and partial degradation of the monomer to be polymerized as well as the polymer produced. One method provided by U.S. Patent no 4,927,755, the disclosure of which is incorporated herein by reference, comprises, after the culture of a bacteria strain and the lysis of the culture thus obtained, the resulting medium should be passed successively on three columns, the first containing an ion exchange resin such as DEAE Sephacel or an equivalent, the second column containing a hydrophobic resin such as phenyl Sepharose or an equivalent and the third column containing a molecular sieve such as SephacrylS300 or Sephadex 200 or any equivalent. Other standard methods for enzyme purification will be known in the art. It has been observed herein that technical limitations may prevent PnPase from yielding compositions of high molecular weight single-stranded RNA polymers which have a relatively low polydispersity (e.g. an Ip value of 2.0 or lower). In a preferred embodiment, a highly purified PnPase (in the present case a recombinant PnPase) is used. Upon reaching a desired expected value of molecular weight of the single-stranded RNA polymers, the polymerization reaction is terminated. By using a highly pure and highly active PnPase, long polymers can be generated without substantial depolymerization of the single-stranded RNA polymers. The resulting single-stranded RNA polymers have a relatively low Ip (e.g. below 3.0, 2.5, 2.0, 1.5, 1.4, 1.3, or 1.2), even for compositions of single-stranded RNA polymers having high Mn values, and at reasonable yields. It will therefore be advantageous to use an essentially pure PnPase composition, preferably an isolated recombinant PnPase polypeptide.

preparation of recombinant PnPase polypeptide can be carried out according to known techniques. A preferred example of a PnPase is obtained from E. Coli, for example. Host cells are made to express a nucleic acid comprising a PnPase operably linked to an expression control sequences that lead to high levels of PnPase production. Use of recombinant PnPase is also believed to resolve technical difficulties relating to pyrogen contaminants. Particularly where a TLR3-directed agonism such as induction of apoptosis is sought rather, it will generally be preferable to avoid unwanted immune reactions. In preferred embodiments, dsRNA compositions are essentially free of pyrogens, preferably wherein endotoxins are below the detection limit.

In preferred embodiments, the method for preparing dsRNA includes the control, determination, or selection of molecular weight or chain length of the single stranded polymers prior to annealing to form the dsRNA. In one embodiment, the disclosure provides that highly bioactive dsRNA compositions can be obtained when a first ssRNA composition used in preparing the dsRNA has an average or mean chain length which is substantially lower than that of a second complementary ssRNA composition, while maintaining an average or mean chain length of the resulting dsRNA not substantially lower than that of the second ssRNA composition. It is believed that multiple shorter ssRNA chains from the lower chain length ssRNA composition will hybridize to the longer ssRNA chain from the higher chain length ssRNA composition, such that the higher chain length ssRNA composition will affect the average chain length of the resulting dsRNA. The resulting dsRNA will have an average or mean chain length which is not substantially less than, or at least 50% of, the ssRNA of higher average or mean chain length, and/or the dsRNA will have an Mn or Mw which is not substantially less than about twice the Mn or Mw of the ssRNA of higher Mn or Mw, or within 50% of about twice the Mn or Mw of the ssRNA of higher Mn or Mw. In another embodiment, highly bioactive dsRNA compositions can be obtained when both ssRNA compositions used in preparing the dsRNA have an average or mean chain length that does not differ by more than 5%, 10% or 20%.

The disclosure also provides dsRNA compositions having particular stoichiometries of first and second complementary ssRNA polymers, wherein the molecular ratio of a first ssRNA polymer to a second complementary ssRNA polymer is greater than 1:1. As further described herein, it has been observed that certain relationships of molecular weight or chain length between a first and second ssRNA composition provide advantageous dsRNA compositions. By way of example, in a given polyAU composition, the stoichiometry of at least one of the complementary ssRNA polymers to the other ssRNA polymers, as present in hybridized form in a dsRNA, was estimated to range from 1:1 to about 1: 10. Thus, a polyAU composition can comprise a ratio of polyA to polyU polymers of 1 to at least 1, 2, 5 or 10, or a ratio of polyU to polyA polymers of 1 to at least 1, 2, 5, or 10. In one embodiment, ratio of polyA to polyU polymers is from about 1:5 to about 1:1, or preferably from about 1:5 to about 3:4.

The present disclosure further provides that in some cases, ssRNA strands can give rise, when annealed, to dsRNA having an average or mean molecular weight which is greater than the sum of the complementary strands used, or that the average or mean chain length of the dsRNA is greater than that of the ssRNA composition having the greater average or mean chain length, and that such dsRNA retain high bioactivity. Without wishing to be bound by theory, it is possible that the combination of a shorter (lower molecular weight) first single-stranded RNA polymer and a longer (higher molecular weight) second single-stranded RNA polymer may favor, upon mixing, the formation of overlapping single-stranded RNA polymers, such that, statistically, the ultimate dsRNA composition comprises a significant number of dsRNA polymers having two or more of at least one of the single-stranded RNA polymer species.

The also describes dsRNA compositions having particular stoichiometries, wherein the molecular ratio of one or both of two complementary ssRNA polymers (as present in hybridized form as dsRNA polymers) to the dsRNA polymers in a composition is greater than 1:1. By way of example, in a given polyAU composition, the stoichiometry of at least one of the complementary ssRNA species to the dsRNA will be greater than 1; e.g. there will be a greater number of polyA and/or polyU molecules that polyAU molecules. This can also be expressed as the presence, on average, in each dsRNA duplex of more than 1 (the average may be less than an integer) ssRNA polymer. For example, a polyAU composition can comprise a ratio of polyA and/or polyU polymers present as hybridized polymers in the dsRNA to polyAU polymers of 1 to at least 1.1, 1.2, 1.3, 1.4, 1.5, 1.6, 1.7, 1.8, 1.9, 2.0 and 3.0.

Preferably, the polyA and polyU polymers used in preparation of a dsRNA will be provided so that the base ratio of A and U are close to 1:1 (e.g. between 1:1 and 1:2 for A:U or U:A base ratio), even when the average chain length of the polyA and polyU polymers differs.

The present invention also describes, as further described herein, that certain molecular weight characteristics and molecular weight ranges for ssRNA compositions provided improved results in the preparation of active dsRNA.

Thus, in one aspect, described is a method of producing a composition comprising high-molecular weight polyAU having TLR3 agonist activity, the method comprising:

a. producing or providing a first composition comprising single-stranded polyA polymers having one or more predetermined molecular weight characteristics, (e.g. having a molecular weight expressed as Mn or Mw, a distribution of molecular weights of fragments, and/or polydispersity within a predetermined range);

b. producing or providing a second composition comprising single-stranded polyU polymers having or more predetermined molecular weight characteristics, (e.g. having a molecular weight expressed as Mn or Mw, a distribution of molecular weights of fragments, and/or polydispersity within a predetermined range); and

c. mixing said first and second compositions comprising single-stranded RNA polymers under conditions suitable to give rise to composition comprising single-stranded RNA polymers.

In certain embodiment, use of ssRNA compositions of predetermined molecular weight characteristics can be used advantageously to give rise to dsRNA compositions having a high molecular weight (e.g. any of the Mn described herein, at least 250 kDa, 600 kDa, 800 kDa, 1000 kDa, 1200 kDa, 1500 kDa etc.), optimal distributions of fragments of particular molecular weight (e.g. less than certain percentages of dsRNA species having a molecular weight less than a predetermined value) and limited polydispersity, e.g. not more than 2.0. In one aspect, the method can further comprise determining the molecular weight parameter (e.g. minimum or desired Mn) and selecting a composition for further processing or ultimately use in therapy if the composition has the required molecular weight parameter. In other embodiments, as described herein, the method can also further comprise assessing TLR3 agonist or binding activity, and selecting a composition that has such activity. Furthermore, the inventors have determined certain optimal combination of polymer lengths are used for the single-stranded RNA polymers that can be used in method of producing long double-stranded polymers, when the single-stranded RNA polymers are annealed without resulting in a significant increase in polydispersity (e.g. Ip values). Importantly, no further fractionation steps were required to isolate double stranded RNA polymers having these desired molecular weights. The present identification of the particular rules relating to selection of the Mn therefore permits low polydispersity, high molecular weight dsRNA compositions to be obtained without the necessity of a step of fractionating the resulting dsRNA compositions.

In one example of a method to control the polymer length of the single-stranded RNA polymers, a standardized protocol is used which results in polymers having a certain mean chain length or Mn in accordance with that of the active high-molecular weight dsRNA of the invention. Such protocols can be developed by determining the conditions necessary during polymerization (e.g. time of polymerization) in order to arrive at a composition of single-stranded RNA polymers having a certain polymer length, generally expressed as a range or in terms of Mn, Mw or Ip. Optionally, two distinct polymerisation protocols or conditions need to be used in optimal preparation of two respective single-stranded RNA polymer compositions; for example, given identical conditions, the same PnPase may have different efficiency in the polymerization of different nucleotide bases (e.g. A and U are not polymerized with the same efficiency). Thus, regardless of whether different lengths are selected for two single-stranded RNA polymers, generally a first and a second polymerization protocol will be used to prepare the first and second single-stranded RNA polymers, respectively.

In another example, the size or length of single-stranded RNA polymers is controlled through the use of a selection step. In such a format, single-stranded RNA polymers are produced from monomers, and the single-stranded RNA polymers are then treated so as to separate or fractionate a subset of the single-stranded RNA polymers. Any means to separate single-stranded RNA polymers having size limitation can be used. For example, electrophoretic methods can be used, high performance liquid chromatographic method (HPLC) combined with gel filtration columns.

The use of nucleic acid templates can also be used to generate double-stranded RNA compositions. Such methods are particularly useful where the polymers are copolymers having specific predetermined sequences. For example, production of predetermined virally-derived RNA sequences or random sequences can be carried out this way.

In one example, the method can generally comprise synthesizing a double-stranded RNA having a strand complementary to a nucleic acid template, comprising synthesizing, separately, a first single stranded RNA polymer and a second single stranded RNA polymer, wherein each polymer is synthesized by contacting, under conditions effective to permit polymerization of the first and second single stranded RNA polymer, respectively, a nucleic acid (e.g. DNA) template with:

i. a polynucleotide linker/primer being sufficiently complementary to the nucleic acid template to hybridize therewith;
ii. an RNA polymerase (e.g. a T7, T3 or SP6 RNA polymerase promoter); and
iii. deoxyribonucleotide triphosphate substrates;

wherein the template and linker/primer are selected such that a single-stranded RNA polymer of a desired length is produced. When the first and second single stranded RNA polymers are synthesized separately, they can then be annealed as further described herein to produce double-stranded polymers. Alternatively, methods can also be envisioned whereby first and second single stranded RNA polymers are synthesized in the same reaction such that double-stranded RNA is produced.

Typically, in vitro transcription generally comprise a purified linear DNA template containing a promoter, ribonucleotide triphosphates, a buffer system that includes DTT and magnesium ions, and an appropriate phage RNA polymerase. The exact conditions used in the transcription reaction depend on the amount of RNA needed for a specific application. The common RNA polymerases used in vitro transcription reactions are SP6, T7 and T3 polymerases, named for the bacteriophages from which they were cloned, for which promoter consensus sequences for each of the phage RNA polymerases are known. The DNA template will contain a double-stranded promoter region where the phage polymerase binds and initiates RNA synthesis. Transcription templates can include for example plasmid constructs engineered by cloning, cDNA templates generated from an RNA precursor, and linear templates generated by PCR or by annealing chemically synthesized oligonucleotides. Typically, plasmid cloning vectors comprises phage polymerase promoters. They often contain two distinct promoters, one on each side of the multiple cloning site, allowing transcription of either strand of an inserted sequence. Examples include Ambion's pDP, Promega's pGEM, Stratagene's pBluescript and Invitrogen's pCRII vectors. Plasmid vectors used as transcription templates are linearized by restriction enzyme digestion, ensuring that RNA transcripts of a defined length and sequence are generated such that a composition of single-stranded RNA polymers having a homogenous length (e.g. low polydispersity) can be obtained. PCR Products. Confirmation that the dsRNA product is the correct size can be carried out using an e.g. a 1% agarose gel with TBE or TAE buffer.

When determining the molecular weight or chain length of any polymer according to the invention, it will be appreciated that many methods exist for providing information about the size or chain length of an polymer, whether a dsRNA or a single-stranded RNA polymer. In specifying the molecular size of nucleic acid, conventional sedimentation constant (i.e. S value) has been widely used in the past. More accurate methods, generally based on relative techniques for comparison to a weight standard, have been developed including electrophoretic methods or a high performance liquid chromatographic method (HPLC) using a gel filtration column, for example followed by comparison with the control which is a double stranded nucleic acid with known base pairs. HPLC results in measurement of the entire molecular weight distribution of the polymers in the composition. In other embodiments, absolute or primary techniques for measuring molecular weight can be used.

In the examples herein, the methods used herein determine the size or chain length of single-stranded (and double-stranded) RNA polymers is Size-Exclusion Chromatography/Multi-Angle Laser Light Scattering (SEC-MALLS). SEC coupled on-line to multi-angle laser light scattering (MALLS), has been demonstrated to be a very powerful method for characterization and analysis of highly polydisperse polymer systems. This technique is based on the absolute detection of light scattered from all the eluted fractions of the polymer after SEC separation (Wyatt, 1993). Consequently, in addition to the molecular weight ($M_w$ and/or $M_n$) and the radius of gyration (Rg); it is possible to obtain the $M_w$ or Rg distribution along the chromatogram (Capron, Grisel & Muller, 1995; Capron, Yvon & Muller, 1996; Picton, Mocanu, Mihai, Carpov & Muller, 1995; Picton, Merle & Muller, 1996). Moreover, from the power law describing the *M*w dependence of *R*g ($Rg \propto M_w^x$) useful information about polymer conformation is available through the value of x (0.3 for globular shape, 0.5 for flexible coil, and1 for rod-like chain). Literature data and our own experience show that SEC-MALLS gave useful information concerning the polydispersity of polyRNA. SEC-MALLS is described for example in Wyatt, P. J. (1993). Journal of Analytical Chemistry Acta, 272, 1.; Capron, I., Grisel, M., & Muller, G. (1995). International Journal of Polymer Analysis and Characterisation, 2, 9; Capron, I., Yvon, M., & Muller, G. (1996). Food Hydrocolloids, 10(2), 239; Picton, L., Mocanu, G., Mihaï, D., Carpov, A., & Muller, G. (1995). Carbohydrate Polymers, 28, 131; and Picton, L., Merle, L., & Muller, G. (1996). International Journal of Polymer Analysis and Characterisation, 2, 103.

$M_n$ (g/mol) and $M_w$ (g/mol) are generally used herein for the purposes of describing polymer characteristics.

Since the presence of a few very large polymers can raise the average molecular weight, it is preferably to describe the polymers in terms of $M_n$, optionally supplemented by the $M_w$ or by the polydispersity index, $M_w/M_n$, also referred to as the Ip.

Preferred methods for producing high molecular weight dsRNA polymers will involve producing a composition comprising single stranded RNA polymers having defined molecular weights. The disclosure also provides single stranded RNA compositions suitable for use as starting materials in the dsRNA production method of the invention. The single stranded RNA compositions include but are not limited to any of the following:

*a)* a composition comprising single-stranded RNA having an $M_n$ for the single stranded RNA polymers of at least 22, 50, 100, 150, 200, 250, 300, 400 or 500 kDa; optionally, between about 10 kDa and about 600 kDa, between 100 kDa and 600 kDa, optionally the single-stranded RNA compositions is further characterized by any one or combination of the following:

*iv.* optionally wherein at least 40%, 50%, 75%, or 90% of the ssRNA fragments in the composition have a molecular weight within about 200 kDa, 100 kDa or 50 kDa of the $M_n$;
v. less than 5% of fragments having a molecular weight less than a specified molecular weight (e.g. 10, 50, 100, 200, 300 or 500 kDa); and
*vi.* optionally, an Ip value of no more than 2.0, 1.8 or 1.6;

*b)* a composition comprising single stranded RNA having an $M_n$ of between about 50 and about 300 KDa;
*c)* a composition comprising single stranded RNA having an $M_n$ of between about 300 and about 600 KDa;
*d)* a composition comprising single stranded RNA having an $M_n$ of between about 150 and about 400 KDa;
*e)* a composition comprising single stranded RNA having an $M_n$ of between about 400 and about 600 KDa;
*f)* a composition comprising single stranded RNA having an $M_n$ of about 46 kDa, or within a range of 20 or 10 kDa of said $M_n$ value;
*g)* a composition comprising single stranded RNA having an $M_n$ of about 81 kDa, or within a range of 50, 20 or 10 kDa of said $M_n$ value;
*h)* a composition comprising single stranded RNA having an $M_n$ of about 149 kDa., or within a range of 50, 20 or 10 kDa of said $M_n$ value;
*i)* a composition comprising single stranded RNA having an $M_n$ of about 410 kDa, or within a range of 50, 20 or 10 kDa of said $M_n$ value;
*j)* a composition comprising single stranded RNA having an $M_n$ of about 426 kDa, or within a range of 50, 20 or 10 kDa of said $M_n$ value;
*k)* a composition comprising single stranded RNA having an $M_n$ of about 500 kDa., or within a range of 50, 20 or 10 kDa of said $M_n$ value;
*l)* a composition comprising single stranded RNA having an $M_n$ of about 467 kDa, or within a range of 50, 20 or 10 kDa of said $M_n$ value;
*m)* a composition comprising single stranded RNA having an $M_n$ of about 551 kDa, or within a range of 50, 20 or 10 kDa of said $M_n$ value;
*n)* a composition comprising single stranded RNA having an $M_n$ of about 198 kDa, or within a range of 50, 20 or 10 kDa of said $M_n$ value; and
*o)* a composition comprising single stranded RNA having an $M_n$ of about 455 kDa, or within a range of 50, 20 or 10 kDa of said $M_n$ value.

Optionally any of the above compositions (b) to (k) can further be characterized by any one of combination of the following: wherein at least 40%, 50%, 75%, or 90% of the ssRNA fragments in the composition have a molecular weight within about 300, 200 kDa, 100 kDa or 50 kDa of the $M_n$ (so long as such is consistent with the $M_n$ of the ssRNA composition); less than 5% of fragments having a molecular weight less than a specified value (e.g. 10, 50, 100, 200, 300 or 500 kDa), and an Ip value of no more than 3.0, 2.0, 1.6, 1.4 or 1.2.

For purposes of example, said single-stranded RNA polymers can be polyA or polyU polymers. Any of said single-stranded RNA polymers can be used to produce a dsRNA polymer. For example, a composition of any one of (a) to (o) wherein the single-stranded RNA polymers is polyA is brought into contact with a composition of any one of (a) to (o) wherein the single stranded polymer is polyU. Thus, each possible combination of (a) to (o) can be envisioned for each of two single-stranded RNA polymers. In one embodiment, a composition of (b) and (c) are combined; in another embodiment, a composition of (d) and (e) are combined; in another embodiment, a composition of either (g) or (h) are combined with a composition of either (i), (j) or (k) are combined. In one embodiment, a composition of (1) or (m) and (n) or (o) are combined; in another embodiment, a composition of (d) or (c) and (e) are combined.

Exemplary single stranded RNA polymer compositions include those of Table 1 below which were annealed to yield dsRNA compositions further described herein.

| Table 1 | | | | |
|---|---|---|---|---|
| **ssRNA (IPH code)** | **Mw (g/mol)** | **Mn (g/mol)** | **Length (b)** | **Ip = Mw/Mn** |
| **Axs** | 170000 | 81 000 | 245 | 2.1 |
| **Am1** | 743 000 | 500 000 | 1515 | 1.5 |
| **Am2** | 861 000 | 426 000 | 1291 | 2.0 |
| **Am3** | 751 000 | 467 200 | 1415 | 1.6 |
| **Am4** | 1012000 | 551 500 | 1671 | 1.8 |
| **Uxs** | 97 800 | 46 200 | 140 | 2.1 |
| **Us** | 206 000 | 149 000 | 452 | 1.4 |
| **Um1** | 302 000 | 198300 | 601 | 1.5 |
| **Um** | 646 000 | 410 000 | 1242 | 1.6 |
| **Um3** | 816 500 | 455 700 | 1381 | 1.8 |

As discussed herein, the combination of single-stranded RNA polymers compositions having certain molecular weights yielded high molecular weight double-stranded RNA composition with molecular weight characteristics as described herein. Generally, the higher molecular weight (longer average chain length) ssRNA composition conditioned $M_n$ values for the double-stranded RNA compositions, and such conditioning ssRNA composition could be combined with a second complementary ssRNA composition having a lower $M_n$ value. It was also observed that the best results were obtained when the molecular weight (e.g. $M_n$) for first single-stranded RNA polymer composition does not differ from that of the second single-stranded RNA polymer composition by more than a certain amount. Large difference in molecular weight between the first and second single-stranded RNA polymer compositions may lead to lower average molecular weights for the resulting dsRNA and/or suggested potentially sub-optimal hybridization quality.

In one aspect, a method for producing a double-stranded RNA composition having an $M_n$ of at least 400, 800, 1000, 1200, 1500 kDa, comprises combining a first and a second single-stranded RNA polymer compositions where the first single-stranded RNA polymer composition has a molecular weight (e.g. $M_n$) that is between about 10% and 100% (e.g. about 10%, 20%, 30, 40%, 50%, 60%, 70%, or 75%) or preferably between about 25% and about 80% of the molecular weight of the second single-stranded RNA polymer composition. In another example, a method for producing double-stranded RNA composition having a $M_n$ of at least 400kDa, or preferably at least 800 kDa, comprises combining single-stranded RNA polymer compositions which differ in $M_n$ by less than 100, 200, 300, 400 or 500 kDa.

Finally, several exemplary predetermined molecular weight ranges are provided for the single-stranded RNA polymer compositions that can be used to produce a dsRNA composition having a predetermined desired molecular weight. For dsRNA compositions having $M_n$ of between about 250 kDa and 2000 kDa, or between about 250 kDa and 1500 KDa, one method comprises combining a first single-stranded RNA polymer composition having an $M_n$ for the ssRNA polymers of less than about 400 kDa, generally between 100 and 300 kDa, with a second single-stranded RNA polymer composition having an $M_n$ for the ssRNA polymers of more than about 300 kDa, generally between 300 and 600 kDa. Another method comprises combining a first and a second single-stranded RNA polymer composition, each having an $M_n$ for the ssRNA polymers of less than about 400 kDa, generally between 100 and 400 kDa. Another method comprises combining a first and a second single-stranded RNA polymer composition, each having an $M_n$ for the ssRNA polymers of more than about 400 kDa, generally between 400 and 600 kDa.

Finally, several exemplary predetermined molecular weight ranges are provided for the single-stranded RNA polymer compositions that can be used to produce a dsRNA composition having a predetermined desired molecular weight. For dsRNA compositions having $M_n$ of at least 1000, 1200, 1500, optionally at least 1600, 1700, 1800, 1900 or 2000 kDa, one method comprises combining a first single-stranded RNA polymer composition having an $M_n$ for the ssRNA polymers of between 100 and 300 kDa, optionally about 200 kDa, with a second single-stranded RNA polymer composition having an $M_n$ for the ssRNA polymers between 400 and 600 kDa, optionally about 500 kDa. Another method comprises combining a first and a second single-stranded RNA polymer composition, each having an $M_n$ for the ssRNA polymers between 400 and 600 kDa, optionally about 500 kDa.

Preferred examples of advantageous combinations of polyA and polyU compositions giving rise to high molecular weights double-stranded RNA compositions are listed in Table 2.

| Table 2 | | | | | | |
|---|---|---|---|---|---|---|
| **dsRNA (IPH code)** | **Mn (g/mol)** | **Length (bp)** | **Mw (g/mol)** | **Ip = Mw/Mn** | **Mn polyA** | **Mn polyU** |
| **Axs:Uxs** | 156000 | 236 | 279 000 | 1.8 | 81 000 | 46 200 |
| **Axs:Us** | 256 000 | 388 | 455 000 | 1.8 | 81 000 | 149 000 |
| **Axs:Um** | 822 000 | 1245 | 1150 000 | 1.4 | 81 000 | 410 000 |
| **Am1:Uxs** | 615 000 | 932 | 717000 | 1.2 | 500 000 | 46 200 |
| **Am1:Us** | 549 000 | 832 | 985 000 | 1.8 | 500 000 | 149 000 |
| **Am1:Um*** | 1680 000 | 2545 | 2090 000 | 1.2 | 500 000 | 410 000 |
| **Am2:Uxs*** | 806 000 | 1221 | 1190 000 | 1.5 | 426 000 | 46 200 |
| **Am2:Us** | 1470 000 | 2227 | 1980 000 | 1.3 | 426 000 | 149 000 |
| **Am2:Um** | 1490 000 | 2258 | 2270 000 | 1.5 | 426 000 | 410 000 |
| **Am3:Um3** | 3 124000 | 4726 | 4019000 | 1.3 | 467 200 | 455 700 |
| **Am4:Um1** | 2 025 000 | 3064 | 2 729 000 | 1.3 | 551 500 | 198 300 |

**[0133]** * indicates, for Am1:Um and Am2:Uxs, the presence of two distinct populations of polymers, and the result in table reflects the higher molecular weight population. The lower molecular weight (a) and combined (b) populations respectively for these compositions was characterized are as follows:

a) Am1:Um: $M_n$ = 152 000 g.mol$^{-1}$ - $M_w$ = 205 000 g.mol$^{-1}$ (Ip = 1,4)
b) Am2:Uxs: $M_n$ < 40 000 g.mol$^{-1}$ - $M_w$ < 60 000 g.mol$^{-1}$ (Ip = 1,6).

Taking together both populations yielded the following results:

a) Am1:Um: $M_n$ = 420 000 g.mol$^{-1}$ - $M_w$ = 1500 000g.mol$^{-1}$ (Ip = 3.6) and
b) Am2:Uxs: $M_n$ = 246 000g.mol$^{-1}$ - $M_w$ = 1080 000 g.mol$^{-1}$ (Ip = 4.4).

Preferred polyAU compositions include compositions comprising complexes of polyA and polyU characterized by an $M_n$ of at least 250 kDa, as determined by SEC-MALLS, produced by mixing a first composition selected from the compositions comprising polyA listed in the leftmost column of Table 2, and a second composition selected from the compositions comprising polyU listed in the middle column of Table 2. Also encompassed is the use of compositions comprising polyA and polyU, respectively, according to Table 2, characterized by an $M_n$, as determined by SEC-MALLS, of within 100, 50, 20 or 10 kDa of the $M_n$ values provided in Table 2. The resulting compositions comprising complexes of polyA and polyU may be characterized by an $M_n$ of at least 250 kDa, 300 kDa, 500 kDa, 800 kDa, 1000 kDa, 1500, 2000 or 3000 kDa or an $M_n$ as specified in Table 2 or within 100, 50, 20 or 10 kDa thereof.

Following polymerisation undertaken on each of the appropriate monomers, they can be allowed to anneal in a conventional manner. Generally, appropriate annealing conditions comprise mixing, in the appropriate ratio, the two single-stranded RNA polymers capable of forming a double-strand in a buffer for a time comprised between 5 minutes and 4 hours, at a temperature comprised between room temperature and 100°C.

One exemplary annealing protocol involves mixing at room temperature, in the appropriate ratio, two single-stranded RNA polymers capable of forming a double-strand, heating for 5 minutes at 95 °C, and cooled at room temperature. Another exemplary annealing protocol involves mixing at room temperature, in a buffer of NaCl 0.15M, in the appropriate ratio, two single-stranded RNA polymers capable of forming a double-strand, heating for between 5 minutes and 2 hours at 60-75 °C, and cooled at room temperature, optionally an organic buffer can be added in the reaction mixture, such buffer will be chosen to maintain the pH of the reaction medium between 6 and 9. In one embodiment, the pH is maintained at a value of about 7. Suitable buffer include, for instance tris/HCl buffer, imidazole buffer, borate buffer, phosphate buffer, HEPES buffer or trismaleate buffer or any other suitable buffer. The single-stranded polymers can be provided in equal volume at the same concentration. Reaction impurities and residues are eliminated from the final product, by dialysis, filtration or any appropriate method. The solution can be stored at -20 °C.

By varying the conditions during annealing, it was observed herein that the same two single-stranded RNA compositions can give rise to more than one type of composition comprising double-stranded RNA polymers. As demonstrated in the Examples herein, annealing of a polyA ssRNA composition having an $M_n$ for the ssRNA polymers of about 500 kDa and a polyU ssRNA composition having an $M_n$ for the ssRNA polymers of about 149 kDa resulted in a composition having an $M_n$ for the double-stranded polymers of 324 kDa, 549 kDa or at least about 1000, 1500 kDa or greater.

As described in the Examples section herein, a method for preparing dsRNA having improved activity as well as increased molecular weight was developed, which involved minimizing the time that the RNA composition remains above the melting temperature (Tm) for the dsRNA during the annealing process. The two single-stranded RNA polymers capable of forming a double-strand are mixed (e.g. at room temperature), in the appropriate ratio, heating the mixture to at least the Tm estimated for the desired dsRNA for the shortest period necessary to allow annealing, and allowing the mixture to cool. In another embodiment, the ssRNA are heated separately and mixed at the desired annealing temperature. The Tm can be estimated based on the nature of the RNA polymers and the desired length of the dsRNA to be obtained. In a preferred embodiment, in order to prepare an exemplary dsRNA composition having an $M_n$ for the dsRNA of at least 300 kDa, or between 300 kDa and 2000 kDa, or higher than 1500, optionally higher than 1600, 1700, 1800, 1900 or 2000 kDa, the method comprises: (a) mixing (e.g. at room temperature), in the appropriate ratio, the two single-stranded RNA polymers capable of forming a double-strand, and (b) heating the mixture to no more than 40 °C, 30 °C, 20 °C or preferably 10 °C above the Tm (e.g. estimated Tm) for the desired dsRNA, for a period of no more than 4 hours, and allowing the mixture to cool. The Tm for a dsRNA of at least 300 kDa, or between 300 kDa and 2000 kDa or higher than 1500, optionally higher than 1600, 1700, 1800, 1900 or 2000 kDa is expected to be generally between about 55 °C and 65 °C. Thus, an exemplary process for producing a polyAU composition having an $M_n$ for the polyAU double stranded polymers of between 300 kDa and 2000 kDa or higher than 1500, optionally higher than 1600, 1700, 1800, 1900 or 2000 kDa comprises mixing in the appropriate ratio (e.g. equimolar) a composition comprising polyA and having an $M_n$ of at least 300 kDa (e.g. between 300 kDa and 600 kDa) with a composition comprising polyU and having an $M_n$ of at least 100KDa (e.g. between 100 kDa and 600 kDa), in a buffer, for a time comprised between 5 minutes and 4 hours, at a temperature comprised between room temperature and 100 °C, optionally for a time comprised between 5 minutes and 4 hours, or between 5 minutes and 2 hours, at a temperature comprised between 60-75 °C.

Optionally, a dsRNA obtained as described above can be treated by a variety of methods. For example, dsRNA may be treated by lyophilization to give a lyophilized product storable for a long period. The lyophilization treatment can be conducted in a conventional manner. For example, a lyophilized product can be obtained as follows: a solution of a dsRNA obtained under the conditions above is sterilized by filtration, the filtrate is then poured on a metal bat previously treated by dry heat sterilization, a pre-freezing is conducted at a shelf temperature from -40 to -20 °C. for about 1 to 4 hours, and primary drying is conducted before the secondary drying which is effected under reduced pressure at a shelf temperature from 15 to 30 °C (for about 10 to 50 hours). Generally, such a lyophilized product can be used after reconstitution (re-dissolution) by the addition of an appropriate solution such as injectable water, distilled water for injection, physiological saline, maltose solution, glucose solution, or the like. The lyophilized preparation of a composition of the present invention can be used after reconstitution by adding an appropriate solution before use. Examples of such a solution for reconstitution include injectable water, distilled water for injection, physiological saline, maltose solution, glucose solution and other general infusion solutions and the like. In another embodiment, the dsRNA compositions are sterilized by filtration or any other suitable method and stored in liquid state.

Preferably, the dsRNA polymer compositions prepared by these methods have a $M_n$ between about 250 kDa and 2000 kDa, between 1500 kDa and 3000 kDa, between 1500 kDa and 4000 kDa or between 1500 kDa and 5000 kDa, and/or any of the characteristics further described herein. In other embodiments, the dsRNA polymer compositions prepared by these methods have a $M_n$ of at least 1500 kDa, optionally at least 1600, 1700, 1800, 1900 or 2000 kDa, and/or any of the characteristics further described herein. Exemplary polyAU compositions were grouped according to increasing TLR3 agonist activity. While each of the four groups were characterized by a high amount of polymers having a weight of at least 100 kDa, and the third most active of four groups (Group 3) was comprised mostly of polymers having a weight of at least 200 kDa (range 62-100%), exemplified by Axs:Us and Am1:Us-2 which had 60-75 % of polymers having a molecular weight of at least 200 kDa, and at least 300 kDa (in the 40-50 % range), respectively. The third most active group (Group 2) exemplified by Am1:Us retained a significant portion of polymers having a molecular weight of at least 300 kDa (66 % range) as well as at least 500 kDa (41.2 %) and 500 kDa (32.5 %), respectively. The second most active group (Group 1) exemplified by composition Am2:Um was almost entirely made of polymers having a molecular weight of at least 600 kDa (99.5 % range) and included high proportion of polymers having a molecular weight of at least 800 kDa (in the 88 % range), 1000 kDa (75.5 %) and even a majority of polymers above 1400 kDa (57 %), respectively. The most active Group 0 exemplified by composition Am3:Um3 and Am4:Um1, was comprised principally of polymers having a molecular weight of at least 1000 kDa (80%), 1500 kDa (75%) or more than 2000 kDa. This subgroup has high bioactivity, as exemplified in Figure 18, where compounds having a Mn of more than 1500 kDa show a 0.5 log improvement in a 293T-T3-ISRE luciferase gene reporter assay over compounds having lower Mn. In summary, the Group 3 was distinguished from least active group (Group 4), exemplified by Axs:Uxs by substantially increased numbers of polymers having 200 and 300 kDa whereas from 600 kDa onwards the two least active groups were of similar composition. The second most active group is distinguished from the immediately less active group (third to last group) by substantially increased numbers of polymers at 300 kDa and above. The second active group is distinguished from the immediately less active group (third most active) by substantially increased numbers of polymers at 600 kDa and higher, and included almost solely polymers above 300 kDa. The most active group is distinguished from the immediately less active group (second most active) by substantially increased numbers of polymers at 1500 kDa and higher, and included almost solely polymers above 1000 kDa.

In one embodiment, a PolyAU composition, is characterized by a $M_n$ of at least about 250 kDa or 300 kDa, optionally an $M_w$ of greater than about 500 kDa, and any one or a combination of:

a) less than about 20%, 10% or more preferably 5 % of fragments having a molecular weight less than about 100 kDa;
b) less than about 20%, 10% or more preferably 5 % of fragments having a molecular weight less than about 300 kDa; and
c) less than about 70%, 60% or more preferably 50 % of fragment having a molecular weight less than about 600 kDa.

Optionally the dsRNA composition is further characterized by an $M_n$ of at least 300, at least 500 or at least 800 kDa, optionally between about 300 kDa and about 600 kDa, optionally between about 300 kDa and about 500 kDa, between about 500 kDa and about 1000 kDa, and between about 1000 kDa and about 2000 kDa.

*Group 3 Compositions*

The examples herein describe Group 3 compositions, preferably polyAU compositions, which have increased activity over other dsRNA compositions. Group 3 compositions can be characterized by a $M_n$ of between about 250 kDa and 500 kDa, and any one or a combination of:

a) less than about 5%, optionally 10% or 25 % of fragments having a molecular weight less than about 100 kDa;
b) less than about 50 %, optionally less than about 40%, of fragments having a molecular weight less than about 200 kDa;
c) less than about 70 %, optionally less than about 60%, of fragments having a molecular weight less than about 300 kDa;
d) an $M_w$ of at greater than 450 kDa, optionally 500 kDa;
e) an Ip of less than 2;
f) a Tm of at least 59 °C;
g) hyperchromicity of at least 40%, preferably at least 50%;
h) an FWHM of less than 5 °C, optionally less than 3° C; and
i) TLR3 activity resulting in a maximum fold increase in same luciferase assay of 50 to 250, with an EC50 of 10 to 50 μg/ml.

The Group 3 compositions can be further or alternatively characterized as comprising complexes of a first composition comprising single-stranded polymers (e.g. polyA) characterized by an $M_n$ of between 50 kDa and 150 kDa, preferably about 81 kDa or within a range of 50, 20 or 10 kDa thereof, and a second composition comprising single-stranded polymers (e.g. polyU) characterized by an $M_n$ of between 100 kDa and 300 kDa, preferably about 149 kDa or within a range of 50, 20 or 10 kDa thereof. In another example, Group 3 compositions can be characterized as comprising complexes of a first composition comprising single-stranded polymers (e.g. polyA) characterized by an $M_n$ of between 300 kDa and 600 kDa, between 400 kDa and 600 kDa, optionally about 500 kDa or within 100, 50, 20 or 10 kDa thereof, and a second composition comprising single-stranded polymers (e.g. polyU) characterized by an $M_n$ of between 150 kDa and 400, optionally about 149 kDa or within 50, 20 or 10 kDa thereof.

*Group 2 Compositions*

The examples herein also describe Group 2 compositions, preferably polyAU compositions, which have increased activity over other dsRNA compositions, including over Group 3 compositions. Group 2 compositions can be characterized by a $M_n$ of between about 500 kDa and 800 kDa, optionally between 600 kDa and 800 kDa, and any one or a combination of:

a) less than about 5 % of fragments having a molecular weight less than about 100 kDa;
b) less than about 20 % of fragments having a molecular weight less than about 200 kDa;
c) less than about 40 % of fragments having a molecular weight less than about 300 kDa;
d) less than about 50 % of fragments having a molecular weight less than about 500 kDa;

e) less than about 70 % of fragments having a molecular weight less than about 600 kDa;
f) less than about 75 % of fragments having a molecular weight less than about 700 kDa;
g) less than about 80 % of fragments having a molecular weight less than about 800 kDa;
h) an $M_w$ of at greater than 700 kDa or 900 kDa;
i) an Ip of less than 2;
j) a Tm of at least 59 °C, preferably at least 60 °C;
k) hyperchromicity of at least 40%, preferably at least 50%;
l) an FWHM of less than 5 °C, optionally less than 3° C; and
m) TLR3 activity resulting in a maximum fold increase in same luciferase assay of 50 to 250, with an EC50 of 1 to 10 μg/ml.

The Group 2 compositions can be further or alternatively characterized as comprising complexes of a first composition comprising single-stranded polymers (e.g. polyA) characterized by an $M_n$ of between 300 kDa and 600 kDa, between 400 kDa and 600 kDa, optionally about 500 kDa or within 100, 50, 20 or 10 kDa thereof, and a second composition comprising single-stranded polymers (e.g. polyU) characterized by an $M_n$ of between 50 kDa and 300 kDa, between 150 kDa and 400 kDa, optionally about 149 kDa or within 50, 20 or 10 kDa thereof.

*Group I Compositions*

The examples herein also describe Group 1 compositions, preferably polyAU compositions, which have increased activity over other dsRNA compositions, including over Group 2 compositions. Group 1 compositions can be characterized by a $M_n$ of between about 800 kDa and 2000 kDa, optionally between about 800 kDa and 1500 kDa, and any one or a combination of:

a) less than about 5 % of fragments having a molecular weight less than about 200 kDa;
b) less than about 10 % of fragments having a molecular weight less than about 300 kDa;
c) less than about 20 % of fragments having a molecular weight less than about 400 kDa;
d) less than about 25 % of fragments having a molecular weight less than about 500 kDa;
e) less than about 30 % of fragments having a molecular weight less than about 600 kDa;
f) less than about 40 % of fragments having a molecular weight less than about 800 kDa;
g) less than about 50 % of fragments having a molecular weight less than about 1000 kDa;
h) less than about 60 % or 70 % of fragments having a molecular weight less than about 1400 or 1500 kDa, respectively;
i) an $M_n$ of at least about 1000 kDa, or at least 1400 kDa or at least 1500 kDa;
j) an $M_w$ of at greater than 1000 kDa;
k) a Tm of at least 59 °C, preferably at least 62 °C;
l) hyperchromicity of at least 40%, preferably at least 50%;
m) an FWHM of less than 5 °C, optionally less than 3° C; and
n) TLR3 activity resulting in a maximum fold increase in same luciferase assay of 50 to 250, with an EC50 of 0.1 to 1 μg/ml.

In one embodiment, the Group 1 compositions can be further or alternatively characterized as comprising complexes of a first composition comprising single-stranded polymers (e.g. polyA) characterized by an $M_n$ of between 300 kDa and 600 kDa, between 400 and 600, optionally about 500 kDa or within 100, 50, 20 or 10 kDa thereof, and a second composition comprising single-stranded polymers (e.g. polyU) characterized by an $M_n$ of between 50 kDa and 300 kDa, between 150 kDa and 400 kDa, optionally about 149 kDa or within 50, 20 or 10 kDa thereof. In another embodiment, the Group 1 compositions can be characterized as comprising complexes of a first and a second composition, each of said compositions comprising single-stranded polymers characterized by an $M_n$ of between 300 kDa and 600 kDa, or between 400 kDa and 600 kDa.

*Group 0 Compositions*

In another aspect, inventors have identified a particularly active group Group 0, including dsRNA compositions having a Mn of more than 1200, more than 1500, optionally more than 1600, 1700, 1800, 1900 or 2000 kDa. Optionally, the Group 0 compositions have a Mn of between 1500 kDa and 3000 kDa, between 1500 kDa and 4000 kDa or between 1500 kDa and 5000 kDa. Optionally, the double-stranded RNA polymer compositions of Group 0 are further characterized by a Tm, hyperchromicity and/or derivative curve of the Tm (FWHM value). In one aspect, the Tm of the composition is greater than 59 °C or preferably 60 °C. In one aspect, the hyperchromicity is at least 45%, or preferably 50%, 51% or 55%. In one aspect the FWHM value is no more than 5.0°, 3.0° or 2.0°. Optionally, the compositions have an Ip for the double-stranded RNA polymers of no more than 2.0, 1.5, 1.4 or 1.2. In an aspect of the present invention, said subgroup is at least 10 times more active in vivo. Accordingly, the therapeutic dose needed for compositions of Group 0 is ten times lower than doses prescribed for less potent dsRNA compositions.

In one embodiment, the Group 0 compositions can be further or alternatively characterized as comprising complexes of a first composition comprising single-stranded polymers (e.g. polyA) characterized by an $M_n$ of between between 400 and 600, optionally about 500 kDa or within 100, 50, 20 or 10 kDa thereof, and a second composition comprising single-stranded polymers (e.g. polyU) characterized by an $M_n$ of between 100 kDa and 300 kDa, optionally about 200 kDa or within 50, 20 or 10 kDa thereof. In another embodiment, the Group 0 compositions can be characterized as comprising complexes of a first and a second composition, each of said compositions comprising single-stranded polymers characterized by an $M_n$ of between 400 kDa and 600 kDa, optionally about 500 kDa. In one embodiment, the compositions of Group 0 the Group 0 compositions can be further or alternatively characterized as obtained using a hybridization protocol similar to Protocol 3 as described in Example 6.

In one embodiment, the Group 0 compositions can be further or alternatively be characterized as comprising complexes presenting a very good hybridization state i.e. a high Tm, and/or high hyperchromicity and/or low FWHM.

In another embodiment, the Group 0 compositions can be further or alternatively be characterized as comprising a very low content in endotoxins.

In another embodiment, the Group 0 compositions can be further or alternatively be characterized as a plurality of dsRNA fragments wherein the majority of dsRNA fragments have a Mn of more than 1500 kDa. In particular, more than 50%, 75%, 95% of the dsRNA fragments have a Mn of more than 1000 kDa, more than 50%, 70%, 90% of the dsRNA fragments have a Mn of more than 1500 kDa.

Any of the dsRNA compositions of the invention can additionally characterized by the nature of the single-stranded RNA polymers that make up the dsRNA. For example, any of the dsRNA compositions of the invention may be characterized as comprising complexes formed from a first and second composition comprising single-stranded RNA polymers, wherein each of said first and second compositions are characterized by an $M_n$ for the single-stranded RNA polymers of between 100 kDa and 600 kDa. In another example, any of the dsRNA compositions of the invention may be characterized as comprising complexes formed from a first and second composition comprising single-stranded RNA polymers, each composition having the characteristics of any of the ssRNA (a) to (k) above. Optionally, the double-stranded RNA polymer compositions are further characterized by a Tm, hyperchromicity and/or derivative curve of the Tm (FWHM value). In one aspect, the Tm of the composition is greater than 59 °C, 60 °C, 61 °C, 62 °C or 63 °C. In one aspect, the hyperchromicity is at least 40%, 48%, or 50%. In one aspect the FWHM value is no more than 5.0 °, 3.0 °, 2.0 ° or 1.5 °. Optionally, the compositions have an Ip for the double-stranded RNA polymers of no more than 2.0, 1.5, 1.4, 1.3 or 1.2.

*Exemplary polyAU compositions*

In further embodiments, preferred polyAU compositions are selected from the polyAU compositions obtained in the Examples herein. The compositions are classified into three ranges of EC50 values (10 to 50 μg/ml, 1 to 10 μg/ml and 0.1 to 1 μg/ml) on TLR3 reporter assays corresponding to the Group 3, Group 2 and Group 1 compounds.

A first polyAU composition is referred to as Am1:Us-2 and can be characterized by an $M_n$ of about 324 kDa, or within a range of 100, 50, 20 or 10 kDa of said $M_n$ value, optionally further having an Ip for the double-stranded polyAU polymers of no more than 3.0, 2.5, 2.0, 1.5, 1.4 or 1.2; preferably the Ip is about 1.6. Optionally, the composition has a Tm of about 59.2 C. Optionally, the dsRNA composition comprises at least about 50% or 75% of dsRNA species having a molecular weight between about 100 kDa and 700 kDa. The composition can be further characterized as inducing a 50 to 250 fold-increase in TLR3 signalling induction compared to control condition, as measured in TLR3 expressing HEK293 cells, by fold increase in reporter gene activity of luciferase under the control of ISRE promoter, when brought into contact at a concentration of at least 100μg/ml. The TLR3 activity can also be defined by resulting in a maximum fold increase in same luciferase assay of 50 to 250, with an EC50 of 10 to 50 μg/ml.

Another polyAU composition is referred to as Am1:Us, and can be characterized by an $M_n$ of about 549 kDa, or within a range of 100, 50, 20 or 10 kDa of said $M_n$ value, optionally further having an Ip for the double-stranded polyAU polymers of no more than 3.0, 2.5, 2.0, 1.5, 1.4 or 1.2; preferably the Ip is about 1.2. Optionally, the composition comprises less than about 5 % of fragments having a molecular weight less than about 100 kDa; less than about 40 % of fragments having a molecular weight less than about 300 kDa; less than about 50 % having a molecular weight less than about 400 kDa; and/or less than about 70 % having a molecular weight less than about 600 kDa. Optionally, the composition has a Tm of about 60.9 °C. Optionally, the dsRNA composition comprises at least about 50%, or optionally 70%, of dsRNA species having a molecular weight between about 200 kDa and 1200 kDa. The composition can be further characterized as inducing a 50 to 250 fold-increase in TLR3 signalling induction compared to control condition, as measured in TLR3 expressing HEK293 cells, by fold increase in reporter gene activity of luciferase under the control of ISRE promoter, when brought into contact at a concentration of at least 100μg/ml. The TLR3 activity can also be defined by resulting in a maximum fold increase in same luciferase assay of 50 to 250, with an EC50 of 1 to 10 μg/ml

Another polyAU composition is referred to as Am1:Us-3, and can be characterized by an $M_n$ of at about 1280 kDa, or within a range of 100, 50, 20 or 10 kDa of said $M_n$ value, optionally an $M_n$ of at about 1690 kDa or within a range of 200, 100 or 50 kDa of said $M_w$ value, optionally further having an Ip for the double-stranded polyAU polymers of no more than 3.0, 2.5, 2.0, 1.5, 1.4 or 1.2. The composition can be further characterized as inducing a 50 to 250 fold-increase in TLR3 signalling induction compared to control condition, as measured in TLR3 expressing HEK293 cells, by fold increase in reporter gene activity of luciferase under the control of ISRE promoter, when brought into contact at a concentration of at least 100μg/ml. The TLR3 activity can also be defined by resulting in a maximum fold increase in same luciferase assay of 50 to 250, with an EC50 of 0.1 to 1μg/ml or lower.

Optionally any of the compositions Am1:Us, Am1:Us-2 and Am1:Us-3 can be further or alternatively characterized as comprising complexes of a first composition comprising single-stranded polyA polymers characterized by an $M_n$ of between 300 kDa and 600 kDa, between 400 and 600, optionally about 500 kDa or within 100, 50, 20 or 10 kDa thereof, and a second composition comprising single-stranded polyU polymers characterized by an $M_n$ of between 50 kDa and 300 kDa, between 150 and 400, optionally about 149 kDa or within 50, 20 or 10 kDa thereof. In another embodiment, the polyAU compositions of the invention may be characterized as comprising complexes of one or more polyA polymers and one or more polyU polymers, wherein the $M_n$ for the polyU polymers is between 300 kDa and 600 kDa, or between 400 and 600, and the $M_n$ for the polyA polymers is between 50 kDa and 300 kDa, or between 150 and 400 kDa.

Another polyAU composition is referred to as Am2:Um, and can be characterized by an $M_n$ of about 1490 kDa, or within a range of 100, 50, 20 or 10 kDa of said $M_n$ value, optionally further having an Ip for the double-stranded polyAU polymers of no more than 3.0, 2.5, 2.0, 1.5, 1.4 or 1.2. Preferably the Ip is about 1.5. Optionally, the composition comprises less than about 5 % of fragments having a molecular weight less than about 10 % of fragments having a molecular weight less than about 300 kDa; less than about 30 % of fragments having a molecular weight less than about 600 kDa; less than about 40 % of fragments having a molecular weight less than about 800 kDa; less than about 50 % having a molecular weight less than about 1000 kDa; and/or less than about 60 % having a molecular weight less than about 1400 kDa. Optionally, the composition has a Tm of about 62.3 °C. The composition can be further or alternatively characterized as comprising complexes of a first composition comprising single-stranded polyA polymers characterized by an $M_n$ of between 300 kDa and 600 kDa, between 400 and 600, optionally about 426 kDa or within 100, 50, 20 or 10 kDa thereof, and a second composition comprising single-stranded polyU polymers characterized by an $M_n$ of between 50 kDa and 300 kDa, between 150 and 400, optionally about 360 kDa or within 50, 20 or 10 kDa thereof. In another embodiment, the polyAU compositions of the invention may be characterized as comprising complexes of one or more polyA polymers and one or more polyU polymers, wherein the $M_n$ for the polyU polymers is between 300 kDa and 600 kDa, between 400 and 600, and the $M_n$ for the polyA polymers is between 50 kDa and 300 kDa, between 150 and 400 kDa.

Another polyAU composition is referred to as Am3:Um3, and can be characterized by an $M_n$ of about 3124kDa, or within a range of 300, 200, 100, 50, 20 or 10 kDa of said $M_n$ value, optionally further having an Ip for the double-stranded polyAU polymers of no more than 3.0, 2.5, 2.0, 1.5, 1.4 or 1.2. Preferably the Ip is about 1.5 or 1.3. Optionally, the composition comprises less than about 1 % of fragments having a molecular weight less than about 500 kDa, less than about 5% of fragments having a molecular weight less than about 1000 kDa; less than about 10 % of fragments having a molecular weight less than about 1500 kDa.

Optionally, the composition has a Tm of about 62.6 °C. Optionally, the composition has a hyperchromocity of 54.4%. Optionally, the composition has an endotoxin content of not more than 3 μg/ml. The composition can be further or alternatively characterized as comprising complexes of a first composition comprising single-stranded polyA polymers characterized by an $M_n$ of between 300 kDa and 600 kDa, between 400 and 500, optionally about 467 kDa or within 100, 50, 20 or 10 kDa thereof, and a second composition comprising single-stranded polyU polymers characterized by an $M_n$ of between 300 kDa and 600 kDa, between 400 and 500, optionally about 455 kDa or within 50, 20 or 10 kDa thereof. In another embodiment, the polyAU compositions of the invention may be characterized as comprising complexes of one or more polyA polymers and one or more polyU polymers, wherein the $M_n$ for the polyU polymers and the Mn for the polyA polymers are within the same range, between 300 kDa and 600 kDa, between 400 and 500 kDa.

Another polyAU composition is referred to as Am4:Um1, and can be characterized by an $M_n$ of about 2025 kDa, or within a range of 200, 100, 50, 20 or 10 kDa of said $M_n$ value, optionally further having an Ip for the double-stranded polyAU polymers of no more than 2.0 or 1.5. Preferably the Ip is about 1.3. Optionally, the composition comprises less than about 10 % of fragments having a molecular weight less than about 500 kDa, 25% of fragments having a molecular weight less than about 1000 kDa; less than about 50 % of fragments having a molecular weight less than about 1500 kDa. Optionally, the composition comprises less than about 5 % of fragments having a molecular weight less than about 500 kDa, 20% of fragments having a molecular weight less than about 1000 kDa; less than about 50 % of fragments having a molecular weight less than about 1500 kDa. Optionally, the composition comprises less than about 5 % of fragments having a molecular weight less than about 500 kDa, 25% of fragments having a molecular weight less than about 1000 kDa; less than about 70 % of fragments having a molecular weight less than about 1500 kDa. Optionally, the composition comprises less than about 1 % of fragments having a molecular weight less than about 500 kDa, 5% of fragments having a molecular weight less than about 1000 kDa; less than about 10 % of fragments having a molecular weight less than about 1500 kDa. Optionally, the composition has a Tm of at least about 62 °C. Optionally, the composition has a hyperchromocity of 51.2%. The composition can be further or alternatively characterized as comprising complexes of a first composition comprising single-stranded polyA polymers characterized by an $M_n$ of between 300 kDa and 600 kDa, between 400 and 600, optionally about 467 kDa or within 100, 50, 20 or 10 kDa thereof, and a second composition comprising single-stranded polyU polymers characterized by an $M_n$ of between 50 kDa and 300 kDa, between 150 and 400, optionally about 198 kDa or within 50, 20 or 10 kDa thereof. In another embodiment, the polyAU compositions of the invention may be characterized as comprising complexes of one or more polyA polymers and one or more polyU polymers, wherein the $M_n$ for the polyA polymers is between 300 kDa and 600 kDa, between 400 and 600 kDa, and the $M_n$ for the polyU polymers is between 50 kDa and 300 kDa, between 150 and 300 kDa.

Another polyAU composition is referred to as Axs:Um, and can be characterized by an $M_n$ of about 822 kDa, or within a range of 100, 50, 20 or 10 kDa of said $M_n$ value, optionally further having an Ip for the double-stranded polyAU polymers of no more than 3.0, 2.5, 2.0, 1.5, 1.4 or 1.2. Preferably the Ip is about 1.4. The composition can be further or alternatively characterized as comprising complexes of a first composition comprising single-stranded polyA polymers characterized by an $M_n$ of between 50 kDa and 300 kDa, preferably about 81 kDa or within a range of 50, 20 or 10 kDa thereof, and a second composition comprising single-stranded polyU polymers characterized by an $M_n$ of between 300 kDa and 600 kDa, preferably about 360 kDa or within a range of 50, 20 or 10 kDa thereof. In another embodiment, the polyAU compositions of the invention may be characterized as comprising complexes of one or more polyA polymers and one or more polyU polymers, wherein the $M_n$ for the polyU polymers is between 50 kDa and 300 kDa, and the $M_n$ for the polyA polymers is between 300 kDa and 600 kDa. The composition can be further characterized as inducing a 50 to 250 fold-increase in TLR3 signalling induction compared to control condition, as measured in TLR3 expressing HEK293 cells, by fold increase in reporter gene activity of luciferase under the control of ISRE promoter, when brought into contact at a concentration of at least 100μg/ml. The TLR3 activity can also be defined by resulting in a maximum fold increase in same luciferase assay of 50 to 250, with an EC50 of 10 to 200 μg/ml.

Another polyAU composition is referred to as Axs:Us, and can be characterized by an $M_n$ of about 256 kDa, or within a range of 100, 50, 20 or 10 kDa of said $M_n$ value, optionally further having an Ip for the double-stranded polyAU polymers of no more than 3.0, 2.5, 2.0, 1.5, 1.4 or 1.2. Preferably the Ip is about 1.8. Optionally, the composition comprises less than about 5 % of fragments having a molecular weight less than about 100 kDa; less than about 30 % of fragments having a molecular weight less than about 200 kDa; less than about 55 % having a molecular weight less than about 300 kDa. Optionally, the composition has a Tm of about 59 °C. Optionally, the dsRNA composition comprises at least about 50% or 75% of dsRNA species having a molecular weight between about 100 kDa and 500 kDa. The composition can be further or alternatively characterized as comprising complexes of a first composition comprising single-stranded polymers (e.g. polyA) characterized by an $M_n$ of between 50 kDa and 150 kDa, preferably about 81 kDa or within a range of 50, 20 or 10 kDa thereof, and a second composition comprising single-stranded polymers (e.g. polyU) characterized by an $M_n$ of between 100 kDa and 300 kDa, preferably about 149 kDa or within a range of 50, 20 or 10 kDa thereof. The composition can be further characterized as inducing a 50 to 250 fold-increase in TLR3 signalling induction compared to control condition, as measured in TLR3 expressing HEK293 cells, by fold increase in reporter gene activity of luciferase under the control of ISRE promoter, when brought into contact at a concentration of at least 100μg/ml. The TLR3 activity can also be defined by resulting in a maximum fold increase in same luciferase assay of 50 to 250, with an EC50 of 10 to 50 μg/ml.

Methods for determining the molecular weights of ssRNA compositions used to generate a given dsRNA polymer composition can be carried out using any method known in the art. Generally, the dsRNA will be denatured and single stranded RNA polymers will be analyzed for molecular weight profile. For example, Gunnarsson et al. (2006) Anal. Biochem. 350(1): 120-127, the disclosure of which is incorporated herein by reference, describe two-dimensional strandness-dependent electrophoresis (2D-SDE) for quantification and length distribution analysis of double-stranded nucleic acids in complex samples. In the first dimension nucleic acid molecules are separated based on strandness and length in the presence of 7 M urea. After the first-dimension electrophoresis all nucleic acid fragments are heat denatured in the gel. During the second-dimension electrophoresis all nucleic acid fragments are single-stranded and migrate according to length.

Any of the dsRNA compositions described herein can be characterized as inducing a 50 to 250 fold increase in TLR3 signalling induction, as measured by fold-increase in reporter gene (e.g. luciferase) activity, under the control of ISRE promoter, when brought into contact at a concentration of at least 100μg/ml. Preferably any of these compositions can also be characterized as not inducing substantial production of interferon, e.g. a type I interferon or an interferon-gamma. Preferably any of the compositions can be further characterized as binding specifically to or inducing selective TLR3 signalling, particularly with respect to MDA5 and/or RIGI and/or PKR.

In any embodiment herein, the embodiment can optionally be characterized so as to specifically exclude any one or combination of (or all of) the following: a dsRNA having a Mw of 500 kDa; a dsRNA having an Mn of 1380 kDa; a dsRNA having a proportion of 2'-5' phosphodiester bonds of between 0.1% and 3% of total phosphodiester bonds; a dsRNA conjugated to a non-polyAU nucleic acid; a dsRNA formulated in an oil in water emulsion, or in an eye drop or otherwise adapted for subconjunctive ocular administration; a dsRNA in a composition containing more than 0.5 or more than 1 EU/mg of endotoxins; a dsRNA containing substantially aggregated polyAU; a dsRNA composition containing platinum, any non-nucleic acid immunomodulatory compounds or any non-polyAU immunomodulatory compounds; or the composition described in Michelson et al. (1985) or the composition referred to as Poludan™ (e.g. the composition having a Mn of 1380 kDa, IP of 1.2, a Mw of 1680 kDda, an FWHM of more than 3.0 °C and/or an endotoxin content of at least 1.9 EU/mg).

*Assaying the ability of the oligonucleotides to stimulate TLR3*

"TLR3", "TLR3 polypeptide" and "TLR3 receptor", used interchangeably, are used herein to refer to Toll-Like Receptor 3, a member of the Toll-like receptor (TLRs) family. Its amino acid sequence of is shown in SEQ ID NO 2 (NCBI accession number NP_003256, the disclosure of which is incorporated herein by reference). As mentioned, it will be appreciated that any TLR3 polypeptide fragment or homologue can be used in accordance with the present methods. In one aspect, the TLR3 polypeptide may comprise an amino acid sequence of at least about 25, 30, 35, 40, 45, 50, 60, 70, 80, 100, 200, 300, 400, 500, 600, 700, 800, 900 or 904 amino acid residues in length, of which at least about 50-80%, preferably at least about 60-70%; more preferably at least about 65%, 75%, 80%, 85% or 90%. 95%, 98%, 99% or 99.5% of the amino acid residues are identical or similar amino acids to the sequence of the full-length native human TLR3 polypeptide (for example SEQ ID NO 2 for human TLR3). Identity or similarity may be determined using any desired algorithm, including the algorithms and parameters for determining homology which are described herein. Toll Like Receptor 3 is a member of the Toll-like receptor (TLR) family which plays a fundamental role in pathogen recognition and activation of innate immunity. TLRs are highly conserved from Drosophila to humans and share structural and functional similarities. They recognize pathogen-associated molecular patterns (PAMPs) that are expressed on infectious agents, and mediate the production of cytokines necessary for the development of effective immunity. The various TLRs exhibit different patterns of expression. TLR is expressed in various cell types, including dendritic cells, NK cells, CD4+ T cells, CD8+ T cells, endothelial cell, epithelial cells, keratinocytes and cancer cells. TLR3 recognizes dsRNA associated with viral infection, and induces the activation of NF-kappaB and the production of type I interferons. It may thus play a role in host defense against viruses. TLR3 mRNA sequence is described in NCBI accession number NM_003265, the sequence of which is shown in SEQ ID No 1. TLR3 is described in WO 98/50547 (the disclosure of which is incorporated herein by reference).

Candidate TLR3 agonist compositions can be assessed in vitro for their ability to bind and/or act as an agonist of TLR3, optionally further their ability to bind and/or act as an agonist of other dsRNA receptors, and/or optionally further their ability to induce any other desirable or undesirable biological effect, such as for example production of cytokines, type I interferons, etc.

Assays useful to detect binding or interaction of a dsRNA polymer composition with a TLR3 polypeptide are well known. Any suitable method can be used, for example detecting binding to immobilized TLR3 polypeptides using Biacore methods described herein. Detection that a dsRNA polymer composition interacts or binds a TLR3 polypeptide indicates that the dsRNA polymer composition is a candidate TLR3 agonist.

The dsRNA polymer compositions of the present invention can be assessed in vitro for their ability to act as an agonist of TLR3. TLR3 agonist activity can advantageously be assessed in cells made to express TLR3, in cell types known to express TLR3, including but not limited to dendritic cells (DCs), NK cells, epithelial cells, endothelial cells, tumor cells, or in other cell types using any of a variety of assays, or in any other cell-free assay where TLR3 agonist activity can be assessed. Such assays can be used, *inter alia,* for testing derivatives of the herein-provided sequences or for assessing novel sequences designed according to the teachings of the present specification for their ability to stimulate TLR3. Such assays can also be used to identify other modulators of TLR3-expressing cells, e.g., using the herein-described dsRNA polymer compositions as standards or controls. In vitro stimulation of DCs, tumor cells or NK cells are also useful, e.g., for evaluating DCs, tumor cells, or NK cells or other TLR3-expressing cells from an individual.

Assessing TLR3 agonist activity can involve, for example, detecting any increase in cellular activity, detected in vitro or in vivo, including for example presence of activation markers on T cells, B cells, NK cells, dendritic cells, the production of cytokines, upregulation of TLR3, proliferation of cells (e.g. T cells, B cells, NK cells, dendritic cells), pro-apoptotic activity, or TLR3 signaling (e.g. MyD88-independent/TRIF dependent signaling).

The present in vitro assays can be performed either with isolated cells that naturally express TLR3, or with cells that do not normally express TLR3 but into which expression constructs encoding TLR3 have been introduced. In one embodiment the cell naturally expresses functional TLR3 and is, e.g., a dendritic cell type; an NK cell, a CD4+ T cell, a CD8+ T cells, an endothelial cell, a keratinocyte or a cancer cell. Examples for tumor cells are human breast tumor cell lines (HCC38, HCC1806) obtained from the American Type Culture Collection and cultured following ATCC instructions. Another suitable cell line is the HCC1806 cell line, used in the Examples herein. Also, suitable murine cells expressing TLR3 can be isolated, e.g., from bone marrow progenitors isolated from C57BL/6, Balb/c, CBA, 129 or other mice, for example as can be obtained from Charles River UK. In humans, suitable cell types expressing TLR3 also include freshly isolated myeloid DC from PBMC and dendritic cells derived in vitro from monocytes.

Any of a large variety of cell types can be made to express TLR3 for the purposes of the present assays. For example, assays employing HEK293 T cells transfected with an expressible TLR3 structural gene may use a threshold of, for example, at least a three-fold increase in a TLR3-mediated biological activity (e.g., NF-KB activation) when the compound is provided at a concentration of, for example, from about 1 microM to about 10 microM for identifying a compound as an agonist of the TLR3 transfected into the cell. Such stably TLR3 transfected HEK-293 cell are commercially available (InvivoGen, San Diego, CA). However, different thresholds and/or different concentration ranges may be suitable in certain circumstances. Also, different thresholds may be appropriate for different assays. TLR3- encoding expression constructs can be made using standard molecular biology methods, typically including regulatory sequences capable of constitutively driving expression of operably linked coding sequences, and a coding sequence encoding all or part of TLR3. Such vectors are standard in the art and are described, e.g., in Molecular Cloning: A Laboratory Manual (Sambrook et al.; Cold Spring Harbor Laboratory Press; 3rd edition (January 15, 2001), or Short Protocols in Molecular Biology (Ausubel et al, Current Protocols; 5 edition (October 18, 2002), each of which is incorporated herein by reference in its entirety.

Constitutive mammalian promoters that can be used to drive TLR3 expression include, but are not limited to, the promoters for the following genes: hypoxanthine phosphoribosyl transferase (HPRT), adenosine deaminase, pyruvate kinase, beta-actin promoter and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, the long terminal repeats (LTR) of Moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art. Typically, the TLR-expressing cells will be introduced into a suitable container, e.g. 96-well plates, together with the oligonucleotide and appropriate culture medium. Typically, a candidate oligonucleotide will be tested in parallel at different concentrations to obtain a different response to the various concentrations. Typically, one of these concentrations serves as a negative control, i.e., at zero concentration of agent or at a concentration of agent below the limits of assay detection. The order of addition of components, incubation temperature, time of incubation, and other parameters of the assay may be readily determined. Such experimentation merely involves optimization of the assay parameters, not the fundamental composition of the assay. Incubation temperatures typically are between 4° C. and 40° C, more typically about 37° C. Incubation times preferably are minimized to facilitate rapid, high throughput screening, and typically are between 1 minute and 48 hours. A variety of other reagents also can be included in the mixture. These include reagents such as salts, buffers, neutral proteins (e.g., albumin), detergents, etc. which may be used to facilitate optimal protein-protein and/or protein-nucleic acid binding. Such a reagent may also reduce non-specific or background interactions of the reaction components. Other reagents that improve the efficiency of the assay such as protease inhibitors, nuclease inhibitors, antimicrobial agents, and the like may also be used. After incubation (for, e.g., 18-20 hours), the activation (or lack thereof) of the cells can be assessed using any of a large number of potential methods.

Assays for detecting TLR3 activation are described, inter alia, in Salaun et al. (2006) J. Immunol. 176: 4894-4901, PCT publication nos. WO 03/31573, WO 04/053057, WO 04/053452, and WO 04/094671; the disclosures of each of which are herein incorporated in their entireties.

**Activation of cells.** In another embodiment, the level of activation of immune or other cells is measured following incubation of the cells with the dsRNA polymer compositions. "Active" or "activated" cells can also be identified by any other property or activity known in the art as associated with the particular cell type, such as cytokine (e.g. IP-10, RANTES, MCP-1 or IL-6.) production (discussed herein) or increases in free intracellular calcium levels. In one example, the cells are isolated following incubation and the level of expression of a cell activation marker (e.g. CD69) can be measured. In one embodiment, activation is measured in the culture medium following incubation of PBMC, or isolated T cells, B cells, NK cells or dendritic cells, or optionally patient derived cells, with the dsRNA polymer compositions. Such assays can also be used in therapeutic or prophylactic methods, where cells are be removed from a patient (e.g. a patient having a cancer), and the ability to induce activation of PBMC or isolated T cells, B cells, NK cells, dendritic cells, or using the present dsRNA polymer composition is assessed. Detection that cells' activation level can be increased indicates that the patient is a suitable candidate for therapeutic or prophylactic methods involving the administration of a TLR3 agonist, particularly the dsRNA polymer composition. It has been determined that tumor cells, including non-immune tumor cells, can produce cytokines in response to treatment with a TLR3 agonist of the invention, such that methods of detection activation of TLR3 in tumor cells can be assessed using similar methods (e.g. detection of cytokines).

**Cytokine production.** In a preferred embodiment, the level of TLR3-responsive cytokines is measured in the culture medium following incubation of the cells (e.g. PBMC, DCs, NK cells, epithelial cells, endothelial cells, tumor cells, or in other cell types) with the dsRNA polymer compositions. For example, the supernatant can be isolated following incubation and the level of a cytokine such as type I IFN, IFNgamma, IL-6, IP-10, and/or MCP-1 (or any other suitable cytokine known to be induced as a result of TLR3 signaling) can be determined using, e.g., sandwich ELISA. In one embodiment, TLR3-responsive cytokines is measured in the culture medium following incubation of the cells (e.g. PBMC or T cells, B cells, NK cells), dendritic cells, optionally patient derived PBMC), tumor cells, with the dsRNA polymer compositions. In another embodiment, TLR3-responsive cytokines is measured in the culture medium following incubation of tumor cells, optionally patient derived tumor cells, with the dsRNA polymer compositions. In another embodiment, the level of TLR3-responsive cytokines can be measured in biological samples (e.g. blood) obtained from an individual (e.g. a mammal, mouse, non-human primate) following administering the dsRNA polymer composition to an individual. Such assays can also be used in therapeutic or prophylactic methods, where cells are be removed from a patient (e.g. a patient having a cancer), and the ability to induce TLR3-responsive cytokines in cells, particularly cancer cells, TLR3-expressing cancer cells, PBMC (e.g. T cells, B cells, NK cells), dendritic cells, or using the present dsRNA polymer composition is assessed. In addition to use in determining whether a candidate dsRNA is a TLR3 agonist, detection that TLR3-responsive cytokines can be induced in cells indicates that the patient is a suitable candidate for therapeutic or prophylactic methods involving the administration of a TLR3 agonist, particularly the dsRNA polymer composition.

In one example, a TLR3 selective agonist of the invention will have similar potency or difference in potency (e.g. expressed as the EC50) of no more than 3-$log_{10}$, 2-$log_{10}$, 1-$log_{10}$ in induction of TLR3 signaling compared to a polyIC composition, as determined by the ability to induce TLR3 signaling in a reporter gene assay. The TLR3 selective agonist will preferably also have reduced potency or reduction in potency (e.g. expressed as the EC50) of at least 1-$log_{10}$, 2-$log_{10}$ or 3-$log_{10}$ in induction of cytokine production, optionally of IP-10, interferon-gamma or a type I interferon (e.g. interferon alpha), compared to said polyIC composition, as determined by the ability to induce cytokines in human PBMC in vitro. The TLR3 selective agonist may also have lower levels of maximum cytokine production than a polyIC composition, particularly for an interferon. In one embodiment, the polyIC composition is a commercial polyIC (e.g. tlrl-pic, Invivogen Corp, or a polyIC having an Mn of about 1130 Kda, an Mw of about 1700 kDa, an Ip of about 1.5 and/or an endotoxin content of about 0.48, or less than 1.0).

**Apoptosis assays**. In another preferred embodiment, pro-apoptotic activity of a dsRNA polymer composition is assessed. This can be observed by determining the number of cells remaining or killed following incubation with dsRNA. Pro-apoptotic activity can also be measured by observing level of indicators of induction of apoptosis following incubation of the cells with the dsRNA polymer compositions. For example, the supernatant can be isolated following incubation and the level of an apoptosis-induced protease or other protein (e.g. a caspase, Bc1-2 family member, p53, Anti-Fas (CD95/Apo-1) can be determined. Assays that measure DNA fragmentation (e.g. DNA ladder kits) can be used. Assays for cytotoxicity can also be used, for example assays that measure plasma membrane leakage (DNA fragmentation kits) or assays that measure metabolic activity (cell proliferation kits). Individual cells can be observed, e.g. using the TUNEL enzymatic labeling assays or membrane alternations in cells can be measured, e.g. using Annexin staining kits. The dsRNA can be assayed for pro-apoptotic activity on any suitable cell, including for example any TLR3 expressing cell, any tumor cell, optionally a tumor cell obtained from a patient having a cancer, an epithelial cell, an endothelial cell, a T cell, a B cell, an NK cell or dendritic cell, a host cell made to express a TLR3 polypeptide. Such assays can also be integrated into therapeutic or prophylactic methods, where cells are be removed from a patient (e.g. a patient having a cancer), and the ability to induce the apoptosis of cancer cells, particularly TLR3-expressing cancer cells, using the present dsRNA polymer composition is assessed. In addition to use in determining whether a candidate dsRNA is a TLR3 agonist, detection that apoptosis can be induced in cells indicates that the patient is a suitable candidate for therapeutic or prophylactic methods involving the administration of a TLR3 agonist, particularly the dsRNA polymer composition.

**TLR3 expression**. In one example, assessing TLR3 agonist activity can involve, for example, detecting the ability of a dsRNA polymer composition to induce or increase expression of TLR3 in a cell. Salaun ct al. (2007) report that type I interferons may induce TLR3 expression. dsRNA polymer compositions may also be used to induce the expression of TLR3 in cells (e.g. tumor cells) Thus, in one embodiment, cells originating from a patient (e.g. a patient having a cancer) are used to assess that ability of the dsRNA polymer composition to stimulate the expression of TLR3 in the cells. If the dsRNA causes an increase in the expression of TLR3 in a cell, the dsRNA can be selected as a TLR3 agonist. In a preferred embodiment, such cells are any tumor cell, optionally a tumor cell obtained from a patient having a cancer, an epithelial cell (optionally carcinoma cells arising therefrom), an endothelial cell, a T cell, a B cell, an NK cell or dendritic cell. Cells can therefore be removed from a patient (e.g. a patient having a cancer), and the ability to stimulate the expression of TLR3 using the present dsRNA polymer composition is assessed. Such assays can also be integrated into therapeutic or prophylactic methods, where cells are removed from a patient (e.g. a patient having a cancer), and the ability to induce or increase TLR3 expression cancer cells, using the present dsRNA polymer composition, is assessed. In addition to use in determining whether a candidate dsRNA is a TLR3 agonist, detection that TLR3 expression can be induced or increased in cells indicates that the patient is a suitable candidate for therapeutic or prophylactic methods involving the administration of a TLR3 agonist, particularly the dsRNA polymer composition.

**Neutralization by blocking TLR3.** One method to assess the activity and selectivity of a TLR3 agonist composition is to determine whether a TLR3 blocking agent will neutralize its biological activity in a particular assay. The antibody named TLR3.7 (catalog ref. 16-9039, eBiosciences, San Diego, CA) reported to be blocking antibody can be used, for example in a PBMC assay to block a TLR3 receptor, whereupon the TLR3 receptor is contacted with a candidate polyAU (or other dsRNA) composition, and the biological activity induced by polyAU is assessed. If the dsRNA retains biological activity this indicates that the composition may have a non-TLR3-specific activity. In another example, it will be possible to inhibit TLR3 signaling by treating a cell with a shRNA that inhibits TRIF as exemplified in the Examples section herein where in order to assess whether the biological response to polyAU is mediated by receptors other than TLR3 in tumor cells, shRNA were used to inhibit TLR3 in HCC38 cells.

**Signal transduction.** TLR3 stimulation can be assessed using any of a number of possible readout systems, most based upon a TLR/IL-1R signal transduction pathway, involving, e.g., the MyD88-independent/TRIF dependent signal transduction pathway, involving, e.g., IRF3, IRF7, IKKε and/or TBK1 (Akira and Takeda (2004) Nature Review Immunol. 4:499-511). These pathways activate kinases including KB kinase complex. TLR3 activation can be assessed by examining any aspect of TLR signaling. For example, activation of TLR signaling triggers alterations in protein-protein associations (e.g., TRIF with TBK and/or IKKε), in intracellular localization of proteins (such as movement of NK-kB into the nucleus), and in gene expression (e.g., in expression of NK-kB sensitive genes), and cytokine production (e.g., production and secretion of IFNgamma, IL-6, IP10, MCP-1). Any such alteration can be detected and used to detect TLR3 activation. In a particularly preferred embodiment, TLR3 stimulation is detected by collecting supernatants after 18-20 hr of culture and measuring levels of IFNgamma, IL-6, IP-10 and/or MCP-1 by sandwich ELISA. In another preferred embodiment, TLR3 stimulation is detected by collecting supernatants after 18-20 hr of culture and measuring levels of IFNgamma, IL-6, IP-10 and/or MCP-1 by sandwich ELISA.

In one embodiment, cells are used that contain a reporter construct that causes the expression of a detectable gene product upon TLR3 stimulation and consequent activation of the signal transduction pathway. Reporter genes and reporter gene constructs particularly useful for the assays include, e.g., a reporter gene operatively linked to a promoter sensitive to NF-kB or to signaling mediated by, particularly TRIF, IRF3, IRF7, IKKε, TBK1. Examples of such promoters include, without limitation, those for IL-1alpha, IL-6, IL-8, IL-12 p40, Tip-10, CD80, CD86, and TNF-alpha. The reporter gene operatively linked to the TLR-sensitive promoter can include, without limitation, an enzyme (e.g., luciferase, alkaline phosphatase, beta-galactosidase, chloramphenicol acetyltransferase (CAT), etc.), a bioluminescence marker (e.g., green-fluorescent protein (GFP, e.g., U.S. Pat. No. 5,491,084), blue fluorescent protein (BFP, e.g., U.S. Pat. No. 6,486,382), etc.), a surface-expressed molecule (e.g., CD25, CD80, CD86), and a secreted molecule (e.g., IL-1, IL-6, IL-8, IL-12 p40, TNF-alpha). See, e.g., Hcker H et al. (1999) EMBO J. 18:6973-82; Murphy TL et al. (1995) Mol Cell Biol 15:5258-67, the disclosures of which are herein incorporated by reference. Reporter plasmids suitable for use are commercially available (InvivoGen, San Diego, CA). In one embodiment, the assay includes determining, in a host cell made to express a human TLR3 polypeptide, whether a test composition induces luciferase expression (or other reporter) under the control of a promoter responsive to TLR3 signalling (e.g. ISRE, IFN-stimulated response element). In one example, a TLR3 agonist of the invention will have similar potency, or a reduction in potency (e.g. expressed as the EC50) of no more than 3-$\log_{10}$, 2-$\log_{10}$, 1-$\log_{10}$ in induction of TLR3 signaling compared to a polyIC composition, as determined by the ability to induce TLR3 signaling in a reporter gene assay, optionally in an ISRE-luciferase reporter gene assay in transfected human 293T cells expressing human TLR3. In one embodiment, the polyIC composition is a commercial polyIC (e.g. tlrl-pic, Invivogen Corp, or a polyIC having an Mn of about 1130 Kda, an Mw of about 1700 kDa, an Ip of about 1.5 and/or an endotoxin content of about 0.48, or less than 1.0).

In assays relying on enzyme activity readout, substrate can be supplied as part of the assay, and detection can involve measurement of chemoluminescence, fluorescence, color development, incorporation of radioactive label, drug resistance, optical density, or other marker of enzyme activity. For assays relying on surface expression of a molecule, detection can be accomplished using flow cytometry (FACS) analysis or functional assays. Secreted molecules can be assayed using enzyme-linked immunosorbent assay (ELISA) or bioassays. Many of these and other suitable readout systems are well known in the art and are commercially available. Preferably, the reporter system, whichever used, is quantifiable.

DsRNA polymer compositions are said to be stimulating if they induce any detectable alteration in the marker used to assess TLR3-mediated activity. For example, the dsRNA polymer compositions can cause an alteration in the marker expression, activity, phosphorylation, secretion, etc., of 5%, 10%, 20%, 30%, 40%, 50%, 60%, 70%, 80%, 90%, 100%, 150%, 200%, 300%, 400%, 500%, 1000%, or greater. Optionally, such alternation can be relative to another dsRNA polymer composition, another polyAU composition, etc.

*Assaying the compositions to stimulate non-TLR pattern recognition receptors*

Using mice deficient in MDA5, Kato et al. (2006) Nature 441: 101-105 showed that MDA5 (OMIM reference 606951) and RIGI (OMIM reference 609631) recognize different types of double-stranded RNAs: MDA5 recognizes polyinosine-polycytidylic acid and RIGI detects in vitro transcribed double-stranded RNAs. RNA viruses are also differentially recognized by RIGI and MDA5. Kato et al. (2006) found that RIGI is essential for the production of interferons in response to RNA viruses including paramyxoviruses, influenza virus, and Japanese encephalitis virus, whereas MDA5 is critical for picornavirus detection. "MDA5" refers to Melanoma Differentiation-Associated Gene 5, a 1,025-amino acid protein containing an N-terminal CARD motif and a C-terminal DExH/D RNA helicase domain closely related to that of RIGI. The cDNA for MDA5 was reported in Kang et al, Proc. Nat. Acad. Sci. 99: 637-642, 2002, the disclosure of which is incorporated herein by reference. "RIG-I", "DEAD/H BOX 58", and "DDX58", used interchangeably, refers to Retinoid Acid Inducible Gene I, is an RNA helicase that belongs to the DEAD/H box family, for which the cDNA was provided in Imaizumi et al. (2004) Life Sci. 75: 1171-1180, the disclosure of which is incorporated herein by reference. Another dsRNA receptor is interferon-induced, double-stranded RNA-activated protein kinase (PRKR or PKR), a serine-threonine kinase of molecular mass 68,000 and 65,000 Da in human and mouse cells, respectively. Activation by dsRNAs leads to autophosphorylation of PKR and allows the kinase to phosphorylate its natural substrate, the alpha subunit of eukaryotic protein synthesis initiation factor-2 (EIF2-alpha), leading to the inhibition of protein synthesis. The nucleotide sequence of the human PKR gene is described in Kuhen et al. (1996) Gene 178: 191-193, 1996, the disclosure of which is incorporated by reference.

The dsRNA polymer compositions can be assessed in vitro for their ability to bind to or act as an agonist of a dsRNA receptor other than TLR3, for example PKR, RIG-I and/or MDA5, another Toll-like receptor (e.g. TLR4), or another receptor for a pathogen-associated molecular pattern (PAMP), and in particular any dsRNA receptor known to have a role in induction of apoptosis or production of cytokines. Additionally, a dsRNA composition may involve not only signaling through non-TLR3 receptors mediated by the dsRNA, but also signaling mediated by non-dsRNA components present in the composition; for example TLR4 recognizes endotoxins (e.g. LPS), and a composition comprising endotoxins may be expected to have TLR4 activity. An endotoxin content of no more than about 1 EU/mg will generally signal a composition which does not have significant TLR4 signaling as a result of endotoxin content. Non-TLR3 receptor (e.g. TLR7, TLR8, RIG-I and/or MDA5) agonist activity can advantageously be assessed in cells made to express the relevant receptor (e.g. TLR7, TLR8, RIG-I and/or MDA5), in cell types known to express the receptor or in other cell types using any of a variety of assays, or in any other cell-free assay where receptor agonist activity can be assessed. Receptor binding can be assessed using any of a range of available assays, for example detecting binding (e.g. on Biacore as demonstrated herein) of a dsRNA composition to the receptor (e.g. RIG-I and/or MDA5) polypeptides immobilized on a solid support. Such assays can be used, *inter alia,* for testing derivatives of the herein-provided compositions or for assessing novel sequences and compositions designed according to the teachings of the present specification for their ability to stimulate RIG-I and/or MDA5. Such assays can also be used to identify other modulators of TLR3-expressing cells, e.g., using the herein-described dsRNA polymer compositions as standards or controls. In vitro stimulation of DCs, tumor cells or NK cells are also useful, e.g., for evaluating DCs, tumor cells, or NK cells or other TLR3-expressing cells from an individual.

Assessing TLR7, TLR8, RIG-I and/or MDA5 agonist activity can involve, for example, detecting any increase in cellular activity, detected in vitro or in vivo, including for example presence of activation markers on T cells, B cells, NK cells, dendritic cells, the production of cytokines, upregulation of RIG-I and/or MDA5, proliferation of cells (e.g. T cells, B cells, NK cells, dendritic cells), pro-apoptotic activity, or RIG-I and/or MDA5 signaling.

The present in vitro assays can be performed either with isolated cells that naturally express TLR7, TLR8, RIG-I and/or MDA5, or with cells that do not normally express TLR7, TLR8, RIG-I and/or MDA5 but into which expression constructs encoding TLR7, TLR8, RIG-I and/or MDA5 have been introduced. In one embodiment the cell naturally expresses functional TLR7, TLR8, RIG-I and/or MDA5 and is, e.g., a dendritic cell type; an NK cell, a CD4+ T cell, a CD8+ T cells, an endothelial cell, a keratinocyte or a cancer cell.

Exemplary assays for TLR7, TLR8, RIG-I and/or MDA5 activity are provided in the Examples section. In one embodiment, the assay includes determining, in a host cell made to express a human TLR3 polypeptide, whether a test composition induces luciferase expression (or other reporter) under the control of a promoter responsive to TLR3 signalling (e.g. ISRE, IFN-stimulated response element). In one example, a TLR3 selective agonist of the invention will have greater potency (e.g. expressed as a lower EC50 value) in induction of TLR3 signaling compared to potency in induction of signaling by another dsRNA receptor, particularly by RIG-1 and/or MDA5, as determined by the ability to induce receptor signaling in a reporter gene assay. In one example, a TLR3 selective agonist of the invention will have (i) similar potency or reduction in potency (e.g. expressed as a similar or higher EC50 value) of no more than 3-$\log_{10}$, 2-$\log_{10}$, 1-$\log_{10}$ difference in induction of TLR3 signaling compared to a polyIC composition, as determined by the ability to induce TLR3 signaling in a reporter gene assay, and (ii) reduced potency, or reduction in potency (e.g. expressed as the EC50) of at least 1-$\log_{10}$, 2-$\log_{10}$ or 3-$\log_{10}$ in induction of non-TLR3 (e.g. dsRNA receptor, RIG-1 and/or MDA5) signaling, compared to said polyIC composition, as determined by the ability to induce receptor signaling in a reporter gene assay. In one embodiment, the polyIC composition is a commercial polyIC (e.g. tlrl-pic, Invivogen Corp, or a polyIC having an Mn of about 1130 Kda, an Mw of about 1700 kDa, an Ip of about 1.5 and/or an endotoxin content of about 0.48, or less than 1.0).

*Screening methods*

In another aspect , methods are disclosed for identifying novel TLR3 agonists. As mentioned above, binding to TLR3 polypeptides, binding to cells expressing TLR3 polypeptides (e.g. host cells made to express TLR3 or naturally expressing TLR3), or functional assays, e.g., the above-described apoptosis, reporter gene or cytokine production assays can be used as a readout in order to assay standard or lead compounds in, in order to assess the ability of test compounds to bind TLR3 or affect its activity. For example, compounds of any type can be screened in the assays in order to identify other compounds that have equal or greater binding affinity or ability to modulate TLR3 activity than the specific compositions described herein. In addition, the compositions described herein can be modified in various ways in order to identify variants with enhanced binding or TLR3 agonist activities or with, e.g., improved specificity, reduced side effects, or enhanced biochemical, pharmacokinetic, or pharmacodynamic properties.

It will be appreciated that any general procedure for screening assays and/or production of agents can be envisaged. In one example, a method screening for a compound can comprise: (a) providing a test compound, (b) contacting a TLR3 polypeptide, preferably a cell expressing a TLR3 polypeptide, with said test compound; and (c) determining whether the test compound induces a TLR3 activity. Optionally the method further comprises (d) selecting a compound that induces a TLR3 activity. Optionally the method further comprises determining whether the test compound induces an activity known to be mediated by a non-TLR3 polypeptide, optionally cytokine production (e.g. type I interferon, IL-6, IP-10), and further optionally selecting a compound that has decreased or does not substantially induce said activity known to be mediated by a non-TLR3 polypeptide.

In another example, a method screening for a compound can comprise: (a) contacting a cell expressing a TLR3 polypeptide with said test compound; and (b) determining whether the test compound induces apoptosis of a cell; and optionally (c) wherein a determination that the test compound induces apoptosis of the cell indicates that the test compound is useful as a therapeutic or prophylactic, e.g. for the treatment of a tumor. The cell can be for example a tumor cell, a cell made to express TLR3, or a cell (e.g. tumor cell) known to express or be capable of expressing TLR3 (e.g. carcinoma, arising from an epithelial cell type). When the test compound is a selective TLR3 agonist and the agonist induces apoptosis of a tumor cell, the tumor may be identified as a TLR3-responsive disease state. The method for can further comprise: (a) contacting a TLR3 polypeptide with a test compound and determining whether the test compound binds TLR3; (b) determining whether the test compound induces apoptosis of the cell; and optionally (c) wherein a determination that the test compound binds TLR3 and induces apoptosis of the cell indicates that the test compound is useful as a therapeutic or prophylactic.

In another example, provided is a method of screening comprises: (a) contacting a TLR3 polypeptide with a TLR3 ligand in the presence of a test compound; (b) contacting a TLR3 polypeptide with a TLR3 ligand in the absence of said test compound; and (c) comparing the binding of TLR3 ligand to said polypeptide in steps a) and b); wherein a detection that said binding differs between steps a) and b) indicates that said test compound is a candidate TLR3 modulator. In one embodiment, the binding of said TLR3 ligand (e.g. a polyIC, a polyAU composition of the invention) to said TLR3 polypeptide is lower in the presence of said compound than it is in the absence of the compound.

In another example, provided is a method of screening or producing an agent, suitable for use in the treatment or prevention of a disorder, said method comprising: i) providing a TLR3-expressing cell and a plurality of test agents; ii) testing the ability of each of said test agents to bind and/or induce TLR3 activity in said cells; iii) selecting an agent from said plurality that bind and/or induces TLR3 activity, iv) optionally, rendering said agent suitable for human administration; and v) optionally, producing a quantity of said agent.

As described herein, the assays may comprise determining whether the test compound selectively binds to TLR3 and/or induces TLR3 activity compared to binding of induction of activity at one or more other dsRNA or ssRNA receptors (e.g. TLR7, TLR8, MDA5 and/or RIGI), and in one embodiment the compound has or is selected to have selectivity for TLR3 over one or more other dsRNA or ssRNA receptors (e.g. TLR7, TLR8, MDA5 and/or RIGI). A composition having selectivity for TLR3 may have increased binding and/or induction of activity at TLR3 compared to another dsRNA or ssRNA receptor, or may have decreased binding and/or induction of activity at another dsRNA receptor than a reference TLR3 agonist/binding compound (e.g. polyIC which has activity at TLR3 as well as other dsRNA and ssRNA receptors).

In one embodiment, disclosed is a method for characterizing, screening or selecting a composition, comprising:

a) bringing a test agent into contact with a TLR3 polypeptide and determining whether the agent binds to TLR3 and/or induces TLR3 activity;

b) bringing a test agent into contact with a dsRNA or ssRNA receptor polypeptide, for example a TLR7, TLR8, RIG-I and/or MDA5 polypeptide, and determining whether the agent binds to said dsRNA polypeptide and/or is induces an activity mediated by said dsRNA receptor polypeptide (e.g. induces dsRNA receptor polypeptide-mediated signalling); and

c) selecting an agent that binds to TLR3 and/or induces TLR3 activity but has decreased or substantially lacks binding to and/or induction of activity of said dsRNA or ssRNA receptor polypeptide.

In any of the assays, or methods of producing an agent, the method can further comprise selecting the candidate compound identified in one of the screening steps for use as a medicament (e.g. if a test compound bind and/or inhibits TLR3, or if it inhibits cell trafficking, etc.). Optionally, any of the methods can further comprise further comprising making the candidate compound suitable for administration to a human (e.g. formulating the compound for use as a medicament, humanizing an antibody, etc.) and/or preparing the quantity of the candidate compound for administration to a human.
In any of the screening methods, a determination that the test compound binds TLR3, induces TLR3-mediated signalling and/or induces apoptosis of the TLR3-expressing cell indicates that the test compound is useful as a therapeutic or prophylactic. In any of the screening methods, the methods can further comprise optionally, rendering said agent suitable for human administration; and/or optionally, producing and/or formulating for human use a quantity of said agent.
In one example, the test agent is a dsRNA composition according to the invention. Preferably the composition is a polyAU composition. Agonist activity can be determined according to the desired parameters. For example a compound can be selected to have TLR3 agonist activity when presenting a 50-250 fold increase in e.g. gene reporter luciferase assay at a concentration of less than 100μg/ml. A compound can be identified as having TLR7, TLR8, RIG-I and/or MDA5 agonist activity when presenting a 2-300 fold increase in a gene reporter luciferase assay at a concentration of less than 100μg/ml.

*Compositions*

Disclosed are compositions comprising one or more of the dsRNA polymer composition of this invention and an acceptable carrier (a "pharmaceutical composition"). Pharmaceutically acceptable solutions typically contain pharmaceutically acceptable concentrations of salt, buffering agents, preservatives, compatible carriers, adjuvants, and optionally other therapeutic ingredients. Pharmaceutically acceptable carriers, adjuvants and vehicles that may be used in the pharmaceutical compositions useful in this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.
For use in therapy, an effective amount of the compound can be administered to a subject by any mode allowing the compound to be taken up by the appropriate target cells, e.g., epithelial cells, tumor cells, DCs, NK cells, T cells. "Administering" the pharmaceutical composition of the present invention can be accomplished by any means known to the skilled artisan. The compositions of the present invention may be administered orally, parenterally, by inhalation spray, topically, transdermally, rectally, nasally, buccally, sublingually, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. An injection can be in a bolus or a continuous infusion. Various methods of preparing and administering therapeutic agents are well known in the art and arc taught, e.g., in Remington's Pharmaceutical Sciences" 15th Edition, the entire disclosure of which is herein incorporated by reference.
The pharmaceutical compositions are preferably prepared and administered in dose units. Such preparative methods include the step of bringing into association with the molecule to be administered ingredients such as the carrier that constitutes one or more accessory ingredients. In general, the compositions are prepared by uniformly and intimately bringing into association the active ingredients with liquid carriers, liposomes or finely divided solid carriers or both, and then if necessary shaping the product.
Liquid dose units are vials or ampoules for injection or other parenteral administration. Solid dose units are tablets, capsules, powders, and suppositories. For treatment of a patient, depending on activity of the compound, manner of administration, purpose of the administration (i.e., prophylactic or therapeutic), nature and severity of the disorder, age and body weight of the patient, different doses may be necessary. The administration of a given dose can be carried out both by single administration in the form of an individual dose unit or else several smaller dose units. Repeated and multiple administration of doses at specific intervals of days, weeks, or months apart are also contemplated by the invention. Effective doses for humans are believed to range from about 10 picomole/kg to about 1000 micromole/kg, or from about 0.1 mg/kg to about 100 mg/kg.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Such injection solutions may be in the form, for example, of a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant such as Ph. Helv or a similar alcohol.

Topical administration of the pharmaceutical compositions of this invention is especially useful when the desired treatment involves areas or organs readily accessible by topical application. For application topically to the skin, the pharmaceutical composition will be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions of this invention may also be topically applied to the lower intestinal tract by rectal suppository formulation or in a suitable enema formulation. Topically-transdermal patches and iontophoretic administration are also included in this invention.

The compositions can be administered per se (neat) or in the form of a pharmaceutically acceptable salt. When used in medicine the salts should be pharmaceutically acceptable, but non-pharmaceutically acceptable salts can conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

Other delivery systems can include time-release, delayed release or sustained release delivery systems (collectively referred to herein as "implantable drug release devices"). Such systems can avoid repeated administrations of the compounds, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109. Delivery systems also include non-polymer systems that are: lipids including sterols such as cholesterol, cholesterol esters and fatty acids or neutral fats such as mono-di-and tri-glycerides; hydrogel release systems; silastic systems; peptide based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Pat. Nos. 4,452,775, 4,675,189, and 5,736,152, and (b) diffusional systems in which an active component permeates at a controlled rate from a polymer such as described in U.S. Pat. Nos. 3,854,480, 5,133,974 and 5,407,686. In addition, pump-based hardware delivery systems can be used, some of which are adapted for implantation.

Disclosed is also a method of impregnating or filling an implantable drug release device comprising the step of contacting said drug release device with a TLR3 agonist dsRNA polymer composition of this invention.

Disclosed is also an implantable drug release device impregnated with or containing a TLR3 agonist dsRNA polymer composition of this invention, such that said TLR3 agonist is released from said device and is therapeutically active.

The present TLR3 agonist dsRNA polymer composition can also be administered (or used in vitro) along with other compounds designed to enhance their ability to reach or enter cells, to increase their stability in vivo, or for other purposes. In a preferred such embodiment, the oligonucleotides are complexed with a cationic compound such as polyethylenimine (PEI), which binds to and compacts nucleic acids, protecting them from degradation and facilitating their uptake into cells (see, e.g., Boussif et al. (1995) PNAS 92: 7297; Godbey (1999) PNAS 96: 5177). It will be appreciated that, while PEI is preferred, other compaction agents or cationic substances can also be used.

Compaction agents also can be used alone, or in combination with, a biological or chemical/physical vector. A "compaction agent", as used herein, refers to an agent, such as a histone, that neutralizes the negative charges on the nucleic acid and thereby permits compaction of the nucleic acid into a fine granule. Compaction of the nucleic acid facilitates the uptake of the nucleic acid by the target cell. The compaction agents can be used alone, i.e., to deliver a nucleic acid in a form that is more efficiently taken up by the cell or, more preferably, in combination with one or more of the above-described vectors.

In other embodiments, the TLR3 agonist dsRNA polymer composition is complexed with liposomes. Liposomes are useful, inter alia, in that they can be targeted to a particular tissue by coupling the liposome to a specific ligand such as a monoclonal antibody, sugar, glycolipid, or protein. Ligands which may be useful for targeting a liposome to an immune cell include, but are not limited to: intact or fragments of molecules which interact with immune cell specific receptors and molecules, such as antibodies, which interact with the cell surface markers of immune cells. Such ligands may easily be identified by binding assays well known to those of skill in the art.

Liposomes fall into two broad classes. Cationic liposomes are positively charged liposomes which interact with the negatively charged RNA molecules to form a stable complex. The positively charged RNA/liposome complex binds to the negatively charged cell surface and is internalized in an endosome. Due to the acidic pH within the endosome, the liposomes are ruptured, releasing their contents into the cell cytoplasm (Wang et at., Biochem. Biophys. Res. Commun., 1987, 147, 980-985).

Liposomes which are pH-sensitive or negatively-charged, entrap ssRNA rather than complex with it. Since both the ssRNA and the lipid are similarly charged, repulsion rather than complex formation occurs. The ssRNA is thus entrapped in the aqueous interior of these liposomes. pH-sensitive liposomes have been used, for example, to deliver ssRNA encoding the thymidine kinase gene to cell monolayers in culture (Zhou et al., Journal of Controlled Release, 1992, 19, 269-274).

One major type of liposomal composition includes phospholipids other than naturally-derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Liposomes that include nucleic acids have been described, for example, in Thierry et al., WO 96/40062 (methods for encapsulating high molecular weight nucleic acids in liposomes); Tagawa et al., U.S. Pat. No. 5,264,221 (protein-bonded liposomes containing RNA); Rahman et al., U.S. Pat. No. 5,665,710 (methods of encapsulating oligodeoxynucleotides in liposomes); Love et al., WO 97/04787 (liposomes that include antisense oligonucleotides).

Another type of liposome, transfersomes are highly deformable lipid aggregates which are attractive for drug delivery vehicles. (Cevc et al., 1998, Biochim Biophys Acta. 1368(2): 201-15.) Transfersomes may be described as lipid droplets which are so highly deformable that they can penetrate through pores which are smaller than the droplet. Transfersomes are adaptable to the environment in which they are used, for example, they are shape adaptive, self-repairing, frequently reach their targets without fragmenting, and often self-loading. Transfersomes can be made, for example, by adding surface edge-activators, usually surfactants, to a standard liposomal composition.

In other embodiments, the TLR3 agonist dsRNA polymer composition is complexed with dendrimers. Dendrimers are useful, inter alia, in that they can be targeted to a particular site through their specific interactions. Dendrimers are perfectly structured molecules with large numbers of cascade branched units emanating from a focal point, resulting in densely packed and groups at the molecular surface (Zhou J et al, 2006; Wu J et al, 2005). Being chemically synthesized, dendrimers can be tailored, through control of size, shape and surface chemistry, to obtain the most efficient properties for delivery to a biological medium. Various dendrimers are suitable for dsRNA transfer comprising but not limited to polyamidoamine (PAMAM), polylsine or poly(propylene imine) (PPI).

Lipid formulations for transfection are commercially available from QIAGEN, for example, as EFFECTENE™. (a non-liposomal lipid with a special DNA condensing enhancer) and SUPERFECT™ (a novel acting dendrimeric technology). Liposomes are commercially available from Gibco BRL, for example, as LIPOFECTIN™ and LIPOFECTACE™, which are formed of cationic lipids such as N-[1-(2,3 dioleyloxy)-propyl]-N,N,N-trimethylammonium chloride (DOTMA), DOTAP and dimethyl dioctadccylammonium bromide (DDAB). Another example is Lipofectamine™ Reagent (Invitrogen Corp., Carlsbad, CA) is suitable for the transfection of DNA into eukaryotic cells (1), and is a 3:1 (w/w) liposome formulation of the polycationic lipid 2,3-diolcyloxy-N-[2(sperminecarboxamido)ethyl]-N,Ndimethyl-1-propanaminium trifluoroacetate (DOSPA) and the neutral lipid dioleoyl phosphatidylethanolamine (DOPE) in membrane-filtered water. Methods for making liposomes are well known in the art and have been described in many publications. Liposomes also have been reviewed by, inter alia, Gregoriadis (1985) Trends Biotechnol 3:235-241, which disclosure is incorporated herein by reference.

The compositions of the invention may comprise other agents useful for the treatment or prevention of the relevant condition, e.g., cancer or infection such as viral infection.

In one embodiment, the TLR3 agonist dsRNA polymer composition of this invention is formulated in a composition together with an antigen (e.g. a vaccine). The antigen may be present in the composition as a discrete component or, alternatively, conjugated to the dsRNA polymer to form a complex. In a complex, the two agents may be either covalently bonded or conjugated directly to one other or attached via a linker or tether moiety. In a preferred embodiment, the antigen is a viral antigen, a cancer antigen or an allergen. In one embodiment, the antigen or vaccine comprise one or a plurality of purified antigens, e.g. recombinantly produced polypeptide antigen(s). Such composition is used to stimulate an antigen-specific response against a disease or condition characterized by that antigen. As used herein, the term "viral antigen" includes, but is not limited to, intact, attenuated or killed whole virus, any structural or functional viral protein, or any peptide portion of a viral protein of sufficient length (typically about 8 amino acids or longer) to be antigenic. As used herein, the terms "cancer antigen" and "tumor antigen" are used interchangeably and refer to antigens that are differentially expressed by cancer cells and can thereby be exploited in order to target cancer cells. Cancer antigens are antigens which can potentially stimulate apparently tumor-specific immune responses. Some of these antigens are encoded, although not necessarily expressed, by normal cells. These antigens can be characterized as those which are normally silent (i.e., not expressed) in normal cells, those that are expressed only at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), fusion proteins resulting from internal deletions or chromosomal translocations. Still other cancer antigens can be encoded by viral genes such as those carried on RNA and DNA tumor viruses. A cancer antigen as used herein is a compound, such as a peptide, protein, or glycoprotein, which is associated with a tumor or cancer cell surface and which is capable of provoking an immune response when expressed on the surface of an antigen-presenting cell in the context of a major histocompatibility complex (MHC) molecule. Cancer antigens can be prepared from cancer cells either by preparing crude extracts of cancer cells, for example, as described in Cohen P A et al. (1994) Cancer Res 54:1055-8, by partially purifying the antigens, by recombinant technology, or by de novo synthesis of known antigens. Cancer antigens include but are not limited to antigens that are recombinantly expressed, an immunogenic portion of, or a whole tumor or cancer or cell thereof. Such antigens can be isolated or prepared recombinantly or by any other means known in the art.

In another embodiment, the dsRNA polymer composition of this invention is formulated in a composition together with another therapeutic agent useful in the treatment of cancer or infectious disease (e.g. viral infection). For example, a number of therapeutic agents are available for the treatment of cancers and infections. The antibody compositions and methods of the present invention may be combined with any other methods generally employed in the treatment of the particular disease, particularly a tumor, cancer disease, infectious disease or other disease or disorder that the patient exhibits. So long as a particular therapeutic approach is not known to be detrimental to the patient's condition in itself, and does not significantly counteract the activity of the dsRNA composition in a pharmaceutical composition of this invention, its combination with the present invention is contemplated. Examples of combinations are described in PCT patent publication no. WO2006/054177 (Andre F. et al.), the disclosure of which is incorporated herein by reference.

The interrelationship of dosages for animals and humans (based on milligrams per meter squared of body surface) is described in Freireich et al., (1966) Cancer Chemother Rep 50: 219. Body surface area may be approximately determined from height and weight of the patient. See, e.g., Scientific Tables, Geigy Pharmaceuticals, Ardley, N.Y., 1970, 537. An effective amount of a compound of this invention can range from about 0.001 mg/kg to about 1000 mg/kg, more preferably 0.01 mg/kg to about 100 mg/kg, more preferably 0.1 mg/kg to about 10 mg/kg; or any range in which the low end of the range is any amount between 0.001 mg/kg and 100 mg/kg and the upper end of the range is any amount between 0.1 mg/kg and 1000 mg/kg (e.g., 0.005 mg/kg and 50 mg/kg, 0.5 mg/kg and 100 mg/kg). Effective doses will also vary, as recognized by those skilled in the art, depending on the diseases treated, route of administration, excipient usage, and the possibility of co-usage with other therapeutic treatments such as use of other agents.

For pharmaceutical compositions that comprise additional therapeutic agents, an effective amount of the additional therapeutic agent is between about 20% and 100% of the dosage normally utilized in a monotherapy regime using just that additional agent. Preferably, an effective amount is between about 70% and 100% of the normal monotherapeutic dose. The normal monotherapeutic dosages of these additional therapeutic agents are well known in the art. See, e.g., Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), each of which references are entirely incorporated herein by reference.

In yet another embodiment, the disclosure provides a composition of matter comprising a TLR3 agonist dsRNA polymer composition and another agent selected from: a therapeutic agent useful in the treatment of cancer (e.g. a type I interferon, as further discussed herein), a therapeutic agent useful in the treatment of infectious disease, a cancer antigen, a viral antigen or an allergen; in separate dosage forms, but associated with one another. The term "associated with one another" as used herein means that the separate dosage forms are packaged together or otherwise attached to one another such that it is readily apparent that the separate dosage forms are intended to be sold and administered as part of the same regimen. The agent and the TLR3 agonist dsRNA polymer composition are preferably packaged together in a blister pack or other multi-chamber package, or as connected, separately sealed containers (such as foil pouches or the like) that can be separated by the user (e.g., by tearing on score lines between the two containers). In still another embodiment, the disclosure provides a kit comprising in separate vessels, a) a TLR3 agonist dsRNA polymer composition of this invention; and b) another agent selected from: a therapeutic agent useful in the treatment of cancer, a therapeutic agent useful in the treatment of infectious disease, a cancer antigen, a viral antigen or an allergen.

*Methods of Treatment*

In numerous embodiments of the present invention, the TLR3 agonist compositions of the invention will be administered in a therapeutically or prophylactically effective amount to a subject in order to achieve a specific outcome. The TLR3 agonist compositions of the invention can be administered as a single agent or in combination with other therapeutic or prophylactic agents. The subject may have or be susceptible to any condition for which the treatment or prevention is effective including but not limited to the conditions described herein. In another embodiment, the condition is any condition in which TLR3 agonism is beneficial. In another embodiment, the condition is a TLR3-responsive disorder, and the patient is treated with a selective TLR3 agonist. Determining which conditions are TLR3-responsive can be determined using the methods and assays described herein. Accordingly, the present invention provides methods of using the herein-described TLR3 agonist polyAU polymer composition for immunostimulation or induction of apoptosis, useful in the treatment or prevention of disorders where an enhanced immune response mediated by TLR3, or apoptosis of TLR3-expressing cells is useful and/or required, such as cancer or infectious disease, e.g., viral infection. Such methods comprise the step of administering to a patient a composition comprising a TLR3 agonist dsRNA polymer composition of this invention. It will be appreciated that the present TLR3 agonist dsRNA polymer composition can be used to treat or prevent any condition that can be beneficially affected by enhanced DC activity, T cell activity, NK cell activity, enhanced monocyte activity, by the elimination of any TLR3-expressing cells in diseases where TLR3 agonist results in apoptosis of the TLR3 expressing cell (e.g. tumor cells), or by the inhibition of angiogenesis.

As discussed, specific human dosage ranges suitable for the administration of the high-molecular weight polyAU compositions are disclosed herein, derived from in vivo experimentation in mice. Preferably, the dsRNA composition is administered in a human subject at a dose comprised between 0.01 and 100 mg/kg, preferably between 0.1 and 50 mg/kg, more preferably between 0.5 and 20 mg/kg, even more preferably between 1 and 10 mg/kg, or about 1, 2, 3, 4 or 5 mg/kg. The present application shows that polyAU compositions with increased potency at TLR3 are not toxic, and can be administered in human equivalent doses of more than 60 mg, 75 mg or 100 mg per patient, whether per day or per week.

It has recently been reported that dsRNA may have a beneficial effect on a range of tumors, by directly inducing apoptotic cell death of tumor cells expressing TLR3, or by favoring primary and memory CD8 T cell responses and antitumor immunity. For example, Salaun et al. (2006) J. Immunol. 176: 4894-4901 report that TLR3 ligands can cause apoptotic death of cancer cells. TLR3 protein can be expressed in human melanoma cells, where it can deliver proapoptotic and antiproliferative signaling (Salaun et al. (2007) Clin. Cancer Res. 13: 4565-74).

Several other reports have focused on the role of TLR3 in inducing antitumor and antiviral immunity. TLR3 signalling augments cross-presentation by dendritic cells (DCs) and leads to upregulation of co-stimulatory molecules and production of immunomodulatory cytokines. TLR3 signalling also augments cross-presentation of cell-associated antigens, playing an important role in regulating CD8 T cell responses to proteins that are not expressed by antigen-presenting cells (APCs). Dendritic cells, which express TLR3, are the principal cross-presenting APCs in vivo. Schultz et al. (2005) Nature 433: 887-892 report that TLR3 promotes cross-priming during viral infections, and that murine CD8alpha dendritic cells are activated by double-stranded dsRNA present in virally infected cells but absent from uninfected cells. They report that DC activation requires phagocytosis of infected material, followed by signalling through the dsRNA receptor, toll-like receptor 3 (TLR3), and that immunization with virus-infected cells or cells containing synthetic dsRNA leads to a striking increase in CTL crosspriming against cell-associated antigens, which is largely dependent on TLR3 expression by antigen-presenting cells. In another example, Salem et al. (2005) J. Immunother. 28(3):220-8 reported enhanced primary and memory CD8 T-cell responses and antitumor immunity mediated by TLR3 when a peptide tumor vaccine was administered in combination with polyIC. In addition, administration of poly(I:C) enhanced the response to a nonimmunogenic self-antigen in a dendritic cell vaccine-based vaccine strategy. Cui et al. (2006) Cancer Immunol. Immunother. 55:1267-79 provide that a polyIC serves as a potent peptide vaccine adjuvant and augments therapeutic activity in human cervical cancer xenografts in mice.

It view of the wide range of cells that express at least low amounts of TLR3, it is anticipated that any of a large number of types of cancer can be treated or prevented using the present TLR3 agonist dsRNA polymer compositions. Examples of cancer types or proliferative diseases that can be treated include carcinoma, including that of the bladder, breast, colon, kidney, liver, lung, ovary, prostate, pancreas, stomach, cervix, thyroid and skin, including squamous cell carcinoma; hematopoietic tumors of lymphoid lineage, including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma and Burketts lymphoma; hematopoietic tumors of myeloid lineage, including acute and chronic myelogenous leukemias and promyelocytic leukemia; tumors of mesenchymal origin, including fibrosarcoma and rhabdomyoscarcoma; other tumors, including melanoma, seminoma, teratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscaroma, and osteosarcoma; and other tumors, including melanoma, xeroderma pigmentosum, keratoacanthoma, seminoma, thyroid follicular cancer and teratocarcinoma.

In one embodiment, a patient can be sensitized to treatment with a TLR3 agonist polyAU polymer composition of the invention. The engagement of TLR3 by TLR3 agonists can directly inhibit cell proliferation and induce tumor cell death, particularly melanoma when treated in combination with either type I IFN or a protein synthesis inhibitor. Thus, in one embodiment, a patient (e.g. a patient having a tumor, optionally a melanoma, malignant melanoma), is administered a type I interferon or protein synthesis inhibitor in combination with a TLR3 agonist of the invention. The type I interferon can be administered in a manner suitable so as to upregulate TLR3 expression. For example the type I interferon is administered sufficiently prior to administration of the TLR3 agonist dsRNA of the invention in order to sensitize tumor cells to apoptosis induce by the TLR3 agonist dsRNA polymer composition. The type I interferon may be any suitable composition; examples include but are not limited to any human leukocyte interferon, interferon alpha (IFN-A), Intron-A™ interferon alpha 2b (Schering-Plough Corp.), Roferon-A™ interferon alpha-2a (Roche), Pegasys™ peginterferon alpha-2a (Roche). The interferon may be administered for example according to manufacturers' instructions, according to any known treatment regimen, including but not limited to low dose treatment regimens. An exemplary low dose regimen is 1-3 MU, optionally 2 MU, or optionally less than 1 MU of Intron-A™. This method does not require a prior selection step is required to select or identify patients whose tumors demonstrate expression TLR3.

It is demonstrated herein that the TLR3 agonist compositions of the invention, including notably selective TLR3 agonist compositions, are capable of inducing the expression of TLR3 polypeptides. In one embodiment of the disclosure, a patient (e.g. a patient having a tumor, optionally a melanoma, malignant melanoma, an ocular angiogenesis disease), is administered a TLR3 agonist dsRNA polymer in an amount effective to induce TLR3 expression in a cell (e.g. an immune cell, a tumor cell, an endothelial cell), followed by a second administration of a TLR3 agonist dsRNA polymer composition. The second administration will generally be within about 72 hours, 48 hours or 24 hours following the first administration of a TLR3 agonist dsRNA polymer. Optionally said second administration of a TLR3 agonist dsRNA is in an amount effective to induce apoptosis of a tumor cell expressing TLR3 and/or an amount effective to induce cytokine production in a cell (e.g. an immune cell, a tumor cell, an endothelial cell) cell expressing TLR3.

It has recently been found (see PCT patent publication no. WO2006/054177 (Andre F. et al.)) that certain classes of patients with cancer treated with dsRNA exhibit greater survival than other patients. One class of patients in which enhanced survival was found were patients having tumors that strongly expressed TLR3 protein. Thus, in one embodiment, the dsRNA polymer compositions are useful for the treatment of such a TLR3-expressing tumor.

Determining whether tumor types express TLR3 can be carried out, e.g. by detecting the presence of one or more TLR3 polypeptides in a biological sample from a cancer patient, generally from a tumor biopsy. Alternatively, when tumors (e.g. types or subtypes, or tumors having been treated according to a particular manner) are known to express TLR3, or known to be capable of upregulating TLR3 (e.g. upon treatment with a TLR3 agonist or type I interferon), or are known to be a TLR3-responsive disease state, a TLR3 detection step is not necessary, and it will be recognized that such tumors will usually have sensitivity to the dsRNA polymer compositions of the invention. Cells are known to upregulate TLR3 in response to type I interferon, and it will be appreciated that interferons can be used in therapy in combination with the TLR3 agonists of the invention is a range of other indications (e.g. cancer, viral infection) as well. For example Tissari et al. (2005) J. Immunol. 174: 4289-4294 report that IFN-$\alpha$-inducedup-regulation of TLR3 expression is involved in dsRNA activated antiviral response in human epithelial and endothelial cells. In specific embodiments, a diagnostic assay can be performed on a tumor sample from a patient to determine whether the tumor sample comprises TLR3-expressing cells. Such assays are described herein; for example antibody-based immunohistochemistry assays can be used advantageously. In one example, a tumor biopsy is performed, yielding a biological sample. A determination that said biological sample comprises TLR3 expressing cells indicates that the patient can benefit from the TLR3 agonist administration. The patient is then treated with the TLR3 agonist. Preferably, the step of determining whether cancer cells in said subject express a TLR3 receptor is performed on a tumoral sample derived from a patient. For example, the sample can be a biopsy of the patient's tumor, a cell or tissue culture, etc. Such sample can be obtained by conventional methods. In a particular embodiment, the sample is obtained by non-invasive methods and/or from tissue collections. Therefore, in one embodiment of the methods and uses according to the present invention, the step of determining whether cancer cells in said subject express a TLR3 receptor comprises: providing a tumoral sample from the patient and detecting the expression of a TLR3. The expression of a TLR3 may be detected at the nucleic acid level or at the polypeptide level. Various techniques known in the art may be used to detect or quantify TLR3, including sequencing, hybridization, amplification and/or binding to specific ligands (such as antibodies). Suitable methods include Southern blot (for DNAs), Northern blot (for RNAs), fluorescent in situ hybridization (FISH), gel migration, ELISA, radio-immunoassays (RIA) and immuno-enzymatic assays (IEMA). Some of these approaches are particularly suited for assessing a polypeptide sequence or expression level, such as Northern blot, ELISA and RIA. These latter require the use of a ligand specific for the polypeptide, more preferably of a specific antibody. Different types of ligands may be used, such as specific antibodies. In a specific embodiment, the sample is contacted with an antibody specific for a TLR3 polypeptide and the formation of an immune complex is determined. Various methods for detecting an immune complex can be used, such as ELISA, radioimmunoassays (RIA) and immuno-enzymatic assays (IEMA).

In an alternative embodiment, the expression of a TLR3 receptor in said cancer cell is determined using a TLR3-specific primer or probe. Such primer or probes are designed to specifically hybridize with a TLR3 gene, under suitable hybridization conditions, thereby allowing detection of a gene or RNA coding for TLR3. A particular embodiment comprises contacting a tumor sample from the patient with a TLR3-specific primer or probe, and determining the existence of a hybrid or amplification product. The presence (or amount) of TLR3 mRNA in a sample can provide an indication as to the expression of said receptor. Such determination may be accomplished by various techniques known in the art, including through RT-PCR. To that purpose, total RNA is isolated from cancer cells using commercially available kits, such as the RNeasy Mini kit (Qiagen, Valencia, CA). DNase I-treated total RNA (3 $\mu$g) is reverse-transcribed by using random primers with RNaseH-free reverse transcriptase (Invitrogen, San Diego, CA). TLR3 can be amplified using specific primers described below. TLR3 (5'-CTCAGAAGATTACCAGCCGCC-3' (SEQ ID No 3) /5'-CCATTATGAGACAGATCTAATG-3' (SEQ ID No 4)) (see US2003/0165479, the disclosure of which is incorporated herein by reference).

In yet another embodiment, the expression of a TLR3 receptor in a cell (e.g. cancer cell) is determined using a TLR3-selective dsRNA composition of the invention, optionally conjugated to a detectable moiety. A particular embodiment comprises contacting a tumor sample from the patient with a TLR3-selective dsRNA composition of the invention, and determining whether the dsRNA composition binds a TLR3 within the tumor sample. The binding of TLR3-selective dsRNA composition in a sample can provide an indication as to the expression of TLR3.

Prior to determining expression of TLR3, the sample may be treated to improve availability of TLR3 nucleic acids or polypeptides. Such treatment may include, for instance, a lysis of the cells or tissue (e.g., mechanical, enzymatic or physical) or treatment with a TLR3 agonist composition (e.g. a dsRNA polymer composition of the invention) to induce TLR3 expression.

The invention also discloses a diagnostic kit comprising a dsRNA polymer composition of the invention for assessing in a tumoral sample from a subject the expression or induction of expression of a TLR3 gene or polypeptide.

If it determined that a tumor expresses TLR3, can be induced to express TLR3 or is otherwise sensitive to treatment with a TLR3 agonist, the dsRNA polymer composition of the invention can be used to treat the tumor, treat the patient having such tumor, or otherwise eliminate the tumor cell, in vitro or in vivo. Alternatively, in any embodiment for the treatment of cancer herein, a method can be TLR3-expression level independent; optionally any of the methods can specifically exclude a step of detecting TLR3 expression or overexpression in tumor sample prior to treatment with a TLR3 agonist.

In another embodiment for treating cancer, a sample of TLR3-expressing cells (e.g. tumor cells, epithelial cells, NK cells, T cells, DCs) is obtained from the patient prior to the administration of the a dsRNA polymer composition, and the ability of the dsRNA polymer compositions to activate the cells will be assessed on a portion of that sample. Following the assessment of activation potential, the patient's cell can be activated in vivo, in which the a dsRNA polymer composition (in an appropriate pharmaceutical formulation) is directly administered to the patient, or in vitro, where the a dsRNA polymer composition of the invention is brought into contact with the patient's cells and the so-treated cells are administered to the patient. The activation can be assessed using any of the methods described supra, e.g. cytokine production, TLR signaling induced gene expression, affect on the proliferation of other cells, etc. In such embodiments, a detection that the TLR-expressing cells are active is an indication that the dsRNA polymer composition is having the desired effect.

When cancer is being treated or prevented using the present polyAU polymer composition, in another embodiment the method of the present invention comprises the additional step of administering to said patient another anti-cancer compound or subjecting the patient to another therapeutic approach. For solid tumor treatment, for example, the administration of a composition of the present invention may be used in combination with classical approaches, such as surgery, radiotherapy, chemotherapy, and the like. The invention therefore provides combined therapies in which the present polyAU polymer compositions are used simultaneously with, before, or after surgery or radiation treatment; or are administered to patients with, before, or after conventional chemotherapeutic, radiotherapeutic or anti-angiogenic agents, or targeted immunotoxins or coaguligands. In a preferred aspect, the polyAU polymer compositions are administered in combination with a type I IFN (e.g. interferon alpha (IFN-A), Intron-A, Schering-Plough Corp.) or protein synthesis inhibitor, optionally wherein the type I IFN or protein synthesis inhibitor is administered prior to administration of the polyAU polymer composition, optionally within 72 hours, 48 hours or 24 hours of, or simultaneously with, administration of the polyAU polymer composition. The type I interferon or protein synthesis inhibitor can be administered in a manner suitable so as to upregulate TLR3 expression.

When infection (e.g. viral infection) is being treated or prevented using the present polyAU polymer composition, the method of the present invention can comprise the additional step of administering to said patient another compound for use in treating or preventing an infection, or subjecting the patient to another therapeutic or prophylactic approach. As discussed, it has been reported that IFN-$\alpha$-induced up-regulation of TLR3 expression is involved in dsRNA activated antiviral response in human epithelial and endothelial cells. Thus, in a preferred aspect, the dsRNA polymer compositions are administered in combination with a type I IFN (e.g. interferon alpha (IFN-A), Intron-A, Schering-Plough Corp.) or protein synthesis inhibitor, optionally wherein the type I IFN or protein synthesis inhibitor is administered prior to administration of the polyAU polymer composition, optionally within 72 hours, 48 hours or 24 hours of, or simultaneously with, administration of the polyAU polymer composition. The type I interferon or protein synthesis inhibitor can be administered in a manner suitable so as to upregulate TLR3 expression.

When the polyAU polymer compositions are administered to a patient with another agent, the two components may be administered either as separately formulated compositions (i.e., as a multiple dosage form), or as a single composition (such as the combination single dosage forms described above containing a polyAU polymer composition of this invention and another therapeutic agent). The present polyAU polymer compositions can also be used in combination with cancer antigens, for the treatment or prevention of cancers. Such methods typically comprise administering to said subject a cancer antigen or viral antigen conjointly with the polyAU polymer composition. The choice of cancer or viral antigen can be made from the cancer or viral antigens set forth herein as useful in combination with polyAU polymer compositions of the present invention.

In one embodiment, the polyAU polymer compositions of this invention is administered in combination with, and/or formulated in a composition together with, an antigen. The antigen may be present in the composition as a discrete component or, alternatively, conjugated to a polyAU polymer to form a complex. In a complex, the two agents may be either covalently bonded or conjugated directly to one other or attached via a linker or tether moiety. In a preferred embodiment, the antigen is a viral antigen or a cancer antigen. Such composition is used to stimulate an antigen-specific response against a disease or condition characterized by that antigen. The compositions can therefore be used to treat an existing condition or to prevent the condition from arising or recurring. The term "antigen" refers to any molecule capable of being recognized by a T-cell antigen receptor or B-cell antigen receptor. The term broadly includes any type of molecule which is recognized by a host immune system as being foreign. Generally, many vaccine compositions will have such properties and will be encompassed by the term "antigen". Antigens generally include but are not limited to cells, cell extracts, proteins, polypeptides, peptides, polysaccharides, polysaccharide conjugates, peptide and non-peptide mimics of polysaccharides and other molecules, small molecules, lipids, glycolipids, polysaccharides, carbohydrates, viruses and viral extracts, and multicellular organisms such as parasites, and allergens. With respect to antigens that are proteins, polypeptides, or peptides, such antigens can include nucleic acid molecules (e.g. viral vectors) encoding such antigens. In one embodiment, the antigen is one for which efficacy requires or is enhanced by augmenting antigen cross presentation by APC, for example most purified peptide and protein antigens, as well as nucleic acid molecules encoding such antigens.

Sources of a viral antigen include, but are not limited to viruses from the families: Retroviridae (e.g., human immunodeficiency viruses, such as HIV-1 (also referred to as HTLV-III, LAV or HTLV-III/LAV, or HIV-III; and other isolates, such as HIV-LP; Picornaviridae (e.g., polio viruses, hepatitis A virus; enteroviruses, human Coxsackie viruses, rhinoviruses, echoviruses); Calciviridae (e.g., strains that cause gastroenteritis); Togaviridae (e.g., equine encephalitis viruses, rubella viruses); Flaviviridae (e.g., dengue viruses, encephalitis viruses, yellow fever viruses); Coronaviridae (e.g., coronaviruses); Rhabdoviridae (e.g., vesicular stomatitis viruses, rabies viruses); Filoviridae (e.g., ebola viruses); Paramyxoviridae (e.g., parainfluenza viruses, mumps virus, measles virus, respiratory syncytial virus); Orthomyxoviridae (e.g., influenza viruses); Bunyaviridae (e.g., Hantaan viruses, bunya viruses, phleboviruses and Nairo viruses); Arenaviridae (hemorrhagic fever viruses); Reoviridae (e.g., reoviruses, orbiviruses and rotaviruses); Bornaviridae; Hepadnaviridae (Hepatitis B virus); Parvoviridae (parvoviruses); Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus; Poxyiridae (variola viruses, vaccinia viruses, pox viruses); and Iridoviridae (e.g., African swine fever virus); and unclassified viruses (e.g., the agent of delta hepatitis (thought to be a defective satellite of hepatitis B virus), Hepatitis C; Norwalk and related viruses, and astroviruses). Alternatively, a viral antigen may be produced recombinantly.

Examples of tumor antigens include MAGE, MART-1/Melan-A, gp100, dipeptidyl peptidase IV (DPPIV), adenosine deaminase-binding protein (ADAbp), cyclophilin b, colorectal associated antigen (CRC)-C017-1A/GA733, carcinoembryonic antigen (CEA) and its immunogenic epitopes CAP-1 and CAP-2, etv6, aml1, prostate specific antigen (PSA) and its immunogenic epitopes PSA-1, PSA-2, and PSA-3, prostate-specific membrane antigen (PSMA), T-cell receptor/CD3-zeta chain, MAGE-family of tumor antigens (e.g., MAGE-A1, MAGE-A2, MAGE-A3, MAGE-A4, MAGE-A5, MAGE-A6, MAGE-A7, MAGE-A8, MAGE-A9, MAGE-A10, MAGE-A1, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, VEGF, VEGF receptors, A-Raf, B-Raf, C-Raf, Raf-1, HSP70, HSP90, PDGF, TGF-alpha, EGF, EGF receptor, a member of the human EGF-like receptor family such as HER-2/neu, HER-3, HER-4 or a heterodimeric receptor comprised of at least one HER subunit, gastrin releasing peptide receptor antigen, Muc-1, CA125, αvß3 integrins, α5ß1 integrins, αIIbß3-integrins, CTLA-4, CD20, CD22, CD30, CD33, CD52, CD56, CD80, PDGF beta receptor, Src, VE-cadherin, IL-8, hCG, IL-6, IL-6 receptor, IL-15, p21ras, RCAS1, α-fetoprotein, E-cadherin, α-catenin, ß-catenin and γ-catenin, p120ctn, gp100.sup.Pme1117, PRAME, NY-ESO-1, cdc27, adenomatous polyposis coli protein (APC), fodrin, Connexin 37, Ig-idiotype, p15, gp75, GM2 and GD2 gangliosides, viral products such as human papillomavirus proteins, Smad family of tumor antigens, imp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2, or any additional protein target. This list is not meant to be limiting.

The present invention also provides compositions for use in treating or preventing an infectious disease in a subject, particularly treating or preventing a viral infection, comprising the step of administering to said patient a polyAU polymer composition of this invention. A subject having an infectious disease is a subject that has been exposed to an infectious organism and has acute or chronic detectable levels of the organism in the body. Exposure to the infectious organism generally occurs with the external surface of the subject, e.g., skin, eye or mucosal membranes and/or refers to the penetration of the external surface of the subject by the infectious organism. In one embodiment, the subject has an ocular viral infection, and the dsRNA polymer composition of this invention is administered (or formulated for such administration) for local application to eye tissues, for example administered to the subconjunctive layer of the eye. In one embodiment, the subject has a viral conjunctivitis caused by an adenovirus or herpes virus. In addition to viral diseases, the present polyAU polymer compositions can also be used to defend against other types of infectious agents, including bacteria, prions, fungi, and various parasites. See, e.g. C. G. A Thomas, Medical Microbiology, Bailliere Tindall, Great Britain 1983, the entire disclosure of which is herein incorporated by reference. A subject requiring prevention of a viral infection is a subject who is a candidate for a vaccination against a viral disease. For certain viral diseases, such a subject is a neonate, infant or adolescent. For other viral diseases, the subject is immunocompromised. For other viral diseases, the subject is any member or the population. As with cancer, the methods of the invention can comprise the addition step of administering to said subject another agent useful for the treatment of infection. Infection medicaments include but are not limited to anti-bacterial agents, anti-viral agents, anti-fungal agents and anti-parasitic agents. When the method of this invention is designed to prevent viral infection, that method typically comprises the additional step of administering to said subject a viral antigen. The choice of viral antigen can be made from the same viral antigens set forth above as useful in combination with polyAU polymer compositions of the present invention.

In one embodiment of the invention where another therapeutic agent is administered to an animal, the effective amount of the compound of this invention is less than its effective amount would be where the other therapeutic agent is not administered. In another embodiment, the effective amount of the conventional agent is less than its effective amount would be where the compound of this invention is not administered. In this way, undesired side effects associated with high doses of either agent may be minimized. Other potential advantages (including without limitation improved dosing regimens and/or reduced drug cost) will be apparent to those of skill in the art.

In another aspect, the compositions of the invention can be used for the treatment of an ocular angiogensis disorder. Wet macular degeneration occurs when abnormal blood vessels grow behind the macula. These vessels are fragile and can leak fluid and blood, which result in scarring of the macula and raise the potential for rapid, severe damage. Bruch's membrane breaks down, usually near drusen deposits. This is where new blood vessel growth, or neovascularization, occurs. Central vision can become distorted or lost entirely in a short period of time, sometimes within days. Recent reports indicate that TLR3 may mediate anti-angiogenic effects, and that TLR3 agonism reduced blinding choriodal neovascularization in a mouse model of disease relating to age-related macular degeneration (Kleinman et al. (2008) Nature and WO 2007/133800). Generally any of TLR3 agonist of the invention can be used for the treatment or prevention of said condition. The method will involve generally inhibiting ocular angiogenesis by exposing a retinal or choroidal cell to a TLR3 agonist described herein. When such exposition is in vivo, the TLR3 agonist will generally be administered locally to the eye, for example by intravitreal administration.

**Specific modes of administration**

The present application discloses that certain administration regimens are advantageous for treatment of subjects with TLR3 agonists, particularly selective TLR3 agonists. In one set of examples it is shown that TLR3 agonist dsRNA, including notably selective TLR3 dsRNA compositions induce the expression of TLR3 in cells. It is also shown that such expression is greatest within the first 72, 48 and preferably 24 hours following treatment with the TLR3 agonist. The disclosure therefore provides repeated administration of polyAU compositions of the invention, wherein two successive administrations of the polyAU compositions are spaced apart by no more than 72, 48 or 24 hours.

In another series of experiments, it is shown that when tumor cells sensitive to TLR3-mediated apoptosis are implanted in a NOD-SCID mouse, a two-step treatment regimen comprising (i) administering to a subject a composition that induces the expression of a TLR3 polypeptide in a cell, and (ii) administering to the subject a TLR3 agonist. The TLR3 agonist is administered to the subject at a period of time when cells (e.g. tumor cells) in the patient express TLR3. The two step of therapy will generally be spaced apart by no more than 72, 48 or 24 hours. The composition of step (i) will be any composition capable of inducing TLR3 expression in a cell (e.g. type I interferon, dsRNA, polyAU of the present invention, polyIC, viral dsRNA), and the composition of step (ii) will be any composition capable of inducing TLR3-mediated apoptosis in cell (e.g. dsRNA, polyAU of the present invention, polyIC, viral dsRNA). Insofar as the dsRNA TLR3 agonist is capable of both inducing TLR3 expression and TLR3-mediated apoptosis, the composition of step (i) and step (ii) may be a dsRNA TLR3 agonist, optionally a TLR3 agonist of the invention. Optionally, expression of TLR3 by tumor cells from a subject may be assessed following the administration of step (i), preferably within 72 hours, 48 hours or 24 hours following the administration of step (i), and optionally further if tumor cells are determined to express TLR3, the patient is treated according to step (ii).

Such methods may have use in treatment of a wide range of tumors, including but not limited to carcinomas, or for example which have not been assessed for their expression of TLR3.

The inventors also demonstrate that the two administrations described above in steps (i) and (ii) have increased efficacy when separated by as few days as possible (e.g. administered more often than once weekly). Thus, the two-step treatment regimen may comprise (i) administering to a subject a composition that induces the expression of a TLR3 polypeptide in a cell, and (ii) within about 72 hours, about 48 hours or about 24 hours of said administration of step (i), administering to the subject a TLR3 agonist. Results showed that regimens using two or five doses per week, separated by about 2 intervening days (e.g. no more than about 72 hours between first and second administration), were more efficacious than once-weekly administration, for delaying tumor growth, particularly by a TLR3-mediated, pro-apoptotic mechanism, which correlated with TLR3 expression at 72 hours following induction by a TLR3 expression inducing composition, which expression was gradually decreasing from 24 hours onwards.

The disclosure therefore provides methods for preventing, managing, treating or ameliorating disease, particularly cancer, said methods comprising administering to a subject in need thereof one or more doses of a prophylactically or therapeutically amount of a TLR3 agonist dsRNA composition, preferably a dsRNA according to the invention. Preferably, the dsRNA is a polyAU composition of the invention, preferably a composition capable of inducing TLR3 expression in a cell and/or capable of inducing apoptosis in a TLR3-expressing cell, and preferably a selective TLR3 agonist of the invention. In one example, the dsRNA is administered by injection (e.g. subcutaneous) or intravenous (i.v.) administration. The administration of a dose can be split into one or more administrations, but will generally take place within the course of one day, for example an injection or an infusion having a duration of 2 to 180 min (e.g. 10 to about 30 min).

In one embodiment, administration of the TLR3 agonist occurs on the first day of a treatment cycle. Preferably a second dose of the TLR3 agonist is administered on a second day within 3 days (e.g. within about 72 hours) of said first day; optionally 3, 4, 5, 6 or 7 doses of the TLR3 agonist are administered, such that a patient is administered the dsRNA compositions on 3, 4, 5, 6 or 7 days within a week of said first day. Preferably a successive administration is separated from the preceding administration by no more than about 72 hours, 48 hours or 24 hours. Optionally, any 2, 3, 4, 5, 6 or 7 of the administrations are on consecutive days. In one exemplary mode of administration, the administration of the TLR3 agonist occurs on days 1 and 4, or days 1, 2, 3, 4 and 5 of a one-weekly treatment cycle. The treatment cycle is repeated at least 2, 3, 4, 5 or 6 times. Optionally, a second agent is administered in combination with the TLR3 agonist; the one example, a type I interferon is administered on at least one or two days within the week starting on the first day of administration of the dsRNA. For example, the administration of the TLR3 agonist occurs on days 1 and 4, or days 1, 2, 3, 4 and 5 and IFN-alpha is administered on day 0 (the day before the first TLR3 agonist treatment on day 1) or on day 0 and day 3.

In one embodiment, the treatment cycle is a 1-weekly to 4-weekly cycle (that is, an about 7 day repeating cycle). In a preferred embodiment, a TLR3 agonist is administered on the first day of the 2-weekly to 8-weekly cycle (that is, an about 14 day to 56 day repeating cycle). As mentioned, a subject will preferably be treated for at least two cycles, or more preferably for at least three cycle. In other aspect, treatment may continue for a greater number of cycles, for example at least 4, 5, 6 or more cycles can be envisioned. At the end of each cycle, the cycle of dosing may be repeated for as long as clinically tolerated and the tumor is under control or until tumor regression.

As discussed, specific dosage ranges suitable for the administration of the dsRNA compositions are disclosed herein, derived from in vivo experimentation in murine tumor models. Preferably, the dsRNA composition is administered in a human subject at a dose comprised between 0.01 and 100 mg/kg, preferably between 0.1 and 50 mg/kg, more preferably between 0.5 and 20 mg/kg, even more preferably between 1 and 10 mg/kg, or about 1, 2, 3, 4 or 5 mg/kg. The present disclosure shows that a significantly higher effect is obtained when the dsRNA composition is administered in more than one administration per week, where administrations are separated by no more than about 72 hours, and that best results were obtained when the dsRNA compositions were obtained on five consecutive days per week.

In one aspect the present disclosure relates especially to the treatment of a disease, especially a tumor, characterized in that a TLR3 agonist dsRNA composition is administered more than once per week, to a human in a dose that is calculated according to the formula (A):

$$(\text{single dose (mg/kg)}=(0.1 \text{ to } 50)) * d\ ) * w \qquad (A)$$

where d is the number of days of treatment, optionally consecutive or non-consecutive, within one week, and where w is the number of weeks of treatment. More preferably, the treatment dose is calculated according to the formula B,

$$(\text{single dose (mg/kg)}=(0.5 \text{ to } 20)) * d\ ) * w \qquad (B)$$

or according to the formula C,

$$(\text{single dose (mg/kg)}=(\text{less than } 1, \text{ or about } 1, 2, 3, 4, 5, 6, 7, 8, 9 \text{ or } 10)) * d\ ) * w \qquad (C)$$

where, in each of formulae A to C, d is about 1 to about 7, preferably about 2 to 5, optionally wherein successive treatments within one week are separated by no more than two intervening days, preferably no more than 72 hours, 48 hours or 24 hours, and where w is the number of weeks of treatment, preferably where w is 2, 3, 4, 5, 6 or greater.

**Detection and targeting of TLR3**

In another embodiment the disclosure provides any of the above-described dsRNA polymer compositions, particularly compositions that selectively bind TLR3, for use in identifying, detecting or targeting TLR3.

In one embodiment, the dsRNA polymer compositions of the invention can be used to deliver a composition to a TLR3-expressing cell (e.g. a tumor cell, an infected cell). For example, the dsRNA polymer compositions of the invention can be associated with a therapeutic agent. The therapeutic agent and dsRNA may be either covalently bonded or conjugated directly to one other or attached via a linker or tether moiety. In one example, the therapeutic agent is a moiety that leads to the elimination of a tumor cell. Examples of such compounds include cytotoxic agents such as a radioactive isotope, a toxic polypeptide, or a toxic small molecule, or polypeptides such as antibodies having the ability to mediate antibody-dependent cytotoxicity (ADCC) or complement-mediated cell cytotoxicity (CDCC). In one embodiment, the therapeutic agent is directly linked to dsRNA polymers.

In another embodiment the disclosure provides any of the above-described dsRNA polymer compositions, particularly compositions that selectively bind TLR3, conjugated to a detectable marker. The term "detectable marker" as used herein refers to any molecule that can be quantitatively or qualitatively observed or measured, in vitro or in vivo.

The dsRNA polymer can be linked to the antibody directly or indirectly, using any of a large number of available methods. For example, an agent can be attached at the hinge region of the reduced antibody component via disulfide bond formation, using cross-linkers such as N-succinyl 3-(2-pyridyldithio)proprionate (SPDP), or via a carbohydrate moiety in the Fc region of the antibody (see, *e.g.,* Yu et al. (1994) Int. J. Cancer 56: 244; Wong, Chemistry of Protein Conjugation and Cross-linking (CRC Press 1991); Upeslacis et al., "Modification of Antibodies by Chemical Methods," in Monoclonal antibodies: principles and applications, Birch et al. (eds.), pages 187-230 (Wiley-Liss, Inc. 1995); Price, "Production and Characterization of Synthetic Peptide-Derived Antibodies," in Monoclonal antibodies: Production, engineering and clinical application, Ritter et al. (eds.), pages 60-84 (Cambridge University Press 1995), Cattel et al. (1989) Chemistry today 7:51-58, Delprino et al. (1993) J. Pharm. Sci 82:699-704; Arpicco et al. (1997) Bioconjugate Chemistry 8:3; Reisfeld et al. (1989) Antibody, Immunicon. Radiopharrn. 2:217. An example of association of a dsRNA with an antibody or polypeptide are also discussed in International patent application no. WO 04/045491 (Yissum), and methods used to prepare a PEI-based carrier can be adapted for use with the dsRNA of the present invention. The entire disclosures of each of the foregoing are herein incorporated by reference.

In one embodiment, dsRNA polymers will be derivatized with a radioactive isotope, such as I-131. Any of a number of suitable radioactive isotopes can be used, including, but not limited to, Indium-111, Lutetium-171, Bismuth-212, Bismuth-213, Astatine-211, Copper-62, Copper-64, Copper-67, Yttrium-90, Iodine-125, Iodine-131, Phosphorus-32, Phosphorus-33, Scandium-47, Silver-111, Gallium-67, Praseodymium-142, Samarium-153, Terbium-161, Dysprosium-166, Holmium-166, Rhenium-186, Rhenium-188, Rhenium-189, Lead-212, Radium-223, Actinium-225, Iron-59, Selenium-75, Arsenic-77, Strontium-89, Molybdenum-99, Rhodium-105, Palladium-109, Praseodymium-143, Promethium-149, Erbium-169, Iridium-194, Gold-198, Gold-199, and Lead-211. In general, the radionuclide preferably has a decay energy in the range of 20 to 6,000 keV, preferably in the ranges 60 to 200 keV for an Auger emitter, 100-2,500 keV for a beta emitter, and 4,000-6,000 keV for an alpha emitter. Also preferred are radionuclides that substantially decay with generation of alpha-particles.

Examples of detectable markers useful in the conjugated oligonucleotides of this invention are radioisotopes, fluorescent dyes, or a member of a complementary binding pair, such as a member of any one of: and antigen/antibody, lectin/carbohydrate; avidin/biotin; receptor/ligand; or molecularly imprinted polymer/print molecule systems. In a related embodiment the invention provides a kit comprising, in separate vessels: a conjugate comprising a dsRNA polymer composition of the invention and a detectable marker; and a TLR3-containing material.

Further aspects and advantages of this invention are disclosed in the following experimental section, which should be regarded as illustrative and not limiting the scope of this application.

## EXAMPLES

### Introduction

The aim of this study is to investigate dsRNA of different types and length obtained using different methods (polymerization of homopolymers using a polynucleotide phophorylase and solid-phase synthesis) and their bioactivity, particularly on human TLR3. Bioactivity was evaluated using the following assays:

- Biacore assay on recombinant huTLR3;
- Gene reporter assay on 293T-TLR3-luciferase;
- Apoptosis induction and cytokine secretion assay on HCC38;
- Endotoxin assay (If some difference in terms of bioactivity is noticed between the dsRNA issued form ssRNA of different length, bioactivity on hematopoïetic cells is further evaluated, provided that endotoxin level is lower than 10 EU /mg);
- Cellular activation and cytokine secretion in vitro on huPBMC;
- Spleen cell activation and cytokine secretion in vivo in B6 mice; and
- Antitumor efficacy in vivo in SCID-NOD mice.

### Materials and methods

### Reagents

*Double-stranded RNA*

Custom RNA oligoribonucleotides were purchased from Dharmacon Inc. (Lafayette, CO). "AU20": 20-mers oligo(A:U) - MW ~13 000 g/mol

5'-AAAAAAAAAAAAAAAAAAAA-3' (SEQ ID NO 5)
5'-UUUUUUUUUUUUUUUUUUUU-3 (SEQ ID NO 6)

19-mers 5'-phosphate-oligo(A:U)

5' -P-AAAAAAAAAAAAAAAAAAA-3' (SEQ ID NO 7)
5'-P- -3 (SEQ ID NO 8)

"AU45": 45-mers oligo(A:U)

5'-AAAAAAAAAAAAAAAAAAAAAAAAAAAAAAAAAAAAAAAAAAAAAAAAAAAAAA-3'(SEQ ID NO 9)
5'-UUUUUUUUUUUUUUUUUUUUUUUUUUUUUUUUUUUUUUUUU-3' (SEQ ID NO 10)

"AxU50": 50-mers oligo(A:U) random sequence - MW ~32 000 g/mol

5' -AAAAUUUUUUAAAAAAAAAUUUUUUUUUUUUAAAUUUUAAAAUUAAUAAU-3' (SEQ ID NO 11)
5'-UUUUAAAAAAUUUUUUUUUAAAAAAAAAAAAUUUAAAAUUUUAAUUAUUA-3' (SEQ ID NO 12)

"AU20-18s-20": 40-mers oligo(A:U) with a 18-atom (hexaethylene glycol) spacer - MW ~26 000 g/mol

5'-AAAAAAAAAAAAAAAAAAAA-18S-AAAAAAAAAAAAAAAAAAAA-3' (SEQ ID NO 13) 5'-UUUUUUUUUUUUUUUUUUUU-18S-UUUUUUUUUUUUUUUUUUUU-3' (SEQ ID NO 14)

The following additional RNA duplexes were prepared from ssRNA purchased from Dharmacon Inc.: 19-mers (A:U), 20-mers (I:C), 45-mers (I:C), 45-mers 5'-Phosphate(A:U) and (I:C). These ssRNA oligos (μg to mg scale) were purified by SDS Page and desalted.

Commercial polyIC was obtained from Invivogen (San Diego, CA, ref #tlrl-pic, batch #28-011-pic, 28-05-pic and 28-08-pic). Powder polyI:C is kept at 4°C. Powder is resuspended in an adequate volume of sterile endotoxin-free NaCl 0.15M, in order to obtain a final concentration of at least 2.5 mg/mL. Solution is kept overnight at 4°C until complete solubilization. Aliquots are distributed in 1.5 mL sterile tubes for long term storage (1 year) at -20°C. The period of validity of thawed polyIC solution is 1 week at 4°C. Commercial polyAU is obtained from Sigma-Aldrich Inc. (St. Louis, MO), product ref. p1537, batch# 25K4004, as a sodium salt. Poludan™ was obtained from Lens Pharma (Russia), comprising 10 mg of dry content per vial, containing about 200 μg of nucleic acid.

PolyA and polyU ssRNA compositions used to prepare the polyAU "Pre-Run", AU690 and polyAU compositions in Example 2 ("Characterization of ssRNA-dsRNA molecular weight relationship") and Example 6 ("Variation of dsRNA annealing protocols") were generated by polymerizing ADP and UDP monomers by incubation with E. Coli polynucleotide polymerase. Powder ssRNA poly A and poly U are stored at -20°C until reconstituted. Powders are resuspended in an adequate volume of endotoxin-free NaCl 0,15M solution, in order to obtain a final concentration of 10 mg/mL if possible. Solution is kept overnight at 4°C until complete solubilization. If necessary, solution is further diluted in endotoxin-free NaCl 0,15M, in order to reach complete solubilization. Specific information for each product is summarized in the table below:

PolyAU "Pre-Run" was obtained by hybridizing a polyA ssRNA composition having an Mw of 588 kDa (IP of 1.492) with the polyU compositions referred to as Uxs, having a Mw of 97.8 kDa, under conditions (b) described in the "Alternative hybridization protocols", involving mixing the polyA and polyU, at the same concentration, at room temperature, and heating for 2 hours at 75 °C and slowly cooled down at room temperature on the bench. This composition (100mg) (Batch # 46K4228) had a Mn of 321.5 kDa.

PolyAU compositions of Example 2 were prepared according to the following protocol: equal volume of polyA and polyU ssRNA at the same concentration (1 to 10mg/ml) in NaCl 0.15M buffer, were mixed at room temperature, heated 5 min at 95 °C and slowly cooled down at room temperature on the bench. Aliquots of hybridized dsRNA are distributed in 1.5 mL sterile tubes for long term storage (1 year) at -20°C.

Pre-hybridized dsRNA: For dsRNA obtained in hybridized form, powder dsRNA poly(A:U) are stored at -20°C until reconstituted. Powders are resuspended in an adequate volume of endotoxin-free NaCl 0,15M solution, in order to obtain a final concentration of 10 or 20 mg/mL. Solution is kept overnight at 4°C until complete solubilization.

Alternative Hybridization protocol for dsRNA used in Example 6: equal volume of polyA ssRNA and polyU ssRNA solutions, at the same concentration (1 or 10mg/ml), are mixed at RT, and annealed according one of the following protocols:

a. polyA and polyU ssRNA at the same concentration (1-10 mg/ml) were mixed at room temperature, heated either 5 min at 95 °C (Am1:Us, the same protocol as compositions in Example 2) or 10 min at 95 °C (Am1:Us-3) and slowly cooled down at room temperature on the bench; or
b. polyA and polyU ssRNA at 5 mg/ml were mixed at room temperature, heated 10 min at 70 °C (for Am1:Us-3) and slowly cooled down at room temperature on the bench. Aliquots of hybridized;

dsRNA are distributed in 1.5 mL sterile tubes for long term storage (1 year) at -20°C.

Interferon-alpha (IntronA™) was purchased from Schering Plough Corp.

*Tumor Cell lines:* All tumor cell lines are purchased from ATCC, including HCC38 (CRL-2314), a primary ductal carcinoma HCC1806 (CRL-2335), a primary acantholytic squamous carcinoma and A3 75 (CRL-1619) malignant melanoma.

*Antibodies (antigen, supplier, reference).* CD3, Beckman Coulter, A07746; CD8, Beckman Coulter, A07757; CD69, Beckman Coulter, IM2656; anti-TLR3 antibody pAb, R&D Systems, ref. AF1487; and CD8β, 5530407; CD69-PE, 553237; CD3, 551163, the latter each from Beckton Dickison.

*Instrumentation.* FACSCalibur™ flow cytometer (BD Biosciences); VmaxTM spectrophotometer (Molecular Devices)

*SEC MALLS.* PolyRNA samples were characterized by size exclusion chromatography (SEC) coupled on line with multiangle laser light scattering (MALLS), quasi elastic light scattering (QELS), and differential refractometer index (DRI). SEC analysis was performed on a HPLC system equipped with a PL Aquagel-OH Mixed 8μm (300 x 7.5mm) column purchased from Polymer Laboratories (Varian SA, France). The mobile phase (0.15 M NaCl solution) was filtered

through 0.1 μm stainless steel filter unit (GSWP 047 Millipore), carefully degassed (Gastorr 154, Flom), and clarified through a 0.45 μm filter unit upstream columns. The column was eluted at a flow rate of 0.5 mL $min^{-1}$ (Intelligent Pump, Flom) with the mobile phase. The sample was filtered beforehand through 0.45 μm filter and injected through a 100 μl full loop (Rheodyne injection valve). The light scattered from each eluted fraction is detected simultaneously at 15 angles (between 15 and 150°) using a Dawn EOS-18 angles photometer from Wyatt Technology Inc (Santa Barbara, USA) fitted with a 50μL K5 cell and a Ga-As 30mW laser source at λ = 690 nm. A QELS detector (Wyatt Technology Inc.) which gives access to the hydrodynamic Radius *(Rh)* of each eluted fraction is connected to photodiode 13 (110° angle) of the MALLS detector. The concentration of each eluted fraction is determined with the DRI detector (ERC 7515A Erma CR Inc., Tokyo, Japan) from the experimental value of the refractive index increment dn/dC. Experimental values of dn/dC are 0.158 $ml.g^{-1}$ for poly(AU), 0.160 $ml.g^{-1}$ for poly(A), 0.176 $ml.g^{-1}$ for polyU and 0.169 $ml.g^{-1}$ for poly(IC). Data acquisition, *Mw* and *Mn* calculations were performed with Astra software v.4.91 from Wyatt Technology Inc using Zimm order 1 calculation method.

*Melting temperature assessments.* Melting temperatures were assessed using the method was based on the "Liautard" method (Liautard et al. (1988) J. Chromatography 454: 195-203), adapted using the parameters as follows.

- $T_m$ = Melting temperature
- Material: Spectrophotometer used was Perkin Elmer Lambda 35 + Peltier / quartz 1 mm cuve
- Buffer: Tris 10 mM NaCl 150 mm pH 7.4
- Product: pApU (e.g. Am1: Us-2 : 8 mg/ml in 1W ($H_2O$+NaCl 150 mM)) diluted at 50 μg/ml in buffer Tris NaCl (1 DO unit approx.)
- The analysis will be performed at 260 nm under an increasing temperature gradient (1°C/min from 30 to 70°C) using UV WinLab and TempLab software.
- Calculation of the $T_m$ (spectrophotometer method): The melting profiles are obtained by plotting the absorbance as function of temperature. The peak height maxima of the first derivative are measured using TempLab software. Tm is expressed in degrees Centigrade.

*(a) Direct Tm measurement from the melting profile (geometric determination):* The melting temperature corresponds to the midpoint of transition where half of the base pairs are dissociated. It is stated that the tangents for the upper and lower parts of the sigmoid curve are never perfectly parallel. A simple geometric construction allows the determination of $T_m$: the bisector line of the acute angle formed by the two tangents crosses the sigmoid curve at the mid-height., The Tm is calculated as follows:

The slope of the bisector line is the mean of the lower and the upper tangent slopes ($a_1$ and $a_2$ respectively):

$$a_3 = \frac{a_2 + a_1}{2} \quad (1)$$

The bisector pass through the lower and the upper tangent intercept and the corresponding abscissa (x) is calculated as follows:

$$x = \frac{b_2 - b_1}{a_1 - a_2} \quad (2)$$

-Using eq. (1) and (2), the coefficient ($b_3$) can be calculated:

$$b_3 = a_1 * \left(\frac{b_2 - b_1}{a_1 - a_2}\right) + b_1 - \left(\frac{a_2 + a_1}{2}\right) * \left(\frac{b_2 - b_1}{a_1 - a_2}\right) \quad (3)$$

The Tm is then determined visually at the intersection between the sigmoid curve and the bisector line.

*(b) Hyperchromicity*

The hyperchromicity is the ratio extrapolated at the melting temperature between the absorbance increase ($A_U$ - $A_L$) and the absorbance of the upper tangent $A_U$ . The hyperchromicity can be calculated as follows:

$$Hyperchromicity = \frac{(a_2 * T_m + b_2) - (a_1 * T_m + b_1)}{a_1 * T_m + b_1} \quad (4).$$

*(c) FWHM.* The full width of half the peak height maxima of the first derivative are measured using TempLab software and expressed in degrees Centigrade.

*Biacore.* For kinetic measurements, various concentrations of the soluble ligand (e.g. dsRNA) (1 x 10-8 to 3x10-10 M) are applied onto the immobilized polypeptides (e.g. TLR3). Measurements are performed at a 40 μl/min continuous flow rate in 10 mM Acetate pH5.6 150 mM NaCl buffer. For each cycle, the surface of the sensor chip is regenerated by 5 μl injection of 10 mM HEPES pH 7.2. The Biacore T100 Evaluation Software is used for data analysis. Blank correction is performed on line by co-injecting the soluble analyte onto the reference dextran flow cell.

*Endotoxin level.* Endotoxin level in each sample is measured using the QCL-1000 Endotoxin Detection Kit following the TDS protocol. The results are expressed in Endotoxin Unit/ml, and can be converted in EU/mg by dividing by the sample concentration, expressed in mg/ml.

*Luciferase reporter assay.* A reporter gene assay using as promoter ISRE (IFN-stimulated response element) and as reporter gene and protein luciferase was set up. Engagement of TLR3 receptor using TLR3-agonists such as poly (I:C) has been reported to activate the type-IFN pathway including the promoter ISRE (Wietek et al. J. Biol. Chem., 278( 51), p50923, 2003). A 293T cell line (ATCC, #CRL-1573) was stably transfected with pISRE-luc plasmid (#219089 - Stratagene), further selected as inducing optimal response to IFN-alha stimulation and referred to as control 293T-ISRE. This cell line was further stably transfected with different plasmids (i) pUNO-hTLR3 plasmid (#puno-htlr3 - InVivogen), (ii) pUNO-hRIG-I plasmid (#puno-hrigi - InVivogen) and (iii) pUNO-hMDA-5 plasmid (#puno-hmda5 - InVivogen), and referred to respectively 293T-TLR3-ISRE, 293T-RIGI-ISRE, 293T-MDA5-ISRE. On day 0, cells are seeded at $4x10^5$ cells/mL in complete culture medium in 96-well culture plate (100μl/well). Cells are first incubated at 37°C for 20 hours, then 50 μL of medium are discarded and cells are activated with 100 μL/well final of various concentrations of dsRNA. Cells incubated with fresh medium will be used as background luciferase activity. Cells are incubated at 37°C for 6 hours. 100 μL of freshly thawed Steady Glo (Promega) are added to each well, plates were incubated 10 min at RT in the dark and the light emitted in each well is quantified as Count Per Second (CPS) on a gamma-counter (TopCount) apparatus.

*dsRNA activity on human cancer cell lines*

*Apoptosis detection.* On day 0, tumor cell lines are seeded at $1x10^5$ to $2x10^5$ cells/ml (depending on the cell line) in appropriate culture medium, in 24-well culture plates (1mL/well). In case IFN-alpha pre-treatment is required, as soon as the cells adhere to the plastic, IFN-alpha is added in the appropriate wells at 1000U/ml. Cells are then incubated at 37°C overnight. On day 1, culture medium is discarded and replaced by fresh culture medium containing various concentrations of dsRNA (serial dilutions with a dilution factor of 10). Cells are then incubated at 37°C for 48 hours. On day 3, cells are harvested using trypsin-EDTA. The apoptosis quantification is performed using Caspase-Glo (see above), or an Annexin V-PI kit wherein cells arc stained following manufacturer instructions. The cells are analyzed on a FACSCalibur® cytometer.

As an alternative method, Caspase Glo 3/7 assay, rcf G8093, from Promega was used. The Caspase-Glo assay is a luminescent assay that measures caspase-3 and caspase-7 activities, playing key effector roles in apoptosis pathways. The assay provides a luminogenic caspase-3/7 substrate, which contains the tetra-peptide sequence DEVD, in a reagent optimized for caspase activity, luciferase activity and cell lysis. Adding a Caspase-Glo 3/7 reagent results in cell lysis followed by caspase-mediated cleavage of the substrate, and generation of a "glow-type" luminescent signal produced by luciferase. The resulting luminescent signal is directly proportional to the amount of caspase activity present in the sample, which is quantified on a TopCount. On day 0, cells are seeded in complete culture medium at 10000 cells/well, unless otherwise indicated, and they are incubated at 37°C for 20 hours. On day 1, 50μL of medium are discarded and cells are activated with 50 μL of various concentrations of dsRNA, prepared in free FBS (Fetal Bovine Serum) medium, for 4h30 at 37°C. This step aims at obtaining a final concentration of as low as 5% FBS, because of the presence of endogenous caspases in FBS. Cells incubated with medium only will be used as background luciferase activity. After incubation for 30mn, plates are equilibrated for 30mn to room temperature. 100μL of room temperature equilibrated Caspase-Glo are added to each well. Read the plate at 30mn to 3 hours on Topcount. The results will be shown as a ratio between experimental CPS (activated cells) and spontaneous CPS (non-activated cells) = fold increase.

*Cytokine secretion.* On day 0, tumor cell lines are counted, adjusted at $1x10^5$ to $2x10^5$ cells/ml (depending on the cells) in appropriate culture medium, in 96-well culture plates (200μL/well). Cells are then incubated at 37°C overnight. On day 1, culture medium is discarded and replaced by fresh culture medium containing various concentrations of dsRNA (serial dilution with a dilution factor of 10). Cells are then incubated at 37°C for 48 hours. On day 3, supernatants of the 96-well plates activated cells are harvested and frozen at -20°C until use. For quantification of the cytokines produced, supernatants are thawed at 37°C and treated with the BD OptEIA Elisa or R&D Elisa, following manufacturer instructions.

*dsRNA activity on huPBMC.* On day 0, PBMC are isolated from blood of healthy donors, counted, adjusted at $1x10^6$ cells per mL and activated with various concentrations of dsRNA. When analysis of phenotypic changes on PBMC is required, cells are activated in 6-well plates (5 mL per well). As per cytokine quantification, cells are activated in duplicate in 96-well plates (200 μL per well). Cells are then incubated for 24 hours at 37°C. On day 1, supernatants of 96-well plate activated cells are harvested and frozen at -20°C until use. For quantification of secreted cytokines, supernatants are thawed at 37°C and treated with ELISA kits following the manufacturer's instruction. As far as phenotypic changes are concerned, 6-well plate activated cells are harvested, stained and analyzed using a FACSCalibur™ flow cytometer. Expression of activation markers is evaluated on CD8+ T cells subpopulation, gated as follows: TCD8 cells: $CD3^+CD8+$ cells.

*Dose-response assay of mouse cytokine production and cellular activation after dsRNA treatment in C57Bl/6 mice.* Specific pathogen-free female C57/B16 mice are purchased from Charles River Laboratory (L'Arbresle, France) and housed in our animal facility. They are used between 6-8 week of age, following internal procedures and guidelines, in respect with good animal experimentation practice. For cellular activation, mice are euthanized at 24h after receiving dsRNA or NaCl 150mM, spleens are collected, spleen cells are isolated and stained, with CD3 and CD8 markers for CD8+ T cell population and CD69 for activation. Blood samples are collected at 2h post dsRNA treatment and stored at + 4°C until processed. All samples are centrifuged at 1800 (rpm) for 10 min. Serum is collected in dry tube, then stored at - 20°C until further assayed for cytokine content. Cytokine content in all collected sera are quantified using specific ELISA (R&D systems) for mouse IFN-alpha and mouse IP10.

*Analyses of in vivo anti tumor efficacy of dsRNA in a xenogenic tumor model.* The anti-tumoral effect of dsRNA in Nod-SCID mice bearing human breast tumors was investigated by treating mice bearing either A375 or HCC1806-originated tumors with dsRNA. Specific pathogen-free Nod-SCID mice (female or male) are purchased from Charles River Laboratory (L'Arbresle, France) and housed under sterile conditions. They are used between 6-8 week of age, following internal procedures and guidelines, in respect with good animal experimentation practice.

At day 0, Nod-SCID mice are inoculated s.c. on the right flank with $3x10^6$ HCC1806 cells or with $5x10^6$ for A375 cells. Starting on day 5 for HCC1806 and day 7 for A375, (time frame in which tumors volume should reach 20 to 50 $mm^3$), mice received intravenous injections of dsRNA (500 μg per mice under 100 μl) or physiological water in control. The treatment schedule for HCC1806 was 5 consecutive daily injections per week, followed by a rest of 2 days, during 6 consecutives weeks. The treatment schedule for A375 was (i) bi-weekly or weekly intravenous injections of either $10^e5$ U/ mice of IFN-alpha (Introna™, Schering Plough) or control NaCl 150mM followed the following day by (ii) intravenous injections of 500μg or 1mg/mice of dsRNA or control NaCl 150mM. This cycle was repeated during 6 consecutives weeks. The follow up of tumor growth was done two times per week. Tumor volume (calculated using the formula $(A \times (B^2)/2))$ and their index evolution was calculated. At the end of the 6 consecutives cycles, if the volume of the tumor does not exceed $800mm^3$, the mice were kept without treatment and tumor growth was analyzed until the volume of tumor exceeds 800 $mm^3$.

*Inhihition with lentivirus shRNA:* A lentivirus construction was made and produced by Vectalys (Toulouse, France), encoding short hairpin RNA (shRNA) targeting control shLaminA/C (GAAGGAGGGTGACCTGATATTCAAGAGATATCAGGTCACCCTCCTTCTTTTT) (SEQ ID NO 15) or human TRIF (AAGACCAGACGCCACUCCAACUCAAGAGGUUGGAGU GGCGUCUGGUCUUUU) (SEQ ID NO 16). Tumor cells were infected with lentivirus preparation and further selected with puromycin to get stable shLamin or shTRIF HCC1806, HCC38 or A375 tumor cells.

## RESULTS

### EXAMPLE 1-INITIAL SIZE-BIOACTIVITY STUDIES

Several polyAU products were obtained and their molecular weight assessed using SEC-MALLS; results are shown in Table 3.

Table 3

| RNA | Mn (g/mol) | Chain Length (bp) | Mw (g/mol) | Ip (Mw/Mn) | Endotoxin (EU/mg) |
|---|---|---|---|---|---|
| **Oligo $(AU)_{20}$** (AU20) Dharmacon | 12 584 | 20 | 12 584 | 1.0 | Not tested |
| **Oligo $(AU)_{45}$** (AU45) Dharmacon | 28 468 | 45 | 28 468 | 1.0 | Not tested |

(continued)

| Table 3 | | | | | |
|---|---|---|---|---|---|
| RNA | Mn (g/mol) | Chain Length (bp) | Mw (g/mol) | Ip (Mw/Mn) | Endotoxin (EU/mg) |
| **Poly(A:U)** Sigma ref. P1537 Batch # 025K4004 | 62 000 | 94 | 100 000 | 1.6 | 78 |
| **Poly(A:U) ref. "Pre-Run"** | 193 200 | 293 | 321 500 | 1.7 | 2.4 |
| **Poludan™** | 1380 000 | 2091 | 1680 000 | 1.2 | Tested between 1.9 and 11 |
| **Poly(I:C)** Invivogen ref. tlrl-pic | 1 130 000 | 1712 | 1 700 000 | 1.5 | 0.48 |
| **Am1:Us-2** | 324 000 | 491 | 515 000 | 1.3 | 1.0 |

Each of these dsRNA compositions in Table 3, as well as polyAxU 50mer (AxU50) and a polyIC 20 mer (IC20), were tested for binding to immobilized human TLR3 polypeptide on Biacore). Each compound bound to TLR3, with differing profiles as shown in Figure 1 and 2, where in Figure 1 the upper (dotted) line shows binding to TLR3 and the lower line shows binding to dextran (control). Figure 2 shows higher chain length dsRNA with similar binding profiles. Among the oligonucleotides, it was observed that IC20, AU20, AU45 and AxU50 bound TLR3 with different profiles. Interestingly, the 40 mer AU20-18s-20 demonstrated good binding. Nevertheless, it was clear that for all of the oligonucleotides (e.g. up to 50 mers, and the AU20-18-20) the diassociation of the TLR3-dsRNA complex was faster for the oligonucleotides than for the longer chain length dsRNA.

However, despite binding to TLR3 on Biacore of each composition, differing potency in induction of TLR3-mediated signaling was observed in reporter gene activity. Shown in Figure 3 are results of activity in a reporter gene activity (293T-TLR3-ISRE) assay. Figure 4A shows results in an apoptosis assay, respectively, for commercial poly(I:C) (pIC), commercial polyAU (Sigma), AU690 and Am1:Us-2. Figures 4B and 4C show cytokine production in vitro on human tumor cells by the same compounds. The compounds demonstrating biological activity had increased potency as a function of their molecular weight. The polyAU 19mers, polyAU 45mers and polyAxU 50mers had no or very low activity in this assay (not shown). Commercial polyAU in turn displayed very low activity compared to other longer chain length dsRNA, Am1:Us-2 showed significantly higher activity. PolyIC, by way of comparison, showed significantly increased potency over each of the polyAU compositions. This first set of experiments therefore suggested that TLR3 binding is not sufficient to induce potent TLR3-mediated signaling in the reporter gene assay, and that chain length of the dsRNA is a factor in TLR3 agonism. Possible explanations include the possibility that dsRNA is required to cross-link multiple TLR3 proteins in order to induce TLR3 signaling, and/or that dsRNA that binds a high number of TLR3 proteins present on a surface (e.g. endosome) will have improved signaling ability as a result of greatly decreased dissociation from the TLR3 proteins. One additional explanation could be that short dsRNA do not enter endocytic pathway as efficiently as longer dsRNA (lack of binding to surface receptor for dsRNA, such as Scavenger receptors recently described), or are too rapidly degraded in this endocytic pathway, compared to longer dsRNA. Thus, in cellular experiment, short dsRNA would not reach and signal through TLR3, located in the endosomes.

## EXAMPLE 2-CHARACTERIZATION OF SSRNA-DSRNA MOLECULAR WEIGHT RELATIONSHIP

As the dsRNA tested in Example 1 were of different origin, hybridized, maintained or otherwise treated according to different conditions which could not be verified, because of differences in endotoxin content, and because the presence of components other than polyAU could not be ruled out, a set of different ssRNA were generated with a view of preparing several different high-molecular weight dsRNA. Three different high MW ssRNA were obtained and Mn, Mw were obtained using SEC MALLS. Chain length was obtained by dividing the Mn by 330 for ssRNA and 660 for dsRNA. The results are shown in Table 4.

| Table 4 | | | | | |
|---|---|---|---|---|---|
| **ssRNA** (IPH code) | **Mw (g/mol)** | **Mn (g/mol)** | **Length (b)** | **Ip** = **Mw/Mn** | **Endotoxin (EU/mg)** |
| **Axs** | 170000 | 81 000 | 245 | 2.1 | |

(continued)

| Table 4 | | | | | |
|---|---|---|---|---|---|
| **ssRNA** (IPH code) | **Mw (g/mol)** | **Mn (g/mol)** | **Length (b)** | **Ip** = **Mw/Mn** | **Endotoxin (EU/mg)** |
| **Am1** | 743 000 | 500 000 | 1515 | 1.5 | 0.67 |
| **Am2** | 861 000 | 426 000 | 1291 | 2.0 | < 1 |
| **Uxs** | 97 800 | 46 200 | 140 | 2.1 | 0.57 |
| **Us** | 206 000 | 149000 | 452 | 1.4 | 1.15 |
| **Um** | 646 000 | 410000 | 1242 | 1.6 | 0.37 |

Using a first defined annealing protocol the dsRNA compositions having the characteristics in Table 5 were obtained.

| Table 5 | | | | | | | |
|---|---|---|---|---|---|---|---|
| **dsRNA** (IPH code) | **Mn (g/mol)** | **Chain Length (bp)** | **Mw (g/mol)** | **Ip = Mw/Mn** | **Endotoxin (EU/mg)** | **Mn polyA** | **Mn polyU** |
| **Axs:Uxs** | 156 000 | 236 | 279 000 | 1.8 | < 1 | 81 000 | 46 200 |
| **Axs :Us** | 256 000 | 388 | 455 000 | 1.8 | 0.32 | 81 000 | 149 000 |
| **Axs:Um** | 822 000 | 1245 | 1 150 000 | 1.4 | 1.2 | 81 000 | 410 000 |
| **Am1:Uxs** | 615 000 | 932 | 717000 | 1.2 | | 500 000 | 46 200 |
| **Am1:Us** | 549 000 | 832 | 985 000 | 1.8 | 0.3 | 500 000 | 149 000 |
| **Am1:Um** | 1680 000 | 2545 | 2090 000 | 1.2 | 1.1 | 500 000 | 410 000 |
| **Am2:Uxs** | 806 000 | 1221 | 1190 000 | 1.5 | < 1 | 426 000 | 46200 |
| **Am2:Us** | 1470 000 | 2227 | 1980 000 | 1.9 | < 1 | 426 000 | 149 000 |
| **Am2:Um** | 1490 000 | 2258 | 2270 000 | 1.5 | < 1 | 426 000 | 410 000 |

Am1:Um and Am2:Uxs each included the presence of two distinct populations of polymers, and the result in table reflects higher molecular weight population. The lower molecular weight populations for these compositions was characterized are as follows:

a) Am1:Um: $M_n$ = 152 000 g.mol$^{-1}$ - $M_w$ = 205 000 g.mol$^{-1}$ (Ip = 1,4) and
b) Am2:Uxs: $M_n$ < 40 000 g.mol$^{-1}$ - $M_w$ < 60 000 g.mol$^{-1}$ (Ip = 1,6).

Taking together both populations yielded the following results:

c) Am1:Um: $M_n$ = 420 000 g.mol$^{-1}$ - $M_w$ = 1500 000g.mol$^{-1}$ (Ip = 3.6) and
d) Am2:Uxs: $M_n$ = 246 000g.mol$^{-1}$ - $M_w$ = 1080 000 g.mol$^{-1}$ (Ip = 4.4).

It was observed that certain combinations of ssRNA results in high MW dsRNA, and with a relatively low polydispersity value less than 2.0 and as low as 1.2. The values for Mn, Mw, Ip, are shown in Table 5 above. The Mn of the dsRNA was significantly greater than the Mn of either of the ssRNA compositions, suggesting that dsRNA polymers in many instances would contain more than one polymer of a given ssRNA species, that is a polyAU molecule would be the product of hybridization of one or more polyU polymers with two or more polyA polymers and/or vise versa, and moreover in many cases would be the product of at least two of each of the complementary single stranded polymers.

The molecular weight profiles of the polyAU compositions were compared as a function of the molecular weight profile of the polyA or polyU ssRNA compositions. Combining Uxs (Mn of 46 kDa) and Axs (Mn of 81kDa) yielded a dsRNA having a Mn of 156 kDa, in turn having lower bioactivity (Example 12) than higher molecular weight compositions. Combining Us (Mn of 149 kDa) and Axs (Mn of 81kDa) yielded a dsRNA at an Mn of 256 kDa, thus having higher molecular weight that Uxs:Axs but still lower than other dsRNA, and demonstrating bioactivity lower than that of higher Mn compositions (Example 12).

However, combining either Uxs (Mn of 46 kDa), Us (Mn of 149 kDa) or Axs (Mn of 81kDa) with various other ssRNA yielded always resulted in a significantly higher molecular weight. It appeared that as long as at least one of the ssRNA compositions had an Mn higher than that of Uxs (Mn of 46 kDa), or preferably at least about than of Us (Mn of 149 kDa) a dsRNA having an Mn of more than 500 kDa could be obtained regardless of whether the complementary ssRNA composition had a low Mn. In particular, in each instance tested where one ssRNA composition had an Mn of greater than about 300 kDa or 400 kDa, a high molecular weight dsRNA (e.g. having an Mn of at least about 500 kDa) was obtained, regardless of whether the complementary ssRNA composition had a low Mn. For example, combining Axs (Mn of 81 kDa) with Um (Mn of 410 kDa) gave rise to a dsRNA having an Mn of 822 kDa. Furthermore, combining a higher molecular weight ssRNA which was in the 400 kDa range (about 426 kDa, Am2) with a ssRNA where having a Mn of at least 149 kDa (e.g. Us or Um) or between about 149 kDa and about 426 kDa gave rise to dsRNA having an Mn greater than about twice that of the longer chain length ssRNA composition. Combining the ssRNA Am2 (426 kDa) and Us (149 kDa) gave rise to a dsRNA composition of 1470 kDa, and Am2 with Um (410 kDa) gave rise to a dsRNA composition of 1490 kDa. Since the same combination of either Us or Um with a higher Mn Am1 yielded lower Mn dsRNA, the Am2:(Us or Um) combination seemed to provide better specifications for a high molecular weight dsRNA under these annealing conditions. For two dsRNA, two distinct populations (two bell curves) were observed. Combining Am2 (Mn of 426 kDa) with Uxs (Mn of 46 kDa) gave rise to a dsRNA composition having two populations with an Mn at 1680 kDa and 152 kDa respectively, and combining Am2 (426 kDa) and Uxs (46 kDa) gave rise to a dsRNA composition having two populations with an Mn at 806 kDa and less than 40 kDa respectively.

In general, the molecular weight and strand length of the polyAU were conditioned by the length or molecular weight of the ssRNA composition of higher average molecular weight and strand length. Figure 5 shows the molecular weight of the dsRNA composition increases as a function of the longer chain length ssRNA composition both when the latter is a polyA or polyU.

## EXAMPLE 3-HYBRIDIZATION QUALITY OF DSRNA COMPOSITIONS

In order to provide an indication of the quality of hybridization, melting temperature profiles were determined for each of the dsRNA compositions prepared from the set of six ssRNA compositions, and for Am1:Us-2 and Poludan for comparison. Melting temperature and hyperchromicity provided an indication about the extent to which the dsRNA polymers are hybridized. The derivative curve of the melting temperature (*FWHM*) provides an indication of the homogeneity of the dsRNA population. Table 6 shows the results of the melting temperature assessment and first derivative of the melting curve.

**Table 6**

| Product name | Mw (g/mol) | Mn (g/mol) | A280/A260 | Tm (°C) (mean value) | Hyperchromicity (%) | 1st derivative FWHM (°C) |
|---|---|---|---|---|---|---|
| -Axs:Uxs | 279 000 | 156000 | 0.422 | 57.3 | 52.4 | 5.4 |
| -Axs:Us | 455 000 | 256 000 | 0.425 | 59.4 (± 0.4) | 54.7 | 2.1 |
| -Axs:Um | 1 150 000 | 822 000 | 0.398 | 61.3 (± 1.5) | 29.9 | 1.8 |
| -Am1:Uxs | 717 000 | 615 000 | 0.437 | 56.9 | Not available | 5.3 |
| -Am1:Us | 985 000 | 549 000 | 0.445 | 60.9 (± 1.8) | 57.0 | 2.1 |
| -Am1:Um | 1680 000 | 2090 000 | 0.403 | 59.9 | Not available | 1.9 |
| -Am2:Uxs | 806 000 | 1190 000 | 0.412 | 58.5 | Not available | 5.0 |
| -Am2:Us | 1980 000 | 1470 000 | 0.422 | 61.0 | Not available | 2.1 |
| -Am2:Um | 2270 000 | 1490 000 | 0.444 | 62,2 (± 1.7) | 48.6 | 1.8 |
| -Poludan™ | 1680 000 | 1380000 | 0.458 | 58.9 | Not available | 3.5 |
| Am1:Us-2 | 515 000 | 324 000 | Not measured | 59.1 | Not available | 3.2 |

It was expected that the Tm (mean value) would increase as a function of the Mn of the polyAU compositions.

However, exceptions were observed; the most notable was Am1:Uxs displayed a Tm of 56.9 °C, four degrees C lower than Am1:Us, despite both compositions having similar Mn and Mw values. Am2:Uxs also demonstrated a somewhat lower Tm than expected. Both Am1:Uxs and Am2:Uxs arise from the combination of populations of ssRNA having a large difference in chain length, from a mixing an ssRNA population having an Mn of 426 kDa or 500 kDa with an ssRNA population having an Mn of 46 kDa. One possible explanation for the variance in Tm between different dsRNA compositions is that the two strands making up the dsRNA can hybridize differently depending on the nature of the single strands used. The ssRNA may hybridize such that the stability of the dsRNA on average is less than that of other compositions having similar or even lower average molecular weight. Tm is expected to provide an overall indication of stability, and will be influenced by multiple factors including total dsRNA chain length, degree of hybridization and the relationship between two hybridized ssRNA. For example, it is possible that a dsRNA comprising several short ssRNA hybridized to a single longer ssRNA may have lower decreased stability at elevated temperature (compared to two ssRNA of identical length) because shorter hybrids are expected to disassociate from their 5' and 3' ends such that a series of short chains hybridized to a single longer chain will have a larger number of total non-hybridized residues compared to two identical long chains. Axs:Uxs also demonstrated a relatively low Tm. Interestingly, as shown in Table 10, each of these compositions having low Tm below about 59 °C, and in particular Am1:Uxs, had lower bioactivity than would have been expected based on their molecular weight. A Tm of at least about 59 °C therefore appears to represent a threshold for optimally hybridized high molecular weight dsRNA.

Another factor considered was hyperchromicity, which provides a quantitative measure of the number of hybridized bases. Interestingly, higher Tm did not necessarily correspond to higher hyperchromicity, and is presumed that some strands were stable but in a less than optimal hybridization state. Axs:Um for example was characterized by a relatively high Tm but low hyperchromicity. Interestingly, as shown in Table 10, Axs:Um has lower bioactivity than would have been expected based on its Tm and molecular weight.

Interestingly, each time two ssRNA compositions having Mn that differ more than a certain amount were combined, the resulting polyAU had poor hybridization indicators - Axs:Um having low hyperchromicity and Am1:Uxs and Am2:Uxs having low Tm. Axs:Um arises from a polyA having Mn of 81 kDa and polyU having Mn of 410 kDa; Am1:Uxs and Am2:Uxs arise from a polyU having Mn or 46 kDa and polyA of either 500 or 426 kDa respectively. Combining ssRNA compositions having ratio of Mn of no more than about 1:5 (the Mn of the lower Mn composition is at least about 20 % of the Mn of the higher Mn composition) was therefore selected as a threshold for polyAU bioactivity.

Finally, the FWHM value, providing a measure of homogeneity of the dsRNA hybridization was assessed. While homogeneity of dsRNA need not necessarily be indicative of potency, greater homogeneity will generally be a desirable quality for a pharmaceutical composition. It was observed that compositions Axs:Uxs, Am1:Uxs, Am2:Uxs had a quality of hybridization different than other compositions, as evidenced by a higher value for the FWHM (about 5° compared to about 2° for best compositions) suggesting a lower or less homogenous state of hybridization. As mentioned above, each of these three compositions also demonstrated a low Tm.

For compositions generated from the ssRNA starting material of Example 2, the Tm and FWHM values appeared to be influenced by the molecular weight of the polyU ssRNA compositions. In particular, all polyAU compositions generated from polyU compositions having lowest molecular weight (that is, the Uxs compositions, with Mn of 46 kDa) correlated with lower Tm and higher FWHM value than would be expected based on the molecular weight of the dsRNA composition. It therefore appeared that there was threshold where polyU having below a threshold of molecular weight is unfavorable to optimally hybridized dsRNA. Based on the FWHM of the 1$^{st}$ derivative of the Tm, it was observed that only Uxs compositions gave rise to values of 5.4 °C, 5.3 °C and 5.0 °C, while the values for the other polymers generated from the three polyA and polyU compositions were all in the range of 1.8 to 2.1 °C. The product Poludan™ yielded a value of 3.5, suggesting that it may arise from the use of a lower molecular weight polyU (e.g. Mn below 149 kDa). The nature of the polyA composition did not appear to be critical to FWHM value or Tm because all polyA compositions, including the polyA of lowest Mn (Axs), resulted in polyAU compositions having higher relative Tm and lower value for the FWHM of the 1$^{st}$ derivative of the Tm. It therefore appeared that FWHM of the 1$^{st}$ derivative of the Tm values above about 2 indicated the use of a polyU starting material of decreased chain length, for example a polyU composition having an Mn of less than about 50 Kda, 100 kDa or 150 Da. It should therefore be useful to generate dsRNA by using as starting material a polyU composition having a predetermined molecular weight profile, generally where polyU ssRNA compositions have a Mn above about 46 kDa, and preferably at least about 149 kDa, combined with a polyA of any suitable chain length, optionally of greater chain length than the polyU composition (e.g. a polyA composition having an Mn of at least 300 kDa, 400 kDa). In one example, optimal conditions for generating a high molecular weight polyAU can comprise for example combining a polyA composition having a Mn of between about 400 kDa and 500 kDa, or between 500 kDa and 600 kDa with a polyU composition having a Mn of between about 150 kDa and 400 kDa.

## EXAMPLE 4-INITIAL SIZE: BIOACTIVITY RELATIONSHIP FOR DSRNA FROM SSRNA COMBINATIONS

The binding to immobilized human TLR3 protein by dsRNA compositions described in Table 5 were assessed by Biacore methods. Each of the compositions demonstrated substantial binding to TLR3.

In a first series of tests for bioactivity, the compositions of Table 5 were subjected to assays for TLR3 reporter gene activity (293T-TLR3-luciferase), cytokine production in PBMC, pro-apoptotic activity on HCC38 cells, and cellular activation of PBMC.

On reporter gene activity assays, Axs:Us at a Mn of 256 kDa has roughly 10-fold better potency that lower Mn compounds (Axs:Us having Mn of 156 kDa). Next, since the lower molecular weight compositions Axs:Uxs and Axs:Us, each had Mn of less than 300 kDa (e.g. the highest of these was Axs:Us at an Mn of 256 kDa and Mw of 455 kDa), it appeared that activity increased when the Mn was beyond the about 300 kDa range (or when the Mw was beyond the about the 500 kDa range), and that as of an Mn of about 500 kDa there was a significant increase in bioactivity mediated by TLR3 in view of Am1:Us (Mn 549) with an Mn in the about 500-600 kDa range demonstrating significantly higher activity than Axs:Us below or near the 300 kDa range. Then, beyond the aforementioned 500 - 600 kDa range for the Mn, from 600 kDa onwards (e.g. 800 kDa to 1000 kDa, 800 kDa to 1500 Kda, 1000 kDa to 1500 kDa), another increase in TLR3 mediated bioactivity was observed in the reporter gene assay, and the composition having Mn above 1000 kDa (e.g. composition Am2:Um in the 1400-1500 kDa range) demonstrated the highest bioactivity. Axs:Uxs had a lower Mn and less favorable Tm profile than Axs:Us, and demonstrated consistently lower activity, with a relatively larger difference observed for pro-apoptotic activity. Among the polyAU compositions considered of "medium" molecular weight profile, Axs:Um, Am1:Uxs, and Am1:Us all demonstrated similar range of activity in all assays. Among the polyAU compositions Am1:Um and Am2:Um considered of "high" molecular weight profile, Am2:Um demonstrated markedly higher activity in all luciferase and apoptosis assays, although Am1:Um's lower bioactivity must be qualified by the presence of a second population of RNA at lower Mn. Am2:Uxs and Am2:Us were not further included in this analysis because the molecular weight observed using an ethidium bromide agarose gel was lower than the molecular weight as determined by SEC-MALLS, suggesting a possible presence of ssRNA. On the whole, it appeared that increasing the molecular weight, as observed by Mn resulted in an increase in bioactivity at TLR3.

## EXAMPLE 5-LOW, MEDIUM AND HIGH MN DSRNA COMPARATIVE BIOACTIVITY

In order to compare polyAU compositions in a more a repeated set of experiments, including in vivo tumor models, the compositions having highest activity polyAU from each of the low, medium or high Mn groups were compared. Each of these compounds were prepared using the same hybridization methods, based on ssRNA of different Mn prepared according to similar protocols. All compositions had a value of about 2 for the FWHM of the 1st derivative of the Tm, as shown in Example 3. Table 7 shows characteristics of these three compositions. Axs:Us, Am1:Us and Am2: Um were compared in luciferase assay, cytokine production and pro-apoptotic activity on HCC38 cells, cellular activation and cytokine production of human PBMC in vitro, cellular activation and cytokine production in mice in vivo, anti-tumoral effect on a xenogenic mouse tumor model.

**Table 7**

| **dsRNA** (IPH code) | **Mn (g/mol)** | **Length (bp)** | **Mw (g/mol)** | **Ip=Mw/Mn** | **Mn polyA** | **Mn polyU** |
|---|---|---|---|---|---|---|
| **Axs :Us** | 256 000 | 388 | 455 000 | 1.8 | 81 000 | 149 000 |
| **Am1:Us** | 549 000 | 832 | 985 000 | 1.8 | 500 000 | 149 000 |
| **Am2:Um** | 1490 000 | 2258 | 2270 000 | 1.5 | 426 000 | 410000 |

*293T-T3-ISRE luciferase*

The dose response for TLR3 signaling activity of compositions Axs:Us, Am1:Us and Am2:Um was tested in a 293T-T3-ISRE luciferase gene reporter assay (range: 0.001 to 1000 μg/ml, 1/10th dilution). There was a clear increase in potency in this assay for the Am2:Um product over the lower molecular weight Am1:Us, and again higher potency for the Am1:Us product compared to the lower molecular weight Axs:Us. The experiments were repeated twice. Results are shown in Figure 6.

*HCC38 tumor cell assays*

Compositions Axs:Us, Am1:Us and Am2:Um were compared either for pro-apoptotic activity (caspase glow) or cytokine production (IL-6 or IP10) on HCC38 tumor cells. IP10 is indicative of MyD88-independent signaling. HCC38 were previously determined to express TLR3. Results demonstrate that in terms of cytokine production Am2:Um was more potent than Am1:Us, which in turn was more potent than Axs:Us. In terms of pro-apoptotic activity, there was a less marked difference in this experiment, and Am1:Us and Am2:Um appeared more similar but both more potent that Axs:Us. Results are shown in Figure 7.

*In vitro cellular activation of PBMC*

Compositions Axs:Us, Am1:Us and Am2:Um were compared either for in vitro cellular activation of CD8+ T cells and cytokine production (IP-10) by PBMC. Results demonstrate that in terms of in vitro cellular activation and cytokine production Am2:Um was more potent than Am1:Us, which in turn was more potent than Axs:Us. Results are shown in Figure 8 for IP-10 and CD8+ T cells.

*In vivo cytokine production and cellular activation*

Compositions Axs:Us, Am1:Us and Am2:Um were compared either for in vivo cellular activation of and cytokine production (IP-10) by PBMC. Results demonstrate that in terms of in vivo cellular activation Am2:Um was much more potent than Am1:Us and Axs:Us, the latter two having similar activity. In terms of cytokine production, there was a less marked difference in this experiment, and Am1:Us and Am2:Um appeared more similar but both more potent that Axs: Us. Results are shown in Figure 9 for IP-10 and CD8+ T cells.

*HCC1806 tumor model in NOD-SCID mice*

Compositions Axs:Us, Am1:Us and Am2:Um were compared for their effect on tumor volume in the HCC1806 xenogenic tumor model. Insofar as the NOD-SCID mouse is immunodeficient, bioactivity will depend on the induction of apoptosis of tumor cells. Results demonstrate that Am2:Um was more potent than Am1:Us, which in turn was more potent than Axs:Us, for the ability to prevent tumor growth. Results are shown in Figure 10.

In summary, Axs:Uxs demonstrated consistently lower activity in all assays, while a less marked difference was observed between Am2:Um and Am1:Us. Long and Medium chain length dsRNA therefore had greater activity, and there seemed to be an activity threshold between chain length of Axs:Us and Am1:Us. Thus, increasing the Mn of the polyAU leads to consistently improved activity, particularly in terms of inducing TLR3-mediated signaling. When taking into account solely pro-apoptotic in tumor cells, on the whole it appears that increasing chain length again corresponds to increased potency, although for compositions having an Mn beyond the range of e.g. 549 kDa the increase in potency may be less dramatic than that observed between medium chain length (e.g. 549 kDa) compositions over lower chain length (e.g. 200 kDa) polyAU such as Axs:Us. The range for medium and high chain length dsRNA, for example compositions characterized by an Mn of between about 250 and 2000 kDa, as well as medium chain length dsRNA such as compositions between about 300 kDa and 900 or 1000 kDa therefore represent compositions having a desirable range of activity for inducing TLR3-mediated signaling.

## EXAMPLE 6-VARIATION OF DSRNA ANNEALING PROTOCOLS

Different parameters in the annealing protocol were tested. The ssRNA polymer compositions designated Am1 and Us having the molecular weight characteristics described in Table 4 were annealed using each of the three protocols. Results are shown in Table 8. One protocol corresponded to that used to generate Am1:Us described in Example 2, another protocol corresponded to that used to generate Am1:Us-2 described in Example 1, and a third protocol described below was used to generate Am1:Us-3. The heating temperature and time were varied, ranging from 5 minutes to 2 hours for the time and from 70 C to 95 C, as shown in Table 8, for a duration of 5 minutes to 2 hours, and each otherwise according to the same conditions.

**Table 8**

| Condition number | dsRNA (IPH code) | Mn (g/mol) | Mw (g/mol) | Ip Mw/Mn | Endotoxin (EU/mg) | Annealing conditions |
|---|---|---|---|---|---|---|
| 1 | Am1:Us | 549 000 | 985 000 | 1.8 | 0.3 | 5 min at 95 °C |
| 2 | Am1:Us-2 | 324 000 | 515 000 | 1.3 | 1.0 | 2 hours at 75 °C |
| 3 | Am1:Us-3 | 1 280 000 | 1 690 000 | 1.3 | 0.6 | 10 min at 70 °C |

Three polyAU compositions having different Mn and distribution of molecular weights of species were obtained. Mn for Am1:Us-2 and Am1:Us were obtained using SEC-MALLS methods. It was observed that in the annealing protocol, time and temperature combinations had an effect on the characteristics of the dsRNA, and in particular with molecular weight characteristics, which in turn correlated with biological activity. It was observed that taking a value as small as possible above the Tm, in this case the estimated Tm based on Am1:Us-2 and heating for as short time as possible (e.g. on the order of minutes was better than hours), the highest molecular weight dsRNA compositions could be obtained.

To investigate the effect of heating during annealing, each of the ssRNA compositions were then tested to determined whether temperature has an effect on the chain length of the ssRNA compositions. It was observed that heating leads to decreased chain length (as expressed in Mn) of the ssRNA compositions, and polyA was particularly sensitive. As shown in Figure 11 for polyA and polyAU, for each ssRNA, at room temperature and at two temperatures, each for two durations of heating, the longer heating time and higher temperature yields decreased Mn for the ssRNA and dsRNA. The polydispersity remained steady during the period; the Ip value was 1.1 for the starting polyA and polyAU, and remained at 1.1 for each polyA, polyU and polyAU composition, with the exception of polyA at 10 minutes and 2 hours at 95 C, where the IP was 1.2. Annealing protocols can therefore be adapted to obtain the desired ssRNA and dsRNA compositions, and heating times and temperatures can be optimized to prevent a decrease in Mn.

**EXAMPLE 7-COMPARISON OF DSRNA ANNEALING PROTOCOLS: LUCIFERASE ASSAY**

Compositions Am1:Us, Am1:Us-2 and Am1:Us-3 described in Example 6 were tested for dose response in a 293T-T3-ISRE luciferase gene reporter assay (range: 0.001 to 1000 μg/ml, 1/10$^{th}$ dilution). Am1:Us-3 consistently demonstrated higher potency than Am1:Us, which in turn consistently demonstrated higher potency that Am1:Us-2. Results arc shown in Figure 12. Commercial polyIC was included in the comparison; results showed that polyIC has similar potency to the polyAU compositions in TLR3 signaling in this assay, and no higher potency that the best polyAU.

**EXAMPLE 8-COMPARISON OF DSRNA PREPARATION PROTOCOLS: HCC38 TUMOR CELL ASSAYS**

Compositions Am1:Us, Am1:Us-2 and Am1:Us-3 described in Example 6 were compared either for pro-apoptotic activity (caspase glow) or cytokine production (IL-6 or IP10) on HCC38 tumor cells in a series of three experiments. Results demonstrated that Am1:Us-3 and Am1:Us had greater potency than Am1:Us-2 in pro-apoptotic (caspase glow), and Am1:Us-3 had greater potency in IP-10 induction but similar potency in IL-6 induction as Am1:Us-2 (Figure 13). Commercial polyIC was included in the comparison for pro-apoptotic activity; results showed that polyIC induced at the same concentration a higher level of pro-apoptotic activity than all polyAU compositions.

**EXAMPLE 9-COMPARISON OF DSRNA PREPARATION PROTOCOLS: IN VITRO HUMAN PBMC ACTIVATION**

Am1:Us, Am1:Us-2 and Am1:Us-3 described in Example 6 were next compared for their ability to activate PBMC (CD8+ T cells, CD4+ T cells, NK cells or B cells), as determined by detecting increase in CD69 cell surface expression and for their ability to induce the production of cytokines (IP-10, IL-6, MCP-1 and IFN-gamma) by human PBMC. Results demonstrated that Am1:Us-3 and Am1:Us have consistently higher potency than Am1:Us-2 and in all assays (Figure 14), and that polyIC has higher potency than all polyAU, although results with polyIC must be qualified by the endotoxin content in commercial polyIC.

**EXAMPLE 10-COMPARISON OF DSRNA PREPARATION PROTOCOLS: IN VIVO CELLULAR ACTIVATION AND CYTOKINE PRODUCTION**

Am1:Us, Am1:Us-2 and Am1:Us-3 described in Example 6, as well as commercially available polyIC, were compared either for their ability to induce the production of cytokines (IP-10, IL-6, MCP-1) or activate spleen cells, in vivo, in B6 mice.

The following parameters were studied: (a) cytokine contents in mice sera; and (b) % of positive cells for selected activation marker (CD69), for each cell sub-population studied. Cellular activation involved comparing compositions' ability to generate, in vivo, activated CD8+ T cells, CD4+ T cells, NK cells or B cells, as determined by detecting increase in CD69 cell surface expression. Results show an increase in cellular activation correlated with increasing Mn; potency was increased for Am1:Us-3 over Am1:Us, and for Am1:Us over Am1:Us-2 throughout the doses, with a very strong increase at the intermediate dose (50 μg/mouse) (Figure 15). PolyIC consistently showed greater cellular activation than all of the polyAU compositions.

Am1:Us-2 was able to activate all spleen populations, in terms of CD69 up-regulation. This activation was comparable to a 2.5 mg/kg dose of commercial polyIC, with an optimal dose of 10 mg/kg for Am1:Us-2. Am1:Us-2 is also able to induce CD86 up-regulation on DC, B Cells and monocytes as 2.5 mg/kg of polyIC, with optimal dose of 25 mg/kg for CD86 activation. Am1:Us-2 induced up-regulation of CD25 on CD8 T cells and on NK cells (no effect on CD4+ T cells and γ8 Cells), with an optimal dose higher than 25 mg/kg.

At equivalent doses (e.g. 2.5 mg/kg) Am1:Us-2 was always less efficient than polyIC in ability to induce cytokine/chemokine secretion. However differential pattern in terms of efficiency in inducing cytokine/chemokine secretion can be distinguished between polyIC and all polyAU. The fact that polyIC has a dramatic increase in type I interferon (murine IFN-alpha) induction compared to the polyAU compositions which do not induce significant IFN-alpha at all, while exhibiting similar potency at TLR3 in terms of inducing TLR3-mediated signaling may help explain why polyAU as TLR3 agonists do not show the toxicity that accompanies polyIC.

This dose response study enabled us to determine an optimal dose for Am1:Us-2 treatment. For most readouts (both cytokine secretion and cell activation), the optimal dose was 200-500 μg/mice (10-25 mg/kg).

In this study, while cell activation (CD69 up-regulation) was still detected at the 50 μg/mouse dose, as far as cytokine/chemokine induction is concerned, only IL12p40, MCP-1, IP-10 and RANTES could be detected at 50 μg/mice dose, at very low level. Spleen B and NK cell activation, as well as IP-10 and MCP-1 secretion could still be detected at the 20 μg/mouse dose for Am1:Us-2. The doses used are shown in Table 9.

**Table 9**

| Am1:Us-2 | Injected dose -in mg/mouse | Dose -in mg/kg | Human Equivalent Dose in mg/kg |
|---|---|---|---|
| MABEL (Minimal Anticipated Biological Effect Level) | <0.02 | <1 | <0.083 |
| Maximum Tolerated Dose | >2 | >100 | >8 |
| **Optimal dose** | **0.2-0.5** | **10-25** | **0.8-2** |

## EXAMPLE 11-IN VIVO ANTI-TUMOR ACTIVITY

Am1:Us, Am1:Us-2 and Am1:Us-3 were compared for their effect on tumor volume in the HCC1806 xenogenic tumor model, indicative of induction of apoptosis of tumor cells by the dsRNA. Results demonstrate significant antitumor activity, where all polyAU compositions had similar range of activity, with a slightly less tumor development in mice treated with Am1:Us-2 over Am1:Us-3 and in Am1:Us-3 over Am1:Us in this experiment. Results are shown in Figure 16.

## EXAMPLE 12-BIOACTIVITY STUDIES

PolyAU compositions Axs:Us, Am1:Us and Am2:Um representing low, medium and high molecular weight groups respectively from Example 5 were compared with other dsRNA compositions polyAU "Pre-Run", Am1:Us-2, Am1:Us-3, Axs:Us, Am1:Uxs and Axs:Um previously tested in only a single series of experiments (Example 1), in repeated series of experiments, for dose response in TLR3-mediated signaling activity in the 293T-T3-ISRE luciferase gene reporter assay (range: 0.001 to 1000 μg/ml, 1/10$^{th}$ dilution). Endotoxin content was below 1 EU/mg for each of the compounds, except for "Pre-run" which had 2.4 EU/mg. Table 10 shows the EC50 values for each of the compositions. Figure 17 shows the EC50 as a function of the Mn of the composition.

**Table 10**

| | **EC50 (μg/ml)** | **pAU** | **Mn (kD)** | **Average value Tm(°C)** n=2 | **Hyperchromicity (%)** | **FWHM** |
|---|---|---|---|---|---|---|
| Group 1 | 2.7 | Am1:Us-3 | 1280 | Nt | Nt | Nt |
| | 3,23 | Am2:Um | 1490 | 62,2 (± 1.7) | 48.6 | 1.8 |
| Group 2 | 9,12 | Am1:Us | 549 | 60.9 (± 1.8) | 57.0 | 2.1 |
| Group 3 | 13.9 | Am1:Us-2 | 324 | 59.1 | Not available | 3.2 |
| | 16,69 | Axs:Us | 256 | 59.4 (± 0.4) | 54.7 | 2.1 |
| | 18.85 | Axs:Um | 822 | 61.3 (± 1.5) | 29.9 | 0.8 |
| | 30.71 | Am1:Uxs | 615 | 56.9 | Not available | 5.3 |
| | 39,47 | PolyAU "Pre-Run" | 321 | Nt | Not available | Nt |
| Group 4 | 170,9 | Axs:Uxs | 156 | 57,3 | 52.4 | 5.4 |

The various polyAU compositions were also characterized based on the distribution of fragment lengths within the compositions, as assessed by SEC-MALLS. For the compositions for which such information was available, Table 11 shows the cumulative distribution of molecular weights, defined by W(M), corresponding to the mass fraction of polymers in the sample having a molecular weight less than M.

The calculation of W(M) was carried out as follows:

$$W(M) = \frac{\sum_{M'<M} C_{M'}}{\sum_{eachM'} C_{M'}}$$

where $C_{M'}$ is the mass concentration per slice, for a slice having the molecular weight M'. The summations are taken over a chromatographic peak, which contains many slices of data. Thus, one has the equation as follows : $W(M_1) = \frac{C_1}{C_1 + .... + C_n}$ , where n is the number of slices (for example, n=172 for Axs:Us and $W(M_1)$=0,001187)

Thus to obtain the molar fraction, $C_i$ is divided by the molar mass $M_i$ of the slice being considered. However, the $M_i$ is an approximation, since the denominator C1+...+Cn would need to be modified. Since it is not possible to obtain individual values for the $C_i$ one can choose to divide C1+...+Cn by Mn, the mean molecular weight of the sample (e.g. Mn=362 000 g/mol for Axs :Us).

To obtain the cumulative distribution of molecular weights $W_m(M_i)$ for each slice, one can carry out the following calculation:

$$W_m(M_{i+1}) = W_m(M_i) + \frac{W(M_{i+1}) - W(M_i)}{M_i} \times M_n$$

Table 11

| | dsRNA | >100 kD | >200 kD | >300 kD | >400 kD | >500 kD | >600 kD | >700 kD | >800 kD | >900 kD | >1000 kD | >1100 kD | >1200 kD | >1300 kD | >1400 kD | >1500 kD |
|---|---|---|---|---|---|---|---|---|---|---|---|---|---|---|---|---|
| Group 1 | Am2:Um | 100 | 100 | 90,9 | 82,7 | 76,8 | 71,8 | 67,1 | 62,8 | 58,5 | 54,5 | 50,7 | 47 | 43,3 | 40 | 37 |
| Group 2 | Am1:Us | 100 | 83,3 | 66 | 52 | 41,2 | 32,5 | 26 | 21,1 | 17,2 | 13,5 | 11,5 | 9,4 | 7,8 | 6,4 | 5,2 |
| Group 3 | Axs:Us | 97,2 | 72 | 47,5 | 30,5 | 20,1 | 13,2 | 8,9 | 6 | 4 | 2,7 | 1,8 | 1,2 | 0,8 | 0,52 | 0,36 |
| - | Am1:Us-2 | 89 | 62 | 41 | 26,9 | 17,9 | 12,2 | 8,4 | 6 | 4,4 | 3,2 | 2,6 | 2,1 | 1,8 | 1,6 | 1,4 |
| - | AU690 | 78,5 | 47,5 | 34,2 | 26,5 | 20,7 | 16,2 | 13,1 | 10,7 | 8,6 | 7,2 | 6 | 5 | 4,2 | 3,5 | 3 |
| -Group 4 | Axs :Uxs | 69,8 | 35,1 | 20,5 | 14,3 | 11,4 | 9,8 | 9 | 8,6 | 8,3 | 8,2 | 8,1 | 8 | 7,99 | 7,96 | 7,95 |

*Results*

Bioactivity studies demonstrated that several factors influenced bioactivity at TLR3 as expressed by the EC50 for induction of human TLR3 signaling. Optimal polyAU compositions had characteristics that had certain minimum Mn (or Mw), certain distribution of dsRNA species (e.g. fewer than x % below y molecular weight), a minimum Tm and minimum hyperchromicity. Additionally, low heterogeneity as expressed by FWHM values could be obtained. As demonstrated in the preceding examples, certain combinations of ssRNA compositions were identified that gave rise to such optimal polyAU compositions.

*Mn and Mw*

As observed in Table 10 and Figure 23, increased Mn of polyAU compositions correlated with increased bioactivity at TLR3, with exceptions for lower than expected activity for those polyAU having either a Tm or hyperchromicity below the threshold for these parameters discussed below. In view of the fact that short (e.g. 45 mer polyAU) bind TLR3, this finding may suggest that the activity of polyAU in the endosome is influenced by its ability to cross-link multiple TLR3 proteins in order to induce TLR3 signaling, that dsRNA that binds a high number of TLR3 proteins present on a surface (e.g. endosome) will have improved signaling ability as a result of greatly decreased dissociation from the TLR3 proteins that longer dsRNA enter the endocytic pathway more efficiently than shorter dsRNA, or are less rapidly degraded in the endocytic pathway, compared to shorter dsRNA.

*Melting temperature.*

For the high molecular weight polyAU, the Tm also correlated with bioactivity at TLR3. All polyAU compositions having low Tm demonstrated poor bioactivity; for example Am1:Uxs shown in Table 10 demonstrated lower EC50 than would have been expected based only on its molecular weight. A Tm of about 59 °C is therefore desirable as a threshold for optimal bioactivity.

*Hyperchromicity*

Hyperchromicity values also appeared to influence the EC50. Axs:Um demonstrated a low hyperchromicity value and as with melting temperature, its EC50 was well below what would have been expected based only on its molecular weight. A value of hyperchromicity above 40%, 48% or 50% is therefore desirable as a threshold for optimal bioactivity.

*Molecular weight distribution of dsRNA species*

*Comparison: Groups 3 and 4.* There was difference in activity between composition Axs:Uxs and the next best set of compositions (Am1:Us-2, Axs:Us and AU690), which each had an Mn in the 300 kDa range. Referring to the distributions of molecular weight species as determined by SEC-MALLS, the latter three compounds were also distinguished by the presence of few species under 100 kDa (from 78% to 97%) and by a difference for dsRNA species in the range of 200-600 kDa. For each of the molecular weight levels assessed in this range, and particular at 300 kDa, 400 kDa, 500 kDa and 600 kDa, the compositions Am1:Us-2, Axs:Us and AU690 had a roughly two-fold or greater number of species falling into this weight range than Axs:Uxs. At molecular weights over 600 kDa, there was not a clear difference between Group 3 and Group 4, and Axs:Us has no less species than Group 3 compositions at molecular weights 700 kDa onwards. Interestingly, within the Group 3, the composition AU690, despite having a higher Mn, was less active than the Am1:Us-2 and Axs:Us. Upon comparison the distributions of molecular weight species as determined by SEC-MALLS, AU690 was found to have less species at each of the 100 to 300 kDa molecular weight ranges than Axs:Uxs, with the greatest difference at 200 kDa. Am1:Us-2 and Axs:Us each had no more than about 10% of dsRNA species less than 100 kDa, less than 40% of dsRNA species less than 200 kDa, and less than 60% of dsRNA species less than 300 kDa.

*Comparison: Groups 2 and 3.* There was another difference in biological activity between the set of compositions (Am1:Us-2, Axs:Us and AU690) and composition Am1:Us. The latter was characterized by a higher Mn (549 kDa) that the former three. Referring to the distributions of molecular weight species as determined by SEC-MALLS, Am1:Us began to differ from each of Am1:Us-2, Axs:Us and AU690 in the number of dsRNA species at about 300 kDa onward. At 400 kDa, Am1:Us contained 1.7-fold the number of species, and at each increment of 100 kDa thereafter Am1:Us contained well over 2-fold the number of dsRNA species. Am1:Us was free of species less than 100 kDa, had no more than about 40% of dsRNA species less than 300 kDa, less than 50% of dsRNA species less than 400 kDa, less than 70% of dsRNA species less than 600 kDa, less than 75% of dsRNA species less than 700 kDa and less than 80% of dsRNA species less than 800 kDa.

*Comparison: Groups I and 2*. There was another difference in biological activity between composition Am2:Um (group 2) and Am1:Us (group 3). The former was characterized by a higher Mn (1490 kDa) that the latter (549 kDa). Referring to the distributions of molecular weight species as determined by SEC-MALLS, Am1:Us began to differ from each of Am2:Um in the number of dsRNA species at about 300 kDa onward. The most marked difference was at about 600 kDa onward, as Am2:Um was still made of mostly of dsRNA species above this weight cutoff, while Am1:Us showed a rapid decrease in percent of species. Am2:Um contained less than 10% of species of less than 600 kDa, less than 20% of species of less than 400 kDa, less than 25% of species of less than 400 kDa, less than 30% of species of less than 600 kDa, less than 40% of species of less than 800 kDa and less than 50% of species under 1000 kDa, and less than 70%, 80% of species less than 1400, 1500 kDa respectively.

**EXAMPLE 13-GROUP 0 DSRNA COMPOSITIONS**

*Characteristics*

Two further DsRNA compositions were prepared by hybridization from four ssRNA compositions, described in Table 12: two polyU ssRNA compositions having a Mn of about 198k Da (Uml) and 456 kDa (Ul) respectively and two polyA ssRNA compositions having a Mn of about 467 kDa (Am3) and 551 kDa (Al) respectively. Hybridisation was conducted as for composition Am1:Us-3 described in Example 6 (about 70°C for about 10 min, condition 3 in Table 8) and allowed to cool.

**Table 12**

| ssRNA (IPH code) | Mw (g/mol) | Mn (g/mol) | Length (b) | Ip= Mw/Mn | Endotoxin Content (EU/mg) |
|---|---|---|---|---|---|
| **Am3** | 751 000 | 467 200 | 1415 | 1.6 | 0.2 |
| **Am4** | 1 012 000 | 551 500 | 1671 | 1.8 | Not done |
| **Um1** | 302 000 | 198 300 | 601 | 1.5 | Not done |
| **Um3** | 816500 | 455 700 | 1381 | 1.8 | < 0.5 |

The molecular weights, molecular weight distribution, polydispersity, melting temperature profiles and hyperchromicity were determined as described for other dsRNA. The characteristics of the resulting dsRNA compositions are shown in Tables 13 to 14. Table 15 shows the cumulative distribution of molecular weights for Group 0 compositions, as described in Example 12 for Group 1, 2 and 3 compositions; Group 1 composition Am2:Um is shown for comparison.

**Table 13**

| dsRNA (IPH code) | Mn (g/mol) | Length (bp) | Mw (g/mol) | Ip= Mw/Mn | Endotoxin (EU/mg) | Mn polyA | Mn polyU | EC50 (μg/ml) |
|---|---|---|---|---|---|---|---|---|
| **Am3:Um3** | 3 124 000 | 4726 | 4019 000 | 1.3 | 2.0 | 467 200 | 455 700 | 7.83 |
| **Am4:Um1** | 2 025 000 | 3064 | 2 729 000 | 1.3 | 5.7 | 551 500 | 198 300 | 7.1 |

**Table 14**

| Product **name** | Mw **(g/mol)** | Mn **(g/mol)** | Tm **(°C)** (mean value) | **Hyperchromicity (%)** | **1st** derivative **FWHM (°C)** |
|---|---|---|---|---|---|
| Am3:Um3 | 3 124000 | 4019000 | 62.6 | 54.4 | ~ 1.4 |
| **Am4:Um1** | 2 025 000 | 2 729 000 | 62 | 51.2 | ~ 2.0 |

| Table 15 | | | |
|---|---|---|---|
| | Group 0 | Groups 0 | Group 1 |
| dsRNA | Am3:Um3 (DB) | Am4:Um1 | Am2:Um |
| >500 kD | 99.4 | 96.6 | 76.8 |
| >1000 kD | 93 | 87.6 | 54.5 |
| >1500 kD | 90.45 | 75.7 | 37 |
| >2000 kD | 83.1 | 61.8 | - |
| >2500 kD | 74.4 | 48.3 | - |
| >3000 kD | 64.7 | 35.8 | - |

*In vitro and in vivo Bioactivity*

The Am3:Um3 dsRNA composition obtained was compared to commercial polyIC and Am1:Us-2 compositions as described in the Materials and Methods, in the following assays:

A. 293T-T3-ISRE luciferase , huTLR7 and huTLR8 gene reporter assays;
B. Dose-response assay of mouse cytokine production and cellular activation after dsRNA treatment in C57B1/6 mice; and
C. Analyses of in vivo anti-tumor efficacy of dsRNA in a xenogenic tumor model.

Figure 18 shows experiments for dose response of gene reporter assay on 293T-T3-ISRE luciferase. Am3:Um3 was 0,5 log times more efficient than Am1:Us-2 in triggering a TLR3 response.

Figure 19 shows dose response for in vivo cellular activation of spleen NK cells (at 24h post injection) (26A), IP-10 (26B), IFN$\alpha$ (26C) production in sera (at 2h post injection) from mice intravenously treated with polyIC, Am1:Us-2 and Am3:Um3. Results demonstrate that Am3:Um3 is significantly more potent than Am1:Us-2 in activation ofNK mice spleen cells, IP-10 and IFN$\alpha$ secretion. Moreover, Am3:Um3 is able to trigger IFNalpha secretion unlike Am1:Us-2 which cannot.

Figure 20 shows EC50 from dose response assays of TLR3 (Figure 20A), MDA-5 (Figure 20B) and RIGI (Figure 20C) gene reporter assay on 293T-T3-ISRE luciferase in comparison with control (Figure 20D) for the dsRNA Am1:Us-2, Am3:Um3 and polyIC delivered with lipofectamine, demonstrating the selectivity of polyAU over polyIC in inducing a selective TLR3 signalling.

Figure 21 shows EC50 dose response in huTLR7 and huTLR8 gene reporter assay (293T-TLR7 (or TLR8)-ISRE luciferase (range: 0.001 to 1000 $\mu$g/ml, 1/10$^{th}$ dilution) for the dsRNA Am1:Us-2, Am3:Um3 and polyIC. DsRNA concentration is indicated on the x-axis and fold increase in luciferase activity is indicated on the y-axis. This data confirms the selectivity of polyAU in inducing a selective TLR3 signalling as polyIC induces significant TLR7 and TLR8 signalling. PolyIC stimulates endogenous receptors on the 293T cells which is not specific to TLR7 or 8.

Figure 22A, B and C show anti-tumor efficacy of Am1:Us and Am3:Um3 in an HCC1806 xenogenic tumor model, in SCID-NOD mice (5 daily intravenous injections/week of 500 $\mu$g/mice dose, starting at day 5 post tumor cells injection). Figure 22A reports the comparison between the mean of tumor size in the mice of the control group (NaCl, full squares, full line), the Am1:Us group (full dots, full line) and the Am3:Um3 group (open dots, dashed line). Figure 22B reports on the x-axis the individual tumor volume in mice in the Am1:Us-3 group, according to time in days on the y-axis. Figure 22C reports on the x-axis the individual tumor volume in mice in the Am3:Um3 group, according to time in days on the y-axis. These results highlight the improved antitumoral effect of Am3:Um3 like compositions, eg of high Mn, compared to smaller dsRNA, ie Am1:Us-3 composition, in a xenogenic mice model.

These results demonstrate that compositions of high molecular weight, and in particular compositions of Group 0 have greater in vitro and in vivo bioactivity, including in the preventing or reducing tumor growth.

**EXAMPLE 14-DS-RNA RESPONSE ON 293T.ISRE.RIGI AND MDA-5 TRANSFECTANTS**

RIGI and MDA-5 are receptors for double-stranded RNA. TLR3 receptor specificity was studied by comparing the activity of different dsRNA (either polyAU or polyIC) based on response to PolyIC and polyAU in 293T cells transfected with an expression vector containing either RIGI or MDA-5 under the control of interferon-stimulated response element (ISRE). Results demonstrated that both polyAU tested (Aml:Us-2 and Am1:Us-3) had selectivity for TLR3 over the other dsRNA receptors RIGI and MDA-5. The more potent Am1:Us-3 demonstrated similar potency in TLR3 reporter activity as polyIC; polyIC however showed significant activity at both RIGI and MDA5 (at least about 100 fold higher than polyAU compositions), and also in control demonstrating that polyIC and polyAU have markedly different receptor specificity and that polyIC likely bind receptors other than TLR3. Results are shown in Figure 23 (standard deviation shown of triplicates wells in one experiment), the y-axis showing fold increase above medium conditions and the x-axis the dsRNA concentration (μg/ml). The control cell line is 293T-ISRE (no receptor, just the reporter gene). Some residual signal on the control cell line is believed to be due to signalling via the TLR3 or other endogenes, which could also be the case for compositions on RIG and MDA-5 transfectants. Figure 24 shows the results of response to PolyIC and polyAU in 293T cells transfected with an expression vector containing either TLR3, RIGI, MDA-5 or no receptor under the control of interferon-stimulated response element (ISRE), in the presence and absence of the transfection agent lipofectamine. It can be seen that even when complexed with transfection agent and brought into a cell, Am1:Us-2 and Am1:Us-3 do not acquire ability to signal through RIG or MDA-5.

## EXAMPLE 15-POLYAU INDUCED APOPTOSIS IS INHIBITED BY TRIF AND TLR3 LENTIVIRUS SHRNA

Am1:Us-3 was tested for TLR3 selectivity in apoptosis induction in various tumor cells (HCC38, HCC1806 and A375) in the presence and absence of pre-treatment of the cells with lentivirus shRNA targeting TRIF (for HCC38 and HCC1806) or both TRIF and TLR3 (A375). Figure 25 shows the results for HCC38 and HCC1806, where apoptosis induction of Am1:Us-3 was inhibited by both TRIF in each cell line, as well as TLR3 shRNA where tested, indicating that apoptosis induction in a variety of tumor cell types is mediated by TLR3, and that the polyAU signaling is selective for TLR3 for induction of apoptosis.

## EXAMPLE 16-INDUCTION OF TLR3 BY AMI: US-2 AND SENSITIZATION WITH IFN-ALPHA

Am1:Us-3 was also tested for apoptosis induction A375 melanoma cells in the presence and absence of pre-treatment (the day preceding treatment with Am1:Us-3) with IFN-alpha. Pretreatment with IFN-alpha induced a dramatic increase in apoptosis mediated by Am1:Us-3, as shown in Figure 26.

## EXAMPLE 17-COMPARISON OF POLYAU DOSING REGIMENS IN MICE FOR APOPTOSIS-INDUCING COMPOSITIONS

Am1:Us-3 was then tested for its anti-tumoral activity in several administration regimens given a fixed dosage. Groups of six Nod-SCID mice per group received A375 human melanoma tumors ($5.10^6$ cells on day 1), and seven days later received the first treatment with either IFN-alpha ($10^5$ IU) or NaCl 0.15M, and in the "5x Am1:Us-3" regimen, also the first dose of Am1:Us-3. On the following day each group except for the "2x IFN-alpha" group received a dose of Am1:Us-3. The different schedules of treatment then followed repeated six times shown in Figure 27, where each hash mark on the horizontal treatment timelines indicates a day 1 to 5 in a week, with days 6 and 7 following (not indicated by hash marks) between each group of 5 hash marks. The arrows above the treatment timeline indicate dosing with Am1:Us-3, and the arrows below the treatment timeline indicate dosing with IFN-alpha. Group "5x Am1:Us-3" received five consecutive injections of Am1:Us-3 per week on days 1 to 5. Group "2x Am1:Us-3" received two injections of Am1: Us-03 per week, on days 2 and 5 respectively. Group "2x Am1:Us-3 + IFN-alpha" received two injections of Am1:Us-03 per week, on days 2 and 5 respectively, and two injections of IFN-alpha, on days 1 and 4 respectively. Group "1x Am1: Us-3 + IFN-alpha" received one injection of Am1:Us-3 per week on day 5, and one injection of IFN-alpha on day 4. Group "2x IFN-alpha" received two injections of IFN-alpha per week, on days 1 and 4 respectively.

Results are shown in Figure 28. Each treatment regimen involving a step of administering a composition that can upregulate TLR3 expression in a cell had efficacy in delaying tumor growth, and best results were obtained when such two-step treatment was repeated with the fewest intervening days. It was observed that 5x Am1:Us-3 induced the greatest delay in tumor growth. Both 2x Am1:Us-3 and 2x Am1:Us-3 + IFN-alpha induced significant delay and tumor growth, and each of these, as well as 5x Am1:Us-3, were more effective than 1x Am1:Us-3 + IFN-alpha and 2x IFN-alpha in delaying tumor growth. It therefore appeared that at least two doses of dsRNA per week were required to yield the best results in this model, and that good results were obtained even when the second dose of the week was administered on the third day after (i.e. 72 hours after) the preceding dose of the week. The Nod-SCID model therefore demonstrates the efficacy of a direct pro-apoptotic mechanism of the dsRNA on tumor cells.

One explanation of these results was that Am1:Us-3 can be used similarly to IFN-alpha to induce upregulation of TLR3, thereby sensitizing such cells to treatment with a TLR3 agonist (e.g. sensitizing cells to TLR3 agonist-induced apoptosis). In order to detect a correlation with TLR3 expression, melanoma cells from the treated mice were examined in an immunohistochemistry assay using an anti-TLR3 antibody. Tissue sections were examined at day 26 following injection of A375 cells, and TLR3 was up-regulated at 24 and 48 hours following treatment with IFN-alpha or Am1:Us-3. TLR3 expression decreased at 72 hours. Preferred treatment regimens will therefore typically involve administering a TLR3 agonist composition (e.g. dsRNA, polyAU) capable of inducing apoptosis of a TLR3-expression cell within 72, 48 or preferably 24 hours following the administration of a first composition capable of inducing TLR3 expression (e.g. dsRNA, polyAU, IFN-alpha).

In order to assess whether the effect of Am1:Us-3 was mediated substantially by, both 2x Am1:Us-3 and 2x Am1:Us-3 + IFN-alpha treatment regimens were repeated in mice who received A375 cells stably infected with shRNA TRIF in order to inhibit TLR3 signaling. It was observed in preliminary studies that both the 2x Am1:Us-3 and 2x Am1: Us-3 + IFN-alpha treatments were unable to induce delay of tumor growth in mice, suggesting that delay of tumor growth is mediated substantially by TLR3. Results are shown in Figure 29.

Finally, in order to assess whether non-apoptotic mechanisms contributed to tumor growth delay induced by Am1:Us-3, both 2x Am1:Us-3 and 2x Am1:Us-3 + IFN-alpha treatment regimens were repeated in the mice receiving A375 tumors, together with administration of 100 μg anti-asialo GM1 antibody (i.p.). The antibody was administered once a week, on the same day as the first day of treatment (e.g. with IFN-alpha or water) in each week. It was observed that both 2x Am1:Us-3 and 2x Am1:Us-3 + IFN-alpha treatments induce delay of tumor growth in mice, suggesting that apoptosis and not an immunomodulatory effect mediated by NK cells are the mechanism by which tumor growth is delayed.

**EXAMPLE 18-OPTIMAL IN VIVO DOSES**

Based on the in vivo experiments for cellular activation and cytokine production, optimal doses for in vivo uses were estimated. Efficient doses in mice were found in the range of about 200 μg for Am1:Us-3, which corresponds to about 10-25 mg/kg in a mouse. Equivalent doses in humans, dividing the mouse dose in mg/kg by a factor of 12 suggests that a dose of 0.8-2 mg/kg in humans should provide TLR3-mediated cellular activation and cytokine production. A second estimate was based on optimal doses in mice from pharmacokinetic and tumor experiments, where 25 mg/kg was found to be effective. A human equivalent dose would be about 2 mg/kg or 4 μmol/kg. However, as toxicity was not limiting, useful doses in humans can be higher than about 2 mg/kg, for example at least about 100 mg per patient dose. Preferred doses in humans are expected to be in the range of 1 to 50 mg/kg, or I to 20 mg/kg or 1 to 10 mg/kg. Doses could also be envisaged up to 100 mg/kg (400μmol).

## Claims

**1.** A pharmaceutical composition comprising (a) an isolated polyAU composition capable of inducing TLR3 mediated signaling and **characterized by** a $M_n$ of at least 250 kDa, wherein less than 5% of fragments have a molecular weight less than 100 kDa, as determined by SEC-MALLS, and an Ip of less than 2, and (b) a pharmaceutically acceptable carrier or 'excipient, wherein the composition has an endotoxin level of no more than about 1.0 EU/mg.

**2.** The composition of claim 1, wherein the isolated polyAU composition capable of inducing TLR3 mediated signaling is **characterized by** a $M_n$ of at least 1500 kDa, as determined by SEC-MALLS, and:

a. less than about 5% of dsRNA species having a molecular weight less than about 500 kDa;

b. less than about 20% of dsRNA species having a molecular weight less than about 1000 kDa:
c. less than about 50% of dsRNA species having a molecular weight less than about 1500 kDa;
d. an Ip of less than 2;
e. a Tm of at least 60°C;
f. a hyperchromicity of at least 40%; and
g. optionally, an FWHM of less than 5 °C, optionally less than 3° C.

**3.** The composition of one of claims 1 or 2, comprising complexes of a first composition comprising single-stranded polymers **characterized by** an $M_n$ of between 400 kDa and 600 kDa, and a second composition comprising single-stranded polymers **characterized by** an $M_n$ of between 100 kDa and 600 kDa.

**4.** A composition according to claim 1. wherein the polyAU composition is **characterized by** a $M_n$ of at least 500 kDa.

**5.** A composition selected from the group consisting of:

a. an isolated polyAU polymer composition capable of inducing TLR3 mediated signaling and **characterized by** an $M_n$ for the double-stranded polyAU polymers of about 1490 kDa, wherein the composition comprises less than about 5 % of fragments having a molecular weight less than about 300 kDa and/or 600 kDa; less than about 15 % of fragments having a molecular weight less than about 800 kDa; less than about 25 % having a molecular weight less than about 1000 kDa; and/or less than about 50 % having a molecular weight less than about 1400 kDa, as determined by SEC-MALLS, and an Ip of less than 2;
b. an isolated polyAU polymer composition capable of inducing TLR3 mediated signaling and **characterized by** an $M_u$ of about 1280 kDa, as determined by SEC-MALLS, and an Ip of less than 2;
c. an isolated polyAU polymer composition capable of inducing TLR3 mediated signalling and **characterized by** an $M_n$ of about 3124 kDa, wherein the composition comprises less than less than about 5% of dsRNA species having a molecular weight less than about 500 kDa, less than about 20% of dsRNA species having a molecular weight less than about 1000 kDa; and less than about 50% of dsRNA species having a molecular weight less than about 1500 kDa as determined by SEC-MALLS, and an Ip of less than 2; and
d. an isolated polyAU polymer composition capable of inducing TLR3 mediated signalling and **characterized by** an $M_n$ for the double-stranded polyAU polymers of about 2025 kDa, wherein the composition comprises less than about 5% of dsRNA species having a molecular weight less than about 500 kDa, less than about 20% of dsRNA species having a molecular weight less than about 1000 kDa; and less than about 50% of dsRNA species having a molecular weight less than about 1500 kDa as determined by SEC-MALLS, and an Ip of less than 2.

**6.** The composition any one of the above claims, wherein the composition is substantially free of endotoxins.

**7.** The composition of any one of the above claims, wherein the composition has (a) an EC50 value for TLR3 agonist activity which is no more than about 2-$log_{10}$ greater than that of a reference polyIC composition, as determined by the ability to induce TLR3 signaling in a reporter gene assay, and (b) an EC50 value for induction of a cytokine which is at least about 2-$log_{10}$ greater than that of said reference polyIC composition, as determined by the ability to induce said cytokine in human PBMC in vitro.

**8.** The composition of claims 1 or 2, wherein the composition has selective agonist and/or binding activity at TLR3 compared to RIGI or MDA5.

**9.** The composition of any one of the above claims, wherein said composition induces cellular activation, cytokine production and/or pro-apoptotic activity that can be at least 50% neutralized by a TLR3-inhibiting agent, optionally wherein the agent is an anti-TLR3 antibody or a TRIF shRNA.

**10.** The composition of any one of the above claims, wherein said composition has a TLR3-binding activity that can be at least 50% neutralized by a TLR3 binding agent, optionally wherein the agent is an anti-TLR3 antibody.

**11.** A pharmaceutical formulation comprising the composition according to any one of the above claims, adapted for parenteral, intravenous, subcutaneous, or intravitreal administration.

**12.** The composition of claim 11, wherein the formulation is adapted for subcutaneous administration.

**13.** The composition of any one of the above claims, wherein said composition comprises a second therapeutic agent.

**14.** The composition of any one of the above claims, wherein said composition comprises one or a plurality of viral or tumor antigen(s).

**15.** A process for producing a polyAU composition comprising mixing a poly A composition **characterized by** an $M_n$ of at least 300 kDa and a poly U composition **characterized by** an $M_n$ of at least 100 kDa as determined by SEC-MALLS, in a buffer for a period of time of between 5 minutes and 4 hours and at a temperature of between room temperature and 100 °C.

**16.** The process of claim 15, wherein said polyA composition is **characterized by** a $M_n$ of between 400 kDa and 600 kDa and polyU composition is **characterized by** a $M_n$ of between 150 kDa and 400 kDa.

**17.** The process of claims 15 or 16, wherein said temperature is between 60 °C and 75 °C and said period of time is between 5 minutes and 4 hours, optionally in a buffer of NaCl 0.15M.

**18.** A composition according to claims 1 to 10, for use in treating or preventing a tumor.

**19.** A composition according to claims 1 to 10, for use in treating or preventing an infection.

**20.** A composition according to claims 1 to 10, for use in treating or preventing an ocular angiogenesis disorder.

**21.** A composition according to claim 1 to 10 for use in treating a tumor, comprising:

a. selecting a patient having a tumor which expresses TLR3, and
b. administering to the patient an effective amount of said composition.

**22.** The composition according to claim 1 to 10, wherein the composition is for administration in combination with a second therapeutic agent.

**23.** The composition according to claim 22, wherein said second therapeutic agent is an antigen.

**24.** The composition according to claim 23, wherein the antigen comprises one or a plurality of purified viral or tumor antigen(s).

**25.** The composition of claims 1 to 10, for administration in a dose for humans of between 1 mg/kg and 50 mg/kg.

## Patentansprüche

**1.** Pharmazeutische Zusammensetzung, umfassend (a) eine isolierte PolyAU-Zusammensetzung mit der Fähigkeit zur Induktion von TLR3-vermittelter Signalgebung und **gekennzeichnet durch** einen $M_n$-Wert von wenigstens 250 kDa, wobei weniger als 5% Fragmente ein Molekulargewicht von weniger als 100 kDa, wie anhand von SEC-MALLS bestimmt, aufweisen, und einen Ip-Wert von weniger als 2 und (b) einen pharmazeutisch unbedenklichen Träger- oder Hilfsstoff, wobei die Zusammensetzung eine Endotoxinkonzentration von nicht mehr als etwa 1,0 EU/mg aufweist.

**2.** Zusammensetzung nach Anspruch 1, wobei die isolierte PolyAU-Zusammensetzung mit der Fähigkeit zur Induktion von TLR3-vermittelter Signalgebung durch einen $M_n$-Wert von wenigstens 1500 kDa, wie anhand von SEC-MALLS bestimmt, und:

a. weniger als etwa 5% dsRNA-Spezies, die ein Molekulargewicht von weniger als etwa 500 kDa aufweisen;
b. weniger als etwa 20% dsRNA-Spezies, die ein Molekulargewicht von weniger als etwa 1000 kDa aufweisen;
c. weniger als etwa 50% dsRNA-Spezies, die ein Molekulargewicht von weniger als etwa 1500 kDa aufweisen;
d. einen Ip-Wert von weniger als 2;
e. einen Tm-Wert von wenigstens 60 °C;
f. eine Hyperchromizität von wenigstens 40%; und
g. gegebenenfalls einen FWHM-Wert von weniger als 5 °C, gegebenenfalls weniger als 3 °C,

gekennzeichnet ist.

**3.** Zusammensetzung nach Anspruch 1 oder 2, umfassend Komplexe einer einzelsträngige Polymere umfassenden ersten Zusammensetzung, **gekennzeichnet durch** einen $M_n$-Wert zwischen 400 kDa und 600 kDa, und einer einzelsträngige Polymere umfassenden zweiten Zusammensetzung, **gekennzeichnet durch** einen $M_n$-Wert zwischen 100 kDa und 600 kDa.

**4.** Zusammensetzung gemäß Anspruch 1, wobei die PolyAU-Zusammensetzung durch einen $M_n$-Wert von wenigstens 500 kDa gekennzeichnet ist.

**5.** Zusammensetzung, ausgewählt aus der Gruppe:

a. eine isolierte PolyAU-Polymer-Zusammensetzung mit der Fähigkeit zur Induktion von TLR3-vermittelter Signalgebung und **gekennzeichnet durch** einen $M_n$-Wert für die doppelsträngigen PolyAU-Polymere von etwa 1490 kDa, wobei die Zusammensetzung weniger als etwa 5% Fragmente, die ein Molekulargewicht von weniger als etwa 300 kDa und/oder 600 kDa aufweisen; weniger als etwa 15% Fragmente, die ein Molekulargewicht von weniger als etwa 800 kDa aufweisen; weniger als etwa 25%, die ein Molekulargewicht von weniger als etwa 1000 kDa aufweisen; und/oder weniger als etwa 50%, die ein Molekulargewicht von weniger als etwa 1400 kDa aufweisen, wie anhand von SEC-MALLS bestimmt, umfasst, und einen Ip-Wert von weniger als 2;
b. eine isolierte PolyAU-Polymer-Zusammensetzung mit der Fähigkeit zur Induktion von TLR3-vermittelter Signalgebung und **gekennzeichnet durch** einen $M_n$-Wert von etwa 1280 kDa, wie anhand von SEC-MALLS bestimmt, und einen Ip-Wert von weniger als 2;
c. eine isolierte PolyAU-Polymer-Zusammensetzung mit der Fähigkeit zur Induktion von TLR3-vermittelter Signalgebung und **gekennzeichnet durch** einen $M_n$-Wert von etwa 3124 kDa, wobei die Zusammensetzung weniger als etwa 5% dsRNA-Spezies, die ein Molekulargewicht von weniger als etwa 500 kDa aufweisen, weniger als etwa 20% dsRNA-Spezies, die ein Molekulargewicht von weniger als etwa 1000 kDa aufweisen; und weniger als etwa 50% dsRNA-Spezies, die ein Molekulargewicht von weniger als etwa 1500 kDa aufweisen, wie anhand von SEC-MALLS bestimmt, umfasst, und einen Ip-Wert von weniger als 2; und
d. eine isolierte PolyAU-Polymer-Zusammensetzung mit der Fähigkeit zur Induktion von TLR3-vermittelter Signalgebung und **gekennzeichnet durch** einen $M_n$-Wert für die doppelsträngigen PolyAU-Polymere von etwa 2025 kDa, wobei die Zusammensetzung weniger als etwa 5% dsRNA-Spezies, die ein Molekulargewicht von weniger als etwa 500 kDa aufweisen, weniger als etwa 20% dsRNA-Spezies, die ein Molekulargewicht von weniger als etwa 1000 kDa aufweisen; und weniger als etwa 50% dsRNA-Spezies, die ein Molekulargewicht von weniger als etwa 1500 kDa aufweisen, wie anhand von SEC-MALLS bestimmt, umfasst, und einen Ip-Wert von weniger als 2.

**6.** Zusammensetzung nach einem der obigen Ansprüche, wobei die Zusammensetzung weitgehend frei von Endotoxinen ist.

**7.** Zusammensetzung nach einem der obigen Ansprüche, wobei die Zusammensetzung (a) einen EC50-Wert für TLR3-Agonisten-Aktivität, der nicht mehr als etwa 2-$\log_{10}$ größer als der Wert einer PolyIC-Referenzzusammensetzung ist, wie anhand des Vermögens, TLR3-Signalgebung in einem Reportergen-Test zu induzieren, bestimmt, und (b) einen EC50-Wert für die Induktion eines Cytokins, der mindestens etwa 2-$\log_{10}$ größer als der Wert der PolyIC-Referenzzusammensetzung ist, wie anhand des Vermögens, das Cytokin in vitro in menschlichen PBMC zu induzieren, bestimmt, aufweist.

**8.** Zusammensetzung nach Anspruch 1 oder 2, wobei die Zusammensetzung selektive Agonisten- und/oder Bindungsaktivität an TLR3 im Vergleich zu RIGI oder MDA5 aufweist.

**9.** Zusammensetzung nach einem der obigen Ansprüche, wobei die Zusammensetzung zelluläre Aktivierung, Cytokinproduktion und/oder proapoptotische Aktivität, die sich zu wenigstens 50% von einem TLR3 hemmenden Agens neutralisieren lässt, induziert, wobei es sich gegebenenfalls bei dem Agens um einen Anti-TLR3-Antikörper oder eine TRIF-shRNA handelt.

**10.** Zusammensetzung nach einem der obigen Ansprüche, wobei die Zusammensetzung eine TLR3-Bindungsaktivität aufweist, die sich zu wenigstens 50% von einem TLR3 bindenden Agens neutralisieren lässt, wobei es sich gegebenenfalls bei dem Agens um einen Anti-TLR3-Antikörper handelt.

**11.** Pharmazeutische Formulierung, umfassend die Zusammensetzung gemäß einem der obigen Ansprüche, angepasst an eine parenterale, intravenöse, subkutane oder intravitreale Verabreichung.

**12.** Zusammensetzung nach Anspruch 11, wobei die Formulierung an eine subkutane Verabreichung angepasst ist.

**13.** Zusammensetzung nach einem der obigen Ansprüche, wobei die Zusammensetzung ein zweites Therapeutikum umfasst.

**14.** Zusammensetzung nach einem der obigen Ansprüche, wobei die Zusammensetzung ein oder mehrere Virus- oder Tumorantigen(e) umfasst.

**15.** Verfahren zur Herstellung einer PolyAU-Zusammensetzung, bei dem man eine durch einen $M_n$-Wert von wenigstens 300 kDa gekennzeichnete PolyA-Zusammensetzung und eine durch einen $M_n$-Wert von wenigstens 100 kDa, wie anhand von SEC-MALLS bestimmt, gekennzeichnete PolyU-Zusammensetzung in einem Puffer über einen Zeitraum zwischen 5 Minuten und 4 Stunden sowie bei einer Temperatur zwischen Raumtemperatur und 100 °C mischt.

**16.** Verfahren nach Anspruch 15, wobei die PolyA-Zusammensetzung durch einen $M_n$-Wert zwischen 400 kDa und 600 kDa und die PolyU-Zusammensetzung durch einen $M_n$-Wert zwischen 150 kDa und 400 kDa gekennzeichnet ist.

**17.** Verfahren nach Anspruch 15 oder 16, wobei die Temperatur zwischen 60 °C und 75 °C liegt und der Zeitraum eine Dauer zwischen 5 Minuten und 4 Stunden aufweist, gegebenenfalls in einem Puffer von 0,15 M NaCl.

**18.** Zusammensetzung gemäß Anspruch 1 bis 10, zur Verwendung bei der Behandlung oder Vorbeugung eines Tumors.

**19.** Zusammensetzung gemäß Anspruch 1 bis 10, zur Verwendung bei der Behandlung oder Vorbeugung einer Infektion.

**20.** Zusammensetzung gemäß Anspruch 1 bis 10, zur Verwendung bei der Behandlung oder Vorbeugung einer Störung der okulären Angiogenese.

**21.** Zusammensetzung gemäß Anspruch 1 bis 10 zur Verwendung bei der Behandlung eines Tumors, wobei man:

a. einen Patienten mit einem Tumor, der TLR3 exprimiert, auswählt und
b. dem Patienten eine wirksame Menge der Zusammensetzung verabreicht.

**22.** Zusammensetzung gemäß Anspruch 1 bis 10, wobei die Zusammensetzung zur Verabreichung in Kombination mit einem zweiten Therapeutikum vorgesehen ist.

**23.** Zusammensetzung gemäß Anspruch 22, wobei es sich bei dem zweiten Therapeutikum um ein Antigen handelt.

**24.** Zusammensetzung gemäß Anspruch 23, wobei das Antigen ein oder mehrere aufgereinigte Virus- oder Tumorantigen(e) umfasst.

**25.** Zusammensetzung nach Anspruch 1 bis 10, zur Verabreichung in einer Dosis für den Menschen zwischen 1 mg/kg und 50 mg/kg.

**Revendications**

**1.** Composition pharmaceutique comprenant (a) une composition de polyAU isolée étant capable d'induire une signalisation ayant lieu par médiation des TLR3 et **caractérisée par** une $M_n$ d'au moins 250 kDa, dans laquelle moins de 5% de fragments ont un poids moléculaire inférieur à 100 kDa, tel que déterminé par SEC-MALLS, et un Ip inférieur à 2, et (b) un véhicule ou excipient acceptable d'un point de vue pharmaceutique, dans laquelle la composition a un taux d'endotoxines de 1,0 EU/mg environ maximum.

**2.** Composition selon la revendication 1, dans laquelle la composition de polyAU isolée étant capable d'induire une signalisation qui a lieu par médiation des TLR3 est **caractérisée par** une $M_n$ d'au moins 1500 kDa, tel que déterminé par SEC-MALLS, et :

a. moins de 5% environ des espèces d'ARNdb ayant un poids moléculaire inférieur à 500 kDa environ ;
b. moins de 20% environ des espèces d'ARNdb ayant un poids moléculaire inférieur à 1000 kDa environ ;
c. moins de 50% environ des espèces d'ARNdb ayant un poids moléculaire inférieur à 1500 kDa environ ;

d. un Ip inférieur à 2 ;
e. une Tm d'au moins 60°C ;
f. une hyperchromicité d'au moins 40% ; et
g. en option, une FWHM inférieure à 5°C, facultativement inférieure à 3°C.

**3.** Composition, selon l'une des revendications 1 ou 2, comprenant des complexes d'une première composition, comprenant des polymères en simple brin étant **caractérisés par** une $M_n$ comprise entre 400 kDa et 600 kDa, et d'une deuxième composition comprenant des polymères en simple brin étant **caractérisés par** une $M_n$ comprise entre 100 kDa et 600 kDa.

**4.** Composition selon la revendication 1, dans laquelle la composition de polyAU est **caractérisée par** une $M_n$ d'au moins 500 kDa.

**5.** Composition choisie dans le groupe constitué par :

a. une composition de polymères de polyAU isolée étant capable d'induire une signalisation ayant lieu par médiation des TLR3 et **caractérisée par** une $M_n$ de 1490 kDa environ pour les polymères de polyAU en double brin, dans laquelle la composition comprend moins de 5% environ de fragments ayant un poids moléculaire inférieur à 300 kDa et/ou 600 kDa environ ; moins de 15% environ de fragments ayant un poids moléculaire inférieur à 800 kDa environ ; moins de 25% environ ayant un poids moléculaire inférieur à 1000 kDa environ ; et/ou moins de 50% environ ayant un poids moléculaire inférieur à 1400 kDa environ, tel que déterminé par SEC-MALLS, et un Ip inférieur à 2 ;
b. une composition de polymères de polyAU isolée étant capable d'induire une signalisation ayant lieu par médiation des TLR3 et **caractérisée par** une $M_n$ de 1280 kDa environ, tel que déterminé par SEC-MALLS, et un Ip inférieur à 2 ;
c. une composition de polymères de polyAU isolée étant capable d'induire une signalisation ayant lieu par médiation des TLR3 et **caractérisée par** une $M_n$ de 3124 kDa environ, dans laquelle la composition comprend moins de 5% environ d'espèces d'ARNdb ayant un poids moléculaire inférieur à 500 kDa environ, moins de 20% environ d'espèces d'ARNdb ayant un poids moléculaire inférieur à 1000 kDa environ ; et moins de 50% environ d'espèces d'ARNdb ayant un poids moléculaire inférieur à 1500 kDa environ, tel que déterminé par SEC-MALLS, et un Ip inférieur à 2 ; et
d. une composition de polymères de polyAU isolée étant capable d'induire une signalisation ayant lieu par médiation des TLR3 et **caractérisée par** une $M_n$ de 2025 kDa environ pour les polymères de polyAU en double brin, dans laquelle la composition comprend moins de 5% environ d'espèces d'ARNdb ayant un poids moléculaire inférieur à 500 kDa environ, moins de 20% environ d'espèces d'ARNdb ayant un poids moléculaire inférieur à 1000 kDa environ ; et moins de 50% environ d'espèces d'ARNdb ayant un poids moléculaire inférieur à 1500 kDa environ, tel que déterminé par SEC-MALLS, et un Ip inférieur à 2.

**6.** Composition selon l'une quelconque des revendications ci-dessus, dans laquelle la composition est substantiellement dépourvue d'endotoxines.

**7.** Composition selon l'une quelconque des revendications ci-dessus, dans laquelle la composition a (a) une valeur d'EC50 pour l'activité d'agoniste des TLR3 qui est d'environ 2-$\log_{10}$ maximum supérieure à celle d'une composition de polyIC de référence, tel que déterminé par la faculté à induire la signalisation des TLR3 dans un dosage de gène rapporteur, et (b) une valeur d'EC50 pour l'induction d'une cytokine qui est d'environ 2-$\log_{10}$ au moins supérieure à celle de ladite composition de polyIC de référence, tel que déterminé par la faculté à induire ladite cytokine dans des PBMC humaines *in vitro.*

**8.** Composition selon les revendications 1 ou 2, dans laquelle la composition a une activité sélective d'agoniste et/ou de liaison au niveau des TLR3, en comparaison avec les RIGI ou MDA5.

**9.** Composition selon l'une quelconque des revendications ci-dessus, dans laquelle ladite composition induit une activation cellulaire, la production de cytokines et/ou une activité pro-apoptotique qui peut être neutralisée, au moins à 50%, par un agent d'inhibition des TLR3, facultativement dans laquelle l'agent est un anticorps anti-TLR3 ou un ARNsh TRIF.

**10.** Composition selon l'une quelconque des revendications ci-dessus, dans laquelle ladite composition a une activité de liaison aux TLR3 qui peut être neutralisée, au moins à 50%, par un agent de liaison aux TLR3, facultativement dans laquelle l'agent est un anticorps anti-TLR3.

**11.** Formulation pharmaceutique comprenant la composition, selon l'une quelconque des revendications ci-dessus, adaptée à une administration parentérale, intraveineuse, sous-cutanée ou intra-vitréenne.

**12.** Composition selon la revendication 11, dans laquelle la formulation est adaptée à une administration sous-cutanée.

**13.** Composition selon l'une quelconque des revendications ci-dessus, dans laquelle ladite composition comprend un deuxième agent thérapeutique.

**14.** Composition selon l'une quelconque des revendications ci-dessus, dans laquelle ladite composition comprend un antigène viral ou tumoral ou bien une pluralité d'entre eux.

**15.** Processus de production d'une composition de polyAU comprenant le mélange d'une composition de poly A, **caractérisée par** une $M_n$ d'au moins 300 kDa, et d'une composition de poly U, **caractérisée par** une $M_n$ d'au moins 100 kDa, tel que déterminé par SEC-MALLS, dans un tampon pendant une période de temps comprise entre 5 minutes et 4 heures ainsi qu'à une température comprise entre la température ambiante et 100°C.

**16.** Processus selon la revendication 15, dans lequel ladite composition de polyA est **caractérisée par** une $M_n$ comprise entre 400 kDa et 600 kDa et la composition de polyU est **caractérisée par** une $M_n$ comprise entre 150 kDa et 400 kDa.

**17.** Processus selon les revendications 15 ou 16, dans laquelle ladite température est comprise entre 60°C et 75°C et ladite période de temps est comprise entre 5 minutes et 4 heures, facultativement dans un tampon de NaCl à 0,15 M.

**18.** Composition, selon les revendications 1 à 10, destinée à être utilisée dans le traitement ou la prévention d'une tumeur.

**19.** Composition, selon les revendications 1 à 10, destinée à être utilisée dans le traitement ou la prévention d'une infection.

**20.** Composition, selon les revendications 1 à 10, destinée à être utilisée dans le traitement ou la prévention d'un trouble de l'angiogenèse oculaire.

**21.** Composition, selon les revendications 1 à 10, destinée à être utilisée dans le traitement d'une tumeur, comprenant les étapes consistant à :

a. sélectionner un patient ayant une tumeur qui exprime des TLR3 et
b. administrer au patient une quantité efficace de ladite composition.

**22.** Composition selon les revendications 1 à 10, dans laquelle la composition est destinée à une administration en combinaison avec un deuxième agent thérapeutique.

**23.** Composition selon la revendication 22, dans laquelle ledit deuxième agent thérapeutique est un antigène.

**24.** Composition selon la revendication 23, dans laquelle l'antigène comprend un antigène viral ou tumoral purifié ou bien une pluralité d'entre eux.

**25.** Composition, selon les revendications 1 à 10, destinée à une administration en une dose pour humains comprise entre 1 mg/kg et 50 mg/kg.

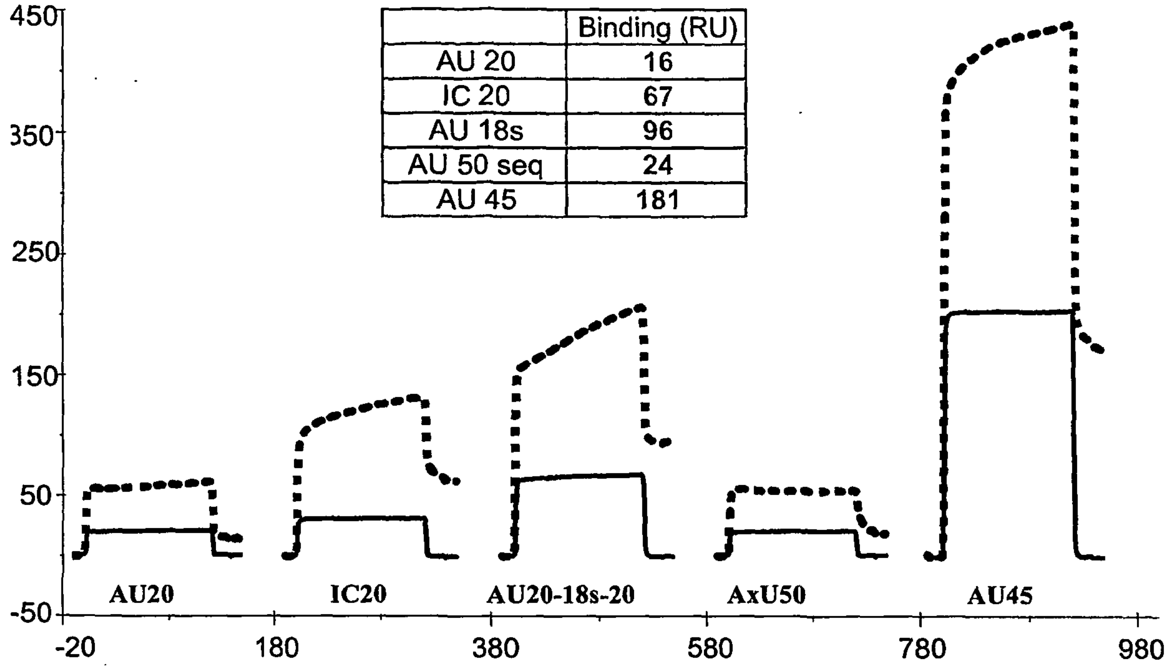

| | Binding (RU) |
|---|---|
| AU 20 | 16 |
| IC 20 | 67 |
| AU 18s | 96 |
| AU 50 seq | 24 |
| AU 45 | 181 |

450
350
250
150
50
-50
AU20
IC20
AU20-18s-20
AxU50
AU45
-20
180
380
580
780
980


Fig. 1

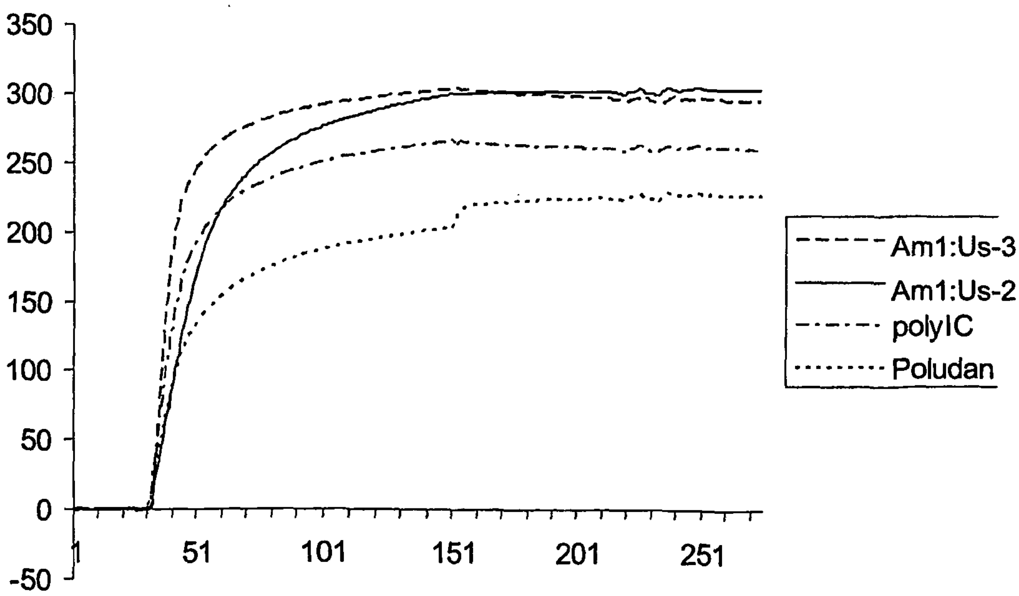

350
300
250
200
150
100
50
0
-50
1
51
101
151
201
251
Am1:Us-3
Am1:Us-2
polyIC
Poludan


Fig. 2

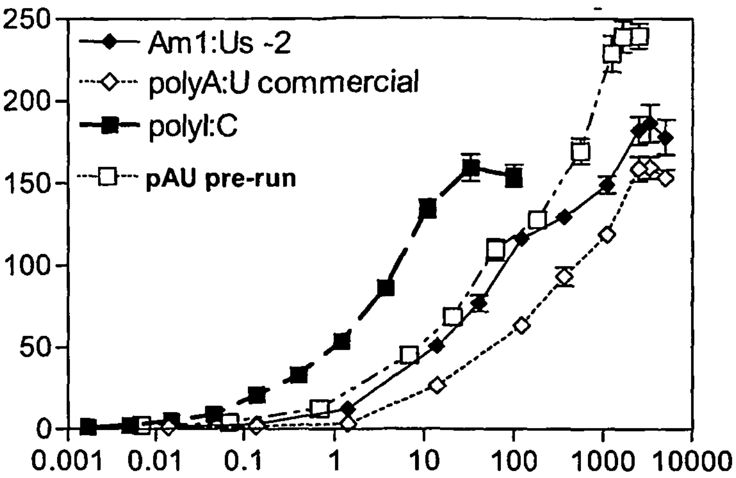


Fig. 3

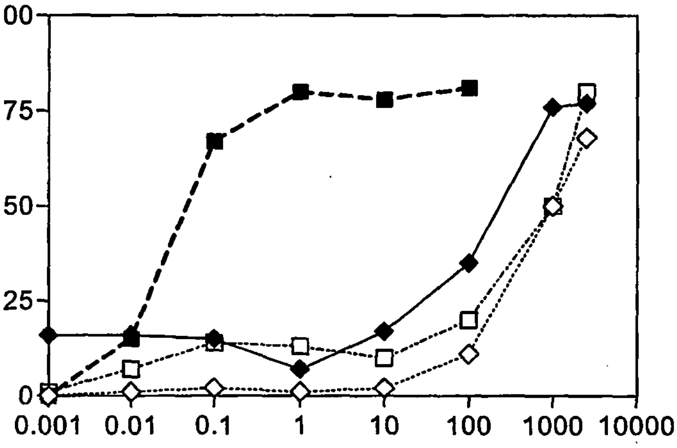


Fig. 4A

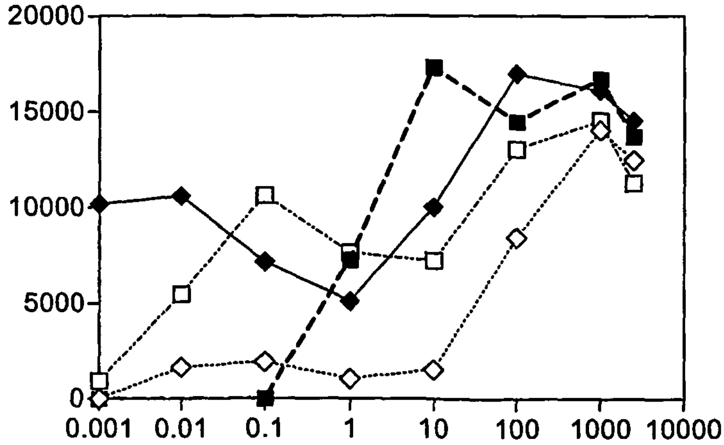


Fig. 4B

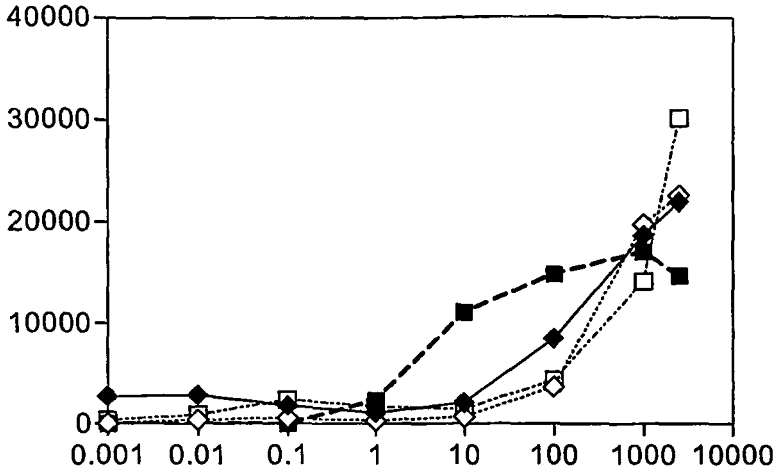
40000
30000
20000
10000
0
0.001
0.01
0.1
1
10
100
1000
10000

Fig. 4C

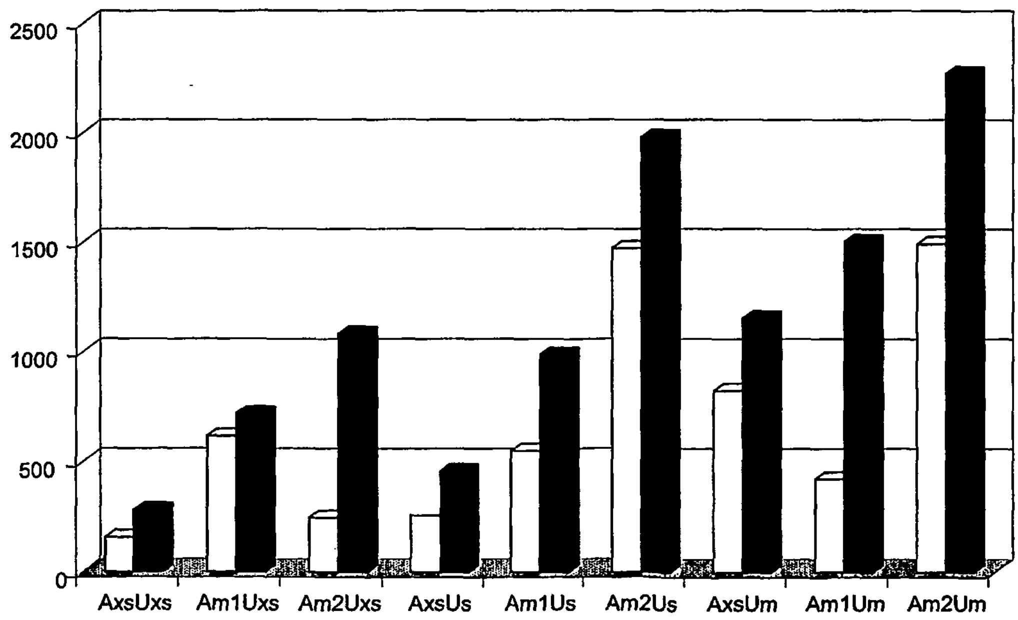
2500
2000
1500
1000
500
0
AxsUxs
Am1Uxs
Am2Uxs
AxsUs
Am1Us
Am2Us
AxsUm
Am1Um
Am2Um

Fig. 5

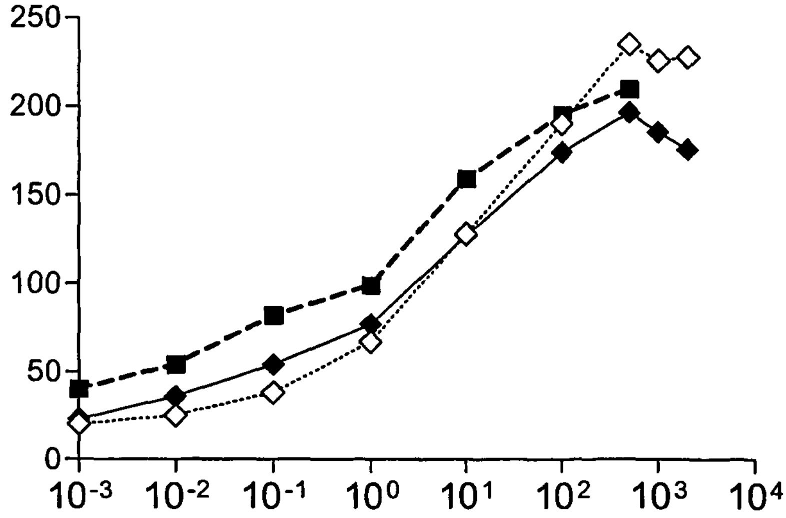
250
200
150
100
50
0
10-3 10-2 10-1 100 101 102 103 104

Fig. 6A

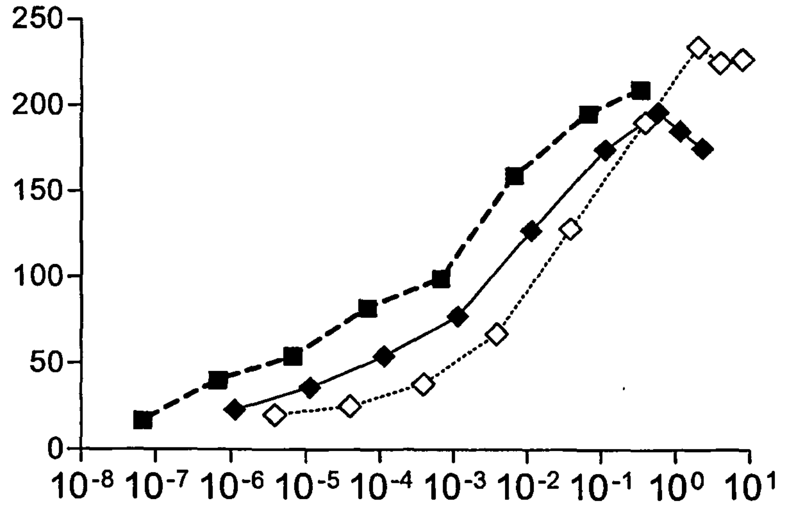
250
200
150
100
50
0
10-8 10-7 10-6 10-5 10-4 10-3 10-2 10-1 100 101

Fig. 6B

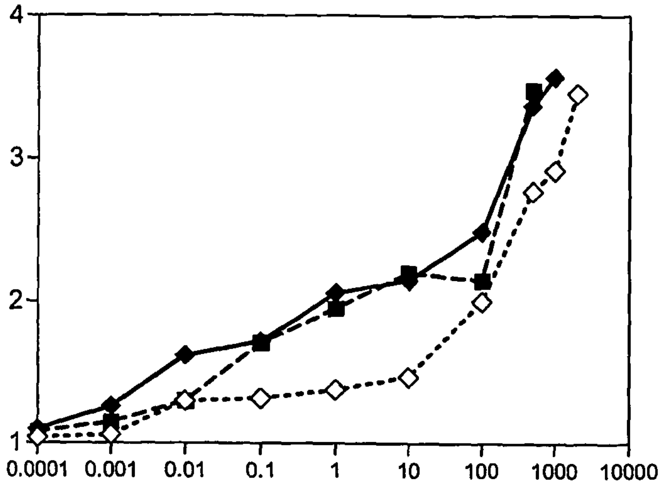


Fig. 7A

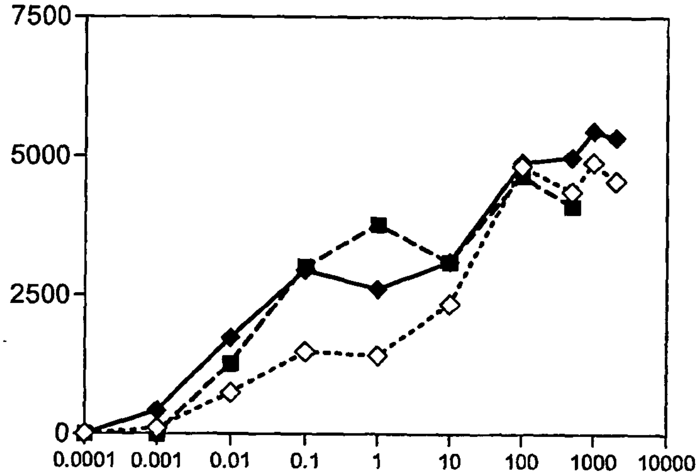


Fig. 7B

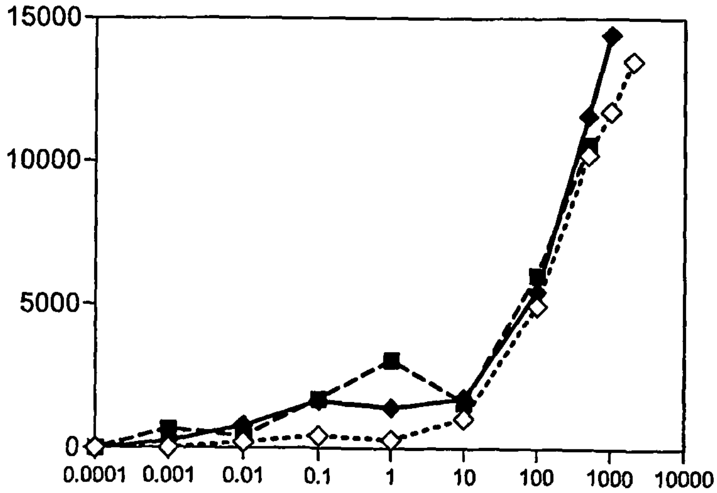


Fig. 7C

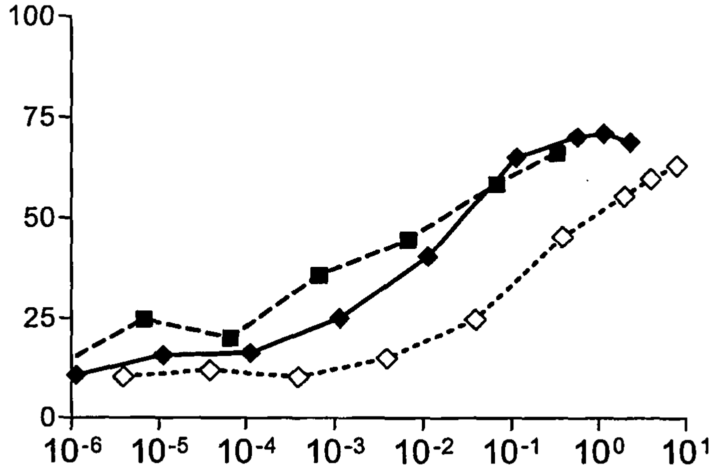
100
75
50
25
0
$10^{-6}$ $10^{-5}$ $10^{-4}$ $10^{-3}$ $10^{-2}$ $10^{-1}$ $10^{0}$ $10^{1}$

Fig. 8A

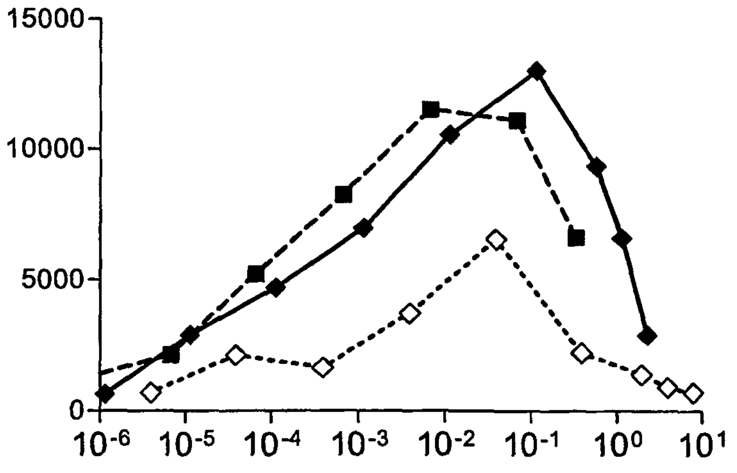
15000
10000
5000
0
$10^{-6}$ $10^{-5}$ $10^{-4}$ $10^{-3}$ $10^{-2}$ $10^{-1}$ $10^{0}$ $10^{1}$

Fig. 8B

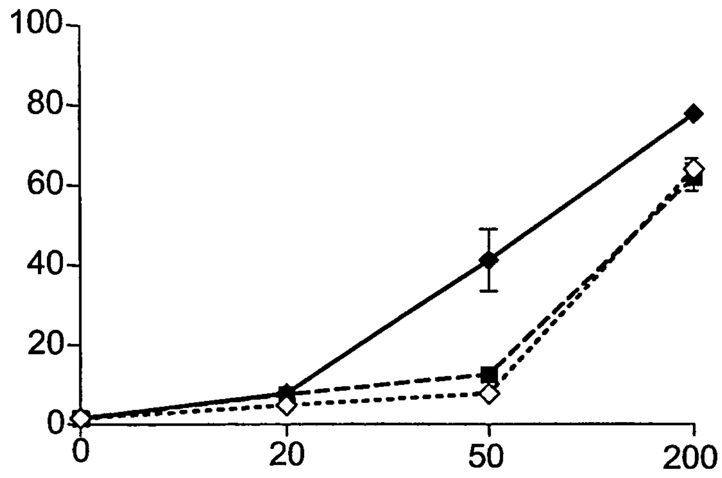


Fig. 9A

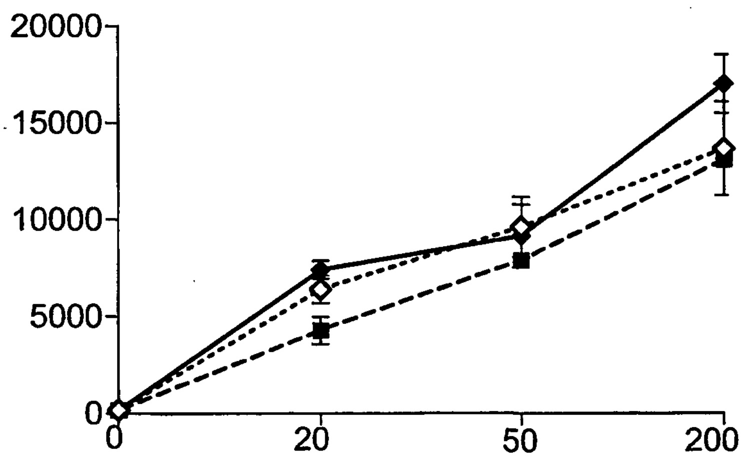


Fig. 9B

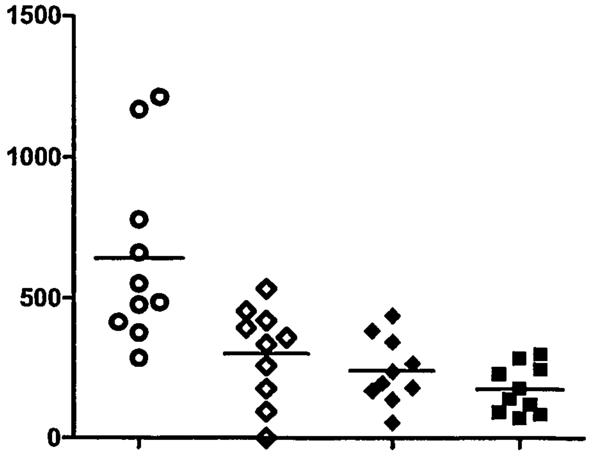


Fig. 10

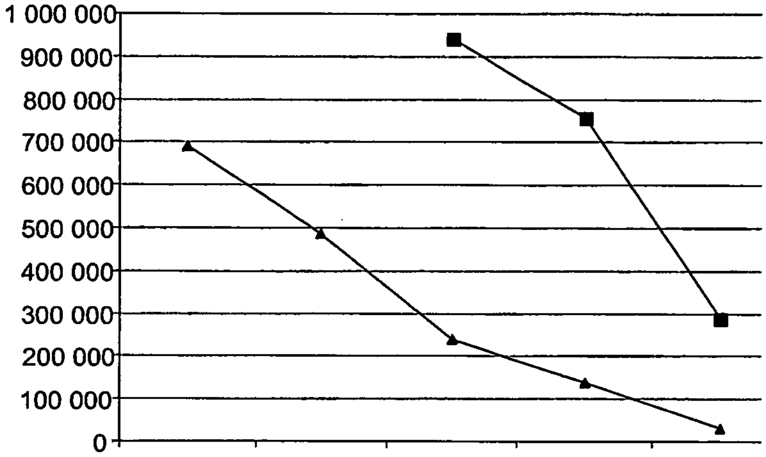


Fig. 11

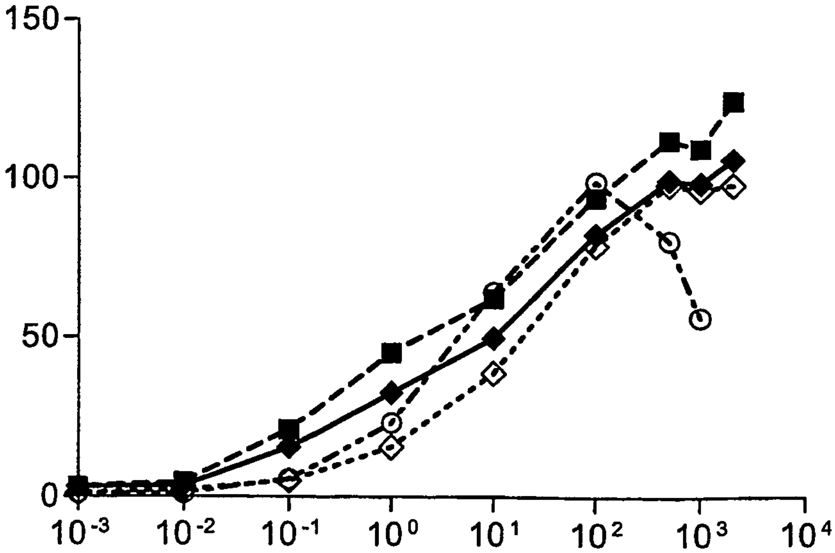


Fig. 12

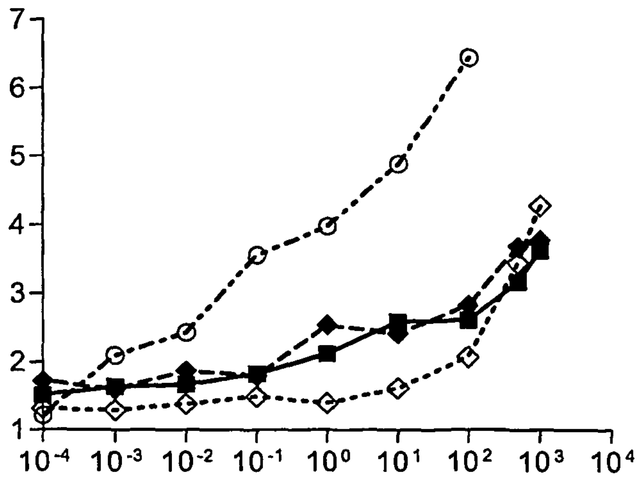


Fig. 13A

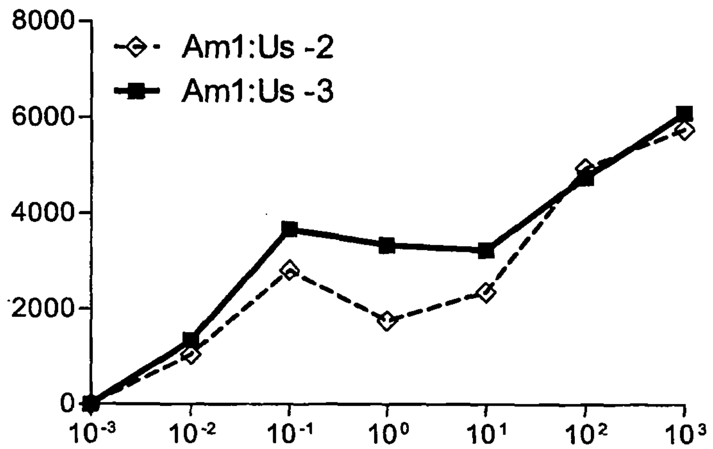


Fig. 13B

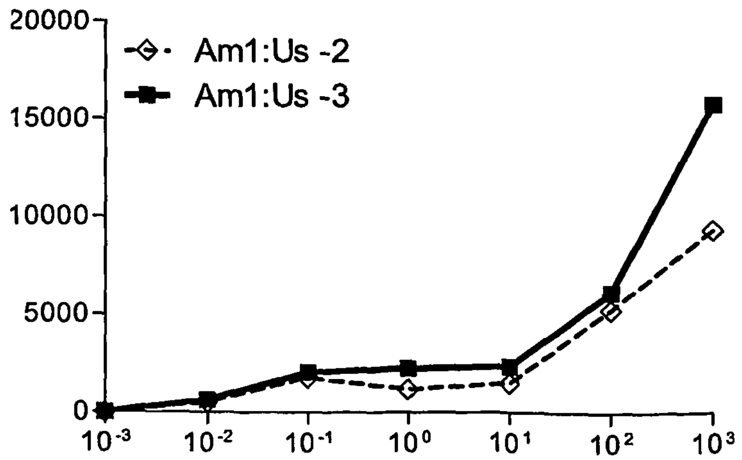


Fig. 13C

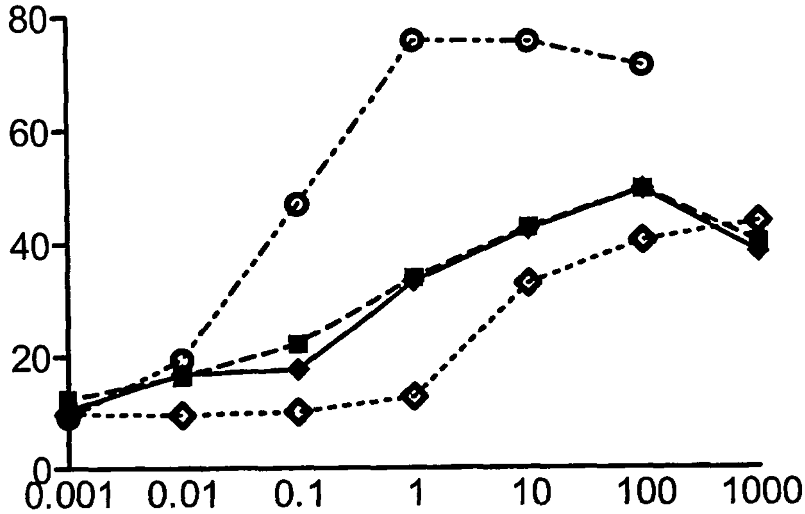


Fig. 14A

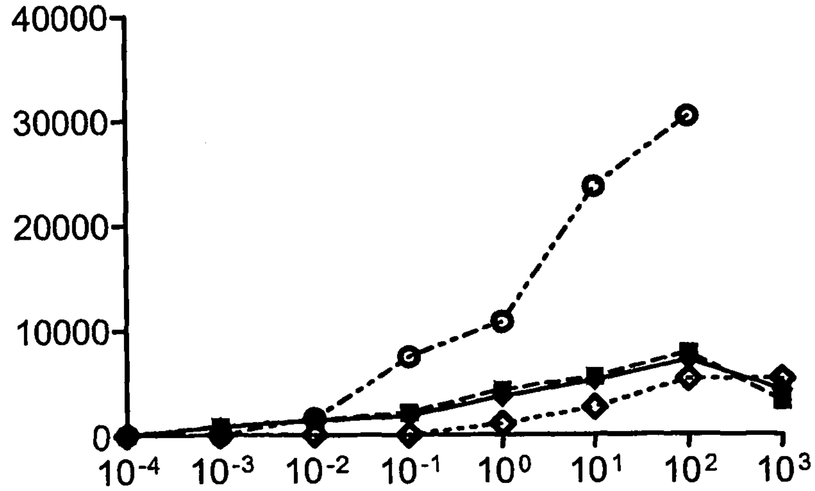


Fig. 14B

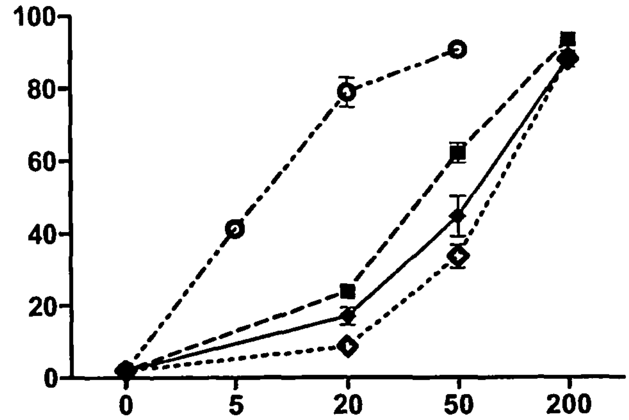


Fig. 15A

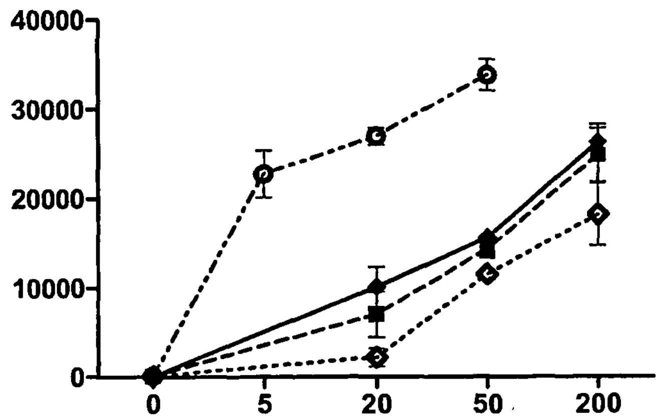


Fig. 15B

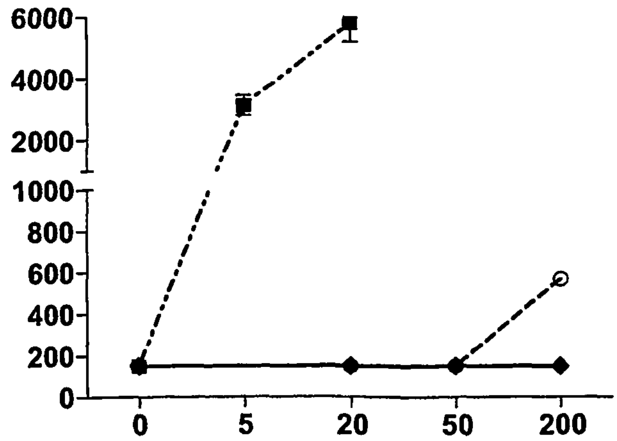


Fig. 15C

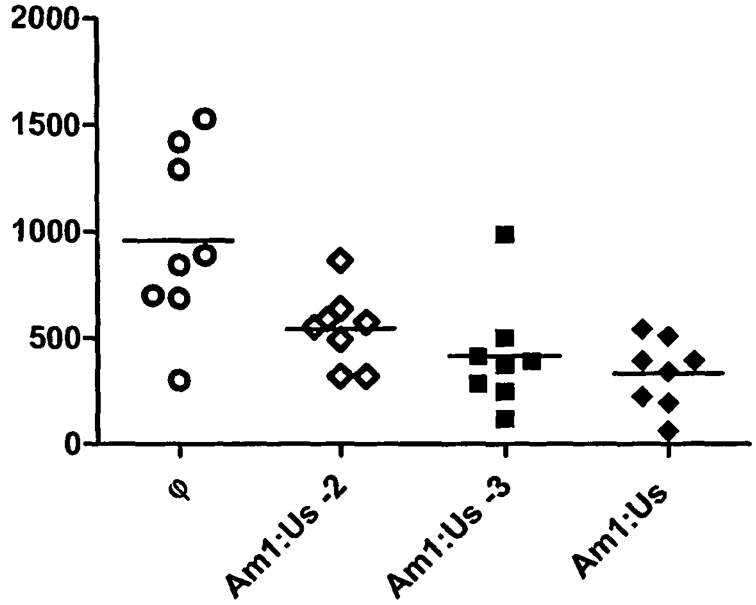


Fig. 16

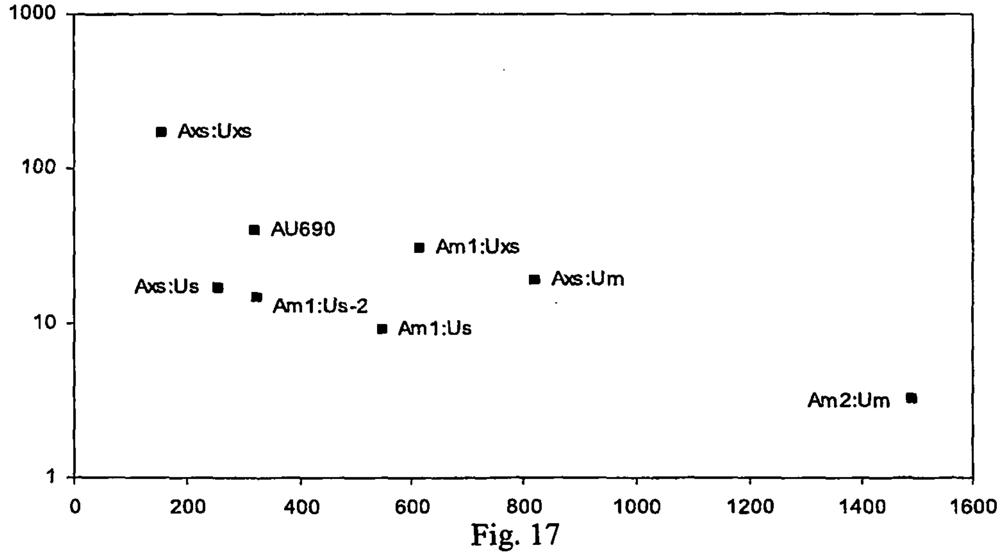


Fig. 17

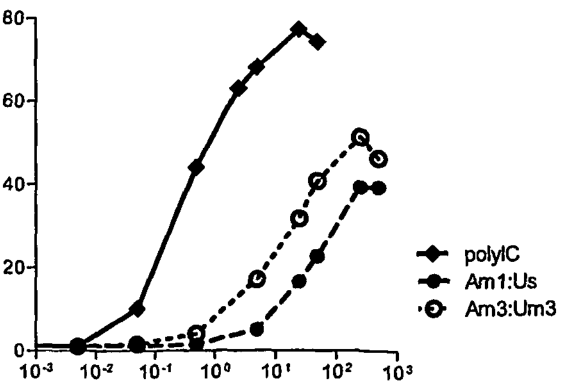


Fig. 18

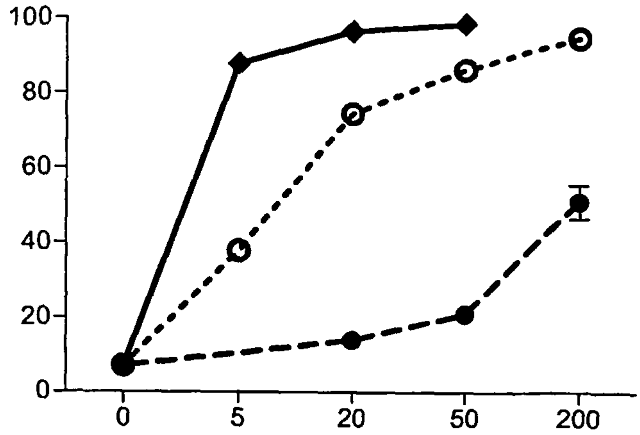


Fig. 19A

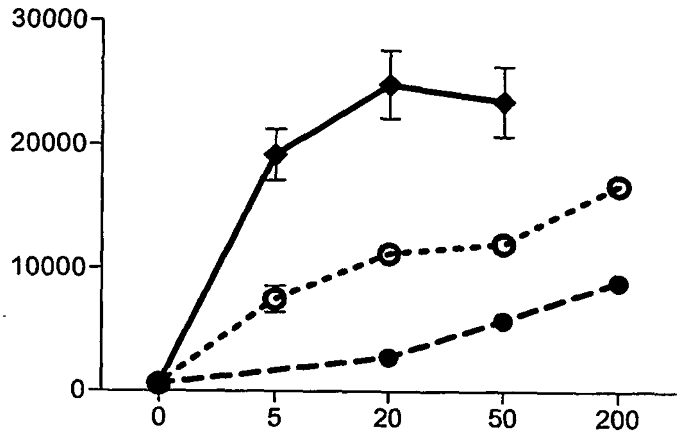


Fig. 19B

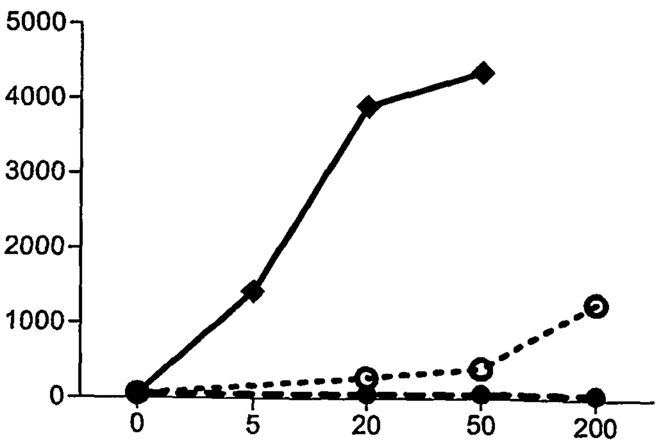


Fig. 19C

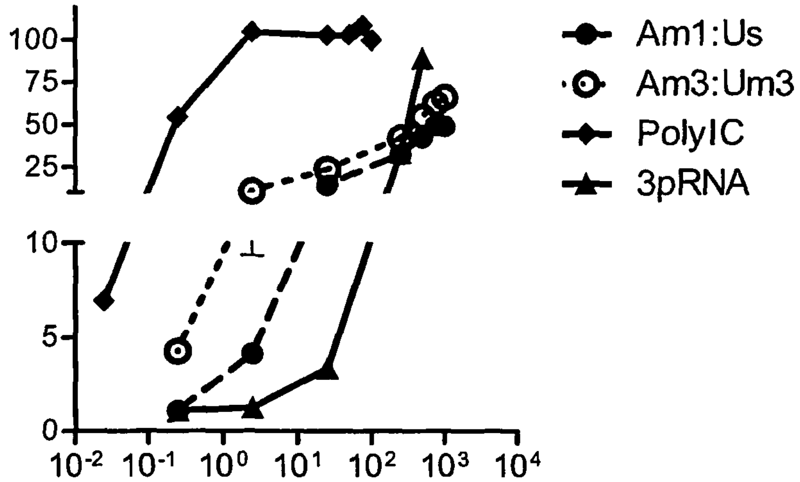


Fig. 20A

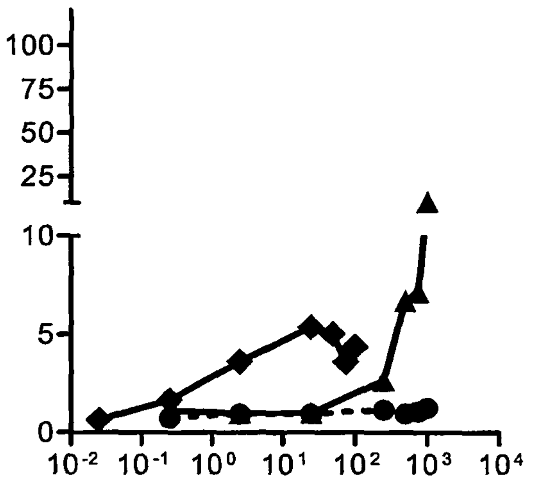


Fig. 20B

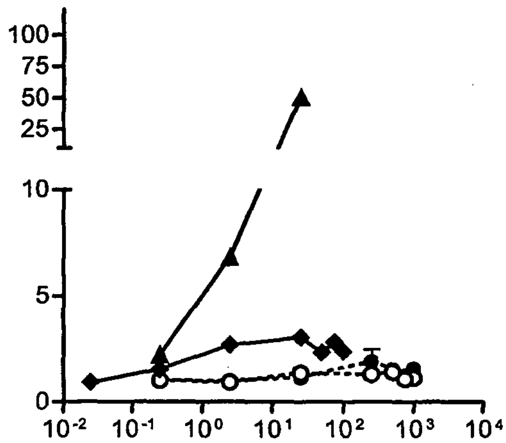


Fig. 20C

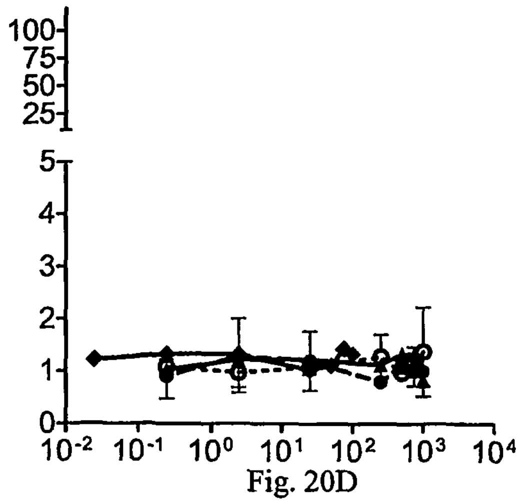


Fig. 20D

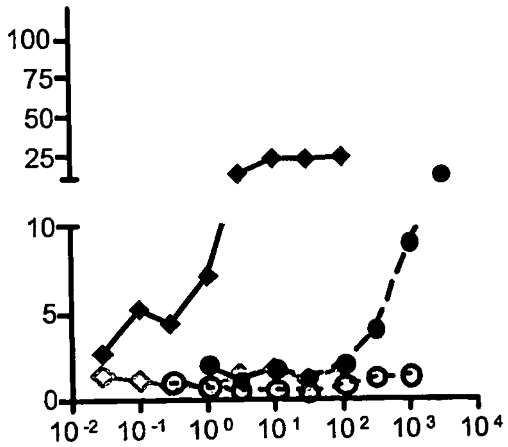


Fig. 21A

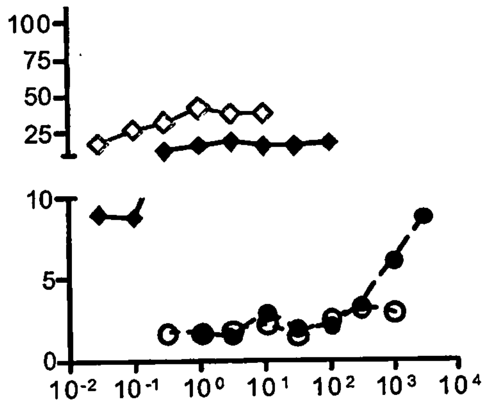


Fig. 21B

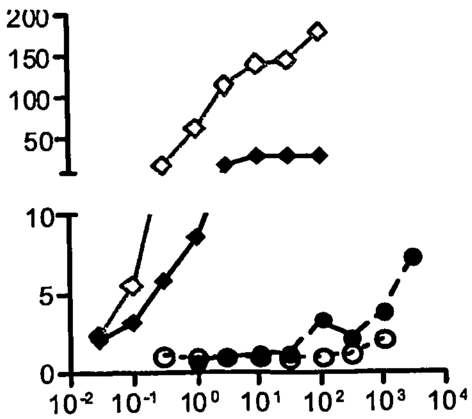


Fig. 21C

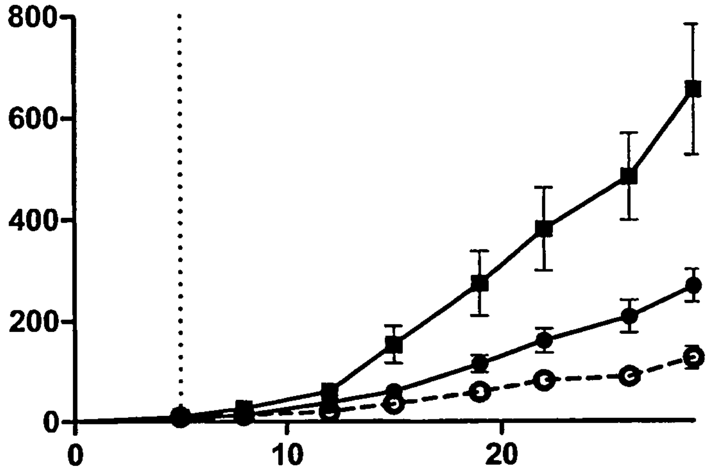


Fig. 22A

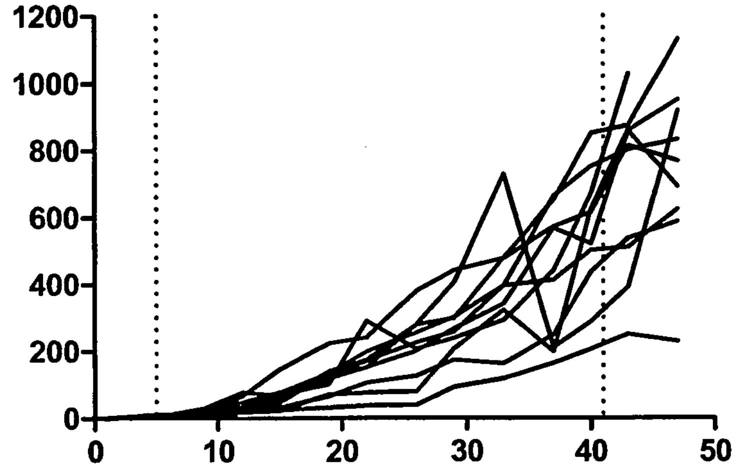


Fig. 22B

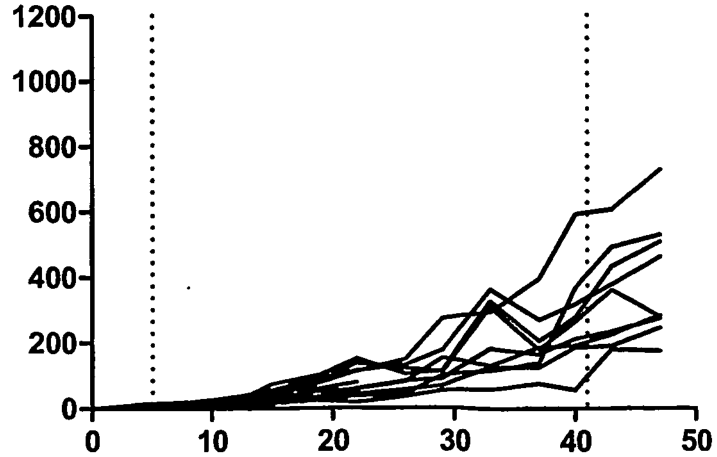


Fig. 22C

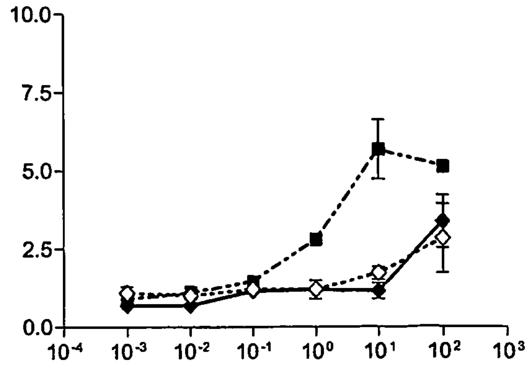


Fig. 23A

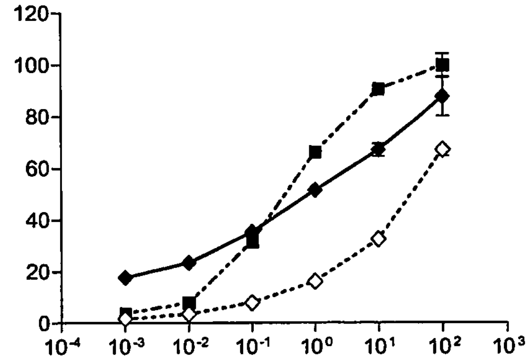


Fig. 23B

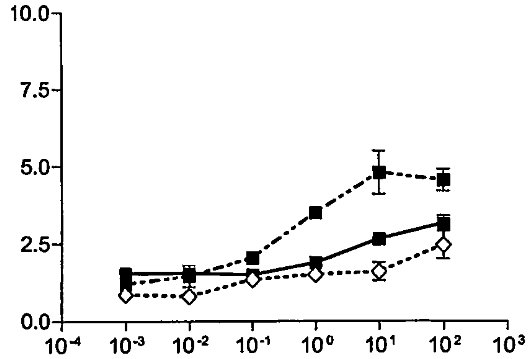


Fig. 23C

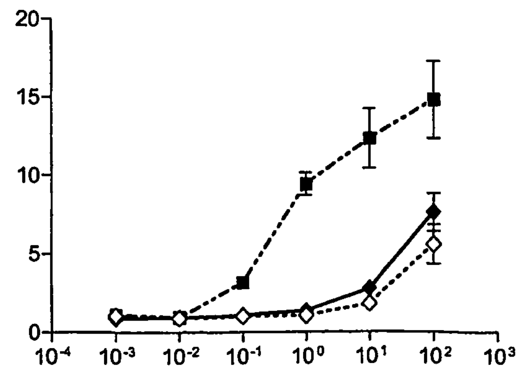


Fig. 23D

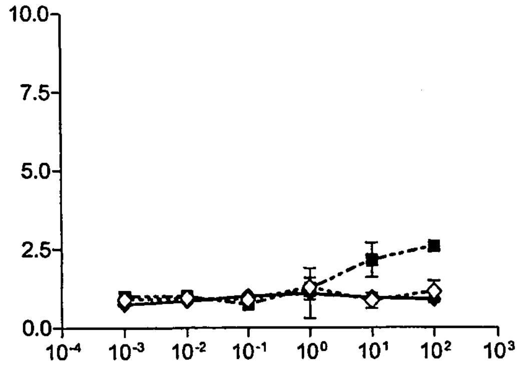


Fig. 24A

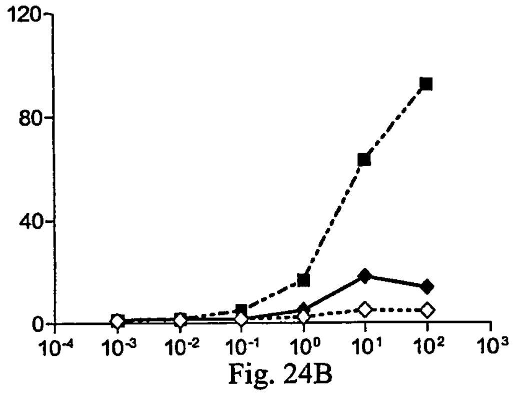


Fig. 24B

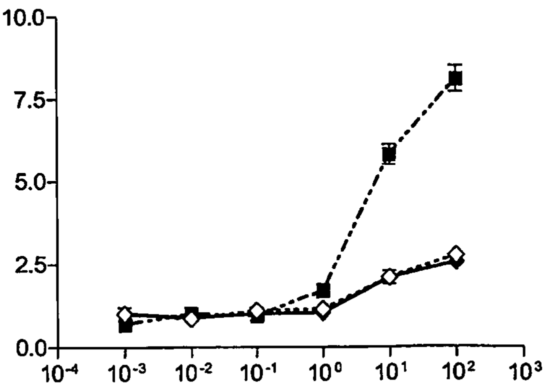


Fig. 24C

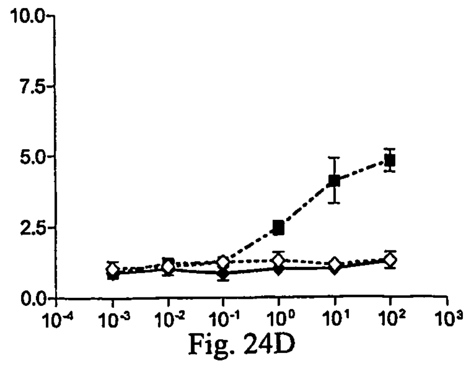


Fig. 24D

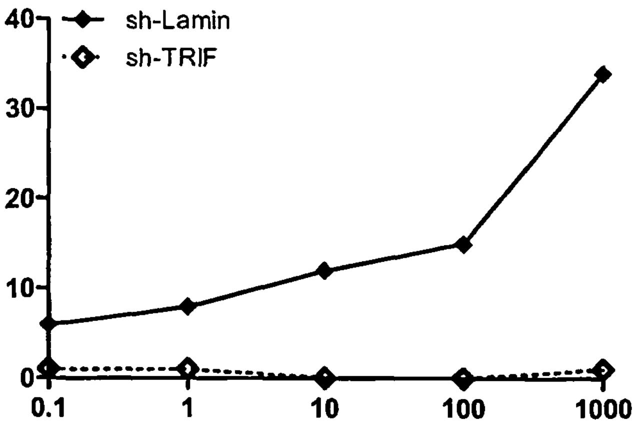


Fig. 25A

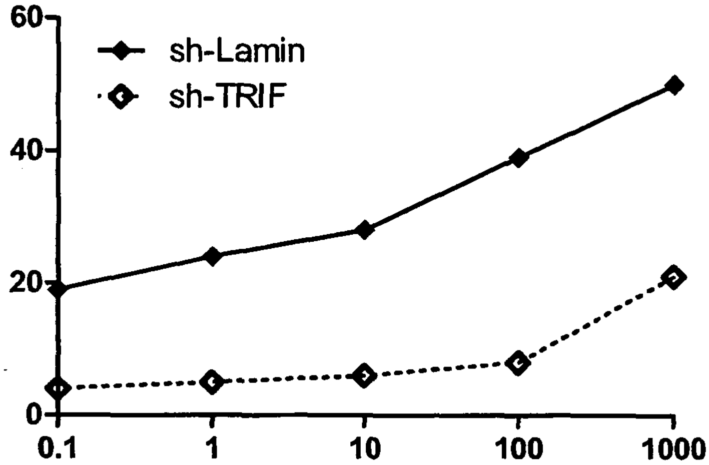


Fig. 25B

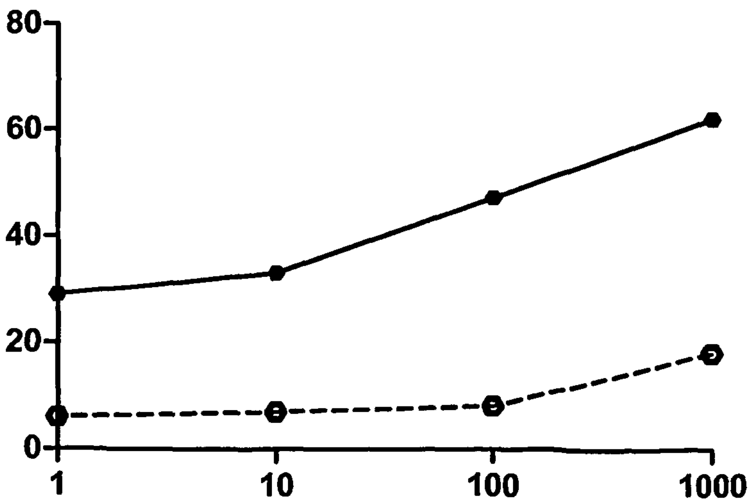


Fig. 26

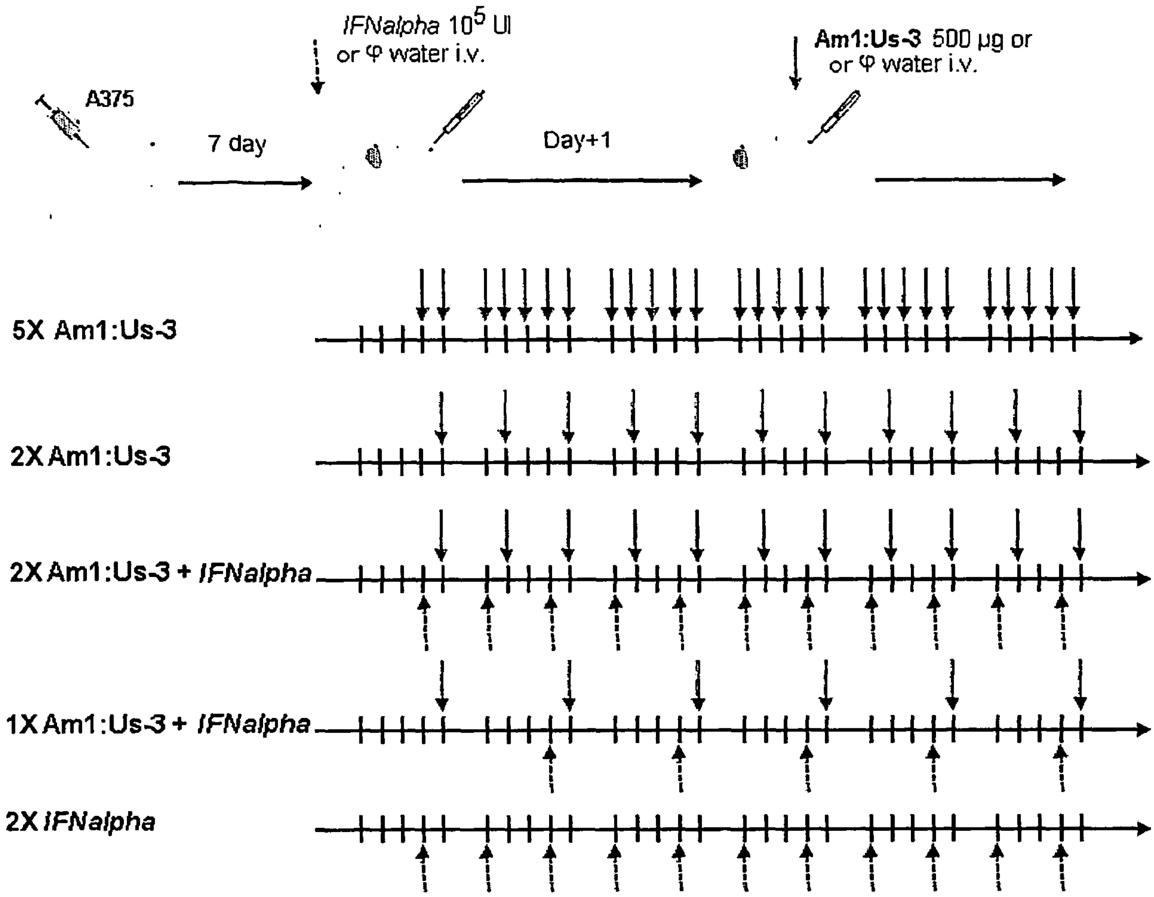
IFNalpha 10^5 UI
or φ water i.v.
A375
7 day
Day+1
Am1:Us-3 500 µg or
or φ water i.v.
5X Am1:Us-3
2X Am1:Us-3
2X Am1:Us-3 + IFNalpha
1X Am1:Us-3 + IFNalpha
2X IFNalpha

Fig. 27

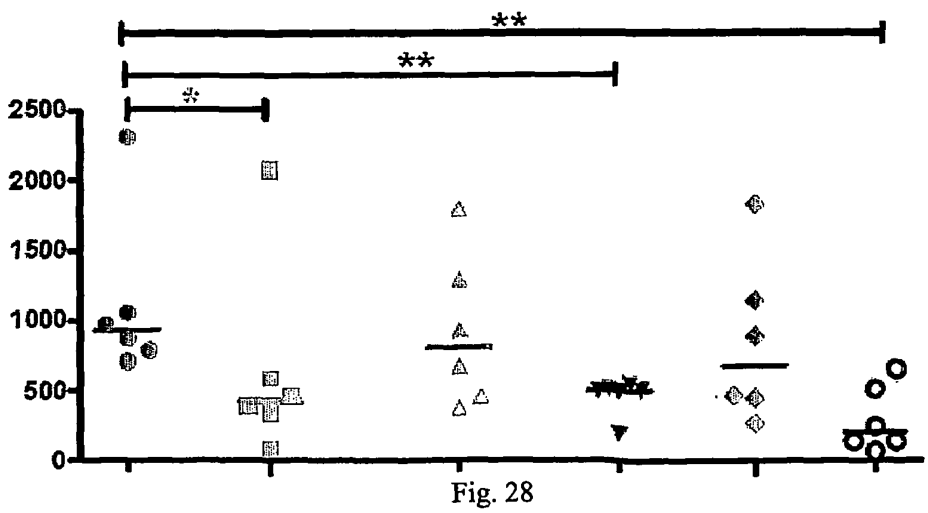
**
**
*
2500
2000
1500
1000
500
0

Fig. 28

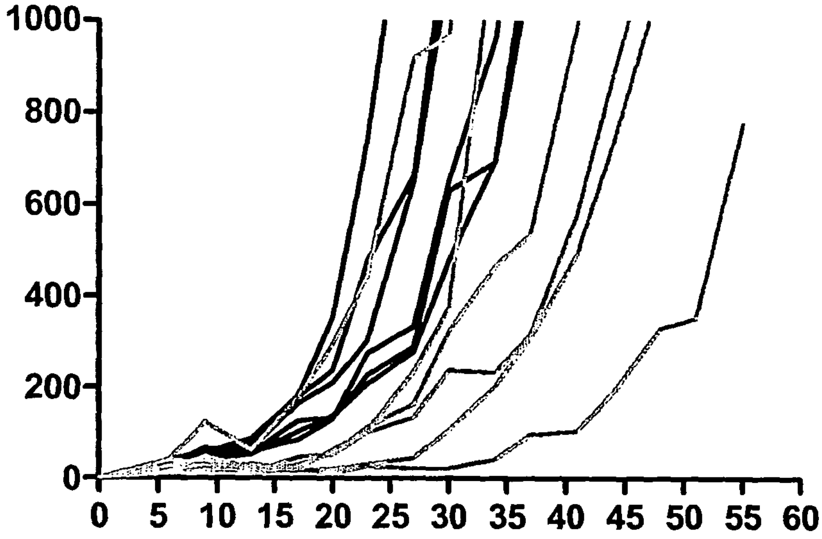

Fig. 29A

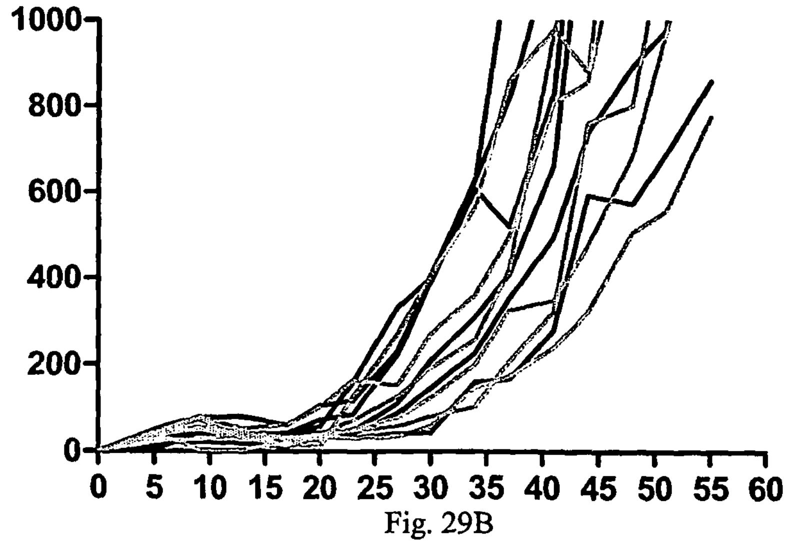

Fig. 29B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03078595 A
- WO 04087877 A
- US 6780429 B
- RU 2165937
- WO 2006014653 A
- WO 2006054177 A, Andre
- US 4927755 A
- WO 9850547 A
- WO 0331573 A
- WO 04053057 A
- WO 04053452 A
- WO 04094671 A
- US 5491084 A
- US 6486382 B
- US 5075109 A
- US 4452775 A
- US 4675189 A
- US 5736152 A
- US 3854480 A
- US 5133974 A
- US 5407686 A
- WO 9640062 A
- US 5264221 A, Tagawa
- US 5665710 A, Rahman
- WO 9704787 A, Love
- US 20030165479 A
- WO 2007133800 A
- WO 04045491 A, Yissum


### Non-patent literature cited in the description


- **ALEXOPOULOU et al.** *Nature,* 2001, vol. 413, 732-738
- **SALAUN et al.** *J. Immunol.,* 2006, vol. 176, 4894-4901
- **KATO et al.** *Nature,* 2006, vol. 441, 101-105
- **KANG et al.** *P.N.A.S. U.S.A.,* 2002, vol. 99 (2), 637-642
- **STEWART et al.** *P.N.A.S. U.S.A.,* 1972, vol. 69 (7), 1851-1854
- **MICHELSON et al.** *Proc. Soc. Exp. Biol. And Med.,* 1985, vol. 179, 1-8
- **DI GIOACCHINO et al.** Ann Clin Lab Sci. Spring, 2004, vol. 34, 195-202
- ASCO Annual Meeting Proceedings. **ANDRE et al.** Journal of Clinical Oncology. 2004, vol. 22, 9619
- **KLEINMAN et al.** *Nature,* 2008
- **KANDIMALLA et al.** *Nucl. Acid. Res.,* 2003, vol. 31 (9), 2393-2400
- **WYATT, P. J.** *Journal of Analytical Chemistry Acta,* 1993, vol. 272, 1
- **CAPRON, I. ; GRISEL, M. ; MULLER, G.** *International Journal of Polymer Analysis and Characterisation,* 1995, vol. 2, 9
- **CAPRON, I. ; YVON, M. ; MULLER, G.** *Food Hydrocolloids,* 1996, vol. 10 (2), 239
- **PICTON, L. ; MOCANU, G. ; MIHAÏ, D. ; CARPOV, A. ; MULLER, G.** *Carbohydrate Polymers,* 1995, vol. 28, 131
- **PICTON, L. ; MERLE, L. ; MULLER, G.** *International Journal of Polymer Analysis and Characterisation,* 1996, vol. 2, 103
- **GUNNARSSON et al.** *Anal. Biochem.,* 2006, vol. 350 (1), 120-127
- **SAMBROOK et al.** Molecular Cloning: A Laboratory Manual. Cold Spring Harbor Laboratory Press, 15 January 2001
- Short Protocols in Molecular Biology. **AUSUBEL et al.** Current Protocols. 18 October 2002
- **AKIRA ; TAKEDA.** *Nature Review Immunol.,* 2004, vol. 4, 499-511
- **HCKER H et al.** *EMBO J.,* 1999, vol. 18, 6973-82
- **MURPHY TL et al.** *Mol Cell Biol,* 1995, vol. 15, 5258-67
- **KANG et al.** *Proc. Nat. Acad. Sci.,* 2002, vol. 99, 637-642
- **IMAIZUMI et al.** *Life Sci.,* 2004, vol. 75, 1171-1180
- **KUHEN et al.** *Gene,* 1996, vol. 178, 191-193
- **BOUSSIF et al.** *PNAS,* 1995, vol. 92, 7297
- **GODBEY.** *PNAS,* 1999, vol. 96, 5177
- **WANG.** *Biochem. Biophys. Res. Commun.,* 1987, vol. 147, 980-985
- **ZHOU et al.** *Journal of Controlled Release,* 1992, vol. 19, 269-274
- **CEVC et al.** *Biochim Biophys Acta,* 1998, vol. 1368 (2), 201-15
- **GREGORIADIS.** *Trends Biotechnol,* 1985, vol. 3, 235-241

- **COHEN P A et al.** *Cancer Res,* 1994, vol. 54, 1055-8
- **FREIREICH et al.** *Cancer Chemother Rep,* 1966, vol. 50, 219
- Scientific Tables. Geigy Pharmaceuticals. Ardley, 1970, 537
- Pharmacotherapy Handbook. Appleton and Lange, 2000
- PDR Pharmacopoeia. Tarascon Pocket Pharmacopoeia. Tarascon Publishing, 2000
- **SALAUN et al.** *Clin. Cancer Res.,* 2007, vol. 13, 4565-74
- **SCHULTZ et al.** *Nature,* 2005, vol. 433, 887-892
- **SALEM et al.** *J. Immunother.,* 2005, vol. 28 (3), 220-8
- **CUI et al.** *Cancer Immunol. Immunother.,* 2006, vol. 55, 1267-79
- **TISSARI et al.** *J. Immunol.,* 2005, vol. 174, 4289-4294
- **C. G. A THOMAS.** *Medical Microbiology,* 1983
- **YU et al.** *Int. J. Cancer,* 1994, vol. 56, 244
- **WONG.** Chemistry of Protein Conjugation and Cross-linking. CRC Press, 1991
- Modification of Antibodies by Chemical Methods. **UPESLACIS et al.** Monoclonal antibodies: principles and applications. Wiley-Liss, Inc, 1995, 187-230
- Production and Characterization of Synthetic Peptide-Derived Antibodies. **PRICE et al.** Monoclonal antibodies: Production, engineering and clinical application. Cambridge University Press, 1995, 60-84
- **CATTEL et al.** *Chemistry today,* 1989, vol. 7, 51-58
- **DELPRINO et al.** *J. Pharm. Sci,* 1993, vol. 82, 699-704
- **ARPICCO et al.** *Bioconjugate Chemistry,* 1997, vol. 8, 3
- **REISFELD et al.** *Antibody, Immunicon. Radiopharrn.,* 1989, vol. 2, 217
- **LIAUTARD et al.** *J. Chromatography,* 1988, vol. 454, 195-203
- **WIETEK et al.** *J. Biol. Chem.,* 2003, vol. 278 (51), 50923